# EUROPEAN PATENT APPLICATION

(11) **EP 2 098 509 A1**
(43) Date of publication of application: **09.09.2009**
(21) Application number: 07829299.2
(22) Date of filing: 05.10.2007
(51) Int. Cl.: C07D 211/58, A61K 31/4468, A61K 31/453, A61K 31/454, A61K 31/4545, A61K 31/496, A61K 31/506, A61K 31/5377, A61P 3/04, A61P 3/06, A61P 3/10, A61P 9/10, A61P 9/12, A61P 25/08, A61P 25/14, A61P 25/18, A61P 25/20, A61P 25/22, A61P 25/24, A61P 25/28

(54) **1-NAPHTHYL ALKYLPIPERIDINE DERIVATIVE**

(30) Priority: 06.10.2006 JP 2006275227; 07.06.2007 JP 2007151270
(71) Applicant: Taisho Pharmaceutical Co. Ltd., Tokyo 170-8633 (JP)
(72) Inventor: SEKIGUCHI, Yoshinori, Tokyo 170-8633 (JP); KANUMA, Kosuke, Tokyo 170-8633 (JP); SAKAGAMI, Kazunari, Tokyo 170-8633 (JP); HAYASHI, Masato, Tokyo 170-8633 (JP); KASHIWA, Shuhei, Tokyo 170-8633 (JP); OMODERA, Katsunori, Tokyo 170-8633 (JP)
(74) Representative: HOFFMANN EITLE
(86) International application number: PCT/JP2007/069561
(87) International publication number: WO 2008/044632

(57) **Abstract**

Disclosed is a pharmaceutical composition comprising a compound represented by the formula (I) 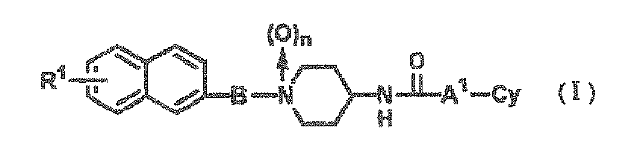 or a pharmaceutically acceptable salt thereof as an active ingredient, which has an antagonistic activity on a melanin-concentrating hormone receptor. The pharmaceutical composition is useful, due to its antagonistic activity on a MCH receptor, for the prevention or treatment of a disease such as depression, anxiety disorders (e.g., generalized anxiety disorder, post traumatic stress disorder, panic disorder, obsessive-compulsive disorder, social anxiety disorder), attention deficit disorder, mania, manic-depressive disorder, schizophrenia, mood disorders, stress, sleep disorder, attacks, memory impairment, cognitive impairment, dementia, amnesia, delirium, obesity, eating disorder, appetite disorder, hyperphagia, bulimia, cibophobia, diabetes, a cardiovascular diseases, hypertension, dyslipidemia, cardiac infarction, movement disorder (e.g., Parkinson's disease, epilepsy, convulsion, tremor), drug abuse and drug addiction.

## Description

### TECHNICAL FIELD

The present invention relates to a compound having a melanin-concentrating hormone receptor antagonistic effect and a pharmaceutically acceptable salt thereof.

### BACKGROUND ART

Depression and anxiety disorders constitute main psychiatric diseases. It is assumed that the lifetime prevalence of depression and anxiety disorders has been steadily increased in recent years. To date, tricyclic antidepressants (TCA), selective serotonin reuptake inhibitors (SSRI), serotonin and noradrenaline reuptake inhibitors (SNRI) and the like based on the monoamine hypothesis have been developed as antidepressants. Benzodiazepines based on the γ-aminobutyric acid mechanism (GABA) have been used as anxiolytics. In recent years, SSRI and SNRI have been demonstrated to be also effective for anxiety disorders such as panic disorder and obsessive-compulsive disorder for which benzodiazepines are not effective, and they are also the first-line treatments for anxiety disorders. However, SSRI and SNRI are not effective in patients with treatment-refractory depression and need to be taken for several weeks for the onset of antidepressive and anxiolytic effects, for example, disadvantageously. Accordingly, it is desirable to develop an antidepressant and anxiolytic based on a mechanism of action differing from that of an existing drug.

Stress is assumed to be a cause of depression and anxiety disorder. In recent years, various neuropeptides biosynthesized in the brain have attracted attention as molecules playing a central role in the stress reaction. Melanin-concentrating hormone (MCH), a neuropeptide, consisting of 19 amino acids is biosynthesized and widely distributed in the limbic system and the like in the bran. The melanin-concentrating hormone-1 receptor (MCH1R) and the melanin-concentrating hormone-2 receptor (MCH2R) have been already known as two MCH receptor subtypes. MCH2R is not expressed in rodents and its physiological functions have not yet been elucidated; however, it has been elucidated that MCH1R is deeply associated with eating behavior and energy metabolism. It is reported that MCH1R is also deeply involved in regulation of stress response and emotion. Activation of the hypothalamus-pituitary-adrenal (HPA) axis by MCH is antagonized by an MCH1R antagonist and a neutralizing antibody against corticotropin-releasing factor (CRF). MCH is presumed to activate the HPA system through facilitation of release of CRF from the hypothalamus. MCH1R is predominantly distributed in the accumbens involved in motivation and reward. When MCH is injected into this site, depressive-like symptoms are observed in a forced swimming test, whereas MCH knockout mice have antidepressive-like symptoms. A study using MCH1R knockout mice shows that MCH1R negatively regulates the activity of dopaminergic neurons involved in reward in the accumbens. Moreover, ATC0175, a nonpeptidic MCH1R antagonist, has antidepressive-like and anxiolytic-like effects in experimental animal models (Non-Patent Document 1). From the above facts, it is suggested that MCH1R is involved not only in eating behavior and energy metabolism but also in depression and anxiety, and it can be expected that an MCH receptor antagonist, in particular, an MCH1R antagonist, may be an antidepressant and anxiolytic having a mechanism of action differing from that of a conventional one.

Recently, Patent Document 1 and Non-Patent Documents 2 and 3 disclose MCH receptor antagonists having a naphthalene skeleton. However, these documents do not disclose nor suggest enhancement of an MCH receptor antagonistic effect by introduction of a substituent into a naphthalene skeleton.
PATENT DOCUMENT 1: WO 2004/046110
NON-PATENT DOCUMENT 1: Drug Development Research, 2005, vol. 65, p. 278-290
NON-PATENT DOCUMENT 2: 224th National Meeting of the American Chemical Society, MEDI-343, 2002
NON-PATENT DOCUMENT 3: Bioorganic & Medicinal Chemistry Letters, 2006, vol. 16, p. 5445-5450

### DISCLOSURE OF THE INVENTION

### PROBLEMS TO BE SOLVED BY THE INVENTION

An object of the present invention is to provide a novel compound useful for preventing or treating a disease such as depression, anxiety disorders (such as generalized anxiety disorder, posttraumatic stress disorder, panic disorder, obsessive-compulsive disorder or social anxiety disorder), attention deficit disorder, mania, manic-depressive illness, schizophrenia, mood disorders, stress, sleep disorders, attacks, memory impairment, cognitive impairment, dementia, amnesia, delirium, obesity, eating disorder, appetite disorder, hyperphagia, bulimia, cibophobia, diabetes, cardiovascular diseases, hypertension, dyslipidemia, myocardial infarction, movement disorder (such as Parkinson's disease, epilepsy, convulsion or tremor), drug abuse or drug addiction, based on an MCH receptor antagonistic effect, or a pharmaceutically acceptable salt thereof.

### MEANS FOR SOLVING THE PROBLEMS

As a result of extensive studies on a novel compound having a naphthalene skeleton, the present inventors have found that the following substituted naphthalene compound represented by the formula (I) has an excellent MCH receptor antagonistic effect. The present invention has been completed based on this finding.

Specifically, the present invention relates to:
1) A compound represented by the formula (I): 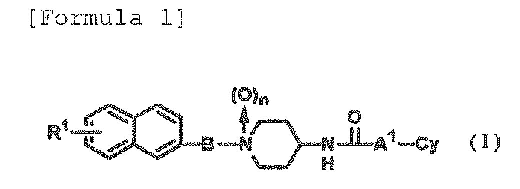 or a pharmaceutically acceptable salt thereof,
   wherein in the formula (I), R¹ is a substituent selected from the group consisting of the formula (II): 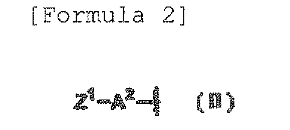 [wherein in the formula (II), Z¹ is a hydroxyl group, a C₁₋₆ alkoxy group (wherein the C₁₋₆ alkoxy group may be substituted with a C₃₋₆ cycloalkyl group), a C₂₋₆ alkenyloxy group, a mono-C₁₋₆ alkylamino group (wherein the mono-C₁₋₆ alkylamino group may be substituted with a C₃₋₆ cycloalkyl group), a di-C₁₋₆ alkylamino group, a formyl group, a carboxyl group, a C₁₋₆ alkoxycarbonyl group or a heterocyclic group {wherein the heterocyclic group is substituted with a hydroxyl group, a C₁₋₆ alkyl group (wherein the C₁₋₆ alkyl group may be substituted with a hydroxyl group or an amino group) or an amino group} and
   A² is a C₁₋₆ alkylene group (wherein the C₁₋₆ alkylene group may be substituted with a C₁₋₆ alkyl group), provided that A² may be a bond when Z¹ is a formyl group],
   the formula (III): 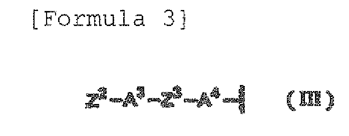 {wherein in the formula (III), Z² is a hydroxyl group, a C₁₋₆ alkoxy group, a C₇₋₁₀ aralkyloxy group, a heterocycloxy group, an amino group, a mono-C₁₋₆ alkylamino group, a di-C₁₋₆ alkylamino group, a carboxyl group, a C₂₋₆ alkanoyl group, a C₇₋₁₀ aralkyloxycarbonyl group, a di-C₁₋₆ alkylaminocarbonyl group or a heterocyclic group,
   Z³ is -O- or -NR³-, wherein R³ is a hydrogen atom or a C₁₋₆ alkyl group, and
   A³ and A⁴ are each independently a C₁₋₆ alkylene group (wherein the C₁₋₆ alkylene group may be substituted with a C₁₋₆ alkyl group), provided that A³ may be a bond when Z² is a heterocyclic group} and
   the formula (IV): 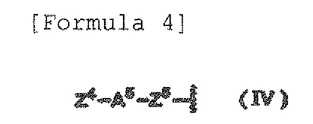 {wherein in the formula (IV), Z⁴ is a C₃₋₆ cycloalkyl group, a C₁₋₆ alkoxy group (wherein the C₁₋₆ alkoxy group may be substituted with a C₁₋₆ alkoxy group), an aryl group or a heterocyclic group, provided that Z⁴ is not a heterocyclic group when Z⁵ is -O-,
   Z⁵ is -O- or -NH-CO- and
   A⁵ is a C₁₋₆ alkylene group (wherein the C₁₋₆ alkylene group may be substituted with a C₁₋₆ alkyl group)},
   B is a C₁₋₆ alkylene group (wherein the C₁₋₆ alkylene group may be substituted with a C₁₋₆ alkyl group),
   A¹ is a bond or a C₁₋₆ alkylene group {wherein the C₁₋₆ alkylene group may be substituted with one or two independent C₁₋₆ alkyl groups (wherein the C₁₋₆ alkyl groups may be bonded together to form a ring) or aryl groups (wherein the aryl groups may each be substituted with a halogen atom)},
   Cy is a C₃₋₆ cycloalkyl group, an aryl group or a heteroaryl group {wherein the aryl group or the heteroaryl group may have 1 to 3 substituents selected from Substituent Group X consisting of a halogen atom,
   a cyano group, a nitro group, a C₁₋₆ alkyl group (wherein the C₁₋₆ alkyl group may be substituted with a halogen atom, a hydroxyl group or a C₁₋₆ alkoxy group), a C₂₋₆ alkenyl group, a hydroxyl group, a C₁₋₆ alkoxy group (wherein the C₁₋₆ alkoxy group may be substituted with a halogen atom), an aryloxy group, a di-C₁₋₆ alkylamino group, a formyl group, a carboxyl group, a carbamoyl group, a C₂₋₆ alkanoyl group, an arylcarbonyl group, a C₁₋₆ alkoxycarbonyl group, a mono-C₁₋₆ alkylcarbonylamino group, a C₁₋₆ alkylcarbonyloxy group, a mono-C₁₋₆ alkylaminocarbonyl group, a di-C₁₋₆ alkylaminocarbonyl group, a C₁₋₆ alkylthio group, a C₁₋₆ alkylsulfonyl group, an aryl group and a heteroaryl group} and
   n is 0 or 1;
2) The compound or pharmaceutically acceptable salt thereof according to 1) above, wherein the compound is represented by the formula (V): 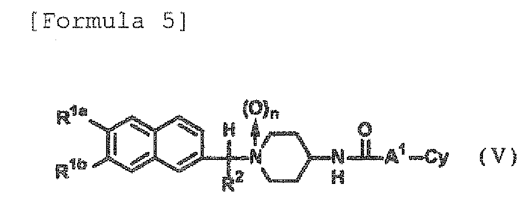 wherein in the formula (V), any one of R^{1a} and R^{1b} is a hydrogen atom and the other is a substituent selected from the group consisting of the formula (II), the formula (III) and the formula (IV),
   R² is a hydrogen atom or a C₁₋₆ alkyl group and
   A¹, Cy and n are as defined in 1) above;
3) The compound or pharmaceutically acceptable salt thereof according to 2) above, wherein in the formula (V),
   R^{1a} is a hydrogen atom,
   R^{1b} is a substituent selected from the group consisting of the formula (II), the formula (III) and the formula (IV) and
   R² is a hydrogen atom;
4) The compound or pharmaceutically acceptable salt thereof according to 3) above, wherein in the formula (V),
   Cy is a C₃₋₆ cycloalkyl group, an aryl group {wherein the aryl group may have 1 to 3 substituents selected from the group consisting of a halogen atom, a cyano group, a nitro group, a C₁₋₆ alkyl group (wherein the C₁₋₆ alkyl group may be substituted with a halogen atom, a hydroxyl group or a C₁₋₆ alkoxy group), a C₂₋₆ alkenyl group, a hydroxyl group, a C₁₋₆ alkoxy group (wherein the C₁₋₆ alkoxy group may be substituted with a halogen atom), an aryloxy group, a di-C₁₋₆ alkylamino group, a formyl group, a C₂₋₆ alkanoyl group, an arylcarbonyl group, a C₁₋₆ alkoxycarbonyl group, a mono-C₁₋₆ alkylcarbonylamino group, a C₁₋₆ alkylcarbonyloxy group, a mono-C₁₋₆ alkylaminocarbonyl group, a C₁₋₆ alkylthio group, as aryl group and a heteroaryl group} or a heteroaryl group (wherein the heteroaryl group may be substituted with a halogen atom);
5) The compound or pharmaceutically acceptable salt thereof according to 4) above, wherein in the formula (V),
   A¹ is a bond or a methylene group {wherein the methylene group may be substituted with one or two independent C₁₋₆ alkyl groups (wherein the C₁₋₆ alkyl groups may be bonded together to form a ring) or aryl groups (wherein the aryl groups may each be substituted with a halogen atom)},
   Cy is an aryl group {wherein the aryl group may have 1 to 3 substituents selected from the group consisting of a halogen atom, a cyano group, a nitro group, a C₁₋₆ alkyl group (wherein the C₁₋₆ alkyl group may be substituted with a halogen atom), a C₂₋₆ alkenyl group, a C₁₋₆ alkoxy group (wherein the C₁₋₆ alkoxy group may be substituted with a halogen atom), an aryloxy group, a di-C₁₋₆ alkylamino group, a C₂₋₆ alkanoyl group, a C₁₋₆ alkoxycarbonyl group and a C₁₋₆ alkylthio group} or a heteroaryl group (wherein the heteroaryl group may be substituted with a halogen atom) and
   n is 0;
6) The compound or pharmaceutically acceptable salt thereof according to 5) above, wherein in the formula (V),
   Cy is an aryl group {wherein the aryl group may have 1 to 3 substituents selected from the group consisting of a halogen atom, a nitro group, a C₁₋₆ alkyl group (wherein the C₁₋₆ alkyl group may be substituted with a halogen atom), a C₂₋₆ alkenyl group, a C₁₋₆ alkoxy group (wherein the C₁₋₆ alkoxy group may be substituted with a halogen atom), a di-C₁₋₆ alkylamino group, a C₂₋₆ alkanoyl group and a C₁₋₆ alkylthio group}, provided that Cy is 3-monosubstituted, 3,4-disubstituted, 3,5-disubstituted or 3,4,5-trisubstituted;
7) The compound or pharmaceutically acceptable salt thereof according to 6) above, wherein in the formula (V),
   R^{1b} is a substituent selected from the group consisting of the formula (II)
   [wherein in the formula (II), Z¹ is a hydroxyl group, a C₁₋₆ alkoxy group (wherein the C₁₋₆ alkoxy group may be substituted with a C₃₋₆ cycloalkyl group), a C₂₋₆ alkenyloxy group, a nono-C₁₋₆ alkylamino group (wherein the mono-C₁₋₆ alkylamino group may be substituted with a C₃₋₆ cycloalkyl group), a formyl group, a C₁₋₆ alkoxycarbonyl group or a heterocyclic group {wherein the heterocyclic group is substituted with a C₁₋₆ alkyl group (wherein the C₁₋₆ alkyl group may be substituted with a hydroxyl group) or an amino group} and
   A² is a C₁₋₆ alkylene group, provided that A² may be a bond when Z¹ is a formyl group],
   the formula (III)
   {wherein in the formula (III), Z² is a hydroxyl group, a C₁₋₆ alkoxy group, a C₇₋₁₀ aralkyloxy group, an amino group, a mono-C₁₋₆ alkylamino group, a di-C₁₋₆ alkylamino group, a C₇₋₁₀ aralkyloxycarbonyl group or a heterocyclic group,
   Z³ is -O- or -NR³-, wherein R³ is a hydrogen atom or a C₁₋₆ alkyl group, and
   A³ and A⁴ are each independently a C₁₋₆ alkylene group (wherein the C₁₋₆ alkylene group may be substituted with a C₁₋₆ alkyl group)} and
   the formula (IV)
   {wherein in the formula (IV), Z⁴ is a C₁₋₆ alkoxy group (wherein the C₁₋₆ alkoxy group may be substituted with a C₁₋₆ alkoxy group) or a heterocyclic group, provided that Z⁴ is not a heterocyclic group when Z⁵ is -O-,
   Z⁵ is -O- or -NH-CO- and
   A⁵ is a C₁₋₆ alkylene group};
8) The compound or pharmaceutically acceptable salt thereof according to 7) above, wherein in the formula (V),
   R^{1b} is a substituent selected from the group consisting of the formula (II)
   {wherein in the formula (II), Z¹ is a hydroxyl group, a C₁₋₆ alkoxy group (wherein the C₁₋₆ alkoxy group may be substituted with a C₃₋₆ cycloalkyl group), a C₂₋₆ alkenyloxy group, a mono-C₁₋₆ alkylamino group (wherein the mono-C₁₋₆ alkylamino group may be substituted with a C₃₋₆ cycloalkyl group), a formyl group, a C₁₋₆ alkoxycarbonyl group or a heterocyclic group (wherein the heterocyclic group is substituted with an amino group) and
   A² is a C₁₋₆ alkylene group, provided that A² may be a bond when Z¹ is a formyl group},
   the formula (III)
   {wherein in the formula (III), Z² is a hydroxyl group, a C₁₋₆ alkoxy group, a C₇₋₁₀ aralkyloxy group, an amino group, a mono-C₁₋₆ alkylamino group, a di-C₁₋₆ alkylamino group or a heterocyclic group,
   Z³ is -O- or -NH- and
   A³ and A⁴ are each independently a C₁₋₆ alkylene group (wherein the C₁₋₆ alkylene group may be substituted with a C₁₋₆ alkyl group)} and
   the formula (IV)
   {wherein in the formula (IV), Z⁴ is a C₁₋₆ alkoxy group
   (wherein the C₁₋₆ alkoxy group may be substituted with a C₁₋₆ alkoxy group),
   Z⁵ is -O- and
   A⁵ is a C₁₋₆ alkylene group} and
   A¹ is a bond or a methylene group {wherein the methylene group may be substituted with an aryl group (wherein the aryl group may be substituted with a halogen atom)};
9) The compound or pharmaceutically acceptable salt thereof according to 8) above, wherein in the formula (V),
   R^{1b} is a substituent selected from the group consisting of the formula (II)
   {wherein in the formula (II), Z¹ is a C₁₋₆ alkoxy group
   (wherein the C₁₋₆ alkoxy group may be substituted with a C₃₋₆ cycloalkyl group) or a C₂₋₆ alkenyloxy group and
   A² is a C₁₋₆ alkylene group},
   the formula (III)
   {wherein in the formula (III), Z² is a hydroxyl group or a C₁₋₆ alkoxy group,
   Z³ is -O- and
   A³ and A⁴ are each independently a C₁₋₆ alkylene group (wherein the C₁₋₆ alkylene group may be substituted with a C₁₋₆ alkyl group)} and
   the formula (IV)
   {wherein in the formula (IV), Z⁴ is a C₁₋₆ alkoxy group
   (wherein the C₁₋₆ alkoxy group may be substituted with a C₁₋₆ alkoxy group),
   Z⁵ is -0- and
   A⁵ is a C₁₋₆ alkylene group} and
   A¹ is a bond;
10) The compound or pharmaceutically acceptable salt thereof according to 1) above, wherein the compound represented by the formula (I) is
   3-methoxy-N-[1-({7-[(2-methoxyethoxy)methyl]-2-naphthyl}methyl)piperidin-4-yl]benzamide,
   N-(1-{[7-(hydroxymethyl)-2-naphthyl]methyl}piperidin-4-yl)-3-methoxybenzamide,
   N-{1-[(7-formyl-2-naphthyl)methyl]piperidin-4-yl}-3-methoxybenzamide,
   3-chloro-N-(1-{[7-(ethoxymethyl)-2-naphthyl]methyl}piperidin-4-yl)-4-fluorobenzamide,
   3,5-dimethoxy-N-(1-{[7-(methoxymethyl)-2-naphthyl]methyl}piperidin-4-yl)benzamide,
   3,5-dimethoxy-N-(1-{[7-(methoxymethyl)-2-naphthyl]methyl}piperidin-4-yl)-4-methylbenzamide,
   3-methoxy-N-(1-{[7-(methoxymethyl)-2-naphthyl]methyl}piperidin-4-yl)benzamide,
   3-methoxy-N-(1-{[7-(methoxymethyl)-2-naphthyl]methyl}piperidin-4-yl)-4-methylbenzamide,
   3-chloro-5-methoxy-N-(1-{[7-(methoxymethyl)-2-naphthyl]methyl}piperidin-4-yl)benzamide,
   N-[1-({7-[(cyclopropylmethoxy)methyl]-2-naphthyl}methyl)piperidin-4-yl]-3-methoxybenzamide,
   3-methoxy-N-[1-({7-[(3-methylbutoxy)methyl]-2-naphthyl}methyl)piperidin-4-yl]benzamide,
   3-methoxy-N-(1-{[7-(propoxymethyl)-2-naphthyl]methyl}piperidin-4-yl)benzamide,
   N-[1-({7-[(allyloxy)methyl]-2-naphthyl}methyl)piperidin-4-yl]-3-methoxybenzamide,
   3-methoxy-N-[1-({7-[(2-methoxyethoxy)methyl]-2-naphthyl}methyl)piperidin-4-yl]benzamide,
   N-{1-[(7-{[2-(benzyloxy)ethoxy]methyl}-2-naphthyl)methyl]piperidin-4-yl}-3-methoxybenzamide,
   N-[1-({7-[(2-ethoxyethoxy)methyl]-2-naphthyl}methyl)piperidin-4-yl]-3-methoxybenzamide,
   3-methoxy-N-[1-({7-[(2-propoxyethoxy)methyl]-2-naphthyl}methyl)piperidin-4-yl]benzamide,
   N-[1-({7-[(2-isopropoxyethoxy)methyl]-2-naphthyl}methyl)piperidin-4-yl]-3-methoxybenzamide,
   3-methoxy-N-[1-({7-[(3-methoxypropoxy)methyl]-2-naphthyl}methyl)piperidin-4-yl]benzamide,
   3-methoxy-N-{1-[(7-{[2-(tetrahydro-2H-pyran-2-yloxy)ethoxy]methyl}-2-naphthyl)methyl]piperidin-4-yl}benzamide,
   N-[1-({7-[(2-hydroxyethoxy)methyl]-2-naphthyl}methyl)piperidin-4-yl]-3-methoxybenzamide,
   3-methoxy-N-[1-({7-[(2-pyrrolidin-1-ylethoxy)methyl]-2-naphthyl}methyl)piperidin-4-yl]benzamide,
   N-{1-[(7-{[2-(dimethylamino)ethoxy]methyl}-2-naphthyl)methyl]piperidin-4-yl}-3-methoxybenzamide,
   3-methoxy-N-{1-[(7-{[(tetrahydrofuran-2-ylmethyl)amino]methyl}-2-naphthyl)methyl]piperidin-4-yl}benzamide,
   3-methoxy-N-[1-({7-[(4-methylpiperazin-1-yl)methyl]-2-naphthyl}methyl)piperidin-4-yl]benzamide,
   3-methoxy-N-{1-[(7-{[(2-pyrrolidin-1-ylethyl)amino]methyl}-2-naphthyl)methyl]piperidin-4-yl}benzamide,
   N-(1-{[7-({[2-(dimethylamino)ethyl]amino}methyl)-2-naphthyl]methyl}piperidin-4-yl)-3-methoxybenzamide,
   3-methoxy-N-{1-[(7-{[(2-morpholin-4-ylethyl)amino]methyl}-2-naphthyl)methyl]piperidin-4-yl}benzamide,
   N-{1-[(7-{[4-(2-hydroxyethyl)piperazin-1-yl]methyl}-2-naphthyl)methyl]piperidin-4-yl}-3-methoxybenzamide,
   N-{1-[(7-{[[2-(dimethylamino)ethyl](methyl)amino]methyl}-2-naphthyl)methyl]piperidin-4-yl}-3-methoxybenzamide,
   3-methoxy-N-{1-[(7-{[(3-methylbutyl)amino]methyl}-2-naphthyl)methyl]piperidin-4-yl}benzamide,
   N-{1-[(7-{[(cyclopropylmethyl)amino]methyl}-2-naphthyl)methyl]piperidin-4-yl}-3-methoxybenzamide,
   3-methoxy-N-{1-[(7-{[(2-methoxyethyl)amino]methyl}-2-naphthyl)methyl]piperidin-4-yl}benzamide,
   3-methoxy-N-{1-[(7-{[(2-piperidin-1-ylethyl)amino]methyl}-2-naphthyl)methyl]piperidin-4-yl}benzamide,
   benzyl N-{[7-({4-[(3-methoxybenzoyl)amino]piperidin-1-yl}methyl)-2-naphthyl]methyl}-beta-alaninate,
   N-{1-[(7-{[(2-aminoethyl)amino]methyl}-2-naphthyl)methyl]piperidin-4-yl}-3-methoxybenzamide,
   3-methoxy-N-(1-{[7-({[2-(methylamino)ethyl]amino}methyl)-2-naphthyl]methyl}piperidin-4-yl)benzamide,
   N-[1-({7-[(3-aminopyrrolidin-1-yl)methyl]-2-naphthyl}methyl)piperidin-4-yl]-3-methoxybenzamide,
   3-methoxy-N-[1-({7-[(pyrrolidin-3-ylamino)methyl]-2-naphthyl}methyl)piperidin-4-yl]benzamide,
   3-methoxy-N-{1-[(7-{[(pyrrolidin-2-ylmethyl)amino]methyl}-2-naphthyl)methyl]piperidin-4-yl}benzamide,
   3-ethoxy-N-[1-({7-[(2-methoxyethoxy)methyl]-2-naphthyl}methyl)piperidin-4-yl]benzamide,
   methyl 3-({[1-({7-[(2-methoxyethoxy)methyl]-2-naphthyl}methyl)piperidin-4-yl]amino}carbonyl)benzoate,
   3-(dimethylamino)-N-[1-({7-[(2-methoxyethoxy)methyl]-2-naphthyl}methyl)piperidin-4-yl]benzamide,
   N-[1-({7-[(2-methoxyethoxy)methyl]-2-naphthyl}methyl)piperidin-4-yl]-3-(trifluoromethyl)benzamide,
   N-[1-({7-[(2-methoxyethoxy)methyl]-2-naphthyl}methyl)piperidin-4-yl]-3-(trifluoromethoxy)benzamide,
   N-[1-({7-[(2-methoxyethoxy)methyl]-2-naphthyl}methyl)piperidin-4-yl]-3-methylbenzamide,
   3-cyano-N-[1-({7-[(2-methoxyethoxy)methyl]-2-naphthyl}methyl)piperidin-4-yl]benzamide,
   N-[1-({7-[(2-methoxyethoxy)methyl]-2-naphthyl}methyl)piperidin-4-yl]-3-phenoxybenzamide,
   N-[1-({7-[(2-methoxyethoxy)methyl]-2-naphthyl}methyl)piperidin-4-yl]-3-vinylbenzamide,
   3-acetyl-N-[1-({7-[(2-methoxyethoxy)methyl]-2-naphthyl}methyl)piperidin-4-yl]benzamide,
   N-[1-({7-[(2-methoxyethoxy)methyl]-2-naphthyl}methyl)piperidin-4-yl]-3-nitrobenzamide,
   3-chloro-N-[1-({7-[(2-methoxyethoxy)methyl]-2-naphthyl}methyl)piperidin-4-yl]benzamide,
   N-[1-({7-[(2-methoxyethoxy)methyl]-2-naphthyl}methyl)piperidin-4-yl]-3-(methylthio)benzamide,
   5-chloro-N-[1-({7-[(2-methoxyethoxy)methyl]-2-naphthyl}methyl)piperidin-4-yl]nicotinamide,
   3-methoxy-N-(1-{[7-(2-methoxyethoxy)-2-naphthyl]methyl}piperidin-4-yl)benzamide,
   3-methoxy-N-[1-({7-[2-(2-methoxyethoxy)ethoxy]-2-naphthyl}methyl)piperidin-4-yl]benzamide,
   3-methoxy-N-(1-{[7-(3-methoxypropoxy)-2-naphthyl]methyl}piperidin-4-yl)benzamide,
   3-methoxy-N-(1-{[7-(2-oxoethyl)-2-naphthyl]methyl}piperidin-4-yl)benzamide,
   N-(1-{[7-(2-hydroxyethyl)-2-naphthyl]methyl}piperidin-4-yl)-3-methoxybenzamide,
   3-methoxy-N-[1-({7-[2-(2-methoxyethoxy)ethyl]-2-naphthyl}methyl)piperidin-4-yl]benzamide,
   3-ethyl-N-[1-({7-[(2-methoxyethoxy)methyl]-2-naphthyl}methyl)piperidin-4-yl]benzamide,
   3-chloro-4-fluoro-N-(1-{[7-(methoxymethyl)-2-naphthyl]methyl}piperidin-4-yl)benzamide,
   3-methoxy-N-[1-(1-{7-[(2-methoxyethoxy)methyl]-2-naphthyl}ethyl)piperidin-4-yl]benzamide,
   3-methoxy-N-(1-{[7-(4-oxobutyl)-2-naphthyl]methyl}piperidin-4-yl)benzamide,
   N-(1-{[7-(4-hydroxybutyl)-2-naphthyl]methyl}piperidin-4-yl)-3-methoxybenzamide,
   ethyl 3-[7-({4-[(3-methoxybenzoyl)amino]piperidin-1-yl}methyl)-2-naphthyl]propanoate,
   N-(1-{[7-(3-hydroxypropyl)-2-naphthyl]methyl}piperidin-4-yl)-3-methoxybenzamide,
   7-({4-[(3-methoxybenzoyl)amino]piperidin-1-yl}methyl)-N-(2-pyrrolidin-1-ylethyl)-2-naphthamide,
   2-(3,4-dichlorophenyl)-N-(1-{[7-(methoxymethyl)naphthalen-2-yl]methyl}piperidin-4-yl)acetamide,
   N-[1-({7-[(2-methoxyethoxy)methyl]naphthalen-2-yl}methyl)piperidin-4-yl]-2,2-diphenylacetamide,
   2,2-bis(4-chlorophenyl)-N-[1-({7-[(2-methoxyethoxy)methyl]naphthalen-2-yl}methyl)piperidin-4-yl]acetamide,
   N-[1-({7-[(2-methoxyethoxy)methyl]naphthalen-2-yl}methyl)piperidin-4-yl]thiophene-3-carboxamide,
   N-[1-({7-[(2-methoxyethoxy)methyl]naphthalen-2-yl}methyl)piperidin-4-yl]-9H-fluorene-9-carboxamide or
   3-methoxy-N-[1-({7-[(3-methoxybutoxy)methyl]naphthalen-2-yl}methyl)piperidin-4-yl]benzamide;
11) A pharmaceutical composition comprising the compound or pharmaceutically acceptable salt thereof according to any one of 1) to 10) above as an active ingredient;
12) The pharmaceutical composition according to 11) above, which is an MCH receptor antagonist; and
13) A pharmaceutical composition for preventing or treating a disease selected from the group consisting of depression, anxiety disorders, attention deficit disorder, mania, manic-depressive illness, schizophrenia, mood disorders, stress, sleep disorders, attacks, memory impairment, cognitive impairment, dementia, amnesia, delirium, obesity, eating disorder, appetite disorder, hyperphagia, bulimia, cibophobia, diabetes, cardiovascular diseases, hypertension, dyslipidemia, myocardial infarction, movement disorder, drug abuse and drug addiction, which comprises the compound or pharmaceutically acceptable salt thereof according to any one of 1) to 10) above as an active ingredient.

### ADVANTAGES OF THE INVENTION

The compound of the present invention has MCH receptor antagonistic activity and is therefore effective for preventing and treating depression, anxiety disorders (such as generalized anxiety disorder, posttraumatic stress disorder, panic disorder, obsessive-compulsive disorder or social anxiety disorder), attention deficit disorder, mania, manic-depressive illness, schizophrenia, mood disorders, stress, sleep disorders, attacks, memory impairment, cognitive impairment, dementia, amnesia, delirium, obesity, eating disorder, appetite disorder, hyperphagia, bulimia, cibophobia, diabetes, cardiovascular diseases, hypertension, dyslipidemia, myocardial infarction, movement disorder (such as Parkinson's disease, epilepsy, convulsion or tremor), drug abuse, drug addiction or the like, based on an MCH receptor antagonistic effect.

### BEST MODE FOR CARRYING OUT THE INVENTION

The terms used herein are as defined below.

The "halogen atom" is a fluorine atom, a chlorine atom, a bromine atom or an iodine atom.

The "C₁₋₆ alkyl group" refers to a linear alkyl group having 1 to 6 carbon atoms or a branched alkyl group having 3 to 6 carbon atoms. Examples of the linear alkyl group include a methyl group, an ethyl group, a propyl group, a butyl group, a pentyl group and a hexyl group. Examples of the branched alkyl group include an isopropyl group, an isobutyl group, a tert-butyl group, an isopentyl group, a 1-ethylpropyl group and an isohexyl group.

The "C₁₋₆ alkylene group" refers to a linear alkylene group having 1 to 6 carbon atoms. Examples of the group include a methylene group, an ethylene group, a propylene group, a butylene group, a pentylene group and a hexylene group.

The "C₃₋₆ cycloalkyl group" refers to a cyclic alkyl group having 3 to 6 carbon atoms. Examples of the group include a cyclopropyl group, a cyclobutyl group, a cyclopentyl group and a cyclohexyl group.

The "C₂₋₆ alkenyl group" refers to a linear alkenyl group having 2 to 6 carbon atoms or a branched alkenyl group having 3 to 6 carbon atoms, which contains at least one double bond. Examples of the linear alkenyl group include a vinyl group, an allyl group, a 3-butenyl group, a 4-pentenyl group and a 5-hexenyl group. Examples of the branched alkenyl group include a 1-methyl-2-butenyl group, a 2-methyl-2-butenyl group, a 2-methyl-3-butenyl group and a 2-methyl-2-pentenyl group.

The "C₁₋₆ alkoxy group" refers to a linear alkoxy group having 1 to 6 carbon atoms or a branched alkoxy group having 3 to 6 carbon atoms. Examples of the linear alkoxy group include a methoxy group, an ethoxy group, a propoxy group, a butoxy group, a pentyloxy group and a hexyloxy group. Examples of the branched alkoxy group include an isopropoxy group, an isobutoxy group, a tert-butoxy group, an isopentyloxy group, a 1-ethylpropoxy group and an isohexyloxy group.

The "C₂₋₆ alkenyloxy group" refers to a linear alkenyloxy group having 2 to 6 carbon atoms or a branched alkenyloxy group having 3 to 6 carbon atoms, which contains at least one double bond. Examples of the linear alkenyloxy group include a vinyloxy group, an allyloxy group, a 3-butenyloxy group, a 4-pentenyloxy group and a 5-hexenyloxy group. Examples of the branched alkenyloxy group include a 1-methyl-2-butenyloxy group, a 2-methyl-2-butenyloxy group, a 2-methyl-3-butenyloxy group and a 2-methyl-2-pentenyloxy group.

The "aryloxy group" is a phenyloxy group or a naphthyloxy group, for example.

The "C₇₋₁₀ aralkyloxy group" refers to an aralkyloxy group having 7 to 10 carbon atoms. Examples of the group include a benzyloxy group and a phenethyloxy group.

Examples of the "heterocycloxy group" include a 3-azetidinyloxy group, a 3-oxetanyloxy group, a 3-thietanyloxy group, a 3-pyrrolidinyloxy group, a 3-tetrahydrofuranyloxy group, a 3-tetrahydrothiofuranyloxy group, a 3-piperidinyloxy group, a 2-tetrahydropyranyloxy group, a 3-tetrahydrothiopyranyloxy group, a 2-piperazinyloxy group and a 2-morpholinyloxy group.

Examples of the "C₁₋₆ alkylcarbonyloxy group" include a methylcarbonyloxy group, an ethylcarbonyloxy group, a propylcarbonyloxy group, a butylcarbonyloxy group, a pentylcarbonyloxy group, a hexylcarbonyloxy group, an isopropylcarbonyloxy group, an isobutylcarbonyloxy group, a tert-butylcarbonyloxy group, an isopentylcarbonyloxy group, a 1-ethylpropylcarbonyloxy group and an isohexylcarbonyloxy group.

The "mono-C₁₋₆ alkylamino group" refers to an amino group having one C₁₋₆ alkyl group. Examples of the group include a methylamino group, an ethylamino group, a propylamino group, a butylamino group, a pentylamino group, a hexylamino group, an isopropylamino group, an isobutylamino group, a tert-butylamino group, an isopentylamino group, a 1-ethylpropylamino group and an isohexylamino group.

The "di-C₁₋₆ alkylamino group" refers to an amino group having two C₁₋₆ alkyl groups independently. Examples of the group include a dimethylamino group, a diethylamino group, a dipropylamino group, a dibutylamino group, a dipentylamino group, a dihexylamino group, an ethylmethylamino group, a diisopropylamino group, a diisobutylamino group, a di-tert-butylamino group, a diisopentylamino group, a di-(1-ethylpropyl)amino group, a diisohexylamino group, an isopropylmethylamino group and an isobutylisopropylamino group.

Examples of the "mono-C₁₋₆ alkylcarbonylamino group" include a methylcarbonylamino group, an ethylcarbonylamino group, a propylcarbonylamino group, a butylcarbonylamino group, a pentylcarbonylamino group, a hexylcarbonylamino group, an isopropylcarbonylamino group, an isobutylcarbonylamino group, a tert-butylcarbonylamino group, an isopentylcarbonylamino group, a 1-ethylpropylcarbonylamino group and an isohexylcarbonylamino group.

The "C₂₋₆ alkanoyl group" refers to a carbonyl group having a C₁₋₅ alkyl group. Examples of the group include a methylcarbonyl group, an ethylcarbonyl group, a propylcarbonyl group, a butylcarbonyl group, a pentylcarbonyl group, a hexylcarbonyl group, an isopropylcarbonyl group, an isobutylcarbonyl group, a tert-butylcarbonyl group, an isopentylcarbonyl group, a 1-ethylpropylcarbonyl group and an isohexylcarbonyl group.

The "C₁₋₆ alkoxycarbonyl group" refers to a carbonyl group having a C₁₋₆ alkoxy group. Examples of the group include a methoxycarbonyl group, an ethoxycarbonyl group, a propoxycarbonyl group, a butoxycarbonyl group, a pentyloxycarbonyl group, a hexyloxycarbonyl group, an isopropoxycarbonyl group, an isobutoxycarbonyl group, a tert-butoxycarbonyl group, an isopentyloxycarbonyl group, a 1-ethylpropoxycarbonyl group and an isohexyloxycarbonyl group.

The "arylcarbonyl group" refers to a carbonyl group having an aryl group. Examples of the group include a phenylcarbonyl group and a naphthylcarbonyl group.

The "C₇₋₁₀ aralkyloxycarbonyl group" refers to an aralkyloxycarbonyl group having 7 to 10 carbon atoms. Examples of the group include a benzyloxycarbonyl group and a phenethyloxycarbonyl group.

The "mono-C₁₋₆ alkylaminocarbonyl group" refers to a carbamoyl group having one C₁₋₆ alkyl group on the nitrogen atom. Examples of the group include a methylcarbamoyl group, an ethylcarbamoyl group, a propylcarbamoyl group, a butylcarbamoyl group, a pentylcarbamoyl group, a hexylcarbamoyl group, an isopropylcarbamoyl group, an isobutylcarbamoyl group, a tert-butyl carbamoyl group, an isopentylcarbamoyl group, a 1-ethylpropylcarbamoyl group and an isohexylcarbamoyl group.

The "di-C₁₋₆ alkylaminocarbonyl group" refers to a carbamoyl group having two C₁₋₆ alkyl groups independently on the nitrogen atom. Examples of the group include a dimethylcarbamoyl group, a diethylcarbamoyl group, a dipropylcarbamoyl group, a dibutylcarbamoyl group, a dipentylcarbamoyl group, a dihexylcarbamoyl group, an ethylmethylcarbamoyl group, a diisopropylcarbamoyl group, a diisobutylcarbamoyl group, a di-tert-butylcarbamoyl group, a diisopentylcarbamoyl group, a di-(1-ethylpropyl)carbamoyl group, a diisohexylcarbamoyl group, an isopropylmethylcarbamoyl group and an isobutylisopropylcarbamoyl group.

The "C₁₋₆ alkylthio group" refers to a linear alkylthio group having 1 to 6 carbon atoms or a branched alkylthio group having 3 to 6 carbon atoms. Examples of the group include a methylthio group, an ethylthio group, a propylthio group, a butylthio group, a pentylthio group, a hexylthio group, an isopropylthio group, an isobutylthio group, a tert-butylthio group, an isopentylthio group, a 1-ethylpropylthio group and an isohexylthio group.

The "C₁₋₆ alkylsulfonyl group" refers to a linear alkylsulfonyl group having 1 to 6 carbon atoms or a branched alkylsulfonyl group having 3 to 6 carbon atoms. Examples of the group include a methylsulfonyl group, an ethylsulfonyl group, a propylsulfonyl group, a butylsulfonyl group, a pentylsulfonyl group, a hexylsulfonyl group, an isopropylsulfonyl group, an isobutylsulfonyl group, a tert-butylsulfonyl group, an isopentylsulfonyl group, a 1-ethylpropylsulfonyl group and an isohexylsulfonyl group.

The "heterocyclic group" refers to a 4- to 6-membered heterocyclic group containing 1 to 3 hetero atoms selected from a nitrogen atom, an oxygen atom or a sulfur atom, in addition to the carbon atoms. Examples of the group include an azetidinyl group, an oxetanyl group, a thietanyl group, a pyrrolidinyl group, a tetrahydrofuranyl group, a tetrahydrothiofuranyl group, a piperidinyl group, a tetrahydropyranyl group, a tetrahydrothiopyranyl group, a piperazinyl group and a morpholinyl group.

The "aryl" refers to an aromatic ring having 6 to 14 carbon atoms. Examples of the ring include benzene, naphthalene, anthracene and 9H-fluorene.

The "heteroaryl" refers to a 5- to 10-membered heteroaromatic ring containing 1 to 3 hetero atoms selected from a nitrogen atom, an oxygen atom, or a sulfur atom, in addition to the carbon atoms. Examples of the ring include pyrrole, pyrazole, imidazole, furan, oxazole, isoxazole, thiophene, thiazole, isothiazole, pyridine, pyrimidine, pyridazine, pyrazine, indole, benzofuran, benzothiophene, benzimidazole, benzoxazole, benzothiazole, benzopyrazole, benzoisoxazole, benzoisothiazole, quinoline, isoquinoline, quinazoline, quinoxaline, phthalazine, cinnoline and 9H-xanthene.

The "aryl group" refers to a monovalent group derived from the "aryl". Examples of the group include a phenyl group, a naphthyl group, an anthracenyl group and a 9H-fluorenyl group.

The "heteroaryl group" refers to a monovalent group derived from the "heteroaryl". Examples of the group include a pyrrolyl group, a pyrazolyl group, an imidazolyl group, a furyl group, an oxazolyl group, an isoxazolyl group, a thienyl group, a thiazolyl group, an isothiazolyl group, a pyridyl group, a pyrimidinyl group, a pyridazinyl group, a pyrazinyl group, an indolyl group, a benzofuryl group, a benzothienyl group, a benzimidazolyl group, a benzoxazolyl group, a benzothiazolyl group, a benzopyrazolyl group, a benzisoxazolyl group, a benzisothiazolyl group, a quinolyl group, an isoquinolyl group, a quinazolinyl group, a quinoxalinyl group, a phthalazinyl group, a cinnolinyl group and a 9H-xanthenyl group.

The "C₁₋₆ alkyl group (wherein the C₁₋₆ alkyl group may be substituted with a halogen atom, a hydroxyl group or a C₁₋₆ alkoxy group)" refers to a C₁₋₆ alkyl group which may be substituted with a halogen atom, a hydroxyl group or a C₁₋₆ alkoxy group. Examples of the group include a methyl group, an ethyl group, a propyl group, a butyl group, a pentyl group, a hexyl group, an isopropyl group, an isobutyl group, a tert-butyl group, an isopentyl group, a 1-ethylpropyl group, an isohexyl group, a fluoromethyl group, a difluoromethyl group, a trifluoromethyl group, a hydroxymethyl group, a 2-hydroxyethyl group, a 1-hydroxypropyl group, a 3-hydroxypropyl group, a 4-hydroxybutyl group, a 5-hydroxypentyl group, a 6-hydroxyhexyl group, a methoxymethyl group, a 2-methoxyethyl group, a 1-methoxypropyl group, a 3-methoxypropyl group, a 4-methoxybutyl group, a 5-methoxypentyl group and a 6-methoxyhexyl group.

The "C₁₋₆ alkyl group (wherein the C₁₋₆ alkyl group may be substituted with a hydroxyl group or an amino group)" refers to a C₁₋₆ alkyl group which may be substituted with a hydroxyl group or an amino group. Examples of the group include a methyl group, an ethyl group, a propyl group, a butyl group, a pentyl group, a hexyl group, an isopropyl group, an isobutyl group, a tert-butyl group, an isopentyl group, a 1-ethylpropyl group, an isohexyl group, a hydroxymethyl group, a 2-hydroxyethyl group, a 1-hydroxypropyl group, a 3-hydroxypropyl group, a 4-hydroxybutyl group, a 5-hydroxypentyl group, a 6-hydroxyhexyl group, an aminomethyl group, a 2-aminoethyl group, a 1-aminopropyl group, a 3-aminopropyl group, a 4-aminobutyl group, a 5-aminopentyl group and a 6-aminohexyl group.

The "C₁₋₆ alkylene group (wherein the C₁₋₆ alkylene group may be substituted with a C₁₋₆ alkyl group)" refers to a linear C₁₋₆ alkylene group which may be substituted with a C₁₋₆ alkyl group.

The "C₁₋₆ alkylene group {wherein the C₁₋₆ alkylene group may be substituted with one or two independent C₁₋₆ alkyl groups (wherein the C₁₋₆ alkyl groups may be bonded together to form a ring) or aryl groups (wherein the aryl groups may be substituted with a halogen atom)}" refers to a linear C₁₋₆ alkylene group which may be substituted with one or two independent C₁₆ alkyl groups (wherein the C₁₋₆ alkyl groups may be bonded together to form a ring) or aryl groups (wherein the aryl groups may be substituted with a halogen atom).

The "methylene group {wherein the methylene group may be substituted with an aryl group (wherein the aryl group may be substituted with a halogen atom)}" refers to a methylene group which may be substituted with an aryl group (wherein the aryl group may be substituted with a halogen atom).

The "methylene group {wherein the methylene group may be substituted with one or two independent C₁₆ alkyl groups (wherein the C₁₋₆ alkyl groups may be bonded together to form a ring) or aryl groups (wherein the aryl groups may each be substituted with a halogen atom)}" refers to a methylene group which may be substituted with one or two independent C₁₋₆ alkyl groups (wherein the C₁₋₆ alkyl groups may be bonded together to form a ring) or aryl groups (wherein the aryl groups may each be substituted with a halogen atom).

The "C₁₋₆ alkoxy group (wherein the C₁₋₆ alkoxy group may be substituted with a halogen atom)" refers to a C₁₋₆ alkoxy group which may be substituted with a halogen atom. Examples of the group include a methoxy group, an ethoxy group, a propoxy group, a butoxy group, a pentyloxy group, a hexyloxy group, an isopropoxy group, an isobutoxy group, a tert-butoxy group, an isopentyloxy group, a 1-ethylpropoxy group, an isohexyloxy group, a fluoromethoxy group, a difluoromethoxy group and a trifluoromethoxy group.

The "C₁₋₆ alkoxy group (wherein the C₁₋₆ alkoxy group may be substituted with a C₃₋₆ cycloalkyl group)" refers to a C₁₋₆ alkoxy group which may be substituted with a C₃₋₆ cycloalkyl group. Examples of the group include a methoxy group, an ethoxy group, a propoxy group, a butoxy group, a pentyloxy group, a hexyloxy group, an isopropoxy group, an isobutoxy group, a tert-butoxy group, an isopentyloxy group, a 1-ethylpropoxy group, an isohexyloxy group, a cyclopropylmethoxy group, a cyclobutylmethoxy group, a 2-methylcyclopropylmethoxy group, a cyclopentylmethoxy group, a 2-methylcyclobutylmethoxy group, a cyclohexylmethoxy group, a 1-cyclopropylethoxy group and a 2-cyclopropylethoxy group.

Examples of the "C₁₋₆ alkoxy group (wherein the C₁₋₆ alkoxy group may be substituted with a C₁₋₆ alkoxy group)" include a methoxy group, an ethoxy group, a propoxy group, a butoxygroup, a pentyloxy group, a hexyloxy group, an isopropoxy group, an isobutoxy group, a tert-butoxy group, an isopentyloxy group, a 1-ethylpropoxy group, an isohexyloxy group, a 2-methoxyethoxy group, a 2-ethoxyethoxy group, a 2-propoxyethoxy group, a 2-butoxyethoxy group, a 2-pentyloxyethoxy group, a 2-hexyloxyethoxy group, a 3-methoxypropoxy group, a 4-methoxybutoxy group, a 5-methoxypentyloxy group, a 6-methoxyhexyloxy group, a 2-(1-ethylpropoxy)ethoxy group, a 2-methoxy-1-methylethoxy group and a 2-methoxy-2-methylethoxy group.

The "mono-C₁₋₆ alkylamino group (wherein the mono-C₁₋₆ alkylamino group may be substituted with a C₃₋₆ cycloalkyl group)" refers to an amino group having one C₁₋₆ alkyl group which may be substituted with a C₃₋₆ cycloalkyl group. Examples of the group include a methylamino group, an ethylamino group, a propylamino group, a butylamino group, a pentylamino group, a hexylamino group, an isopropylamino group, an isobutylamino group, a tert-butylamino group, an isopentylamino group, a 1-ethylpropylamino group, an isohexylamino group, a cyclopropylmethylamino group, a cyclobutylmethylamino group, a 2-methylcyclopropylmethylamino group, a cyclopentylmethylamino group, a 2-methylcyclobutylmethylamino group, a cyclohexylmethylamino group, a 1-cyclopropylethylamino group and a 2-cyclopropylethylamino group.

The "heterocyclic group (wherein the heterocyclic group is substituted with an amino group)" refers to a heterocyclic group substituted with an amino group. Examples of the group include a 3-aminoazetidinyl group, a 3-aminooxetanyl group, a 3-aminothietanyl group, a 3-aminopyrrolidinyl group, a 3-aminotetrahydrofuranyl group, a 3-aminotetrahydrothiofuranyl group, a 3-aminopiperidinyl group, a 3-aminotetrahydropyranyl group, a 3-aminotetrahydrothiopyranyl group, a 2-aminopiperazinyl group and a 2-aminomorpholinyl group.

The "heterocyclic group {wherein the heterocyclic group is substituted with a C₁₋₆ alkyl group (wherein the C₁₋₆ alkyl group may be substituted with a hydroxyl group) or an amino group}" refers to a heterocyclic group substituted with a C₁₋₆ alkyl group which may be substituted with a hydroxyl group or with an amino group. Examples of the group include a 3-methylazetidinyl group, a 3-methyloxetanyl group, a 3-methylthietanyl group, a 3-methylpyrrolidinyl group, a 3-methyltetrahydrofuranyl group, a 3-methyltetrahydrothiofuranyl group, a 3-methylpiperidinyl group, a 3-methyltetrahydropyranyl group, a 3-methyltetrahydrothiopyranyl group, a 4-methylpiperazinyl group, a 2-methylmorpholinyl group, a 3-hydroxymethylazetidinyl group, a 3-hydroxymethyloxetanyl group, a 3-hydroxymethylthietanyl group, a 3-hydroxymethylpyrrolidinyl group, a 3-hydroxymethyltetrahydrofuranyl group, a 3-hydroxymethyltetrahydrothiofuranyl group, a 4-hydroxymethylpiperidinyl group, a 3-hydroxymethyltetrahydropyranyl group, a 3-hydroxymethyltetrahydrothiopyranyl group, a 4-hydroxymethylpiperazinyl group, a 2-hydroxymethylmorpholinyl group, a 3-(2-hydroxyethyl)azetidinyl group, a 3-(2-hydroxyethyl)oxetanyl group, a 3-(2-hydroxyethyl)thietanyl group, a 3-(2-hydroxyethyl)pyrrolidinyl group, a 3-(2-hydroxyethyl)tetrahydrofuranyl group, a 3-(2-hydroxyethyl)tetrahydrothiofuranyl group, a 3-(2-hydroxyethyl)piperidinyl group, a 3-(2-hydroxyethyl)tetrahydropyranyl group, a 3-(2-hydroxyethyl)tetrahydrothiopyranyl group, a 4-(2-hydroxyethyl)piperazinyl group, a 2-(2-hydroxyethyl)morpholinyl group, a 3-aminoazetidinyl group, a 3-aminooxetanyl group, a 3-aminothietanyl group, a 3-aminopyrrolidinyl group, a 3-aminotetrahydrofuranyl group, a 3-aminotetrahydrothiofuranyl group, a 3-aminopiperidinyl group, a 3-aminotetrahydropyranyl group, a 3-aminotetrahydrothiopyranyl group, a 2-aminopiperazinyl group and a 2-aminomorpholinyl group.

The "heterocyclic group {wherein the heterocyclic group is substituted with a hydroxyl group, a C₁₋₆ alkyl group (wherein the C₁₋₆ alkyl group may be substituted with a hydroxyl group or an amino group) or an amino group}" refers to a heterocyclic group substituted with a hydroxyl group, with a C₁₋₆ alkyl group which may be substituted with a hydroxyl group or an amino group or with an amino group. Examples of the group include a 3-hydroxyazetidinyl group, a 3-hydroxyoxetanyl group, a 3-hydroxythietanyl group, a 3-hydroxypyrrolidinyl group, a 3-hydroxytetrahydrofuranyl group, a 3-hydroxytetrahydrothiofuranyl group, a 4-hydroxypiperidinyl group, a 3-hydroxytetrahydropyranyl group, a 3-hydroxytetrahydropyranyl group, a 2-hydroxypiperazinyl group, a 2-hydroxymorpholinyl group, a 3-methylazetidinyl group, a 3-methyloxetanyl group, a 3-methylthietanyl group, a 3-methylpyrrolidinyl group, a 3-methyltetrahydrofuranyl group, a 3-methyltetrahydrothiofuranyl group, a 3-methylpiperidinyl group, a 3-methyltetrahydropyranyl group, a 3-methyltetrahydropyranyl group, a 4-methylpiperazinyl group, a 2-methylmorpholinyl group, a 3-hydroxymethylazetidinyl group, a 3-hydroxymethyloxetanyl group, a 3-hydroxymethylthietanyl group, a 3-hydroxymethylpyrrolidinyl group, a 3-hydroxymethyltetrahydrofuranyl group, a 3-hydroxymethyltetrahydrothiofuranyl group, a 4-hydroxymethylpiperidinyl group, a 3-hydroxymethyltetrahydropyranyl group, a 3-hydroxymethyltetrahydropyranyl group, a 4-hydroxymethylpiperazinyl group, a 2-hydroxymethylmorpholinyl group, a 3-(2-hydroxyethyl)azetidinyl group, a 3-(2-hydroxyethyl)oxetanyl group, a 3-(2-hydroxyethyl)thietanyl group, a 3-(2-hydroxyethyl)pyrrolidinyl group, a 3-(2-hydroxyethyl)tetrahydrofuranyl group, a 3-(2-hydroxyethyl)tetrahydrothiofuranyl group, a 3-(2-hydroxyethyl)piperidinyl group, a 3-(2-hydroxyethyl)tetrahydropyranyl group, a 3-(2-hydroxyethyl)tetrahydropyranyl group, a 4-(2-hydroxyethyl)piperazinyl group, a 2-(2-hydroxyethyl)morpholinyl group, a 3-aminomethylazetidinyl group, a 3-aminomethyloxetanyl group, a 3-aminomethylthietanyl group, a 3-aminomethylpyrrolidinyl group, a 3-aminomethyltetrahydrofuranyl group, a 3-aminomethyltetrahydrothiofuranyl group, a 4-aminomethylpiperidinyl group, a 3-aminomethyltetrahydropyranyl group, a 3-aminomethyltetrahydropyranyl group, a 4-aminomethylpiperazinyl group, a 2-aminomethylmorpholinyl group, a 3-aminoazetidinyl group, a 3-aminooxetanyl group, a 3-aminothietanyl group, a 3-aminopyrrolidinyl group, a 3-aminotetrahydrofuranyl group, a 3-aminotetrahydrothiofuranyl group, 3-aminopiperidine, a 3-aminotetrahydropyranyl group, a 3-aminotetrahydropyranyl group, a 2-aminopiperazinyl group and a 2-aminomorpholinyl group.

The "aryl group {wherein the aryl group may have 1 to 3 substituents selected from the group consisting of a halogen atom, a nitro group, a C₁₋₆ alkyl group (wherein the C₁₋₆ alkyl group may be substituted with a halogen atom), a C₂₋₆ alkenyl group, a C₁₋₆ alkoxy group (wherein the C₁₋₆ alkoxy group may be substituted with a halogen atom), a di-C₁₋₆ alkylamino group, a C₂₋₆ alkanoyl group and a C₁₋₆ alkylthio group}" refers to an aryl group which may have 1 to 3 substituents selected from the group consisting of a halogen atom, a nitro group, a C₁₋₆ alkyl group (wherein the C₁₋₆ alkyl group may be substituted with a halogen atom), a C₂₋₆ alkenyl group, a C₁₋₆ alkoxy group (wherein the C₁₋₆ alkoxy group may be substituted with a halogen atom), a di-C₁₋₆ alkylamino group, a C₂₋₆ alkanoyl group and a C₁₋₆ alkylthio group.

The "aryl group {wherein the aryl group may have 1 to 3 substituents selected from the group consisting of a halogen atom, a cyano group, a nitro group, a C₁₋₆ alkyl group (wherein the C₁₋₆ alkyl group may be substituted with a halogen atom), a C₂₋₆ alkenyl group, a C₁₋₆ alkoxy group (wherein the C₁₋₆ alkoxy group may be substituted with a halogen atom), an aryloxy group, a di-C₁₋₆ alkylamino group, a C₂₋₆ alkanoyl group, a C₁₋₆ alkoxycarbonyl group and a C₁₋₆ alkylthio group}" refers to an aryl group which may have 1 to 3 substituents selected from the group consisting of a halogen atom, a cyano group, a nitro group, a C₁₋₆ alkyl group (wherein the C₁₋₆ alkyl group may be substituted with a halogen atom), a C₂₋₆ alkenyl group, a C₁₋₆ alkoxy group (wherein the C₁₋₆ alkoxy group may be substituted with a halogen atom), an aryloxy group, a di-C₁₋₆ alkylamino group, a C₂₋₆ alkanoyl group, a C₁₋₆ alkoxycarbonyl group and a C₁₋₆ alkylthio group.

The "aryl group {wherein the aryl group may have 1 to 3 substituents selected from the group consisting of a halogen atom, a cyano group, a nitro group, a C₁₋₆ alkyl group (wherein the C₁₋₆ alkyl group may be substituted with a halogen atom, a hydroxyl group or a C₁₋₆ alkoxy group), a C₂₋₆ alkenyl group, a hydroxyl group, a C₁₋₆ alkoxy group (wherein the C₁₋₆ alkoxy group may be substituted with a halogen atom), an aryloxy group, a di-C₁₋₆ alkylamino group, a formyl group, a C₂₋₆ alkanoyl group, an arylcarbonyl group, a C₁₋₆ alkoxycarbonyl group, a mono-C₁₋₆ alkylcarbonylamino group, a C₁₋₆ alkylcarbonyloxy group, a mono-C₁₋₆ alkylaminocarbonyl group, a C₁₋₆ alkylthio group, an aryl group and a heteroaryl group}" refers to an aryl group which may have 1 to 3 substituents selected from the group consisting of a halogen atom, a cyano group, a nitro group, a C₁₋₆ alkyl group (wherein the C₁₋₆ alkyl group may be substituted with a halogen atom, a hydroxyl group or a C₁₋₆ alkoxy group), a C₂₋₆ alkenyl group, a hydroxyl group, a C₁₋₆ alkoxy group (wherein the C₁₋₆ alkoxy group may be substituted with a halogen atom), an aryloxy group, a di-C₁₋₆ alkylamino group, a formyl group, a C₂₋₆ alkanoyl group, an arylcarbonyl group, a C₁₋₆ alkoxycarbonyl group, a mono-C₁₋₆ alkylcarbonylamino group, a C₁₋₆ alkylcarbonyloxy group, a mono-C₁₋₆ alkylaminocarbonyl group, a C₁₋₆ alkylthio group, an aryl group and a heteroaryl group.

The "aryl group or heteroaryl group {wherein the aryl group or the heteroaryl group may have 1 to 3 substituents selected from substituent group X consisting of a halogen atom, a cyano group, a nitro group, a C₁₋₆ alkyl group (wherein the C₁₋₆ alkyl group may be substituted with a halogen atom, a hydroxyl group or a C₁₋₆ alkoxy group), a C₂₋₆ alkenyl group, a hydroxyl group, a C₁₋₆ alkoxy group (wherein the C₁₋₆ alkoxy group may be substituted with a halogen atom), an aryloxy group, a di-C₁₋₆ alkylamino group, a formyl group, a carboxyl group, a carbamoyl group, a C₂₋₆ alkanoyl group, an arylcarbonyl group, a C₁₋₆ alkoxycarbonyl group, a mono-C₁₋₆ alkylcarbonylamino group, a C₁₋₆ alkylcarbonyloxy group, a mono-C₁₋₆ alkylaminocarbonyl group, a di-C₁₋₆ alkylaminocarbonyl group, a C₁₋₆ alkylthio group, a C₁₋₆ alkylsulfonyl group, an aryl group and a heteroaryl group}" refers to an aryl group or a heteroaryl group which may have 1 to 3 substituents selected from substituent group X consisting of a halogen atom, a cyano group, a nitro group, a C₁₋₆ alkyl group (wherein the C₁₋₆ alkyl group may be substituted with a halogen atom, a hydroxyl group or a C₁₋₆ alkoxy group), a C₂₋₆ alkenyl group, a hydroxyl group, a C₁₋₆ alkoxy group (wherein the C₁₋₆ alkoxy group may be substituted with a halogen atom), an aryloxy group, a di-C₁₋₆ alkylamino group, a formyl group, a carboxyl group, a carbamoyl group, a C₂₋₆ alkanoyl group, an arylcarbonyl group, a C₁₋₆ alkoxycarbonyl group, a mono-C₁₋₆ alkylcarbonylamino group, a C₁₋₆ alkylcarbonyloxy group, a mono-C₁₋₆ alkylaminocarbonyl group, a di-C₁₋₆ alkylaminocarbonyl group, a C₁₋₆ alkylthio group, a C₁₋₆ alkylsulfonyl group, an aryl group and a heteroaryl group.

The "heteroaryl group (wherein the heteroaryl group may be substituted with a halogen atom)" refers to a heteroaryl group which may be substituted with a halogen atom.

An embodiment of the compound of the present invention relates to a substituted naphthalene compound represented by the formula (I): 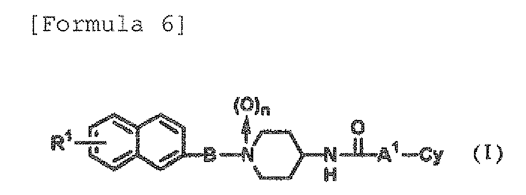 and a pharmaceutically acceptable salt thereof,
wherein in the formula (I), R¹, R^{1a}, R^{1b}, R², R³, Z¹, Z², Z³, Z⁴, Z⁵, A¹, A², A³, A⁴, A⁵, B, Cy, n and substituent group X are as defined above.

Another preferred embodiment of the compound of the present invention relates to the compound and pharmaceutically acceptable salt thereof according to the above embodiment, wherein the compound is represented by the formula (V): 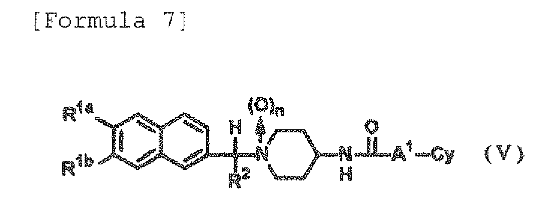 wherein any one of R^{1a} and R^{1b} is a hydrogen atom and the other is a substituent elected from the group consisting of the formula (II), the formula (III) and the formula (IV), R² is a hydrogen atom or a C₁₋₆ alkyl-group and A¹, Cy and n are as defined in the above embodiment.

Another preferred embodiment of the compound of the present invention relates to the compound or pharmaceutically acceptable salt thereof according to the above embodiment, wherein in the formula (V), R^{1a} is a hydrogen atom, R^{1b} is a substituent selected from the group consisting of the formula (II), the formula (III) and the formula (IV) and R² is a hydrogen atom.

Another preferred embodiment of the compound of the present invention relates to the compound or pharmaceutically acceptable salt thereof according to the above embodiment, wherein in the formula (V), Cy is a C₃₋₆ cycloalkyl group, an aryl group {wherein the aryl group may have 1 to 3 substituents selected from the group consisting of a halogen atom, a cyano group, a nitro group, a C₁₋₆ alkyl group (wherein the C₁₋₆ alkyl group may be substituted with a halogen atom, a hydroxyl group or a C₁₋₆ alkoxy group), a C₂₋₆ alkenyl group, a hydroxyl group, a C₁₋₆ alkoxy group (wherein the C₁₋₆ alkoxy group may be substituted with a halogen atom), an aryloxy group, a di-C₁₋₆ alkylamino group, a formyl group, a C₂₋₆ alkanoyl group, an arylcarbonyl group, a C₁₋₆ alkoxycarbonyl group, a mono-C₁₋₆ alkylcarbonylamino group, a C₁₋₆ alkylcarbonyloxy group, a mono-C₁₋₆ alkylaminocarbonyl group, a C₁₋₆ alkylthio group, an aryl group and a heteroaryl group} or a heteroaryl group (wherein the heteroaryl group may be substituted with a halogen atom).

Another preferred embodiment of the compound of the present invention relates to the compound or pharmaceutically acceptable salt thereof according to the above embodiment, wherein in the formula (V), A¹ is a bond or a methylene group {wherein the methylene group may be substituted with one or two independent C₁₆ alkyl groups (wherein the C₁₋₆ alkyl groups may be bonded together to form a ring) or aryl groups (wherein the aryl groups may each be substituted with a halogen atom)}, Cy is an aryl group {wherein the aryl group may have 1 to 3 substituents selected from the group consisting of a halogen atom, a cyano group, a nitro group, a C₁₋₆ alkyl group (wherein the C₁₋₆ alkyl group may be substituted with a halogen atom), a C₂₋₆ alkenyl group, a C₁₋₆ alkoxy group (wherein the C₁₋₆ alkoxy group may be substituted with a halogen atom), an aryloxy group, a di-C₁₋₆ alkylamino group, a C₂₋₆ alkanoyl group, a C₁₋₆ alkoxycarbonyl group and a C₁₋₆ alkylthio group} or a heteroaryl group (wherein the heteroaryl group may be substituted with a halogen atom) and n is 0.

Another preferred embodiment of the compound of the present invention relates to the compound or pharmaceutically acceptable salt thereof according to the above embodiment, wherein in the formula (V), Cy is an aryl group {wherein the aryl group may have 1 to 3 substituents selected from the group consisting of a halogen atom, a nitro group, a C₁₋₆ alkyl group (wherein the C₁₋₆ alkyl group may be substituted with a halogen atom), a C₂₋₆ alkenyl group, a C₁₋₆ alkoxy group (wherein the C₁₋₆ alkoxy group may be substituted with a halogen atom), a di-C₁₋₆ alkylamino group, a C₂₋₆ alkanoyl group and a C₁₋₆ alkylthio group}, provided that Cy is 3-monosubstituted, 3,4-disubstituted, 3,5-disubstituted or 3,4,5-trisubstituted.

Another preferred embodiment of the compound of the present invention relates to the compound or pharmaceutically acceptable salt thereof according to the above embodiment, wherein in the formula (V), R^{1b} is a substituent selected from the group consisting of the formula (II) [wherein in the formula (II), Z¹ is a hydroxyl group, a C₁₋₆ alkoxy group (wherein the C₁₋₆ alkoxy group may be substituted with a C₃₋₆ cycloalkyl group), a C₂₋₆ alkenyloxy group, a mono-C₁₋₆ alkylamino group (wherein the mono-C₁₋₆ alkylamino group may be substituted with a C₃₋₆ cycloalkyl group), a formyl group, a C₁₋₆ alkoxycarbonyl group or a heterocyclic group {wherein the heterocyclic group is substituted with a C₁₋₆ alkyl group (wherein the C₁₋₆ alkyl group may be substituted with a hydroxyl group) or an amino group} and A² is a C₁₋₆ alkylene group, provided that A² may be a bond when Z¹ is a formyl group], the formula (III) {wherein in the formula (III), Z² is a hydroxyl group, a C₁₋₆ alkoxy group, a C₇₋₁₀ aralkyloxy group, an amino group, a mono-C₁₋₆ alkylamino group, a di-C₁₋₆ alkylamino group, a C₇₋₁₀ aralkyloxycarbonyl group or a heterocyclic group, Z³ is -O- or -NR³-, wherein R³ is a hydrogen atom or a C₁₋₆ alkyl group, and A³ and A⁴ are each independently a C₁₋₆ alkylene group (wherein the C₁₆ alkylene group may be substituted with a C₁₋₆ alkyl group)} and the formula (IV){wherein in the formula (IV), Z⁴ is a C₁₋₆ alkoxy group (wherein the C₁₋₆ alkoxy group may be substituted with a C₁₋₆ alkoxy group) or a heterocyclic group, provided that Z⁴ is not a heterocyclic group when Z⁵ is -O-, Z⁵ is -O- or -NH-CO- and A⁵ is a C₁₋₆ alkylene group}.

Another preferred embodiment of the compound of the present invention relates to the compound or pharmaceutically acceptable salt thereof according to the above embodiment, wherein in the formula (V), R^{1b} is a substituent selected from the group consisting of the formula (II) {wherein in the formula (II), Z¹ is a hydroxyl group, a C₁₋₆ alkoxy group (wherein the C₁₋₆ alkoxy group may be substituted with a C₃₋₆ cycloalkyl group), a C₂₋₆ alkenyloxy group, a mono-C₁₋₆ alkylamino group (wherein the mono-C₁₋₆ alkylamino group may be substituted with a C₃₋₆ cycloalkyl group), a formyl group, a C₁₋₆ alkoxycarbonyl group or a heterocyclic group (wherein the heterocyclic group is substituted with an amino group) and A² is a C₁₋₆ alkylene group, provided that A² may be a bond when Z¹ is a formyl group}, the formula (III) {wherein in the formula (III), Z² is a hydroxyl group, a C₁₋₆ alkoxy group, a C₇₁₀ aralkyloxy group, an amino group, a mono-C₁₋₆ alkylamino group, a di-C₁₋₆ alkylamino group or a heterocyclic group, Z³ is -O- or -NH- and A³ and A⁴ are each independently a C₁₋₆ alkylene group (wherein the C₁₆ alkylene group may be substituted with a C₁₋₆ alkyl group)} and the formula (IV) {wherein in the formula (IV), Z⁴ is a C₁₋₆ alkoxy group (wherein the C₁₋₆ alkoxy group may be substituted with a C₁₋₆ alkoxy group), Z⁵ is -O- and A⁵ is a C₁₋₆ alkylene group} and A¹ is a bond or a methylene group {wherein the methylene group may be substituted with an aryl group (wherein the aryl group may be substituted with a halogen atom)}.

Another preferred embodiment of the compound of the present invention relates to the compound or pharmaceutically acceptable salt thereof according to the above embodiment, wherein in the formula (V), R^{1b} is a substituent selected from the group consisting of the formula (II) {wherein in the formula (II), Z¹ is a C₁₋₆ alkoxy group (wherein the C₁₋₆ alkoxy group may be substituted with a C₃₋₆ cycloalkyl group) or a C₂₋₆ alkenyloxy group and A² is a C₁₋₆ alkylene group}, the formula (III) {wherein in the formula (III), Z² is a hydroxyl group or a C₁₋₆ alkoxy group, Z³ is -O- and A³ and A⁴ are each independently a C₁₋₆ alkylene group (wherein the C₁₋₆ alkylene group may be substituted with a C₁₋₆ alkyl group)} and the formula (IV) {wherein in the formula (IV), Z⁴ is a C₁₋₆ alkoxy group (wherein the C₁₋₆ alkoxy group may be substituted with a C₁₋₆ alkoxy group), Z⁵ is -O- and A⁵ is a C₁₋₆ alkylene group} and A¹ is a bond.

A preferred specific compound of the present invention is
3-methoxy-N-[1-({7-[(2-methoxyethoxy)methyl]-2-naphthyl}methyl)piperidin-4-yl]benzamide,
N-(1-{[7-(hydroxymethyl)-2-naphthyl]methyl}piperidin-4-yl)-3-methoxybenzamide,
N-{1-[(7-formyl-2-naphthyl)methyl]piperidin-4-yl}-3-methoxybenzamide,
3-chloro-N-(1-{[7-(ethoxymethyl)-2-naphthyl]methyl}piperidin-4-yl)-4-fluorobenzamide,
3,5-dimethoxy-N-(1-{[7-(methoxymethyl)-2-naphthyl]methyl}piperidin-4-yl)benzamide,
3,5-dimethoxy-N-(1-{[7-(methoxymethyl)-2-naphthyl]methyl}piperidin-4-yl)-4-methylbenzamide,
3-methoxy-N-(1-{[7-(methoxymethyl)-2-naphthyl]methyl}piperidin-4-yl)benzamide,
3-methoxy-N-(1-{[7-(methoxymethyl)-2-naphthyl]methyl}piperidin-4-yl)-4-methylbenzamide,
3-chloro-5-methoxy-N-(1-{[7-(methoxymethyl)-2-naphthyl]methyl}piperidin-4-yl)benzamide,
N-[1-({7-[(cyclopropylmethoxy)methyl]-2-naphthyl}methyl)piperidin-4-yl]-3-methoxybenzamide,
3-methoxy-N-[1-({7-[(3-methylbutoxy)methyl]-2-naphthyl}methyl)piperidin-4-yl]benzamide, 3-methoxy-N-(1-{[7-(propoxymethyl)-2-naphthyl]methyl}piperidin-4-yl)benzamide,
N-[1-({7-[(allyloxy)methyl]-2-naphthyl}methyl)piperidin-4-yl]-3-methoxybenzamide,
3-methoxy-N-[1-({7-[(2-methoxyethoxy)methyl]-2-naphthyl}methyl)piperidin-4-yl]benzamide,
N-{1-[(7-{[2-(benzyloxy)ethoxy]methyl}-2-naphthyl)methyl]piperidin-4-yl}-3-methoxybenzamide,
N-[1-({7-[(2-ethoxyethoxy)methyl]-2-naphthyl}methyl)piperidin-4-yl]-3-methoxybenzamide,
3-methoxy-N-[1-({7-[(2-propoxyethoxy)methyl]-2-naphthyl}methyl)piperidin-4-yl]benzamide,
N-[1-({7-[(2-isopropoxyethoxy)methyl]-2-naphthyl}methyl)piperidin-4-yl]-3-methoxybenzamide,
3-methoxy-N-[1-({7-[(3-methoxypropoxy)methyl]-2-naphthyl}methyl)piperidin-4-yl]benzamide,
3-methoxy-N-{1-[(7-{[2-(tetrahydro-2H-pyran-2-yloxy)ethoxy]methyl}-2-naphthyl)methyl]piperidin-4-yl}benzamide,
N-[1-({7-[(2-hydroxyethoxy)methyl]-2-naphthyl}methyl)piperidin-4-yl]-3-methoxybenzamide, 3-methoxy-N-[1-({7-[(2-pyrrolidin-1-ylethoxy)methyl]-2-naphthyl}methyl)piperidin-4-yl]benzamide,
N-{1-[(7-{[2-(dimethylamino)ethoxy]methyl}-2-naphthyl)methyl]piperidin-4-yl}-3-methoxybenzamide,
3-methoxy-N-{1-[(7-{[(tetrahydrofuran-2-ylmethyl)amino]methyl}-2-naphthyl)methyl]piperidin-4-yl}benzamide,
3-methoxy-N-[1-({7-[(4-methylpiperazin-1-yl)methyl]-2-naphthyl}methyl)piperidin-4-yl]benzamide,
3-methoxy-N-{1-[(7-{[(2-pyrrolidin-1-ylethyl)amino]methyl}-2-naphthyl)methyl]piperidin-4-yl}benzamide,
N-(1-{[7-({[2-(dimethylamino)ethyl]amino}methyl)-2-naphthyl]methyl}piperidin-4-yl)-3-methoxybenzamide,
3-methoxy-N-{1-[(7-{[(2-morpholin-4-ylethyl)amino]methyl}-2-naphthyl)methyl]piperidin-4-yl}benzamide,
N-{1-[(7-{[4-(2-hydroxyethyl)piperazin-1-yl]methyl}-2-naphthyl)methyl]piperidin-4-yl}-3-methoxybenzamide,
N-{1-[(7-{[[2-(dimethylamino)ethyl](methyl)amino]methyl}-2-naphthyl)methyl]piperidin-4-yl}-3-methoxybenzamide,
3-methoxy-N-{1-[(7-{[(3-methylbutyl)amino]methyl}-2-naphthyl)methyl]piperidin-4-yl}benzamide,
N-{1-[(7-{[(cyclopropylmethyl)amino]methyl}-2-naphthyl)methyl]piperidin-4-yl}-3-methoxybenzamide,
3-methoxy-N-{1-[(7-{[(2-methoxyethyl)amino]methyl}-2-naphthyl)methyl]piperidin-4-yl}benzamide,
3-methoxy-N-{1-[(7-{[(2-piperidin-1-ylethyl)amino]methyl}-2-naphthyl)methyl]piperidin-4-yl}benzamide,
benzyl N-{[7-({4-[(3-methoxybenzoyl)amino]piperidin-1-yl}methyl)-2-naphthyl]methyl}-beta-alaninate,
N-{1-[(7-{[(2-aminoethyl)amino]methyl}-2-naphthyl)methyl]piperidin-4-yl}-3-methoxybenzamide, 3-methoxy-N-(1-{[7-({[2-(methylamino)ethyl]amino}methyl)-2-naphthyl]methyl}piperidin-4-yl)benzamide,
N-[1-({7-[(3-aminopyrrolidin-1-yl)methyl]-2-naphthyl}methyl)piperidin-4-yl]-3-methoxybenzamide,
3-methoxy-N-[1-({7-[(pyrrolidin-3-ylamino)methyl]-2-naphthyl}methyl)piperidin-4-yl]benzamide,
3-methoxy-N-{1-[(7-{[(pyrrolidin-2-ylmethyl)amino]methyl}-2-naphthyl)methyl]piperidin-4-yl}benzamide,
3-ethoxy-N-[1-({7-[(2-methoxyethoxy)methyl]-2-naphthyl}methyl)piperidin-4-yl]benzamide,
methyl 3-({[1-({7-[(2-methoxyethoxy)methyl]-2-naphthyl}methyl)piperidin-4-yl]amino}carbonyl)benzoate,
3-(dimethylamino)-N-[1-({7-[(2-methoxyethoxy)methyl]-2-naphthyl}methyl)piperidin-4-yl]benzamide,
N-[1-({7-[(2-methoxyethoxy)methyl]-2-naphthyl}methyl)piperidin-4-yl]-3-(trifluoromethyl)benzamide,
N-[1-({7-[(2-methoxyethoxy)methyl]-2-naphthyl}methyl)piperidin-4-yl]-3-(trifluoromethoxy)benzamide,
N-[1-({7-[(2-methoxyethoxy)methyl]-2-naphthyl}methyl)piperidin-4-yl]-3-methylbenzamide,
3-cyano-N-[1-({7-[(2-methoxyethoxy)methyl]-2-naphthyl}methyl)piperidin-4-yl]benzamide,
N-[1-({7-[(2-methoxyethoxy)methyl]-2-naphthyl}methyl)piperidin-4-yl]-3-phenoxybenzamide,
N-[1-({7-[(2-methoxyethoxy)methyl]-2-naphthyl}methyl)piperidin-4-yl]-3-vinylbenzamide,
3-acetyl-N-[1-({7-[(2-methoxyethoxy)methyl]-2-naphthyl}methyl)piperidin-4-yl]benzamide,
N-[1-({7-[(2-methoxyethoxy)methyl]-2-naphthyl}methyl)piperidin-4-yl]-3-nitrobenzamide,
3-chloro-N-[1-({7-[(2-methoxyethoxy)methyl]-2-naphthyl}methyl)piperidin-4-yl]benzamide,
N-[1-({7-[(2-methoxyethoxy)methyl]-2-naphthyl}methyl)piperidin-4-yl]-3-(methylthio)benzamide,
5-chloro-N-[1-({7-[(2-methoxyethoxy)methyl]-2-naphthyl}methyl)piperidin-4-yl]nicotinamide,
3-methoxy-N-(1-{[7-(2-methoxyethoxy)-2-naphthyl]methyl}piperidin-4-yl)benzamide,
3-methoxy-N-[1-({7-[2-(2-methoxyethoxy)ethoxy]-2-naphthyl}methyl)piperidin-4-yl]benzamide,
3-methoxy-N-(1-{[7-(3-methoxypropoxy)-2-naphthyl]methyl}piperidin-4-yl)benzamide,
3-methoxy-N-(1-{[7-(2-oxoethyl)-2-naphthyl]methyl}piperidin-4-yl)benzamide,
N-(1-{[7-(2-hydroxyethyl)-2-naphthyl]methyl}piperidin-4-yl)-3-methoxybenzamide,
3-methoxy-N-[1-({7-[2-(2-methoxyethoxy)ethyl]-2-naphthyl}methyl)piperidin-4-yl]benzamide,
3-ethyl-N-[1-({7-[(2-methoxyethoxy)methyl]-2-naphthyl}methyl)piperidin-4-yl]benzamide,
3-chloro-4-fluoro-N-(1-{[7-(methoxymethyl)-2-naphthyl]methyl}piperidin-4-yl)benzamide,
3-methoxy-N-[1-(1-{7-[(2-methoxyethoxy)methyl]-2-naphthyl}ethyl)piperidin-4-yl]benzamide,
3-methoxy-N-(1-{[7-(4-oxobutyl)-2-naphthyl]methyl}piperidin-4-yl)benzamide,
N-(1-{[7-(4-hydroxybutyl)-2-naphthyl]methyl}piperidin-4-yl)-3-methoxybenzamide,
ethyl 3-[7-({4-[(3-methoxybenzoyl)amino]piperidin-1-yl}methyl)-2-naphthyl]propanoate,
N-(1-{[7-(3-hydroxypropyl)-2-naphthyl]methyl}piperidin-4-yl)-3-methoxybenzamide,
7-({4-[(3-methoxybenzoyl)amino]piperidin-1-yl}methyl)-N-(2-pyrrolidin-1-ylethyl)-2-naphthamide,
2-(3,4-dichlorophenyl)-N-(1-{[7-(methoxymethyl)naphthalen-2-yl]methyl}piperidin-4-yl)acetamide,
N-[1-({7-[(2-methoxyethoxy)methyl]naphthalen-2-yl}methyl)piperidin-4-yl]-2,2-diphenylacetamide,
2,2-bis(4-chlorophenyl)-N-[1-({7-[(2-methoxyethoxy)methyl]naphthalen-2-yl}methyl)piperidin-4-yl]acetamide,
N-[1-({7-[(2-methoxyethoxy)methyl]naphthalen-2-yl}methyl)piperidin-4-yl]thiophene-3-carboxamide,
N-[1-({7-[(2-methoxyethoxy)methyl]naphthalen-2-yl}methyl)piperidin-4-yl]-9H-fluorene-9-carboxamide or
3-methoxy-N-[1-({7-[(3-methoxybutoxy)methyl]naphthalen-2-yl}methyl)piperidin-4-yl]benzamide,
or a pharmaceutically acceptable salt thereof.

An embodiment of the compound of the present invention relates to a pharmaceutical composition comprising at least one compound described herein or a pharmaceutically acceptable salt thereof as an active ingredient.

Another preferred embodiment of the compound of the present invention relates to a pharmaceutical composition comprising at least one compound described herein or a pharmaceutically acceptable salt thereof as an active ingredient, which is an MCH receptor antagonist.

Another preferred embodiment of the compound of the present invention relates to a pharmaceutical composition for preventing or treating a disease selected from the group consisting of depression, anxiety disorders (such as generalized anxiety disorder, posttraumatic stress disorder, panic disorder, obsessive-compulsive disorder or social anxiety disorder), attention deficit disorder, mania, manic-depressive illness, schizophrenia, mood disorders, stress, sleep disorders, attacks, memory impairment, cognitive impairment, dementia, amnesia, delirium, obesity, eating disorder, appetite disorder, hyperphagia, bulimia, cibophobia, diabetes, cardiovascular diseases, hypertension, dyslipidemia, myocardial infarction, movement disorder (such as Parkinson's disease, epilepsy, convulsion or tremor), drug abuse and drug addiction, which comprises at least one compound described herein or a pharmaceutically acceptable salt thereof as an active ingredient.

The compounds (I) to (I-IV) and (V) to (V-XVII) of the present invention and pharmaceutically acceptable salts thereof can be synthesized by various methods of organic synthesis known to a person skilled in the art. The following preparation methods are shown as examples; however, the synthesis methods are not intended as a limitation. R¹, R^{1a}, R^{1b}, R², R³, Z¹, Z², Z³, Z⁴, Z⁵, A¹, A², A³, A⁴, A⁵, B and Cy in the reaction formulas below are as defined above.

Examples of the "inert solvent" include aromatic solvents such as benzene, toluene, xylene and pyridine; hydrocarbon solvents such as hexane, pentane and cyclohexane; halogenated hydrocarbon solvents such as dichloromethane, chloroform, 1,2-dichloroethane and carbon tetrachloride; ether solvents such as tetrahydrofuran, diethyl ether, 1,2-dimethoxyethane and 1,4-dioxane; ester solvents such as ethyl acetate and ethyl formate; alcohol solvents such as methanol, ethanol, isopropyl alcohol, tert-butyl alcohol and ethylene glycol; ketone solvents such as acetone and methyl ethyl ketone; amide solvents such as N,N-dimethylformamide, N-methylpyrolidone and N,N-dimethylacetamide; sulfoxide solvents such as dimethyl sulfoxide; nitrile solvents such as acetonitrile and propionitrile; and water, as well as their homogeneous and heterogeneous mixed solvents. These inert solvents are appropriately selected according to various reaction conditions known to a person skilled in the art.

Examples of the "base" include alkali metal or alkali earth metal hydrides such as lithium hydride, sodium hydride, potassium hydride and calcium hydride; alkali metal or alkali earth metal amides such as lithium amide, sodium amide, lithium diisopropylamide, lithium dicyclohexylamide, lithium hexamethyldisilazide, sodium hexamethyldisilazide and potassium hexamethyldisilazide; alkali metal or alkali earth metal lower alkoxides such as sodium methoxide, sodium ethoxide and potassium tert-butoxide; alkyllithiums such as butyllithium, sec-butyllithium, tert-butyllithium and methyllithium; alkali metal or alkali earth metal hydroxides such as sodium hydroxide, potassium hydroxide, lithium hydroxide and barium hydroxide; alkali metal or alkali earth metal carbonates such as sodium carbonate, potassium carbonate and cesium carbonate; alkali metal or alkali earth metal bicarbonates such as sodium bicarbonate and potassium bicarbonate; amines such as triethylamine, N-methylmorpholine, N,N-diisopropylethylamine, 1,8-diazabicyclo[5.4.0]undec-7-ene (DBU), 1,5-diazabicyclo[4.3.0]non-5-ene (DBN) and N,N-dimethylaniline; and basic heterocyclic compounds such as pyridine, imidazole and 2,6-lutidine. These bases are appropriately selected according to various reaction conditions known to a person skilled in the art.

Examples of the acid include inorganic acids such as hydrochloric acid, hydrobromic acid, sulfuric acid, nitric acid and phosphoric acid; and organic acids such as p-toluenesulfonic acid, methanesulfonic acid, trifluoroacetic acid, formic acid and acetic acid. These acids are appropriately selected according to various reaction conditions known to a person skilled in the art.

### [Preparation Method 1]

The compound (I-I) of the present invention can be prepared by subjecting an amine compound (4) and a compound represented by the formula (5) to alkylation reaction.

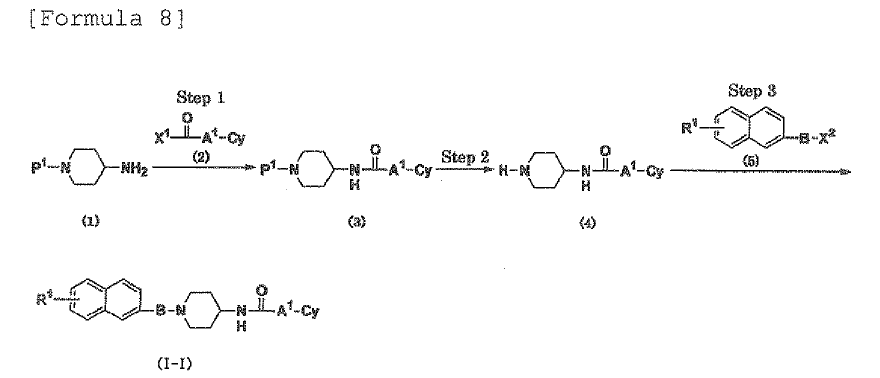

In the formula, P¹ represents an amino protecting group such as a methoxycarbonyl group, an ethoxycarbonyl group, a tert-butoxycarbonyl group, a benzyloxycarbonyl group, an acetyl group or a benzyl group [see Protective Groups in Organic Synthesis, 3rd ed., John Wiley & Sons, Inc.].

X¹ represents a halogen atom or a hydroxyl group.

X² represents a leaving group such as a halogen atom, a methanesulfonyloxy group, a trifluoromethanesulfonyloxy group or a p-toluenesulfonyloxy group.

Step 1: A compound (3) can be obtained by subjecting a commercially available or known amine compound (1) and a commercially available or known compound (2), wherein X¹ is a halogen atom, to amidation reaction in an inert solvent in the presence or absence of a base. Alternatively, a compound (3) can be obtained by subjecting a commercially available or known amine compound (1) and a commercially available or known compound (2), wherein X¹ is a hydroxyl group, to various amidation reactions known to a person skilled in the art. Examples of the amidation reaction here include amidation reaction using a condensing agent such as N,N'-dicyclohexylcarbodiimide, 1-ethyl-3-(3-dimethylaminopropyl)carbodiimide hydrochloride, diphenylphosphoryl azide or carbonyldiimidazole in an inert solvent in the presence or absence of a base; and amidation reaction through a mixed acid anhydride using ethyl chlorocarbonate, isobutyl chlorocarbonate, pivaloyl chloride or the like (see Pepuchido Gosei No Kiso To Jikken (Fundamentals and Experiments of Peptide Synthesis), 1985, Maruzen Co., Ltd.). Here, an additive such as 1-hydroxybenzotriazole may be used as necessary in the amidation reaction using a condensing agent.

Step 2: A compound (4) can be obtained by removing the amino protecting group P¹ of the compound (3) by various methods of organic synthesis known to a person skilled in the art [see Protective Groups in Organic Synthesis, 3rd ed., John Wiley & Sons, Inc.]. A compound (4) can also be directly obtained by performing the same amidation reaction as in Step 1 of Preparation Method 1, when P¹ is a hydrogen atom in the compound (1).

Step 3: The compound (I-I) of the present invention can be obtained by subjecting the amine compound (4) and a compound (5) to alkylation reaction in an inert solvent in the presence or absence of a base [see Comprehensive Organic Transformations, 1989, VCH Publishers, Inc.]. Here, the compound (5) used may be a commercially available compound, a known compound or a compound synthesized from a commercially available compound or a known compound using various methods of organic synthesis known to a person skilled in the art.

A compound (9) not commercially available or not known can be prepared as follows, for example.

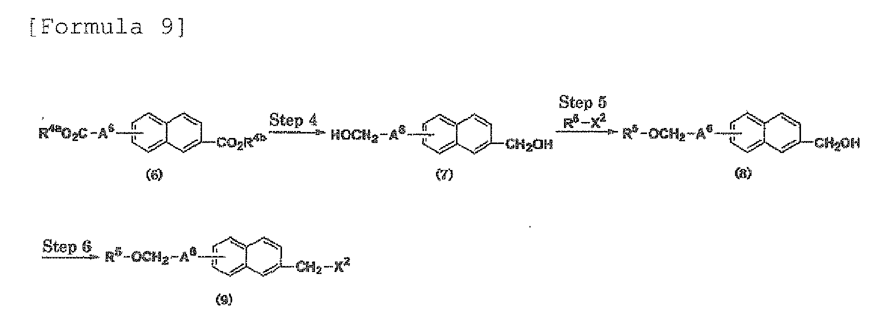

In the formula, X² is as defined above.

R^{4a} and R^{4b} each independently represent P² or a hydrogen atom. Here, P² represents a carboxyl protecting group such as a methyl group, an ethyl group, a tert-butyl group, a trimethylsilyl group or a benzyl group [see Protective Groups in Organic Synthesis, 3rd ed., John Wiley & Sons, Inc.].

R⁵ represents a C₁₋₆ alkyl group (wherein the C₁₋₆ alkyl group may be substituted with a C₃₋₆ cycloalkyl group), a C₂₋₆ alkenyl group or a Z²-A³-group.

A⁶ represents a C₁₋₅ alkylene group (wherein the C₁₋₅ alkylene group may be substituted with a C₁₋₆ alkyl group) or a bond.

Step 4: A compound (7) can be obtained by reacting a commercially available or known compound (6) with a reducing agent in an inert solvent (see Shin Jikken Kagaku Koza (New Courses in Experimental Chemistry), vol. 15, Sanka To Kangen (Oxidation and Reduction) [II], 1977, Maruzen Co., Ltd.). Here, the reducing agent is a reagent which can convert an acyl group or a carboxyl group to an alcohol by reduction. Examples of the reducing agent include lithium borohydride, sodium borohydride, calcium borohydride, zinc borohydride, lithium aluminum hydride, sodium aluminum hydride and diisobutylaluminum hydride.

Step 5: A compound (8) can be obtained by subjecting the compound (7) and a compound represented by the formula R⁵-X² to alkylation reaction in an inert solvent in the presence of a base [see Comprehensive Organic Transformations, 1989, VCH Publishers, Inc.]. An additive such as sodium iodide or tetrabutylammonium iodide may be used as necessary in the alkylation reaction.

Step 6: When X² represents a halogen atom, a compound (9) can be obtained by performing halogenation reaction of the hydroxyl group of the compound (8) with a halogenating agent such as carbon tetrachloride, carbon tetrabromide, N-chlorosuccinimide, N-bromosuccinimide, bromine or oxalyl chloride in an inert solvent in the presence of trimethylphosphine, tributylphosphine, triphenylphosphine or the like, or by performing halogenation reaction of the hydroxyl group with a halogenating agent such as thionyl chloride, phosphorus trichloride, phosphorus pentachloride, phosphorus tribromide, phosphorus pentabromide or phosphorus oxychloride in an inert solvent or without a solvent in the presence or absence of a base. When X² represents a leaving group such as a methanesulfonyloxy group or a p-toluenesulfonyloxy group, a compound (9) can be obtained by reacting the hydroxyl group of the compound (8) with methanesulfonyl chloride, methanesulfonic anhydride or p-toluenesulfonyl chloride, for example, in an inert solvent in the presence or absence of a base [see Comprehensive Organic Transformations, 1989, VCH Publishers, Inc.].

### [Preparation Method 2]

The compounds (I-II) and (V-I) of the present invention can be prepared by subjecting an amine compound (4) and a carbonyl compound (10) or (10-1) to reductive amination reaction.

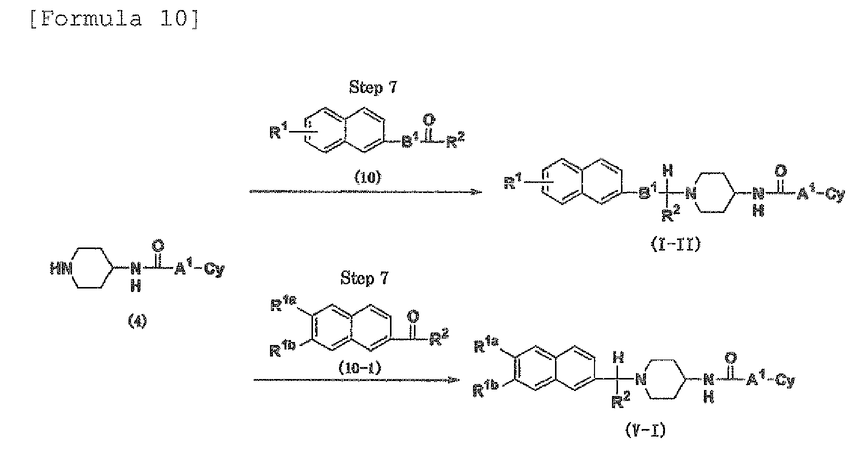

In the formula, B¹ represents a C₁₋₅ alkylene group (wherein the C₁₋₅ alkylene group may be substituted with a C₁₋₆ alkyl group).

Step 7: The compounds (I-II) and (V-I) of the present invention can be obtained by subjecting an amine compound (4) and a carbonyl compound (10) or (10-1) to reductive amination reaction using a reducing agent in an inert solvent in the presence or absence of an acid [see Comprehensive Organic Transformations, 1989, VCH Publishers, Inc.]. Here, the carbonyl compounds (10) and (10-1) used may each be a commercially available compound, a known compound or a compound synthesized from a commercially available compound or a known compound using various methods of organic synthesis known to a person skilled in the art. Examples of the reducing agent here include sodium triacetoxyborohydride, sodium cyanoborohydride and sodium borohydride.

Carbonyl compounds (11) and (13) not commercially available or not known can be prepared as follows, for example.

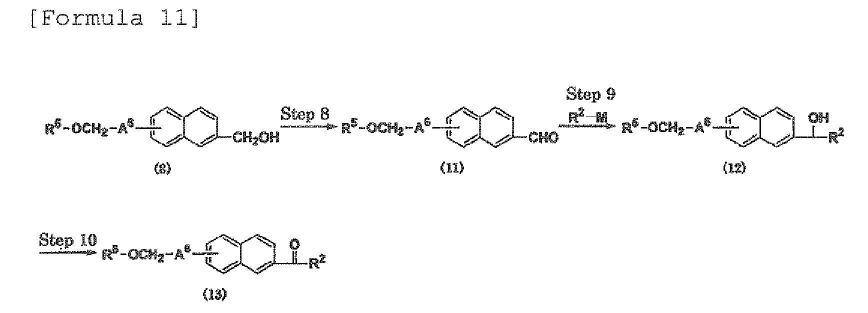

In the formula, R⁵ and A⁶ are as defined above.

M represents a metal used in alkylation reaction. Here, the metal refers to a metal such as lithium or magnesium halide.

Step 8: A compound (8) can be converted to a carbonyl compound (11) in an inert solvent by an oxidation reaction known to a person skilled in the art [see Oxidations in Organic Chemistry, 1990, American Chemical Society]. Examples of the oxidation reaction known to a person skilled in the art here include chromium oxidation reaction using pyridinium dichromate, pyridinium chlorochromate or the like; manganese oxidation reaction using manganese dioxide or the like; dimethyl sulfoxide oxidation reaction using oxalyl chloride (Swern oxidation), dicyclohexylcarbodiimide (Moffatt oxidation) or the like as an activator; 2,2,6,6-tetramethyl-1-piperidinyloxy oxidation reaction using a co-oxidizing agent such as sodium hypochlorite (TEMPO oxidation); and oxidation reaction using a Dess-Martin reagent.

Step 9: A compound (12) can be obtained by performing alkylation reaction of the carbonyl compound (11) with an organometallic reagent represented by the formula R²-M [see Comprehensive Organic Transformations, 1989, VCH Publishers, Inc.] in an inert solvent.

Step 10: The compound (12) can be converted to a carbonyl compound (13) by the same method as in Step 8 of Preparation Method 2.

### [Preparation Method 3]

The compounds (I-II) and (V-I) of the present invention can be prepared by subjecting an amine compound (4) and a carbonyl compound (14) or (14-1) to reductive amination reaction and then converting R⁶, R^{6a} and R^{6b} to R¹, R^{1a} and R^{1b}, respectively.

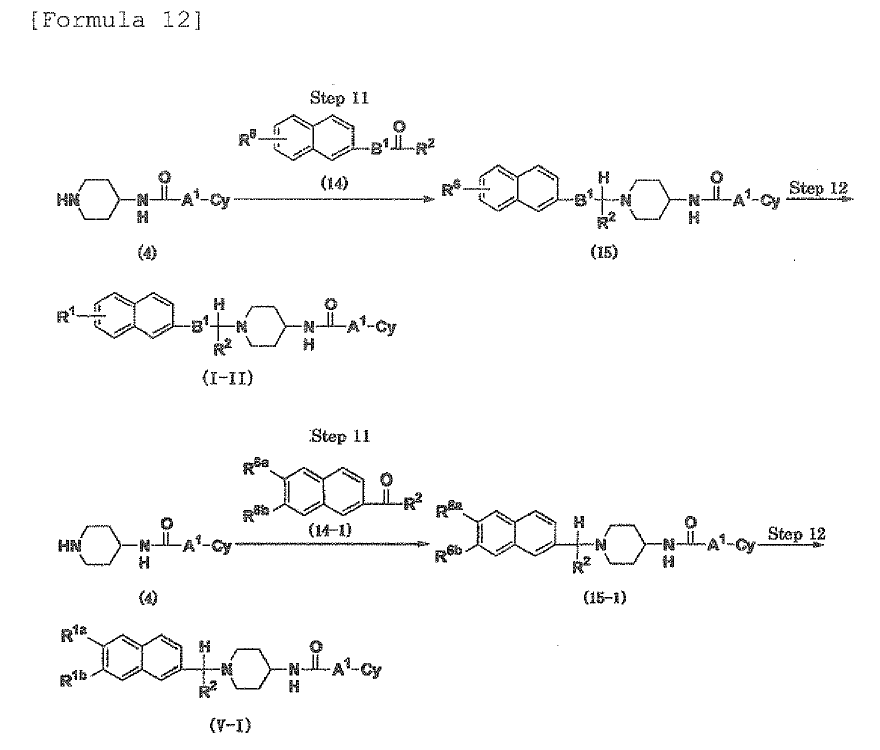

In the formula, B¹ is as defined above.

R⁶, R^{6a} and R^{6b} represent functional groups which can be converted to R¹, R^{1a} and R^{1b} by performing functional group conversion using various methods of organic synthesis known to a person skilled in the art.

Step 11: Compounds (15) and (15-1) can be obtained by subjecting an amine compound (4) and a carbonyl compound (14) or (14-1) to reductive amination reaction by the same method as in Step 7 of Preparation Method 2. Here, the carbonyl compounds (14) and (14-1) used may each be a commercially available compound, a known compound or a compound synthesized from a commercially available compound or a known compound using various methods of organic synthesis known to a person skilled in the art.

Step 12: The compounds (I-II) and (V-I) of the present invention can be obtained by converting R⁶, R^{6a} and R^{6b} of the compounds (15) and (15-1) to R¹, R^{1a} and R^{1b} using various methods of organic synthesis known to a person skilled in the art [see Comprehensive Organic Transformations, 1989, VCH Publishers, Inc.)]. Detailed synthesis examples of converting R⁶, R^{6a} and R^{6b} to R¹, R^{1a} and R^{1b} are shown in Formula 11 and Formula 12.

The compounds (V-II) and (V-III) of the present invention can be prepared from a synthetic precursor (20). The synthetic precursor (20) can be prepared by subjecting the amine compound (4) and a carbonyl compound (17) or (19) to reductive amination reaction.

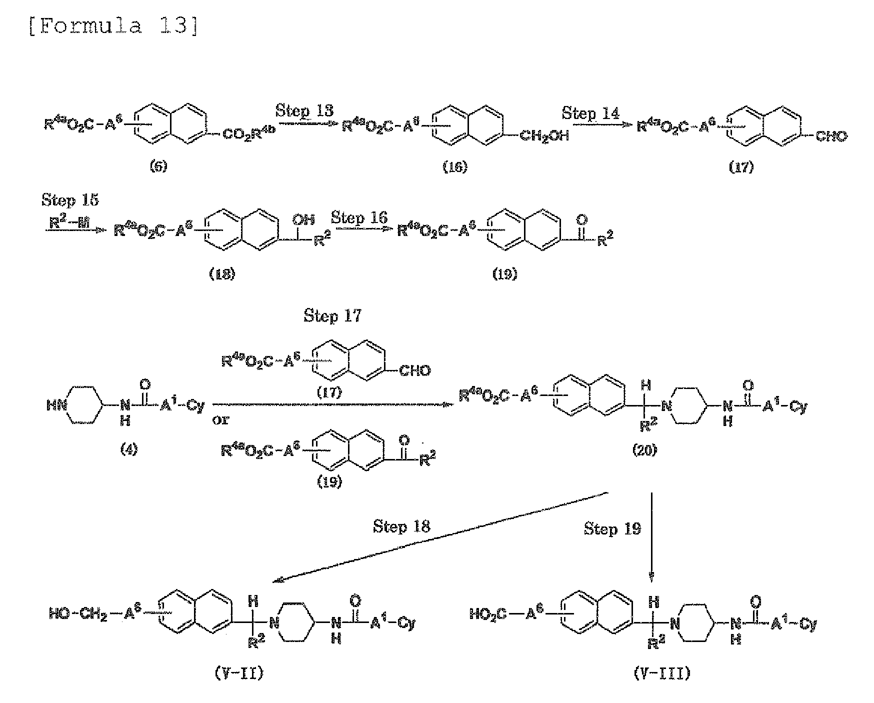

In the formula, M, R^{4a}, R^{4b} and A⁶ are as defined above.

Step 13: A compound (16) having one acyl group or carboxyl group selectively reduced can be obtained by reacting a commercially available or known compound (6) with a reducing agent in an inert solvent in the presence or absence of a lower alcohol, with the molar equivalent of the reducing agent, the reaction time, the reaction temperature and the like controlled (see Shin Jikken Kagaku Koza (New Courses in Experimental Chemistry), vol. 15, Sanka To Kangen (Oxidation and Reduction) [II], 1977, Maruzen Co., Ltd.). Examples of the lower alcohol here include methanol, ethanol, 1-propanol and 1-butanol. The reducing agent is a reagent which can convert an acyl group or a carboxyl group to an alcohol by reduction. Examples of the reducing agent include lithium borohydride, sodium borohydride, calcium borohydride, zinc borohydride, lithium aluminum hydride, sodium aluminum hydride and diisobutylaluminum hydride.

Step 14: The compound (16) can be converted to a compound (17) by the same method as in Step 8 of Preparation Method 2. Alternatively, the commercially available or known compound (6) can be directly converted to a compound (17) by reacting the compound with a reducing agent in an inert solvent, with the molar equivalent of the reducing agent, the reaction time, the reaction temperature and the like controlled (see Shin Jikken Kagaku Koza (New Courses in Experimental Chemistry), vol. 15, Sanka To Kangen (Oxidation and Reduction) [II], 1977, Maruzen Co., Ltd.). Here, the reducing agent is a reagent which can convert an acyl group or a carboxyl group to a formyl group by reduction. Examples of the reducing agent include diisobutylaluminum hydride and sodium aluminum hydride.

Step 15: The compound (17) can be converted to a compound (18) by the same method as in Step 9 of Preparation Method 2.

Step 16: The compound (18) can be converted to a compound (19) by the same method as in Step 8 of Preparation Method 2.

Step 17: The compound (4) and the carbonyl compound (17) or (19) can be converted to a synthetic precursor (20) by the same method as in Step 7 of Preparation Method 2.

Step 18: The compound (20) can be converted to the compound (V-II) of the present invention by the same method as in Step 4 of Preparation Method 1.

Step 19: The compound (V-II) of the present invention can be obtained by removing the carboxyl protecting group R^{4a} of the compound (20) by various methods of organic synthesis known to a person skilled in the art [see Protective Groups in Organic Synthesis, 3rd ed., John Wiley & Sons, Inc.].

The compound (V-IV) of the present invention can be prepared from a synthetic precursor (29). The synthetic precursor (29) can be prepared by subjecting the amine compound (4) and a carbonyl compound (26) or (28) to reductive amination reaction.

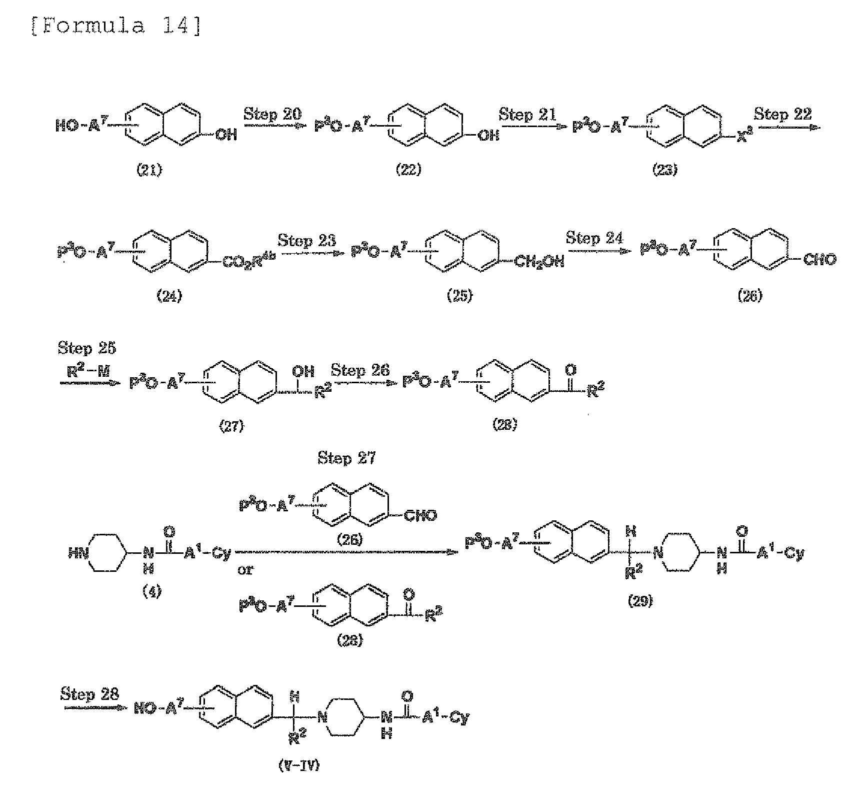

In the formula, M and R^{4b} are as defined above.

A⁷ represents a C₁₋₆ alkylene group (wherein the C₁₋₆ alkylene group may be substituted with a C₁₋₆ alkyl group) or a bond.

P³ represents a hydroxyl protecting group known to a person skilled in the art such as a tertbutyldimethylsilyl group, a tert-butyldiphenylsilyl group, a tetrahydropyranyl group, a methoxymethyl group, an acetyl group, a benzoyl group or a benzyl group [see Protective Groups in Organic Synthesis, 3rd ed., John Wiley & Sons, Inc.].

X³ represents a leaving group such as a halogen atom or a trifluoromethanesulfonyloxy group.

Step 20: A compound (22) can be obtained by protecting one hydroxyl group of a commercially available or known compound (21) with a protecting group P³ by various methods of organic synthesis known to a person skilled in the art [see Protective Groups in Organic Synthesis, 3rd ed., John Wiley & Sons, Inc.].

Step 21: When X³ represents a halogen atom, a compound (23) can be obtained by performing halogenation reaction of the hydroxyl group of the compound (22) with a halogenating agent such as bromine or oxalyl chloride in an inert solvent in the presence of trimethylphosphine, tributylphosphine, triphenylphosphine or the like, or by performing halogenation reaction of the hydroxyl group with a halogenating agent such as thionyl chloride, phosphorus trichloride, phosphorus pentachloride, phosphorus tribromide, phosphorus pentabromide or phosphorus oxychloride in an inert solvent or without a solvent in the presence or absence of a base. Alternatively, when X³ represents a trifluoromethanesulfonyloxy group, a compound (23) can be obtained by reacting the hydroxyl group of the compound (22) with a trifluoromethanesulfonylating agent in an inert solvent in the presence or absence of a base. Examples of the trifluoromethanesulfonylating agent here include trifluoromethanesulfonic anhydride and N-phenyl-bis(trifluoromethanesulfonimide) [see Comprehensive Organic Transformations, 1989, VCH Publishers, Inc.].

Step 22: A compound (24) can be obtained by reacting the compound (23) with carbon monoxide and R^{4b}OH in an inert solvent in the presence of a transition metal catalyst and a base [see Comprehensive Organic Transformations, 1989, VCH Publishers, Inc.]. Here, the transition metal catalyst is a zerovalent palladium reagent, for example, and can be prepared in the reaction from a divalent palladium such as palladium (II) acetate and a ligand such as triphenylphosphine, 1,3-bis(diphenylphosphino)propane (dppp) or 2,2'-bis(diphenylphosphino)-1,1'-binaphthyl (BINAP). Alternatively, a zerovalent palladium reagent such as tetrakistriphenylphosphine palladium (0) may be directly used.

Step 23: The compound (24) can be converted to a compound (25) by the same method as in Step 4 of Preparation Method 1.

Step 24: The compound (25) can be converted to a compound (26) by the same method as in Step 8 of Preparation Method 2. Alternatively, the compound (24) can be directly converted to a compound (26) by reacting the compound with a reducing agent in an inert solvent, with the molar equivalent of the reducing agent, the reaction time, the reaction temperature and the like controlled (see Shin Jikken Kagaku Koza (New Courses in Experimental Chemistry), vol. 15, Sanka To Kangen (Oxidation and Reduction) [II], 1977, Maruzen Co., Ltd.). Here, the reducing agent is a reagent which can convert an acyl group or a carboxyl group to a formyl group by reduction. Examples of the reducing agent include diisobutylaluminum hydride and sodium aluminum hydride.

Step 25: The compound (26) can be converted to a compound (27) by the same method as in Step 9 of Preparation Method 2.

Step 26: The compound (27) can be converted to a compound (28) by the same method as in Step 8 of Preparation Method 2.

Step 27: The compound (4) and the carbonyl compound (26) or (28) can be converted to a synthetic precursor (29) by the same method as in Step 7 of Preparation Method 2.

Step 28: The compound (V-IV) of the present invention can be obtained by removing the hydroxyl protecting group P³ by various methods of organic synthesis known to a person skilled in the art [see Protective Groups in Organic Synthesis, 3rd ed., John Wiley & Sons, Inc.].

### [Preparation Method 4]

The compound (I-I) of the present invention can be further converted to the compound (I-III) of the present invention by performing functional group conversion or the like by various methods of organic synthesis known to a person skilled in the art as follows.

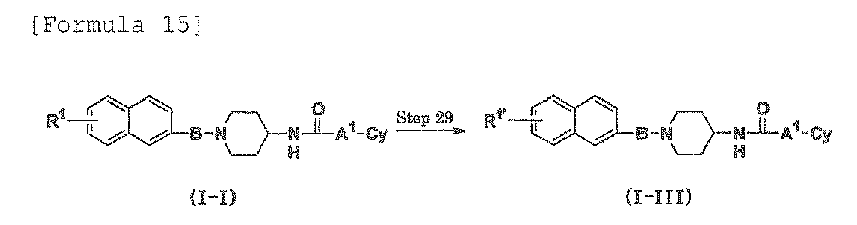

In the formula, R^{1'} is as defined for R¹, provided that R^{1'} represents a functional group differing from R¹.

Step 29: The compound (I-I) of the present invention can be further converted to the compound (I-III) of the present invention by converting R¹ to R^{1'} by various methods of organic synthesis known to a person skilled in the art [see Comprehensive Organic Transformations, 1989, VCH Publishers, Inc.]. Detailed synthetic examples of converting R¹ to R^{1'} are shown in Formula 14 to Formula 19.

The compound (V-II) of the present invention can be further converted to the compound (V-V) of the present invention by subjecting the compound to alkylation reaction with a compound represented by the formula R⁵-X². The compound (V-IV) of the present invention can be further converted to the compound (V-VI) of the present invention by subjecting the compound to alkylation reaction with a compound represented by the formula R⁶-X².

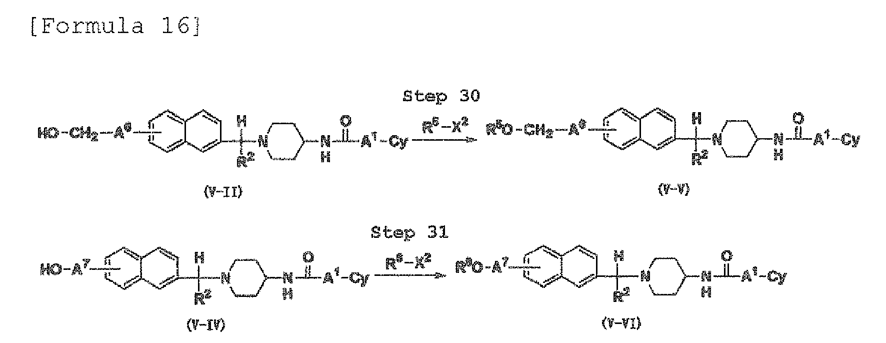

In the formula, R⁵, A⁶, A⁷ and X² are as defined above.

R⁶ is as defined for R⁵ when A⁷ is a C₁₋₆ alkylene group (wherein the C₁₋₆ alkylene group may be substituted with a C₁₋₆ alkyl group), and is a Z⁴-A⁵-group when A⁷ is a bond.

Step 30: The compound (V-II) of the present invention can be converted to the compound (V-V) of the present invention by the same method as in Step 5 of Preparation Method 1.

Step 31: The compound (V-IV) of the present invention can be converted to the compound (V-VI) of the present invention by the same method as in Step 5 of Preparation Method 1 (provided that a compound represented by the formula R⁶-X² is used instead of a compound represented by the formula R⁵-X²).

The compound (V-II) of the present invention can be further converted to the compounds (V-VII), (V-VIII) and (V-IX) of the present invention by performing functional group conversion shown below.

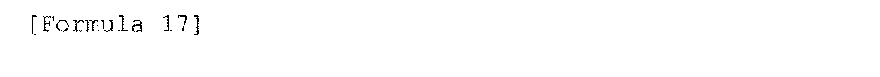

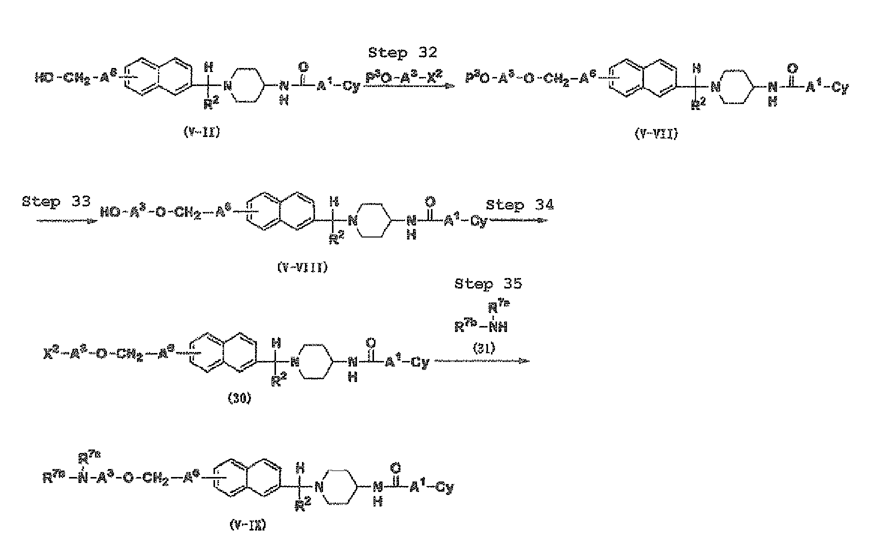

In the formula, A⁶, P³ and X² are as defined above.

R^{7a} and R^{7b} each independently represent a C₁₋₆ alkyl group or a hydrogen atom. Here, R^{7a} and R^{7b} may be bonded together to form a heterocyclic group.

Step 32: The compound (V-II) of the present invention can be converted to the compound (V-VII) of the present invention by the same method as in Step 5 of Preparation Method 1 (provided that a compound represented by the formula P³O-A³-X² is used instead of a compound represented by the formula R⁵-X²).

Step 33: The compound (V-VII) of the present invention can be converted to the compound (V-VIII) of the present invention by the same method as in Step 28 of Preparation Method 3.

Step 34: The compound (V-VIII) of the present invention can be converted to a compound (30) by the same method as in Step 6 of Preparation Method 1.

Step 35: The compound (V-IX) of the present invention can be obtained by reacting the compound (30) with a compound (31) by the same method as in Step 3 of Preparation Method 1.

The compound (V-II) of the present invention can be further converted to the compound (V-X) of the present invention by an oxidation reaction known to a person skilled in the art [see Oxidations in Organic Chemistry, 1990, American Chemical Society]. The compound (V-X) of the present invention can be further converted to the compound (V-XI) of the present invention by subjecting the compound to reductive amination reaction with a compound (32).

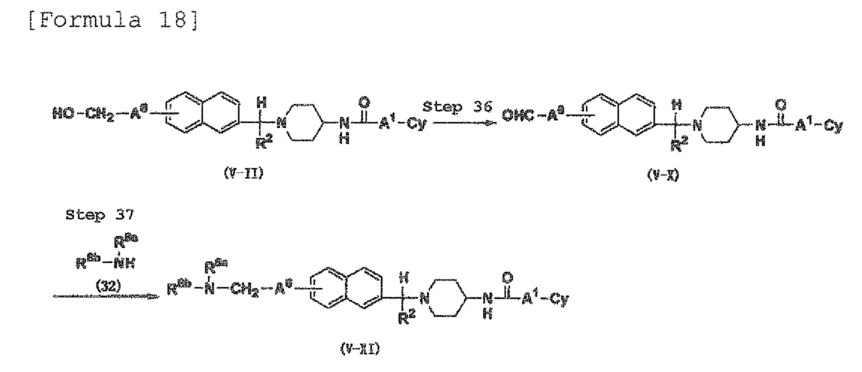

In the formula, A⁶ is as defined above.

R^{8a} and R^{8b} each independently represent a hydrogen atom, a C₁₋₆ alkyl group (wherein the C₁₋₆ alkyl group may be substituted with a C₃₋₆ cycloalkyl group) or a Z²-A³- group, provided that when any one of R^{8a} and R^{8b} is a Z²-A³- group, the remaining substituent is a hydrogen atom or a C₁₋₆ alkyl group. R^{8a} and R^{8b} may be bonded together to form a heterocyclic group {wherein the heterocyclic group is substituted with a hydroxyl group, C₁₋₆ alkyl group (wherein the C₁₋₆ alkyl group may be substituted with a hydroxyl group or an amino group) or an amino group}.

Step 36: The compound (V-II) of the present invention can be converted to the compound (V-X) of the present invention by the same method as in Step 8 of Preparation Method 2. Alternatively, a compound (20) can be directly converted to the compound (V-X) of the present invention by reacting the compound with a reducing agent in an inert solvent, with the molar equivalent of the reducing agent, the reaction time, the reaction temperature and the like controlled (see Shin Jikken Kagaku Koza (New Courses in Experimental Chemistry), vol. 15, Sanka To Kangen (Oxidation and Reduction) [II], 1977, Maruzen Co., Ltd.). Here, the reducing agent is a reagent which can convert an acyl group or a carboxyl group to a formyl group by reduction. Examples of the reducing agent include diisobutylaluminum hydride and sodium aluminum hydride.

Step 37: The compound (V-X) of the present invention can be converted to the compound (V-XI) of the present invention by the same method as in Step 7 of Preparation Method 2. When R^{8a} and R^{8b} each have an amino group, a carboxyl group or a hydroxyl group as a substituent, a protecting group P¹, P² or P³ known to a person skilled in the art may be introduced into such a group (wherein P¹, P² and P³ are as defined above). In this case, the compound (V-X) of the present invention can be converted to the compound (V-XI) of the present invention by removing the protecting group by various methods of organic synthesis known to a person skilled in the art [see Protective Groups in Organic Synthesis, 3rd ed., John Wiley & Sons, Inc.] as necessary after the reductive amination reaction.

The compound (V-XII) of the present invention can be converted to the compound (V-XIII) of the present invention with the substituent on the naphthalene ring group having an additional carbon atom by reaction with a Witting reagent or a Horner-Emmons reagent.

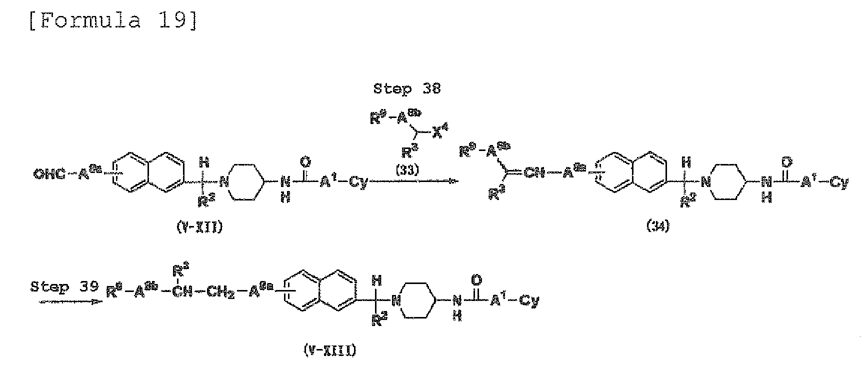

In the formula, R⁹ represents a formyl protecting group such as a C₁₋₆ alkoxy group, a R^{4a}O₂C-group or 1,3-dioxolane.

A^{8a} and A^{8b} each independently represent a C₁₋₄ alkylene group (wherein the C₁₋₄ alkylene group may be substituted with a C₁₋₆ alkyl group) or a bond, provided that A^{8a} to A^{8b} have 6 or less carbon atoms in total.

X⁴ represents a group (such as a phosphonium salt or a phosphorous acid diester) used in a Wittig reagent or a Horner-Emmons reagent.

Step 38: An olefin compound (34) can be obtained by reacting the compound (V-XII) of the present invention with a Wittig reagent or a Horner-Emmons reagent (33) in an inert solvent in the presence of a base [see Comprehensive Organic Transformations, 1989, VCH Publishers, Inc.].

Step 39: The compound (V-XIII) of the present invention can be obtained by hydrogenating the olefin compound (34) in an inert solvent in the presence of a transition metal catalyst [see Comprehensive Organic Transformations, 1989, VCH Publishers, Inc.]. Examples of the transition metal catalyst here include palladium-activated carbon, palladium black, palladium hydroxide, platinum (IV) oxide and Raney nickel.

A compound (35) which is the compound (34), wherein A^{8b} represents a bond and R⁹ represents a C₁₋₆ alkoxy group, can be converted to the compounds (V-XIV) and (V-XV) of the present invention as follows.

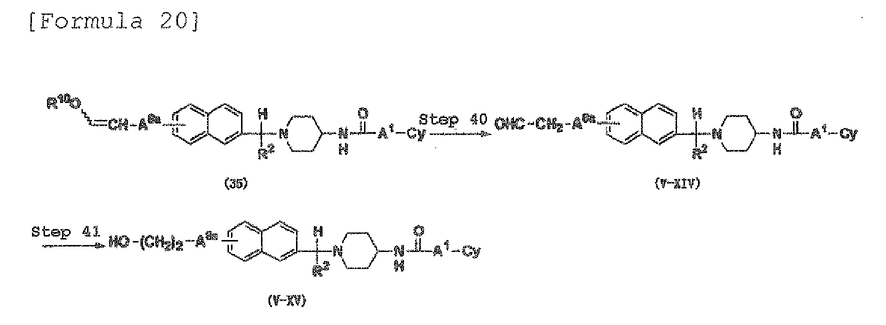

In the formula, A^{8a} is as defined above.

R¹⁰ represents a C₁₋₆ alkyl group.

Step 40: A compound (35) can be converted to the compound (V-XIV) of the present invention by various hydrolysis reactions known to a person skilled in the art [see Protective Groups in Organic Synthesis, 3rd ed., John Wiley & Sons, Inc.].

Step 41: The compound (V-XIV) of the present invention can be converted to the compound (V-XV) of the present invention by the same method as in Step 4 of Preparation Method 1.

The compound (V-III) of the present invention can be converted to the compound (V-XVI) of the present invention by subjecting the compound to amidation reaction with a compound (36).

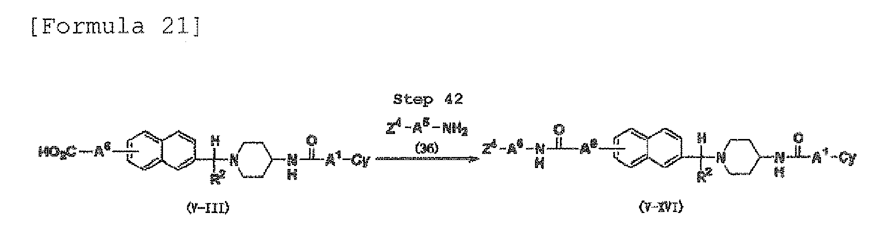

In the formula, A⁶ is a bond.

Step 42: The compound (V-III) of the present invention and the compound (36) can be converted to the compound (V-XVI) of the present invention by the same method as in Step 1 of Preparation Method 1.

### [Preparation Method 5]

The compounds (I-I) and (I-II) of the present invention can be prepared by subjecting an amine compound (39) or (39-1) and a compound (2) to amidation reaction.

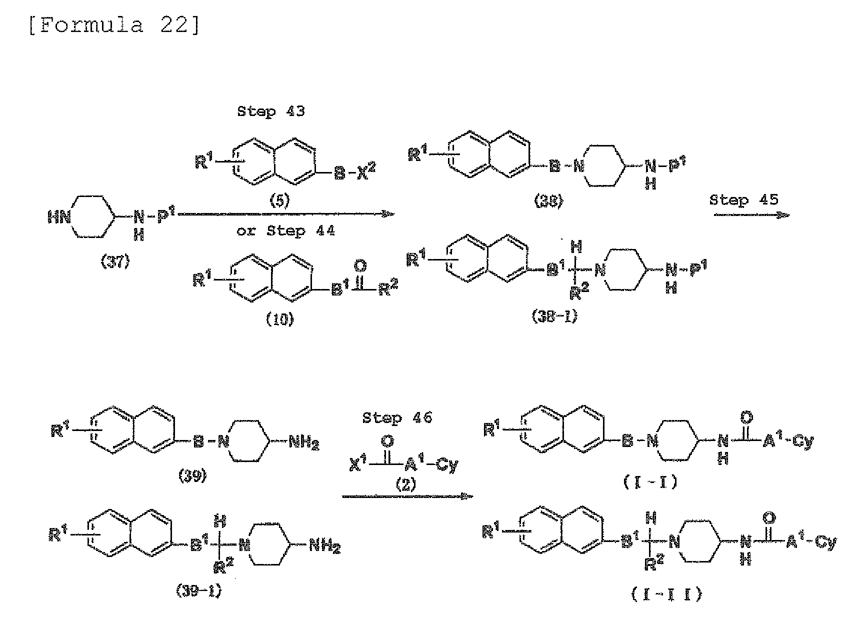

In the formula, B¹, P¹, X¹ and X² are as defined above.

Step 43: A compound (38) can be obtained by reacting an amine compound (37) with a compound (5) by the same method as in Step 3 of Preparation Method 1.

Step 44: A compound (38-1) can be obtained by reacting an amine compound (37) with a compound (10) by the same method as in Step 7 of Preparation Method 2.

Step 45: The compound (38) or (38-1) can be converted to an amine compound (39) or (39-1) by the same method as in Step 2 of Preparation Method 1. Compounds (39) and (39-1) can also be directly obtained by performing the same reaction as in Step 43 or Step 44, when P¹ is a hydrogen atom in the compound (37).

Step 46: The amine compounds (39) and (39-1) can be converted to the compounds (I-I) and (I-II) of the present invention by the same method as in Step 1 of Preparation Method 1.

Further, other compounds of the present invention can be synthesized by performing functional group conversion of the substituent in Cy of the compound (I-I) of the present invention by various methods of organic synthesis known to a person skilled in the art.

The carbonyl compound (11) not commercially available or not known, which is used for preparing the compounds (I-II) and (V-I) of the present invention in Preparation Method 2, can also be prepared as follows.

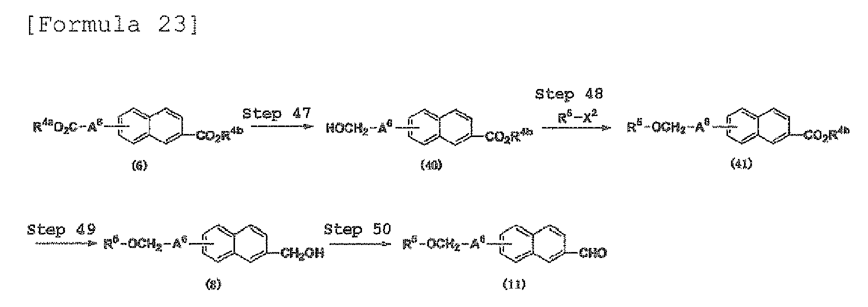

In the formula, A⁶, R^{4a}, R^{4b}, R⁵ and X² are as defined above.

Step 47: A compound (6) can be converted to a compound (40) by the same method as in Step 13 of Preparation Method 3.

Step 48: The compound (40) can be converted to a compound (41) by the same method as in Step 5 of Preparation Method 1.

Step 49: The compound (41) can be converted to a compound (8) by the same method as in Step 4 of Preparation Method 1.

Step 50: The compound (8) can be converted to a carbonyl compound (11) by the same method as in Step 8 of Preparation Method 2.

A carbonyl compound (45) not commercially available or not known can be prepared as follows, for example.

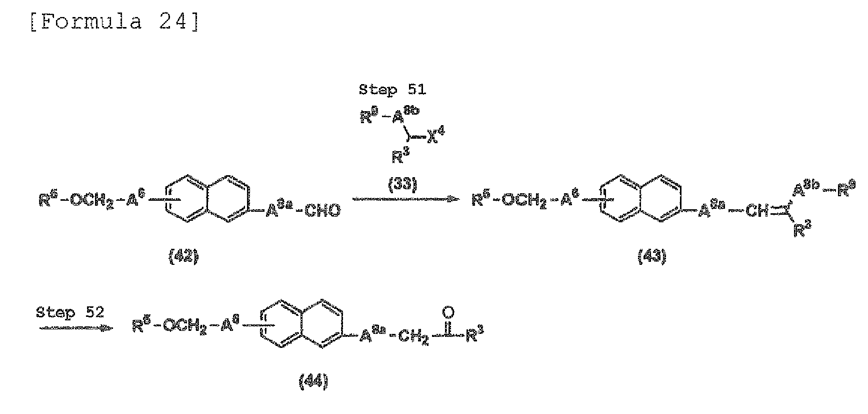

In the formula, A⁶, A^{8a}, A^{8b}, R⁵, R⁹ and X⁴ are as defined above.

Step 51: A compound (43) can be obtained by reacting a compound (42) with a Witting reagent or Horner-Emmons reagent (33) by the same method as in Step 38 of Preparation Method 4.

Step 52: The compound (43), wherein A^{8b} represents a bond and R⁹ represents a C₁₋₆ alkoxy group, can be converted to a carbonyl compound (44) by the same method as in Step 40 of Preparation Method 4.

### [Preparation Method 6]

The compounds (I-IV) and (V-XVII) of the present invention can be prepared by oxidizing the nitrogen atom in piperidine of the compounds (I-I) and (V-I) of the present invention.

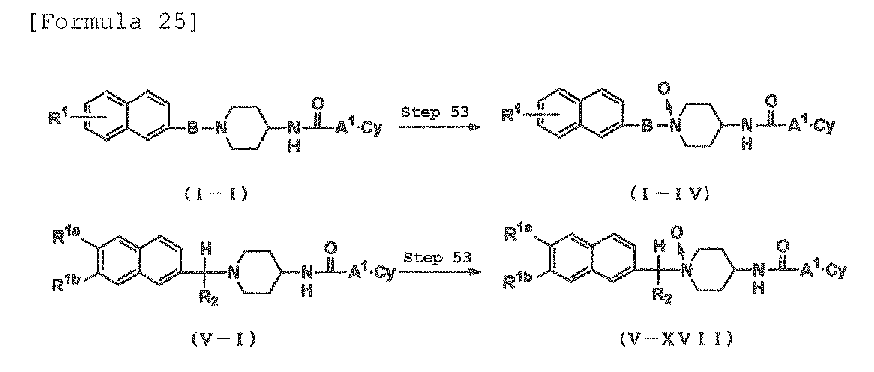

Step 53: The compounds (I-IV) and (V-XVII) of the present invention which are piperidine N-oxides can be obtained by reacting the compounds (I-I) and (V-I) of the present invention with a tertiary amine oxidizing agent known to a person skilled in the art [see Oxidations in Organic Chemistry, 1990, American Chemical Society] in an inert solvent. Examples of the oxidizing agent known to a person skilled in the art include 3-chloroperbenzoic acid, hydrogen peroxide, peracetic acid, and t-butyl hydroperoxide in the presence of a vanadium or molybdenum catalyst.

When the compound (I) of the present invention forms a salt and it is used as a pharmaceutical agent, the salt is preferably a pharmaceutically acceptable salt. Examples of the pharmaceutically acceptable salt used include, but are not limited to, salts with inorganic acids such as hydrochlorides, sulfates, hydrobromides, nitrates and phosphates; and salts with organic acids such as acetates, oxalates, lactates, citrates, malates, tartrates, maleates, fumarates, succinates, methanesulfonates, ethanesulfonates, benzenesulfonates and p-toluenesulfonates.

Examples of the pharmaceutically acceptable salt also include salts with inorganic bases and salts with organic bases. Suitable examples of the salts with inorganic bases include alkali metal salts (such as sodium salts and potassium salts), alkali earth metal salts (such as calcium salts, magnesium salts and barium salts), aluminum salts and ammonium salts. Suitable examples of the salts with organic bases include trimethylamine, triethylamine, pyridine, picoline, ethanolamine, diethanolamine, triethanolamine, dicyclohexylamine and N,N-dibenzylethylenediamine.

When the compound (I) of the present invention includes an optical isomer, a stereoisomer, a regioisomer and a rotamer, single compounds and mixtures thereof are included in the compounds of the present invention. When the compound (I) of the present invention forms a hydrate or a solvate, they are also within the scope of the present invention. Further, the compound (I) of the present invention may be labeled with an isotope (such as D, ³H, ¹³C, ¹⁴C, ¹⁵N, ³⁵S or ¹²⁵I).

The MCH receptor antagonist and the pharmaceutical composition of the present invention are respectively prepared by formulating the compound (I) or the pharmaceutically acceptable salt of the present invention as is or together with a pharmacologically acceptable carrier according to means known per se. Examples of the pharmacologically acceptable carrier include various organic or inorganic carrier substances conventionally used as formulation materials, for example, excipients (such as lactose, saccharose, D-mannitol, starch, corn starch, microcrystalline cellulose and light anhydrous silicic acid), lubricants (such as magnesium stearate, calcium stearate, talc and colloidal silica), binders (such as microcrystalline cellulose, saccharose, D-mannitol, dextrin, hydroxypropylcellulose, hydroxypropylmethylcellulose, polyvinylpyrrolidone, starch, sucrose, gelatin, methylcellulose and sodium carboxymethylcellulose) and disintegrants (such as starch, carboxymethylcellulose, calcium carboxymethylcellulose, croscarmellose sodium, sodium carboxymethylstarch and low-substituted hydroxypropylcellulose) in solid formulations, and solvents (such as water for injection, alcohols, propylene glycol, macrogol, sesame oil and corn oil), solubilizing agents (such as polyethylene glycol, propylene glycol, D-mannitol, benzyl benzoate, ethanol, trisaminomethane, cholesterol, triethanolamine, sodium carbonate and sodium citrate), suspending agents (e.g. surfactants such as stearyltriethanolamine, sodium lauryl sulfate, laurylaminopropionic acid, lecithin, benzalkonium chloride, benzethonium chloride and glyceryl monostearate, or hydrophilic polymers such as polyvinyl alcohol, polyvinylpyrrolidone, sodium carboxymethylcellulose, methylcellulose, hydroxymethylcellulose and hydroxypropylcellulose), tonicity adjusting agents (such as glucose, D-sorbitol, sodium chloride, glycerol and D-mannitol), buffers (such as phosphates, acetates, carbonates and citrates) and soothing agents (such as benzyl alcohol) in liquid formulations. Preservatives (such as p-hydroxybenzoates, chlorobutanol, benzyl alcohol, phenethyl alcohol, dehydroacetic acid and sorbic acid), antioxidants (such as sulfites and ascorbic acid), colorants, sweeteners, adsorbents, wetting agents and the like may also be used in formulation as necessary.

The MCH receptor antagonist and the pharmaceutical composition of the present invention can be administered orally or parenterally (for example, intravenously, topically or rectally). Examples of the dosage form include tablets (including sugar-coated tablets and film-coated tablets), powders, granules, powder materials, troches, capsules (including soft capsules), liquids, injections (such as subcutaneous injections, intravenous injections, intramuscular injections and intraperitoneal injections), external formulations (such as nasal formulations, dermal formulations, ointments and creams), suppositories (such as rectal suppositories and vaginal suppositories), controlled release formulations (such as controlled release microcapsules), pellets and drops, all of which can be prepared by a conventional formulation technique (such as a method described in The Japanese Pharmacopoeia Fifteenth Edition).

The dose of the MCH receptor antagonist and the pharmaceutical composition of the present invention is appropriately selected according to the administration subject, the administration route, the disease, and the age, the body weight and the symptom of the patient. For example, in treatment of an adult patient, the dose is 1 to 2000 mg per day and is administered in one dose or several doses per day.

It should be noted that an MCH receptor antagonist as an active ingredient of a pharmaceutical composition is not intended to be used only for humans, and is also used for mammals other than humans. For example, the recent progress in the field of animal health care would make it possible to use an MCH receptor antagonist for treating diabetes in livestocks (such as cats and dogs) and furthermore to use an MCH receptor antagonist for other livestocks (e.g. edible animals such as cattle, chicken and fish) in which a disease or disorder is not apparent.

### EXAMPLES

The present invention will be described in more detail by the following examples; however, these examples do not limit the present invention and may be changed without departing from the scope of the present invention.

The "room temperature" in the examples refers to a temperature of 0°C to 40°C. The "Silica gel 60 N" and "Chromatorex NH" commercially available from Kanto Chemical Co., Inc. and Fuji Silysia Chemical Ltd., respectively, were used in purification by column chromatography. Silica gel 60 F₂₅₄ commercially available from Merck Ltd. was used for TLC in purification by PTLC.

The instrumental data described in the examples were measured using the following measuring instruments.
MS spectra: Shimadzu LCMS-2010EV, micromass Platform LC or micromass GCT
NMR spectra: 600 MHz (JNM-ECA600, JEOL Ltd.), 300 MHz (INOVA 300, Varian Inc.) or 200 MHz (GEMINI 2000/200, Varian Inc.)

The compounds in the examples were named by ACD/Name (ACD/Labs 8.00, Advanced Chemistry Development Inc.).

The abbreviations used in the examples are shown below.
AcOH (acetic acid), APCI (atmospheric pressure Chemical ionization), brs (broad singlet), BuLi (butyllithium), CBr₄ (carbon tetrabromide), CDCl₃ (deuterated chloroform), CHCl₃ (chloroform), CI (chemical ionization), d (doublet), d d (double doublet), DMF (N,N-dimethylformamide), DMSO (dimethyl sulfoxide), DMSO-d₆ (deuterated dimethyl sulfoxide), dppp [1,3-bis(diphenylphosphino)propane], d t (double triplet), EDC [1-ethyl-3-(3-dimethylaminopropyl)carbodiimide], EI (electronic ionization), ESI (electrospray ionization), EtI (ethyl iodide), EtMgBr (ethylmagnesium bromide), Et₃N (triethylamine), Et₂O (diethyl ether), EtOAc (ethyl acetate), EtOH (ethanol), FAB (fast atom bombardment), free (free form), H (proton), HBr (hydrobromic acid), HCl (hydrogen chloride or hydrochloric acid), H₂O (water), H₂O₂ (hydrogen peroxide), HOBt (1-hydroxybenzotriazole), Hz (hertz), IPA (isopropyl alcohol), J (coupling constant), K₂CO₃ (potassium carbonate), KH (potassium hydride), KOtBu (potassium tert-butoxide), LiAlH₄ (lithium aluminum hydride), LiOH (lithium hydroxide), m (multiplet), MeI (methyl iodide), MeMgBr (methylmagnesium bromide), MeNH₂ (methylamine), Me₂NH (dimethylamine), MeOH (methanol), MeOH-d₃ (deuterated methanol), MgSO₄ (magnesium sulfate), MnO₂ (manganese dioxide), MS (mass spectrometry), MsCl (methanesulfonyl chloride), NaBH₄ (sodium borohydride), NaH (sodium hydride), NaHCO₃ (sodium bicarbonate), Na₂SO₄ (sodium sulfate), NH₄Cl (ammonium chloride), NMR (nuclear magnetic resonance spectroscopy), NaBH₃CN (sodium cyanoborohydride), NaBH(OAc)₃ (sodium triacetoxyborohydride), NaOH (sodium hydroxide), Pd/C (palladium-activated carbon), Pd(OAc)₂ [palladium (II) acetate], PPh₃ (triphenylphosphine), PTLC (preparative thin-layer chromatography), Py (pyridine), q (quartet), s (singlet), t (triplet), TBAI (tetrabutylammonium iodide), TBSCl (tertbutyldimethylsilyl chloride), TFA (trifluoroacetic acid), Tf₂O (trifluoromethanesulfonic anhydride), THF (tetrahydrofuran), v/v (volume/volume)

### Example 1: Synthesis of 3-methoxy-N-[1-({7-[(2-methoxyethoxy)methyl]-2-naphthyl}methyl)piperidin-4-yl]benzamide hydrochloride

Step 1-1: A solution of dimethyl naphthalene-2,7-dicarboxylate (250 g) in THF (2.50 L) was added to a suspension of LiAlH₄ (81.6 g) in THF (1.25 L) under nitrogen atmosphere with ice-cooling, and the mixture was warmed up to room temperature. After stirring for one hour, the reaction solution was ice-cooled again. Na₂SO₄·10H₂O was added and the mixture was stirred at room temperature for 15 minutes. The reaction solution was filtered through celite and washed with heated THF (12.0 L). Then, the filtrate was concentrated under reduced pressure. CHCl₃ (1.00 L) was added to the resulting crude product, followed by stirring for 10 minutes. Then, the solid was collected by filtration to obtain naphthalene-2,7-diyldimethanol (151 g, colorless solid).
¹H NMR (200 MHz, DMSO-d₆, δ): 4.66 (d, J = 5.7 Hz, 4H), 5.31 (t, J = 5.7 Hz, 2H), 7.42 (dd, J = 8.6, 1.5 Hz, 2H), 7.73-7.88 (m, 4H); EI MS m/z 188, M⁺.

Step 1-2: A solution of the compound obtained in Step 1-1 (181 g) in DMSO (800 mL) was added dropwise to a suspension of NaH (60% in oil, 38.5 g) in DMSO (800 mL) under nitrogen atmosphere. After stirring at room temperature for 30 minutes, 1-bromo-2-methoxyethane (160 g) was added dropwise and the mixture was stirred at the same temperature for 22 hours. H₂O (1.50 L) was added to the reaction solution, followed by extraction with EtOAc (1.50 L, 500 mL, 500 mL) three times. The organic layers were combined, washed with H₂O (500 mL), dried over MgSO₄ and then concentrated under reduced pressure. CHCl₃ (1.00 L) was added to the residue, followed by stirring for one hour. Then, the solid was collected by filtration to obtain naphthalene-2,7-diyldimethanol (58.8 g, colorless solid) as a recovered raw material. The filtrate was concentrated under reduced pressure. Then, the residue was purified by column chromatography (Silica gel 60 N, mobile phase: MeOH/CHCl₃ = 1/99 to 5/95; v/v). The resulting crude product was dissolved in EtOAc (1.00 L), followed by washing with H₂O (1.00 L) four times. The organic layer was dried over MgSO₄. Then, the filtrate was concentrated under reduced pressure to obtain {7-[(2-methoxyethoxy)methyl]-2-naphthyl}methanol (83.6 g, yellow oil).
¹H NMR (200 MHz, CDCl₃, δ): 3.41 (s, 3H), 3.56-3.70 (m, 4H), 4.74 (s, 2H), 4.85 (d, J = 6.2 Hz, 2H), 7.47 (dt, J = 8.4, 1.8 Hz, 2H), 7.68-7.91 (m, 4H); ESI MS m/z 269, (M+Na)⁺.

Step 1-3: MnO₂ (295 g) was added to a solution of the compound obtained in Step 1-2 (83.5 g) in CHCl₃ (1.25 L), and the mixture was stirred at room temperature for four hours. The reaction solution was filtered through celite. Then, the filtrate was concentrated under reduced pressure to obtain 7-[(2-methoxyethoxy)methyl]-2-naphthaldehyde (82.3 g, pale yellow oil).
¹H NMR (200 MHz, CDCl₃, δ): 3.42 (s, 3H), 3.58-3.66 (m, 2H), 3.67-3.74 (m, 2H), 4.77 (s, 2H), 7.65 (dd, J = 8.4, 1.8 Hz, 1H), 7.82-8.01 (m, 4H), 8.33 (d, J = 0.9 Hz, 1H), 10.16 (s, 1H); ESI MS m/z 267, (M+Na)⁺.

Step 1-4: Et₃N (130 mL), HOBt·H₂O (71.7 g) and EDC·HCl (82.8 g) were added to a suspension of tert-butyl 4-aminopiperidine-1-carboxylate (78.0 g) and 3-methoxybenzoic acid (65.2 g) in DMF (780 mL), and the mixture was stirred at room temperature for 12 hours. H₂O (1.56 L) was added and the mixture was stirred in a water bath for 1.5 hours. Then, the solid was collected by filtration to obtain tert-butyl 4-[(3-methoxybenzoyl)amino]piperidine-1-carboxylate (126 g, colorless solid). 4 M HCl/EtOAc solution (900 mL) was added to a suspension of the compound obtained by the above method in EtOAc (900 mL), and the mixture was stirred at room temperature for four hours. The reaction solution was concentrated under reduced pressure. Then, CHCl₃ (2.00 L) and 2 M aqueous NaOH solution (1.00 L) were added to the residue, followed by stirring for 15 hours. The aqueous layer was separated and extracted with CHCl₃ (800 mL) twice. The combined organic layers were dried over Na₂SO₄ and then concentrated under reduced pressure to obtain 3-methoxy-N-piperidin-4-ylbenzamide (87.8 g, pale yellow solid).
¹H NMR (200 MHz, CDCl₃, δ): 1.30-1.52 (m, 2H), 1.97-2.12 (m, 2H), 2.75 (dt, J = 12.0, 2.4 Hz, 2H), 3.11 (dt, J = 12.8, 3.5 Hz, 2H), 3.85 (s, 3H), 3.96-4.18 (m, 1H), 6.00 (d, J = 7.9 Hz, 1H), 6.98-7.07 (m, 1H), 7.21-7.38 (m, 3H); ESI MS m/z 235, (M+H)⁺.

Step 1-5: AcOH (13.6 mL) and NaBH(OAc)₃ (68.7 g) were added to a solution of the compound obtained in Step 1-3 (52.8 g) and the compound obtained in Step 1-4 (50.6 g) in CHCl₃ (530 mL), and the mixture was stirred at room temperature for 12 hours. After adding saturated aqueous NaHCO₃ solution, the aqueous layer was separated. The organic layer was dried over Na₂SO₄ and then concentrated under reduced pressure. EtOAc (500 mL) was added to the residue, followed by stirring for one hour. Then, the solid was collected by filtration to obtain 3-methoxy-N-[1-({7-[(2-methoxyethoxy)methyl]-2-naphthyl}methyl)piperidin-4-yl]benzamide (76.1 g, colorless solid).
¹H NMR (600 MHz, CDCl₃, δ): 1.52-1.61 (m, 2H), 1.98-2.05 (m, 2H), 2.23 (t, J = 10.6 Hz, 2H), 2.88 (d, J = 11.5 Hz, 2H), 3.40 (s, 3H), 3.57-3.60 (m, 2H), 3.63-3.67 (m, 4H), 3.84 (s, 3H), 3.97-4.05 (m, 1H), 4.73 (s, 2H), 5.94 (d, J = 7.8 Hz, 1H), 7.02 (dd, J = 8.3, 2.8 Hz, 1H), 7.23 (d, J = 7.8 Hz, 1H), 7.29-7.34 (m, 2H), 7.45 (dd, J = 8.3, 1.4 Hz, 1H), 7.47 (dd, J = 8.3, 1.4 Hz, 1H), 7.71 (s, 1H), 7.76 (s, 1H), 7.78 (d, J = 8.3 Hz, 1H), 7.80 (d, J = 8.3 Hz, 1H); ESI MS m/z 463, (M+H)⁺.

Step 1-6: 4 M HCl/EtOAc solution (1.10 mL) was added to a suspension of the compound obtained in Step 1-5 (1.00 g) in EtOAc (10.0 mL), and the mixture was stirred at room temperature for five hours. The generated solid was collected by filtration to obtain the title compound (1.06 g, colorless solid).
¹H NMR (600 MHz, CDCl₃, δ): 2.12-2.18 (m, 2H), 2.46-2.56 (m, 2H), 2.81-2.89 (m, 2H), 3.40 (s, 3H), 3.51-3.57 (m, 2H), 3.58-3.61 (m, 2H), 3.65-3.68 (m, 2H), 3.82 (s, 3H), 4.19-4.27 (m, 1H), 4.31 (s, 2H), 4.73 (s, 2H), 6.64 (d, J = 7.8 Hz, 1H), 6.99-7.03 (m, 1H), 7.27-7.30 (m, 2H), 7.31-7.32 (m, 1H), 7.55-7.58 (m, 1H), 7.78 (dd, J = 8.7, 1.8 Hz, 1H), 7.84-7.87 (m, 2H), 7.91 (d, J = 8.3 Hz, 1H), 8.02 (s, 1H), 12.65 (brs, 1H); ESI/APCI MS m/z 463, [M (free)+H]⁺.

### Example 2: Synthesis of 3-methoxy-N-[1-({7-[(2-methoxyethoxy)methyl]-2-naphthyl}methyl)piperidin-4-yl]benzamide hydrobromide

48% HBr solution (730 mg) was added to a suspension of the compound obtained in Step 1-5 (1.00 g) in EtOAc (10.0 mL), and the mixture was stirred at room temperature for 2.5 hours. The generated solid was collected by filtration to obtain the title compound (1.09 g, colorless solid).
¹H NMR (600 MHz, CDCl₃, δ): 2.18-2.24 (m, 2H), 2.53-2.63 (m, 2H), 2.93-3.01 (m, 2H), 3.43 (s, 3H), 3.58-3.64 (m, 4H), 3.68-3.71 (m, 2H), 3.84 (s, 3H), 4.26-4.34 (m, 1H), 4.37 (d, J = 5.0 Hz, 2H), 4.76 (s, 2H), 6.59 (d, J = 8.3 Hz, 1H), 7.02-7.05 (m, 1H), 7.28-7.33 (m, 3H), 7.59-7.62 (m, 1H), 7.82 (dd, J = 8.3, 1.8 Hz, 1H), 7.87-7.90 (m, 2H), 7.94 (d, J = 8.3 Hz, 1H), 8.10 (s, 1H), 11.62 (brs, 1H); ESI/APCI MS m/z 463, [M (free)+H]⁺.

### Example 3: Synthesis of methyl 7-({4-[(3-methoxybenzoyl)amino]piperidin-1-yl}methyl)-2-naphthoate

Step 3-1: NaBH₄ (30.9 g) was added to a solution of dimethyl naphthalene-2,7-dicarboxylate (100 g) in THF (1.60 L) under nitrogen atmosphere, and then the mixture was heated to 60°C. MeOH was added dropwise, and the mixture was heated under reflux for six hours and further stirred at room temperature for 12 hours. Concentrated hydrochloric acid (70.0 mL) was added dropwise with ice-cooling, followed by stirring for 30 minutes. The generated solid was separated by filtration through celite, and the filtrate was concentrated under reduced pressure. EtOAc (2.00 L) and CHCl₃ (500 mL) were added to the residue, and the generated solid was separated by filtration. The filtrate was washed with H₂O, dried over MgSO₄ and then concentrated under reduced pressure. CHCl₃ (1.30 L) was added to the resulting residue. The mixture was stirred at room temperature for 12 hours and then the solid was separated by filtration. The filtrate was concentrated under reduced pressure and CHCl₃ (1.00 L) was added to the residue, followed by stirring for one hour, to obtain a solid A. The filtrate was concentrated under reduced pressure again and a solid B was similarly obtained. The filtrate was concentrated under reduced pressure. Then, the residue was purified by column chromatography (Silica gel 60 N, mobile phase: MeOH/CHCl₃ = 1/99 to 4/96; v/v) to obtain a solid C. The solids A, B and C were combined to obtain methyl 7-(hydroxymethyl)-2-naphthoate (57.2 g, colorless solid).
¹H NMR (200 MHz, CDCl₃, δ): 1.87 (brs, 1H), 3.98 (s, 3H), 4.89 (s, 2H), 7.60 (dd, J = 8.4, 1.8 Hz, 1H), 7.82-7.94 (m, 3H), 8.05 (dd, J = 8.4, 1.8 Hz, 1H), 8.59 (s, 1H); ESI MS m/z 239, (M+Na)⁺.

Step 3-2: MnO₂ (184 g) was added to a suspension of the compound obtained in Step 3-1 (57.1 g) in toluene (1.38 L), and the mixture was heated under reflux for 30 minutes. The reaction solution was left to cool to room temperature and filtered through celite. The filtrate was concentrated under reduced pressure to obtain methyl 7-formyl-2-naphthoate (52.2 g, colorless solid).
¹H NMR (200 MHz, CDCl₃, δ): 4.02 (s, 3H), 7.92-8.02 (m, 2H), 8.07 (dd, J = 8.4, 1.8 Hz, 1H), 8.23 (dd, J = 8.8, 1.8 Hz, 1H), 8.45 (s, 1H), 8.76 (s, 1H), 10.19 (s, 1H); EI MS m/z 214, M⁺.

Step 3-3: The title compound (92.3 g, colorless solid) was obtained from the compound obtained in Step 3-2 (52.1 g) and the compound obtained in Step 1-4 (59.8 g) by the same method as in Step 1-5.
¹H NMR (600 MHz, CDCl₃, δ): 1.53-1.62 (m, 2H), 1.98-2.06 (m, 2H), 2.24 (t, J = 11.0 Hz, 2H), 2.88 (d, J = 11.5 Hz, 2H), 3.68 (s, 2H), 3.84 (s, 3H), 3.97 (s, 3H), 3.98-4.06 (m, 1H), 5.96 (d, J = 7.8 Hz, 1H), 7.02 (dd, J = 8.3, 2.8 Hz, 1H), 7.24 (dt, J = 7.8, 1.4 Hz, 1H), 7.29-7.34 (m, 2H), 7.60 (dd, J = 8. 3, 1.4 Hz, 1H), 7.82-7.87 (m, 3H), 8.02 (dd, J = 8.3, 1.8 Hz, 1H), 8.57 (s, 1H); ESI MS m/z 433, (M+H)⁺.

### Example 4: Synthesis of N-(1-{[7-(hydroxymethyl)-2-naphthyl]methyl}piperidin-4-yl)-3-methoxybenzamide

The title compound (74.5 g, colorless solid) was obtained from the compound obtained in Step 3-3 (91.0 g) by the same method as in Step 1-1.
¹H NMR (600 MHz, CDCl₃, δ): 1.53-1.62 (m, 2H), 1.78 (brs, 1H), 2.02 (d, J = 11.0 Hz, 2H), 2.23 (t, J = 11.0 Hz, 2H), 2.85-2.92 (m, 2H), 3.67 (s, 2H), 3.84 (s, 3H), 3.97-4.05 (m, 1H), 4.85 (s, 2H), 5.94 (d, J = 7.3 Hz, 1H), 7.00-7.03 (m, 1H), 7.23 (d, J = 7.8 Hz, 1H), 7.29-7.34 (m, 2H), 7.47 (dd, J = 18.1, 8.5 Hz, 2H), 7.72 (s, 1H), 7.76-7.84 (m, 3H); ESI MS m/z 403, (M-H)⁻.

### Example 5: Synthesis of N-{1-[(7-formyl-2-naphthyl)methyl]piperidin-4-yl}-3-methoxybenzamide

The title compound (34.7 g, colorless solid) was obtained from the compound obtained in Example 4 (35.0 g) by the same method as in Step 1-3.
¹H NMR (600 MHz, CDCl₃, δ): 1.57-1.66 (m, 2H), 2.01-2.08 (m, 2H), 2.23-2.31 (m, 2H), 2.87-2.94 (m, 2H), 3.71 (s, 2H), 3.84 (s, 3H), 3.99-4.07 (m, 1H), 5.96 (d, J = 7.3 Hz, 1H), 7.02 (dd, J = 7.8, 2.3 Hz, 1H), 7.24 (d, J = 7.8 Hz, 1H), 7.30-7.34 (m, 2H), 7.67 (d, J = 8.3 Hz, 1H), 7.87 (d, J = 8.3 Hz, 1H), 7.90-7.94 (m, 3H), 8.31 (s, 1H), 10.15 (s, 1H); ESI MS m/z 403, (M+H)⁺.

### Example 6: Synthesis of 3-chloro-N-(1-{[7-(ethoxymethyl)-2-naphthyl]methyl}piperidin-4-yl)-4-fluorobenzamide

Step 6-1: [7-(Ethoxymethyl)-2-naphthyl]methanol (1.03 g) was obtained from the compound obtained in Step 1-1 (2.34 g) and EtI (1.94 g) by the same method as in Step 1-2.
¹H NMR (200 MHz, CDCl₃, δ): 1.27 (t, J = 7.0 Hz, 3H), 1.79 (brs, 1H), 3.59 (q, J = 7.0 Hz, 2H), 4.66 (s, 2H), 4.84 (s, 2H), 7.41-7.51 (m, 2H), 7.73-7.86 (m, 4H); ESI MS m/z 239, (M+Na)⁺.

Step 6-2: 7-(Ethoxymethyl)-2-naphthaldehyde (580 mg) was obtained from the compound obtained in Step 6-1 (980 mg) by the same method as in Step 3-2.
¹H NMR (200 MHz, CDCl₃, δ): 1.30 (t, J = 7.0 Hz, 3H), 3.63 (q, J = 7.0 Hz, 2H), 4.71 (s, 2H), 7.64 (dd, J = 8.8, 1.8 Hz, 1H), 7.84-8.00 (m, 4H), 8.33 (s, 1H), 10.16 (s, 1H); CI MS m/z 215, (M+H)⁺.

Step 6-3: Et₃N (122 mL) and 3-chloro-4-fluorobenzoyl chloride (37.1 g) were added to a solution of tert-butyl 4-aminopiperidine-1-carboxylate (35.0 g) in CHCl₃ (350 mL) with ice-cooling, and the mixture was stirred at the same temperature for 1.5 hours. Saturated aqueous NaHCO₃ solution was added, followed by extraction with CHCl₃ three times. The combined organic layers were dried over MgSO₄ and then concentrated under reduced pressure to obtain tert-butyl 4-[(3-chloro-4-fluorobenzoyl)amino]piperidine-1-carboxylate (62.0 g). 4 M HCl/EtOAc solution (300 mL) was added to a suspension of the resulting compound in EtOAc (300 mL), and the mixture was stirred at room temperature for four hours. The reaction solution was concentrated under reduced pressure and 1 M aqueous NaOH solution (300 mL) was added to the residue, followed by extraction with CHCl₃ three times. The combined organic layers were dried over MgSO₄ and then concentrated under reduced pressure. The residue was suspended in EtOAc/hexane (200 mL, 1/1; v/v), followed by stirring for one hour. Then, the solid was collected by filtration to obtain 3-chloro-4-fluoro-N-piperidin-4-ylbenzamide (37.7 g, colorless solid).
¹H NMR (200 MHz, CDCl₃, δ): 1.30-1.53 (m, 2H), 1.94-2.12 (m, 2H), 2.75 (td, J = 12.0, 2.4 Hz, 2H), 3.12 (dt, J = 12.6, 3.7, 3.4 Hz, 2H), 3.93-4.17 (m, 1H), 5.87-6.09 (m, 1H), 7.19 (t, J = 8.6 Hz, 1H), 7.59-7.70 (m, 1H), 7.83 (dd, J = 7.0, 2.2 Hz, 1H); ESI MS m/z 257, (M+H)⁺.

Step 6-4: The title compound (457 mg) was obtained from the compound obtained in Step 6-2 (300 mg) and the compound obtained in Step 6-3 (300 mg) by the same method as in Step 1-5.
¹H NMR (600 MHz, CDCl₃, δ): 1.26 (t, J = 6.9 Hz, 3H), 1.53-1.62 (m, 2H), 1.97-2.04 (m, 2H), 2.22 (t, J = 10.8 Hz, 2H), 2.89 (d, J = 11.0 Hz, 2H), 3.58 (q, J = 6.9 Hz, 2H), 3.66 (s, 2H), 3.95-4.02 (m, 1H), 4.66 (s, 2H), 5.85 (d, J = 7.3 Hz, 1H), 7.18 (t, J = 8.5 Hz, 1H), 7.45 (s, 2H), 7.60-7.63 (m, 1H), 7.69-7.83 (m, 5H); ESI MS m/z 455, (M+H)⁺.

### Example 7: Synthesis of 3-fluoro-4-methoxy-N-(1-{[7-(methoxymethyl)-2-naphthyl]methyl}piperidin-4-yl)benzamide

Step 7-1: Methyl 7-(methoxymethyl)-2-naphthoate (12.7 g) was obtained from the compound obtained in Step 3-1 (19.0 g) and MeI (18.7 g) by the same method as in Step 6-1.
¹H NMR (200 MHz, CDCl₃, δ): 3.45 (s, 3H), 3.98 (s, 3H), 4.64 (s, 2H), 7.57 (dd, J = 8.8, 1.3 Hz, 1H), 7.80-7.93 (m, 3H), 8.05 (dd, J = 8.4, 1.8 Hz, 1H), 8.60 (s, 1H); ESI MS m/z 253, (M+Na)⁺.

Step 7-2: [7-(Methoxymethyl)-2-naphthyl]methanol (9.68 g) was obtained from the compound obtained in Step 7-1 (11.5 g) by the same method as in Step 1-1.
¹H NMR (200 MHz, CDCl₃, δ): 1.74 (brs, 1H), 3.43 (s, 3H), 4.63 (s, 2H), 4.87 (s, 2H), 7.45 (dd, J = 3.5, 1.8 Hz, 1H), 7.49 (dd, J = 4.0, 1.8 Hz, 1H), 7.74-7.88 (m, 4H); ESI MS m/z 225, (M+Na)⁺.

Step 7-3: 7-(Methoxymethyl)-2-naphthaldehyde (8.65 g) was obtained from the compound obtained in Step 7-2 (9.66 g) by the same method as in Step 3-2.
¹H NMR (200 MHz, CDCl₃, δ): 3.47 (s, 3H), 4.65 (s, 2H), 7.62 (dd, J = 8.4, 1.8 Hz, 1H), 7.84-7.97 (m, 4H), 8.32 (s, 1H), 10.16 (s, 1H); EI MS m/z 223, (M+Na)⁺.

Step 7-4: tert-Butyl (1-{[7-(methoxymethyl)-2-naphthyl]methyl}piperidin-4-yl)carbamate (10.1 g) was obtained from the compound obtained in Step 7-3 (8.55 g) and tert-butyl piperidin-4-ylcarbamate (7.13 g) by the same method as in Step 1-5.
¹H NMR (200 MHz, CDCl₃, δ): 1.37-1.56 (m, 2H), 1.44 (s, 9H), 1.82-2.00 (m, 2H), 2.13 (dt, J = 11.4, 2.2 Hz, 2H), 2.75-2.90 (m, 2H), 3.40-3.55 (m, 1H), 3.43 (s, 3H), 3.63 (s, 2H), 4.35-4.49 (m, 1H), 4.62 (s, 2H), 7.37-7.51 (m, 2H), 7.66-7.87 (m, 4H); ESI MS m/z 407, (M+Na)⁺.

Step 7-5: 4M HCl/EtOAc solution (100 mL) was added to a solution of the compound obtained in Step 7-4 (10.1 g) in EtOAc (100 mL) with ice-cooling, and the mixture was stirred at room temperature for 14 hours. The reaction solution was concentrated under reduced pressure and 1 M aqueous NaOH solution was added to the residue, followed by extraction with CHCl₃ twice. The combined organic layers were dried over Na₂SO₄ and then concentrated under reduced pressure to obtain 1-{[7-(methoxymethyl)-2-naphthyl]methyl}piperidin-4-amine (7.44 g, colorless solid).
¹H NMR (600 MHz, CDCl₃, δ): 1.21-1.35 (m, 2H), 1.34-1.44 (m, 2H), 1.77 (d, J = 12.8 Hz, 2H), 2.05 (t, J = 10.8 Hz, 2H), 2.59-2.69 (m, 1H), 2.85 (d, J = 11.9 Hz, 2H), 3.41 (s, 3H), 3.63 (s, 2H), 4.60 (s, 2H), 7.41 (dd, J = 8.7, 1.4 Hz, 1H), 7.46 (dd, J = 8.3, 1.4 Hz, 1H), 7.69 (s, 1H), 7.73 (s, 1H), 7.77 (d, J = 8.3 Hz, 1H), 7.79 (d, J = 8.3 Hz, 1H); ESI MS m/z 285, (M+H)⁺.

Step 7-6: 3-Fluoro-4-methoxybenzoic acid (179 mg), Et₃N (300 µL), HOBt·H₂O (202 mg) and EDC·HCl (202 mg) were added to a solution of the compound obtained in Step 7-5 (250 mg) in DMF (5.00 mL), and the mixture was stirred at room temperature for 15 hours. H₂O was added, followed by extraction with CHCl₃ three times. The organic layers were combined, dried over MgSO₄ and then concentrated under reduced pressure. The residue was purified by column chromatography (Silica gel 60 N, mobile phase: MeOH/CHCl₃ = 0/100 to 3/97; v/v). The resulting solid was suspended in Et₂O, followed by stirring for two hours. Then, the solid was collected by filtration to obtain the title compound (252 mg, colorless solid).
¹H NMR (600 MHz, CDCl₃, δ): 1.52-1.61 (m, 2H), 1.98-2.04 (m, 2H), 2.19-2.25 (m, 2H), 2.86-2.92 (m, 2H), 3.42 (s, 3H), 3.66 (s, 2H), 3.92 (s, 3H), 3.95-4.03 (m, 1H), 4.61 (s, 2H), 5.81-5.85 (m, 1H), 6.96 (t, J = 8.5 Hz, 1H), 7.41-7.44 (m, 1H), 7.46-7.51 (m, 3H), 7.71 (s, 1H), 7.74 (s, 1H), 7.79 (d, J = 8.3 Hz, 1H), 7.81 (d, J = 8.3 Hz, 1H); ESI MS m/z 437, (M+H)⁺.

The compounds of Example 8 to Example 21 were obtained by the same method as in Example 7.

**[Table 1-1]**

| Example No. | Compound name | Physical data |
|---|---|---|
| **8** | 3-Fluoro-N-(1-{[7-methoxymethyl)-2-naphthyl]methyl}piperidin-4-yl)-4-methylbenzamide | **¹H NMR (600 MHz, CDCl₃, δ): 1.52-1.61 (m, 2H), 1.98-2.04 (m, 2H), 2.18-2.26 (m, 2H), 230 (d, J = 1.8 Hz, 3H), 2.85-2.92 (m, 2H), 3.42 (s, 3H), 3.66 (s, 2H), 3.96-4.04 (m, 1H), 4.61 (s, 2H), 5.86-5.90 (m, 1H), 7.22 (t, J = 7.6 Hz, 1H), 7.36-7.44 (m, 3H), 7.46-7.49 (m, 1H), 7.72 (s, 1H), 7.74 (s, 1H), 7.79 (d, J = 8.3 Hz, 1H), 7.81 (d, J = 8.3 Hz. 1H); ESI MS m/z 421, (M+H)⁺.** |
| **9** | 3,4-Dimethoxy-N-(1-{[7-methoxymethyl)-2-naphthyl]methyl}piperidin-4-yl)benzamide | **¹H NMR (600 MHz, CDCl₃, δ): 1.57-1.68 (m, 2H), 2.00-2.06 (m, 2H), 2.22-2.30 (m, 2H), 2.89-2.96 (m, 2H), 3.42 (s, 3H), 3.70 (s, 2H), 3.91 (s, 3H), 3.92 (s, 3H), 3.98-4.06 (m, 1H), 4.61 (s, 2H), 5.88-5.93 (m, 1H), 6.84 (d, J = 8.7 Hz, 1H), 7.22 (dd J = 8.3, 2.3 Hz, 1H), 7.39 (d, J = 1.8 Hz, 1H), 7.42-7.45 (m, 1H), 7.47-7.51 (m 1H), 7.73 (s, 1H), 7.75 (s, 1H), 7.80 (t, J = 8.9 Hz, 2H); ESI MS m/z 449, (M+H)⁺.** |
| **10** | 3,5-Dimethoxy-N-(1-{[7-(methoxymethyl)-2-naphthyl]methyl}piperidin-4-yl)benzamide | **¹H NMR (600 MHz, CDCl₃, δ): 1.52-1.61 (m, 2H), 1.98-2.04 (m, 2H), 2.19-2.27 (m, 2H), 2.84-2.91 (m, 2H), 3.42 (s, 3H), 3.66 (s, 2H), 3.81 (s, 6H), 3.95-4.03 (m, 1H) , 4.61 (s, 2H), 5.88-5.92 (m, 1H), 6.56 (t, J = 2.3 Hz, 1H), 6.84 (d, J = 2.3 Hz, 2H), 741-7.44 (m, 1H), 7.46-7.49 (m, 1H), 7.72 (s, 1H), 7.75 (s, 1H), 7.79 (d, J = 8.7 Hz, 1H), 7.80 (d, J = 8.3 Hz, 1H); ESI MS m/z 449, (M+H)⁺.** |
| **11** | 3,4,5-Trimethoxy-N-(1-{[7-methoxymethyl)-2-naphthyl]methyl}piperidin-4- yl)benzamide | **¹H NMR (600 MHz, CDCl₃, δ): 1.54-1.63 (m, 2H), 2.00-2.05 (m, 2H), 2.19-2.26 (m, 2H), 2.86-2.93 (m, 2H), 3.42 (s, 3H), 3.67 (s, 2H), 3.86 (s, 3H), 3.89 (s, 6H), 3.96-4.04 (m, 1H), 4.61 (s, 2H), 5.83-5.87 (m, 1H), 6.94 (s, 2H), 7.41-7.44 (m, 1H), 7.46-7.49 (m, 1H), 7.72 (s, 1H), 1.74 (s, 1H), 7.78 (d, J = 8.3 Hz, 1H), 7.81 (d, J = 8.7 Hz, 1H); ESI MS m/z 479, (M+H)⁺.** |
| **12** | 3,5-Dimethoxy-N-(1-{[7-(methoxymethyl)-2-naphthyl]methyl}piperidin-4-yl)-4-methylbenzamide | **¹H NMR (600 MHz, CDCl₃ δ): 1.65-1.64 (m, 2H), 2.01-2.07 (m, 2H), 2.10 (s, 3H), 2.20-2.26 (m, 2H), 2.87-2.93 (m, 2H), 3.42 (s, 3H), 3.67 (s, 2H), 3.85 (s, 6H), 3.97-4.06 (m, 1H), 4.61 (s, 2H), 5.86-5.90 (m, 1H), 6.86 (s, 2H), 7.41-7.44 (m, 1H), 7.48 (dd, J = 83, 1.8 Hz, 1H), 7.72 (s, 1H), 7.75 (s, 1H), 7.78 (d, J = 8.3 Hz, 1H), 7.81 (d, J = 8.7 Hz, 1H); ESI MS m/z 463, (M+H)⁺.** |

**[Table 1-2]**

| Example No. | Compound name | Physical data |
|---|---|---|
| **13** | 3-Methoxy-N-(1-{[7-(methoxymethyl)-2-naphthyl]methyl}piperidin-4-yl)benzamide | **¹H NMR (600 MHz, CDCl_{3,} δ): 1.54-1.65 (m, 2H), 1.99-2.06 (m, 2H), 2.20-2.27 (m, 2H), 2.83-7.94 (m, 2H), 3.42 (s, 3H), 3.67 (s, 2H), 3.84 (s, 3H), 3.97-4.06 (m, 1H), 4.61 (s, 2H), 5.93 (d, J = 7.8 Hz, 1H), 7.02 (dd, J = 8.0, 2.1 Hz, 1H), 7.23 (d, J = 7.8 Hz, 1H), 7.29-7.33 (m, 2H), 7.43 (d, J = 8.3 Hz, 1H), 7.48 (d, J = 8.7 Hz, 1H), 7.69-7.76 (m, 2H), 7.76-7.84 (m, 2H); ESI MS m/z 419, (M+H)⁺.** |
| **14** | 3-Methoxy-N-(1-{[7-(methoxymethyl)-2-naphthyl]methyl}piperidin-4-yl)-4-methylbenzamide | **¹H NMR (600 MHz, CDCl₃. δ): 1.50-1.63 (m, 2H), 2.03 (d, J = 12.8 Hz, 2H), 2.18-2.28 (m, 5H), 2.89 (d, J = 11.0 Hz, 2H), 3.42 (s, 3H), 3.67 (s, 2H), 3.87 (s, 3H), 3.96-4.05 (m, 1H), 4.61 (s, 2H), 5.93 (d, J = 8.3 Hz, 1H), 7.09 (dd, J = 7.8, 1.83 Hz, 1H), 7.13 (d, J = 7.8 Hz, 1H), 7.32 (d, J = 1.4 Hz, 1H), 7.43 (d, J = 8.3 Hz, 1H), 7.48 (d, J = 8.3 Hz, 1H), 7.72 (s, 1H), 7.75 (s, 1H), 7.79 (d, J = 8.3 Hz, 1H), 7.81 (d, J = 8.3 Hz, 1H); ESI MS m/z 433. (M+H)⁺.** |
| **15** | 3-Chloro-4-methoxy-N-(1-{[7-(methoxymethyl)-2-naphthyl]methyl}piperidin4-yl)benzamide | **¹H NMR (600 MHz, CDCl₃, δ): 1.52-1.61 (m, 2H), 1.96-2.04 (m, 2H), 2.22 (t, J = 11.0 Hz, 2H), 2.89 (d, J = 11.5 Hz, 2H), 3.42 (s, 3H), 3.66 (s, 2H), 3.93 (s, 3H), 3.95-4.03 (m, 1H), 4.61 (s, 2H), 5.85 (d, J = 7.8 Hz, 1H), 6.93 (d, J = 8.7 Hz, 1H), 7.43 (dd, J = 8.3, 1.4 Hz, 1H), 7.47 (dd, J = 8.3, 1.8 Hz, 1H), 7.65 (dd, J = 8.7, 2.3 Hz, 1H), 7.71 (s, 1H), 7.74 (s, 1H), 7.75 (d, J = 2.3 Hz, 1H), 7.78 (d, J = 8.3 Hz, 1H), 7.80 (d, J = 8.3 Hz, 1H); ESI MS m/z 453, (M+H)⁺.** |
| **16** | 3-Chloro-5-methoxy-N-(1-{[7-(methoxymethyl)-2-naphthyl]methyl}piperidin-4-yl)benzamide | **¹H NMR (600 MHz, CDCl₃, δ): 1.47-1.57 (m, 2H), 1.91-2.01 (m, 2H), 2.17 (t, J = 10.8 Hz, 2H), 2.84 (d, J = 11.5 Hz, 2H), 3.37 (s, 3H), 3.61 (s, 2H), 3.78 (s, 3H), 3.88-3.98 (m, 1H), 4.56 (s, 2H), 5.86 (d, J = 7.8 Hz, 1H), 6.94-6.96 (m, 1H), 7.14 (dd, J = 2.3, 1.4 Hz, 1H), 7.16-7.18 (m, 1H), 7.38 (dd, J = 8.3, 1.4 Hz, 1H), 7.43 (dd, J = 8.3, 1.4 Hz, 1H), 7.67 (s, 1H), 7.70 (s, 1H), 7.74 (d, J = 8.3 Hz, 1H), 7.76 (d, J = 8.7 Hz, 1H); ESI MS m/z 453, (M+H)⁺.** |
| **17** | 3-Chloro-4-hydroxy-N-(1-{[7-(methoxymethyl)-2-naphthyl]methyl}piperidin-4-yl)benzamide | **¹H NMR (600 MHz, CDCl₃, δ): 1.52-1.64 (m, 2H), 1.97-2.05 (m, 2H), 2.18-2.27 (m, 2H). 2.87-2.94 (m, 2H). 3.42 (s, 3H), 3.68 (s, 2H), 3.92-4.03 (m, 1H), 4.61 (s, 2H), 5.84 (d, J = 7.8 Hz, 1H), 7.02 (d, J = 8.7 Hz, 1H), 7.43 (dd, J = 8.3, 1.4 Hz, 1H), 7.48 (dd, J = 8.3, 1.4 Hz, 1H), 7.54 (dd, J = 8.5, 2.1 Hz, 1H), 7.68-7.83 (m, 5H); ESI MS m/z 439, (M+H)⁺.** |

**[Table 1-3]**

| Example No. | Compound name | Physical data |
|---|---|---|
| **18** | 4-Hydroxy-3-methoxy-N-(1-{[7-(methoxymethyl)-2-naphthyl]methyl}piperidin-4-yl)benzamide | **¹H NMR (600 MHz, CDCl₃, δ): 1.51-1.64 (m, 2H), 1.95-2.06 (m, 2H), 2.23 (t, J = 11.2 Hz, 2H), 2.85-2.92 (m, 2H), 3.42 (s, 3H), 3.67 (s, 2H), 3.93 (s, 3H), 3.95-4.04 (m, 1H), 4.61 (s, 2H), 5.88 (d, J = 7.8 Hz, 1H), 5.90 (brs, 1H), 6.90 (d, J = 8.3 Hz, 1H), 7.14 (d, J = 8.7 Hz, 1H), 7.43 (s, 2H), 7.48 (d, J = 8.7 Hz, 1H), 7.72 (s, 1H), 7.74 (s, 1H), 7.78 (d, J = 8.3 Hz, 1H), 7.81 (d, J = 8.3 Hz, 1H); ESI MS m/z 435, (M+H)⁺.** |
| **19** | 3-Hydroxy-4-methoxy-N-(1-{[7-(methoxymethyl)-2-naphthyl]methyl}piperidin-4-yl)benzamide | **¹H NMR (600 MHz, CDCl₃, δ): 1.52-1.61 (m, 2H), 1.97-2.04 (m, 2H), 2.22 (t, J = 11.0 Hz, 2H), 2.88 (d, J = 9.6 Hz, 2H), 3.42 (s, 3H), 3.66 (s, 2H), 3.92 (s, 3H), 3.94-4.05 (m, 1H), 4.61 (s, 2H), 5.76 (brs, 1H), 5.85 (d, J = 7.3 Hz, 1H), 6.86 (d, J = 8.3 Hz, 1H), 7.28 (d, J = 2.1 Hz, 1H), 7.31 (dd, J = 8.5, 2.1 Hz, 1H), 7.43 (d, J = 8.3 Hz, 1H), 7.48 (d, J = 8.3 Hz, 1H), 7.72 (s, 1H), 7.74 (s, 1H). 7.78 (d, J = 8.7 Hz, 1H), 7.80 (d, J = 8.7 Hz, 1H); ESI MS m/z 435, (M+H)⁺.** |
| **20** | 3-Hydroxy-N-(1-{[7-(methoxymethyl)-2- naphthyl]methyl}piperidin-4-yl)-4-methylbenzamide | **¹H NMR (600 MHz, CDCl₃, δ): 1,49-1.58 (m, 2H), 1.93-1.99 (m, 2H), 2.16-2.25 (m, 2H). 2.23 (s, 3H), 2.86 (d, J = 11.5 Hz, 2H), 3.39 (s, 3H), 3.64 (s, 2H), 3.93-4.01 (m, 1H), 4.58 (s, 2H), 5.98 (d, J = 7.8 Hz, 1H), 7.02 (dd, J = 7.8, 1.4 Hz, 1H), 7.08 (d, J = 7.8 Hz, 1H), 7.40 (dd, J = 8.3, 1.8 Hz, 1H). 7.43-7.46 (m, 2H), 7.69 (s, 1H), 7.70 (s, 1H), 7.75 (d, J = 8.3 Hz, 1H), 7.77 (d, J = 8.3 Hz, 1H); ESI MS m/z 419, (M+H)⁺.** |
| **21** | 4-Methoxy-N-(1-{[7-(methoxymethyl)-2-naphtthyl]methyl}piperidin-4-yl)-3-methylbenzamide | **¹H NMR (600 MHz, CDCl₃, δ): 1.51-1.66 (m, 2H), 1.97-2.03 (m, 2H), 2.17-2.25 (m, 5H), 2.83-2.91 (m, 2H), 3.40 (s, 3H), 3.65 (s, 2H), 3.84 (s, 3H), 3.95-4.03 (m, 1H), 4.60 (s, 2H), 5.81-5.86 (m, 1H), 6.80 (d, J = 8.3 Hz, 1H), 7.39-7.43 (m, 1H), 7.45-7.48 (m, 1H), 7.49-7.52 (m, 1H), 7.56 (dd, J = 8.7, 2.3 Hz, 1H), 7.70 (s, 1H), 7.73 (s, 1H), 7.77 (d, J = 8.3 Hz, 1H), 7.79 (d, J = 8.3 Hz, 1H); ESI MS m/z 433, (M+H)⁺.** |

### Example 22: Synthesis of N-[1-({7-[(cyclopropylmethoxy)methyl]-2-naphthyl}methyl)piperidin-4-yl]-3-methoxybenzamide

The title compound (116 mg) was obtained from the compound obtained in Example 4 (250 mg) and (bromomethyl)cyclopropane (92.0 mg) by the same method as in Step 1-2.
¹H NMR (600 MHz, CDCl₃, δ): 0.19-0.22 (m, 2H), 0.52-0.57 (m, 2H), 1.08-1.16 (m, 1H), 1.56-1.68 (m, 2H), 2.00-2.06 (m, 2H), 2.22-2.30 (m, 2H), 2.87-2.96 (m, 2H), 3.34 (d, J = 6.9 Hz, 2H), 3.69 (s, 2H), 3.84 (s, 3H), 3.99-4.07 (m, 1H), 4.69 (s, 2H), 5.93-5.98 (m, 1H), 7.02 (dd, J = 8.3, 1.8 Hz, 1H), 7.21-7.25 (m, 1H), 7.29-7.34 (m, 2H), 7.44-7.47 (m, 1H), 7.47-7.50 (m, 1H), 7.73 (s, 1H), 7.75 (s, 1H), 7.79 (t, J = 8.3 Hz, 2H) ESI MS m/z 459, (M+H)⁺.

The Compounds of Example 23 to Example 34 were obtained by the same method as in Example 22.

**[Table 2-1]**

| Example No. | Compound name | Physical data |
|---|---|---|
| **23** | 3-Methoxy-N-[1-({7-[(3-methylbutoxy)methyl]-2-naphthyl}methyl)piperdin-4-yl]benzamide | **¹H NMR (600 MHz, CDCl₃, δ): 0.90 (d, J = 6.4 Hz, 6H), 1.47-1.70 (m, 2H), 1.53 (q, J = 6.9 Hz, 2H), 1.71-1.78 (m, 1H), 2.00-2.06 (m, 2H), 2.21-2.32 (m, 2H), 2.87-2.98 (m, 2H), 3.53 (t, J = 6.9 Hz, 2H), 3.70 (s, 2H), 3.84 (s, 3H), 3.99-4.07 (m, 1H), 4.65 (s, 2H), 5.93-5.98 (m, 1H), 7.02 (dd, J = 8.3, 2.8 Hz, 1H), 7.23 (d, J = 7.8 Hz, 1H), 7.29-7.33 (m, 2H), 7.42-7.46 (m, 1H), 7.46-7.51 (m, 1H), 7.73 (s, 1H), 7.75 (s, 1H), 7.77-7.81 (m, 2H); ESI MS m/z 473, (M-H)⁺.** |
| **24** | 3-Methoxy-N-(1-{[7-(propoxymethyl)-2-naphthyl]methyl}piperidin-4-yl)benzamide | **¹H NMR (600 MHz, CDCl₃, δ): 0.95 (t, J = 7.6 Hz, 3H), 1.52-1.60 (m, 2H), 1.62-1.70 (m, 2H), 1.99-2.05 (m, 2H), 2.23 (t, J = 11.0 Hz, 2H), 2.88 (d, J = 11.5 Hz, 2H), 3.47 (t, J = 6.9 Hz, 2H), 3.66 (s, 2H), 3.84 (s, 3H), 3.96-4.05 (m, 1H), 4.66 (s, 2H), 5.94 (d, J = 7.8 Hz, 1H), 7.02 (dd, J = 8.0, 2.5 Hz, 1H), 7.23 (d, J = 7.8 Hz, 1H), 7.31 (d, J = 7.8 Hz, 1H), 7.32-7.33 (m, 1H), 7.44 (dd, J =8.2, 1.8 Hz, 1H), 7.47 (dd, J = 8.3, 1.8 Hz, 1H), 7.71 (s, 1H), 7.75 (s, 1H), 7.78 (d, J = 9.2 Hz, 1H), 7.80 (d, J = 9.2 Hz, 1H); ESI MS m/z 447, (M+H)⁺.** |
| **25** | N-[1-({7-[(Allyloxy)methyl]-2-naphthyl}methyl)piperidin4-yl]-3-methoxybenzamide | **¹H NMR (600 MHz, CDCl_{3,} δ): 1.54-1.61 (m, 2H), 1,99-2.05 (m, 2H), 2.23 (t, J = 11.0 Hz, 2H), 2.89 (d, J = 11.0 Hz, 2H), 3.67 (s, 2H), 3.84 (s, 3H), 3.97-4.05 (m, 1H), 4.06 (dt, J = 5.5, 1.4 Hz, 2H), 4.68 (s, 2H), 5.22 (s, 1H), 5.32 (s, 1H), 5.92-6.01 (m, 2H), 7.00-7.03 (m, 1H), 7.23 (d, J = 7.8 Hz, 1H), 7.29-7.33 (m, 2H), 7.44 (dd, J = 8.3, 1.8 Hz, 1H), 7.47 (dd, J = 8.3, 1.8 Hz, 1H), 7.72 (s, 1H), 7.76 (s, 1H), 7.78 (d, J = 8.3 Hz, 1H), 7.80 (d, J = 8.3 Hz, 1H); ESI MS m/z 445, (M+H)⁺.** |
| **26** | 3-Methoxy-N-[1-({7-[(2-methoxyethoxy)methyl]-2-naphthyl}methyl)piperidin-4-yl]benzamide | **¹H NMR (600 MHz, CDCl₃, δ): 1.53-1.63 (m, 2H), 2.03 (d, J = 10.6 Hz, 2H), 2.23 (t, J = 10.6 Hz, 2H), 2.84-2.92 (m, 2H), 3.40 (s, 3H), 3.56-3.61 (m, 2H), 3.63-3.69 (m, 4H), 3.84 (s, 3H), 3.96-4.06 (m, 1H), 4.73 (s, 2H), 5.93 (d, J = 7.8 Hz, 1H), 7.02 (dd, J = 8.3, 2.8 Hz, 1H), 7.23 (d, J = 7.8 Hz, 1H), 7.28-7.34 (m, 2H), 7.45 (dd, J = 8.3, 1.4 Hz, 1H), 7.47 (d, J = 8.3 Hz, 1H), 7.71 (s, 1H), 7.76 (s, 1H), 7.78 (d, J = 8.7 Hz, 1H), 7.80 (d, J = 8.7 Hz, 1H); ESI MS m/z 463, (M+H)⁺.** |
| **27** | N-{1-[(7-{[2-(Benzyloxy)ethoxy]methyl}-2-naphthyl)methyl]piperidin-4-yl}-3-methoxybenzamide | **¹H NMR (600 MHz, CDCl₃, δ): 1.52-1.62 (m, 2H), 1.98-2.07 (m, 2H), 2.23 (t, J = 10.8 Hz, 2H), 2.89 (d, J = 11.5 Hz, 2H), 3.64- 3.72 (m, 6H), 3.84 (s, 3H), 3.98-4.06 (m, 1H), 4.59 (s, 2H), 4.73 (s, 2H), 5.94 (d, J = 7.3 Hz, 1H), 7.02 (ddd, J = 8.3, 2.8, 0.9 Hz, 1H), 7.20-7.37 (m, 8H), 7.45 (d, J = 8.3 Hz, 1H), 7.47 (d, J = 8.3 Hz, 1H), 7.70 (s, 1H), 7.75-7.81 (m, 3H); ESI MS m/z 539, (M +H)⁺.** |

**[Table 2-2]**

| Example No. | Compound name | Physical data |
|---|---|---|
| **28** | N-[1-({7-[(2-Ethoxyethoxy)methyl]-2-naphthyl}methyl)piperidin-4-yl]-3-methoxybenzamide | **¹H NHR (600 MHz, CDCl₃, δ): 1.31 (t, J = 7.0 Hz, 3H), 1.60-1.71 (m, 2H), 2.07-2.13 (m, 2H), 2.31 (t, J = 11.0 Hz, 2H), 2.96 (d, J = 11.5 Hz, 2H), 3.62 (q, J = 7.0 Hz, 2H), 3.68-3.76 (m, 6H), 3.92 (s, 3H), 4-06-4.15 (m, 1H), 4.81 (s, 2H), 6.20 (d, J = 7.6 Hz, 1H), 7.10 (dd, J = 8.0, 2.5 Hz, 1H), 7.31 (d, J = 7.8 Hz, 1H), 7.36-7.42 (m, 2H), 7.53 (d, J = 8.3 Hz, 1H), 7.55 (d, J = 8.3 Hz, 1H), 7.19(s, 1H), 7.84 (s, 1H), 7.85-7.39 (m, 2H); ESI MS m/z 477, (M+H)⁺.** |
| **29** | 3-Mothoxy-N-[1-({7-[(2-propoxyethoxy)methyl]-2-naphthyl}methyl)piperidin-4-yl]benzamide | **¹H NMR (600 MHz, CDCl₃, δ): 0.92 (t, J = 7.6 Hz, 3H), 1.52-1,66 (m,4H), 1.99-2.05 (m, 2H), 2.23 (t, J = 10.8 Hz, 2H), 2.88 (d, J = 11.0 Hz, 2H), 3.43 (t, J = 6.9 Hz, 2H), 3.60-3.68 (m, 6H), 3.84 (s, 3H), 3.97-4.05 (m, 1H), 4.73 (s, 2H), 5.94 (d, J = 7.8 Hz, 1H), 7.02 (dd, J = 7.8, 2.8 Hz, 1H), 7.23 (d, J = 7.8 Hz, 1H), 7.29-7.34 (m, 2H), 7.45 (d, J = 8.3 Hz, 1H), 7.47 (d, J = 8.3 Hz. 1H), 7.71 (s, 1H), 7.76 (s, 1H), 7.77-7.82 (m, 2H); ESI MS m/z 491, (M+H)⁺.** |
| **30** | N-[1-({7-[(2-Isopropoxyethoxy)methyl]-2-naphthyl}methyl)piperidin-4-yl]-3-methoxybenzamide | **¹H NMR (600 MHz, CDCl₃, δ)_{:} 1.18 (d, J = 6.4 Hz, 6H), 1.50-1.62 (m, 2H), 1.98-2.06 (m, 2H), 2.17-2.27 (m, 2H), 2.85-2.93 (m, 2H), 3.57-3.65 (m, 5H), 3.67 (s, 2H), 3.84 (s, 3H), 3.95-4.07 (m, 1H), 4.73 (s, 2H), 5.94 (d, J = 7.3 Hz, 1H), 7.02 (dd, J = 8.3, 2.3 Hz, 1H), 7.23 (d, J = 7.8 Hz, 1H), 7.30-7.34 (m, 2H), 7.45 (d, J = 8.3 Hz, 1H), 7.47 (d, J = 8.3 Hz, 1H), 7.71 (s, 1H), 7.76 (s, 1H), 7.77-7.81 (m, 2H); ESI MS m/z 491, (M+H)⁺.** |
| **31** | 3-Methoxy-N-[1-({7-[(3-methoxypropoxy)methyl]-2-naphthyl}methyl)piperidin-4-yl]benzamide | **¹H NMR (600 MHz, CDCl₃, δ): 1.53-1.62 (m, 2H), 1.86-1.94 (m, 2H), 1.99-2.05 (m, 2H), 2.23 (t, J = 11.0 Hz, 2H), 2.89 (d, J = 11.0 Hz, 2H), 3.33 (s, 3H), 3.49 (t, J = 6.4 Hz, 2H), 3.59 (t,J= 6.4 Hz, 2H), 3.66 (s, 2H), 3.84 (s, 3H), 3.97-4.05 (m, 1H), 4.66 (s, 2H), 5.94 (d, J = 7.8 Hz, 1H), 7.02 (dd, J = 8.3, 2.8 Hz, 1H), 7.23 (d, J = 8.3 Hz, 1H), 7.29-7.33 (m, 2H), 7.43 (dd, J = 8.3, 1.8 Hz, 1H), 7.47 (dd, J = 8.3, 1.8 Hz, 1H), 7.71 (s, 1H), 7.74 (s, 1H), 7.78 (d, J = 8.7 Hz, 1H), 7.80 (d, J = 8.7 Hz, 1H); ESI MS m/z 477, (M+H)⁺.** |
| **32** | 3-Methoxy-N-{1-[7-{[2-(tetrahydro-2H-pyran-2-yloxy)ethoxy]methyl}-2-naphthyl)methyl]piperidin-4-yl}benzamide | **¹H NMR (600 MHz, CDCl₃, δ): 1.48-1.67 (m, 6H), 1.70-1.77 (m, 1H), 1.81-1.91 (m, 1H), 1,99-2.05 (m, 2H), 2.22 (t, J = 10.8 Hz, 2H), 2.88 (d, J = 11.0 Hz, 2H), 3.46-3.52 (m, 1H), 3.61-3.73 (m, 5H), 3.81-3.93 (m, 5H), 3.97-4.06 (m, 1H), 4.65 (t, J = 4.1 Hz, 1H), 4.72 (d, J = 12.8 Hz, 1H), 4.76 (d, J = 12.8 Hz, 1H), 5.95 (d, J = 7.8 Hz, 1H), 7.02 (dd, J = 8.3, 2.8 Hz, 1H), 7.23 (d, J = 7.8 Hz, 1H), 7.29-7.33 (m, 2H), 7.43-7.49 (m, 2H), 7.71 (s, 1H), 7.76 (s, 1H), 7.78 (d, J = 9.2 Hz, 1H), 7.80 (d, J = 9.2 Hz, 1H); ESI/APCI MS m/z 533, (M+H)⁺.** |

**[Table 2-3]**

| Example No. | Compound name | Physical data |
|---|---|---|
| **33** | N-{1-[(7-{[2-(Dimethylamino)-2-oxoethoxy]methyl}-2-naphthyl)methyl]piperidin-4-yl}-3-methoxybenzamide | **¹H NMR (600 MHz, CDCl₃, δ): 1-53-1.66 (m, 2H), 1.99-2.05 (m, 2H), 2.20-2.27 (m, 2H), 2.85-2.92 (m, 2H), 2.96 (s, 3H), 2.97 (s, 3H), 3.66 (s, 2H), 3.84 (s, 3H), 3.98-4.05 (m, 1H), 4.20 (s, 2H), 4.77 (s, 2H), 5.93-5.97 (m, 1H), 7.00-7.03 (m, 1H), 7.22-7.25 (m, 1H), 7.31 (d, J = 8.3 Hz, 1H), 7.32-7.33 (m, 1H), 7.44-7.50 (m, 2H). 7.72 (s, 1H), 7.76-7.80 (m, 2H), 7.81 (d, J = 8.3 Hz, 1H); ESI MS m/z 490, (M+H)⁺.** |
| **34** | N-[1-({7-[(3,3-Dimethyl-2-oxohutoxy)methyl]-2-naphthyl}methyl)piperidin-4-yl]-3-methoxybenzamide | **¹H NMR (600 MHz, CDCl₃, δ): 1.14 (s, 9H), 1.50-1.65 (m, 2H), 2.00-2.06 (m, 2H), 2.21-2.28 (m, 2H). 2.86-2.93 (m, 2H), 3.68 (s, 2H), 3.84 (s, 3H), 3.98-4.06 (m, 1H). 4.36 (s, 2H), 4,75 (s, 2H), 5.92-5.97 (m, 1H), 7.01-7.03 (m, 1H), 7.22-7.26 (m, 1H), 7.30-7.34 (m, 2H), 7.46-7.51 (m, 2H), 7.73 (s, 1H), 7.77 (s, 1H), 7.79 (d, J = 8.3 Hz, 1H), 7.81 (d, J = 8.7 Hz, 1H); ESI MS m/z 503, (M+H)⁺.** |

### Example 35: Synthesis of N-[1-({7-[(2-hydroxyethoxy)methyl]-2-naphthyl}methyl)piperidin-4-yl]-3-methoxybenzamide

Step 35-1: (7-{[2-(Tetrahydro-2H-pyran-2-yloxy)ethoxy]methyl}-2-naphthyl)methanol (10.7 g) was obtained from the compound obtained in Step 1-1 (13.8 g) and 2-(2-bromoethoxy)tetrahydro-2H-pyrane (15.3 g) by the same method as in Step 1-2.
¹H NMR (200 MHz, CDCl₃, δ): 1.33-1.95 (m, 6H), 3.37-3.60 (m, 2H), 3.59-3.77 (m, 2H), 3.79-3.96 (m, 2H), 4.61-4.70 (m, 1H), 4.75 (s, 2H), 4.85 (s, 2H), 7.47 (dd, J = 8.4, 1.8 Hz, 2H), 7.74-7.86 (m, 4H); ESI MS m/z 339, (M+Na)⁺.

Step 35-2: 7-{[2-(Tetrahydro-2H-pyran-2-yloxy)ethoxy]methyl}-2-naphthaldehyde (3.65 g) was obtained from the compound obtained in Step 35-1 (10.7 g) by the same method as in Step 1-3. ¹H NMR (200 MHz, CDCl₃, δ): 1.45-1.95 (m, 6H), 3.44-3.59 (m, 1H), 3.62-3.78 (m, 3H), 3.81-4.00 (m, 2H), 4.67 (dd, J = 4.0, 3.1 Hz, 1H), 4.79 (s, 2H), 7.65 (dd, J = 8.4, 1.8 Hz, 1H), 7.85-8.01 (m, 4H), 8.32 (s, 1H), 10.16 (s, 1H); ESI/APCI MS m/z 337, (M+Na)⁺.

Step 35-3: The compound obtained in Example 32 (5.27 g) was obtained from the compound obtained in Step 35-2 (3.64 g) and the compound obtained in Step 1-4 (2.71 g) by the same method as in Step 1-5.
¹H NMR (600 MHz, CDCl₃, δ): 1.48-1.68 (m, 6H), 1.70-1.77 (m, 1H), 1.81-1.89 (m, 1H), 1.99-2.05 (m, 2H), 2.23 (t, J = 11.0 Hz, 2H), 2.88 (d, J = 11.5 Hz, 2H), 3.47-3.51 (m, 1H), 3.62-3.72 (m, 5H), 3.82-3.93 (m, 5H), 3.97-4.05 (m, 1H), 4.65 (t, J = 3.7 Hz, 1H), 4.72 (d, J = 12.4 Hz, 1H), 4.76 (d, J = 12.4 Hz, 1H), 5.94 (d, J = 8.3 Hz, 1H), 7.02 (dd, J = 8.3, 2.8 Hz, 1H), 7.23 (d, J = 8.3 Hz, 1H), 7.29-7.33 (m, 2H), 7.43-7.49 (m, 2H), 7.71 (s, 1H), 7.76 (s, 1H), 7.78 (d, J = 8.7 Hz, 1H), 7.80 (d, J = 8.7 Hz, 1H); ESI/APCI MS m/z 533, (M+H)⁺.

Step 35-4: A mixed solution of the compound obtained in Example 32 (4.96 g) in AcOH (84.0 mL), THF (42.0 mL) and H₂O (21.0 mL) was stirred at 50°C for 2.5 days. The reaction solution was concentrated under reduced pressure and then CHCl₃ and saturated aqueous NaHCO₃ solution were added to the residue. The organic layer was separated, dried over Na₂SO₄ and then concentrated under reduced pressure. The residue was purified by column chromatography (Silica gel 60 N, mobile phase: MeOH/CHCl₃ = 2/98 to 5/95; v/v) to obtain the title compound (3.54 g, colorless solid).
¹H NMR (600 MHz, CDCl₃, δ): 1.51-1.62 (m, 2H), 1.98-2.06 (m, 2H), 2.08 (brs, 1H), 2.22 (t, J = 10.8 Hz, 2H), 2.88 (d, J = 11.5 Hz, 2H), 3.62-3.65 (m, 2H), 3.66 (s, 2H), 3.78 (t, J = 4.6 Hz, 2H), 3.84 (s, 3H), 3.95-4.06 (m, 1H), 4.72 (s, 2H), 5.97 (d, J = 7.8 Hz, 1H), 7.02 (dd, J = 8.3, 1.8 Hz, 1H), 7.23 (d, J = 7.8 Hz, 1H), 7.29-7.33 (m, 2H), 7.43 (dd, J = 8.3, 1.8 Hz, 1H), 7.48 (dd, J = 8.3, 1.4 Hz, 1H), 7.72 (s, 1H), 7.75 (s, 1H), 7.79 (d, J = 8.3 Hz, 1H), 7.81 (d, J = 8.3 Hz, 1H); ESI/APCI MS m/z 449, (M+H)⁺.

### Example 36: Synthesis of 3-Methoxy-N-[1-({7-[(2-pyrrolidin-1-ylethoxy)methyl]-2-naphthyl}methyl)piperidin-4-yl]benzamide

Et₃N (150 µL) and MsCl (123 mg) were added to a solution of the compound obtained in Example 35 (322 mg) in THF (6.40 mL) under nitrogen atmosphere with ice-cooling, and the mixture was stirred at room temperature for 45 minutes. Saturated aqueous NaHO₃ solution, H₂O and EtOAc were added and the aqueous layer was separated. The organic layer was dried over Na₂SO₄ and then concentrated under reduced pressure. NaHCO₃ (60.0 mg) and pyrrolidine (51.0 mg) were added to a solution of the resulting residue in DMF (4.40 mL), and the mixture was stirred at room temperature for one hour. The mixture was further stirred at 80°C for 18 hours. H₂O was added to the reaction solution, followed by extraction with EtCAc twice. The organic layers were combined, dried over Na₂SO₄ and then concentrated under reduced pressure. The residue was sequentially purified by column chromatography (Silica gel 60 N, mobile phase: MeOH/CHCl₃ = 10/90 to 90/10; v/v, and subsequently Chromatorex NH, mobile phase: MeOH/CHCl₃ = 0/100 to 1/99; v/v) to obtain the title compound (89 mg).
¹H NMR (600 MHz, CDCl₃, δ): 1.52-1.64 (m, 2H), 1.75-1.80 (m, 4H), 1.98-2.06 (m, 2H), 2.23 (t, J = 10.8 Hz, 2H), 2.52-2.60 (m, 4H), 2.74 (t, J = 6.0 Hz, 2H), 2.88 (d, J = 11.5 Hz, 2H), 3.63 (t, J = 6.0 Hz, 2H), 3.66 (s, 2H), 3.84 (s, 3H), 3.97-4.05 (m, 1H), 4.70 (s, 2H), 5.94 (d, J = 7.8 Hz, 1H), 7.02 (ddd, J = 8.3, 2.8, 0.9 Hz, 1H), 7.23 (d, J = 7.8 Hz, 1H), 7.29-7.34 (m, 2H), 7.44 (d, J = 8.3 Hz, 1H), 7.47 (dd, J = 8.3, 1.8 Hz, 1H), 7.71 (s, 1H), 7.74 (s, 1H), 7.78 (d, J = 9.2 Hz, 1H), 7.79 (d, J = 9.2 Hz, 1H); ESI/APCI MS m/z 502, (M+H)⁺.

### Example 37: Synthesis of N-{1-[(7-{[2-(dimethylamino)ethoxy]methyl}-2-naphthyl)methyl]piperidin-4-yl}-3-methoxybenzamide

The title compound (58.0 mg) was obtained from the compound obtained in Example 35 (1.00 g) and 2 M Me₂NH/THF solution (1.12 mL) by the same method as in Example 36.
¹H NMR (600 MHz, CDCl₃, δ): 1.53-1.61 (m, 2H), 1.99-2.05 (m, 2H), 2.22 (t, J = 11.0 Hz, 2H), 2.27 (s, 6H), 2.55 (t, J = 5.7 Hz, 2H), 2.88 (d, J = 11.0 Hz, 2H), 3.58 (t, J = 5.7 Hz, 2H), 3.66 (s, 2H), 3.84 (s, 3H), 3.98-4.05 (m, 1H), 4.69 (s, 2H), 5.94 (d, J = 8.3 Hz, 1H), 7.02 (dd, J = 8.3. 2.5 Hz, 1H), 7.23 (d, J = 7.8 Hz, 1H), 7.29-7.34 (m, 2H), 7.46 (ddd, J = 14.4, 8.3, 1.6 Hz, 2H), 7.71 (s, 1H), 7.74 (s, 1H), 7.78 (d, J = 8.7 Hz, 1H), 7.80 (d, J = 8.7 Hz, 1H); ESI/APCI MS m/z 476, (M+H)⁺.

### Example 38: Synthesis of N-[1-({7-[(dimethylamino)methyl]-2-naphthyl}methyl)piperidin-4-yl]-3-methoxybenzamide

The title compound (97.0 mg) was obtained from the compound obtained in Example 5 (250 mg) and a 5.6 M Me₂NH/EtOH (125 µL) by the same method as in Step 1-5.
¹H NMR (600 MHz, CDCl₃, δ): 1.53-1.71 (m, 2H), 1.99-2.06 (m, 2H), 2.20-2.28 (m, 2H), 2.30 (s, 6H), 2.86-2.93 (m, 2H), 3.60 (s, 2H), 3.67 (s, 2H), 3.84 (s, 3H), 3.98-4.05 (m, 1H), 5.92-5.96 (m, 1H), 7.02 (dd, J = 8.3, 2.8 Hz, 1H), 7.23 (d, J = 7.8 Hz, 1H), 7.29-7.34 (m, 2H), 7.46 (t, J = 8.3 Hz, 2H), 7.70 (s, 2H), 7.78 (dd, J = 8.5, 2.1 Hz, 2H); ESI MS m/z 432, (M+H)⁺.

The compounds of Example 39 to Example 62 were obtained by the same method as in Example 38.

**[Table 3-1]**

| Example No. | Compound name | physical data |
|---|---|---|
| **39** | 3-Methoxy-N-(1-{[7-(pyrrolidin-1-ylmethyl)-2-naphthyl]methyl}piperidin-4-yl)benzamide | **¹H NMR (600 MHz, CDCl₃, δ): 1.52-1.62 (m, 2H), 1.77-1.84 (m,** 4H), **1.99-2.05 (m, 2H). 2.19-2.26 (m, 2H), 2.52-2.61 (m, 4H), 2.85-2.91 (m, 2H), 3.66 (s, 2H), 3.78 (s, 2H), 3.84 (s, 3H), 3.97 4.05 (m, 1H), 5.91-5.95 (m, 1H), 7.02 (dd, J = 7.3, 2.3 Hz, 1H), 7.22-7.24 (m, 1H), 7.31 (d, J = 8.3 Hz 1H), 7-32-7.33 (m, 1H), 7.43-7.49 (m, 2H), 7.70 (s, 1H), 7.73 (s, 1H), 7.77 (d, J = 8.3 Hz, 2H); ESI MS m/z 458, (M+H)⁺.** |
| **40** | N-{1-[(7-{[(2-Hydroxyethyl)(methyl)amino]methyl}-2-naphthyl)methyl]piperidin-4-yl}-3-methoxybenzamide | **¹H NMR (600 MHz, CDCl₃, δ): 1.69-1.81 (m, 2H), 2.02-2.09 (m, 2H), 2.30-2.39 (m, 5H), 2.70-2.15 (m, 2H), 2.96-3.05 (m, 2H), 3.67-3.71 (m, 2H), 3.77 (s, 2H), 3.80 (s, 2H), 3.84 (s, 3H), 4.02-4.09 (m, 1H), 6.00-6.05 (m, 1H), 7.00-7.04 (m, 1H), 7.22-7.25 (m, 1H), 7.30-7.34 (m, 2H), 7.47-7.51 (m, 1H), 7.52-7.55 (m, 1H), 7.74 (s, 1H), 7.78 (s, 1H), 7.81 (d, J = 8.7 Hz, 2H); ESI MS m/z 462, (M+H)⁺.** |
| **41** | 3-Methoxy-N-(1-{[7-(Piperidin-1-ylmethyl)-2-naphthyl]methyl} piperidin-4-yl)benzamide | **¹H NMR (600 MHz, CDCl₃, δ); 1.40-1.47 (m, 2H), 1.53-1.63 (m,** 8H), **1.99-2.05 (m, 2H), 2.20-2.26 (m, 2H), 2.37-2.48 (m, 2H), 2.86-2.91 (m, 2H), 3.62 (s, 2H), 3.66 (s, 2H), 3.84 (s, 3H), 3.98-4.05 (m, 1H), 5.91-5.95 (m, 1H), 7.02 (dd, J = 8.3, 2.8 Hz, 1H). 7.22-7.25 (m, 1H), 7.31 (d, J = 8.3 Hz, 1H), 7.32-7.33 (m, 1H). 7.45 (t, J = 7.6 Hz, 2H), 7.70 (s. 2H), 7.76 (d, J = 3.7 Hz, 1H), 7.77 (d, J = 3.7 Hz, 1H); ESI MS m/z 472, (M+H)⁺.** |
| **42** | 3-Methoxy-N-[1-({7-[(tetrahydro-2H-pyran-4-ylamino)methyl]-2-naphtlyl}methyl)piperidin-4-yl]benzamide | **¹H NMR (600 MMz, CDCl₃ δ): 1.44-1.64 (m, 4H), 1.86-1.91 (m, 2H), 1.99-2.06 (m, 2H), 2.20-2.28 (m, 2H), 2.74-2.80 (m, 1H), 2.86-2.93 (m, 2H), 3.35-3.41 (m, 2H), 3.67 (s, 2H), 3.84 (s, 3H), 3.95-4.00 (m, 4H), 3.97-4.05 (m, 1H), 5.93-5.97 (m, 1H), 7.02 (dd, J = 8.3, 2.8 Hz, 1H), 7.22-7.25 (m, 1H), 7.30-7.34 (m, 2H), 7.42-7.45 (m, 1H), 7.45-7.48 (m, 1H), 7.71 (s, m), 7.73 (s, 1H), 7.78 (d, J = 4.6 Hz, 1H), 7.79 (d, J = 5.0 Hz, 1H); ESI MS m/z 488, (M+H)⁺.** |
| **43** | 3-Methoxy-N-{1-[(7-{[(tetrahydrofuran-2-ylmethyl)amino]methyl}-2-naphthyl)methyl]pipeeridin-4-yl}benzamide | **¹H NMR (600 MHz, CDCl₃, δ): 1.52-1.71 (m, 3H), 1.84-1.90 (m, 2H), 1.93-1.99 (m, 1H), 2.00-2.05 (m, 2H), 2.21-2.29 (m, 2H), 2.66-2.71 (m, 1H), 2.73-2.77 (m, 1H), 2.87-2.94 (m, 2H), 3.68 (s, 2H), 3.72-3.76 (m, 1H), 3.81-3.86 (m, 4H), 3.99 (s, 2H), 4.00-4.08 (m, 2H), 5.93-5.98 (m, 1H), 7.02 (dd, J = 8.3, 2.8 Hz, 1H), 7.22-7.25 (m, 1H), 7.31 (d, J = 8.3 Hz, 1H), 7.32-7.33 (m, 1H), 7.43-7.48 (m, 2H), 7.70 (s, 1H), 7.74 (s, 1H), 7.76-7.77 (m, 1H), 7.78 (d, J = 1.8 Hz, 1H); ESI MS m/z 488, (M+H)⁺.** |

**[Table 3-2]**

| Example No. | Compound name | Physical data |
|---|---|---|
| **44** | 3-Methoxy-N-[1-({7-[(4-methylpiperazin-1-yl)methyl]-2-naphthyl}methyl)piperidin-4-yl]benzamide | **¹H NMR (600 MHz, CDCl₃,** δ**): 1.50-1.63 (m, 2H), 1.99-2.05 (m, 2H), 2.20-2.27 (m, 2H), 2.31 (s, 3H), 2.37-2.72 (m, 8H), 2.86-2.92 (m, 2H), 3.66 (s, 4H), 3.84 (s, 3H), 3.98-4.05 (m, 1H), 5.91-5.95 (m, 1H), 7.00-7.03 (m, 1H), 7.22-7.24 (m, 1H), 7.30-7.33 (m, 2H), 7.44-7.47 (m, 2H), 7.70 (s, 2H), 7.76 (d, J = 1.8 Hz, 1H), 7.78 (d, J = 1.8 Hz, 1H); ESI MS m/z 487, (M+H)⁺.** |
| **45** | N-[1-({7-[(3-Hydroxypyrrolidin-1-yl)methyl]-2-naphthyl}methyl)piperidin-4-yl]-3-methoxybenzamide | **¹H NMR (600 MHz, CDCl₃,** δ**): 1.53-1.61 (m, 2H), 1.72-1.8.0 (m, 1H), 1.99-2.05 (m, 2H), 2.16-2.26 (m, 3H), 2.33-2.39 (m, 1H), 2.59 (dd, J = 9.6, 5.0 Hz, 1H), 2.72 (d, J = 9.6 Hz, 1H), 2.85-2.93 (m, 3H), 3.66 (s, 2H), 3.78 (s, 2H), 3.84 (s, 3H), 3.96-4.05 (m, 1H), 4.31-4.37 (m, 1H), 5.95 (d, J = 7.8 Hz, 1H), 7.02 (dd, J = 7.8 2.3 Hz, 1H), 7.23 (d, J = 7.8 Hz, 1H), 7.28-7.34 (m, 2H), 7.45 (d, J = 8.3 Hz, 2H), 7.70 (d, J = 9.2 Hz, 2H), 7.77 (d, J = 8.7 Hz, 2H); ESI MS m/z 496, (M+Na)⁺.** |
| **46** | 3-Methoxy-N-{1-[(7-{[(2-methoxyethyl)(methyl)amino]methyl}-2-naphthyl)methyl]piperidin-4-yl}benzamide | **¹H NMR (600 MHz, CDCl₃,** δ**): 1.53-1.62 (m, 2H), 1.99-2.05 (m, 2H), 2.23 (t, J = 10.8 Hz, 2H), 2.30 (s, 3H), 2.64 (t, J = 6.0 Hz, 2H), 2.89 (d, J = 11.0 Hz, 2H), 3.34 (s, 3H), 3.53 (t, J = 6.0 Hz, 2H), 3.66 (s, 2H), 3.70 (s, 2H), 3.84 (s, 3H), 3.97-4.06 (m, 1H), 5.95 (d, J = 7.8 Hz, 1H), 7.02 (dd, J = 7.8, 2.3 Hz, 1H), 7.23 (d, J = 7.8 Hz, 1H), 7.29-7,34 (m, 2H), 7.41-7.48(m, 2H), 7.70 (d, J = 5.5 Hz, 2H), 7.77 (d, J = 8.3 Hz, 2H); ESI MS m/z 498, (M+Na)⁺.** |
| **47** | N-{1-[(7-{[4-(hydroxymethyl)piperidin-1-yl]methyl}-2-naphthyl)methyl]piperidin-4-yl}-3-methoxybenzamide | **¹H NMR (600 MHz, CDCl₃,** δ**): 1.26-1.35 (m, 2H), 1.43-1.66 (m, 3H), 1.71 (d, J = 11.9 Hz, 2H), 1.96-2.05 (m, 4H), 2.18-2.26 (m, 2H), 2.89 (d, J = 12.4 Hz, 2H), 2.95 (d, J = 11.9 Hz, 2H), 3.49 (d, J = 6.9 Hz, 2H), 3.64 (s, 2H), 3.66 (s, 2H), 3.84 (s, 3H), 3.97-4.05 (m, 1H), 5.95 (d, J = 8.3 Hz, 1H), 7.02 (dd, J = 8.3, 2.8 Hz, 1H), 7.23 (d, J = 7.8 Hz, 1H), 7.29-7.34 (m, 2H), 7.42-7.47 (m, 2H), 7.70 (s, 2H), 7.74-7.79 (m, 2H); ESI MS m/z 524, (M+Na)⁺.** |
| **48** | 3-Methoxy-N-{1-[(7-{[(2-pyrrolidin-1-ylethyl)amino]methyl}-2-naphthyl)methyl]piperidin-4-yl}benzamide | **¹H NMR (600 MHz, CDCl₃,** δ**): 1.53-1.61 (m, 2H), 1.65-1.80 (m, 4H), 1.99-2.05 (m, 2H), 2.19-2.26 (m, 2H), 2.50 (s, 4H), 2.64 (t, J = 6.2 Hz, 2H), 2.78 (t J = 6.4 Hz, 2H), 2.85-2.91 (m, 2H), 3.66 (s, 2H), 3.84 (s, 3H), 3.97 (s, 2H), 3.98-4.05 (m, 1H), 5.91-5.95 (m, 1H), 7.00-7.03 (m, 1H), 7.22-7.25 (m, 1H), 7.31 (d, J = 7.8 Hz, 1H), 7.32-7.34 (m, 1H); 7-42-7.46 (m, 2H), 7.69 (s, 1H), 7.72 (s, 1H), 7.76 (d, J = 3.2 Hz, 1H), 7.78 (d, J = 3.7 Hz, 1H); ESI MS m/z 501, (M+H)⁺.** |

**[Table 3-3]**

| Example No. | Compound name | Physical data |
|---|---|---|
| **49** | N-(1-{[7-({[2-(Dimethylamino)ethyl]amino}methyl)-2-naphthyl]methyl}piperidin-4-yl)-3-methoxybenzamide | **¹H NMR (600 MHz, CDCl₃,** δ**): 1.52-1.61 (m, 2H), 1.991-2.05 (m, 2H), 2.18-2.26 (m, 8H), 2.45 (t, J = 6.2 Hz, 2H), 2.72 (t, J = 6.2 Hz, 2H), 2.88 (d, J = 11.0 Hz, 2H), 3.66 (s, 2H), 3.84 (s, 3H), 3.96 (s, 2H), 3.98-4.05 (m, 1H), 5.93 (d, J = 7.8 Hz, 1H), 7.02 (dd, J = 7.8, 2.3 Hz, 1H), 7.23 (d, J = 7.8 Hz, 1H), 7.29-7.34 (m, 2H), 7.41-7.46 (m, 2H), 7.69 (s, 1H), 7.72 (s, 1H), 7.75-7.79 (m, 2H); ESI MS m/z 497, (M+Na)⁺.** |
| **50** | 3-Methoxy-N-{1-[(7-{[(2-morpholin-4-ylethyl)amino]methyl}-2-naphthyl)methyl]piperidin-4-yl}benzamide | **¹H NMR (600 MHz, CDCl₃,** δ**): 1.55-1.98 (m, 4H), 2.01-2.07 (m, 2H), 2.23-2.31 (m, 2H), 2.38-2.45 (m, 2H), 2.55 (t, J = 6.0 Hz, 2H), 2.77 (t, J = 6.0 Hz, 2H), 2.88-2.95 (m, 2H), 3.67-3.73 (m, 6H), 3.86 (s, 3H, 3.99-4.07 (m, 3H), 5.95-5.99 (m, 1H), 7.03 (dd, J = 8.3, 2.3 Hz, 1H), 7.25 (d, J = 7.3 Hz, 1H), 7.31-7.35 (m, 2H), 7.43-7.47 (m, 1H), 7,47-7.50 (m, 1H), 7.73 (s, 1H), 7.75 (s, 1H), 7.78-7.82 (m, 2H); ESI MS m/z 539, (M+Na)⁺.** |
| **51** | N-{1-[(7-{[4-(2-Hydroxyethyl)piperazin-1-yl]methyl}-2-naphthyl)methy]piperidin-4-yl}-3-methoxybenzamide | **¹H NMR (600 MHz, CDCl₃,** δ**): 1.50-1.71 (m, 4H), 2.00-2.06 (m, 2H), 2.21-2.29 (m, 2H), 2.46-2.70 (m, 8H), 2.87-2.94 (m, 2H), 3.59-3.63 (m, 2H), 3.67 (s, 2H), 3.68 (s, 2H), 3.84 (s, 3H), 3.98-4.06 (m, 1H), 5.92-5.96 (m, 1H), 7.02 (dd, J = 8.3, 2.8 Hz, 1H), 7.23 (d, J = 7.3 Hz, 1H), 7.29-7.34 (m, 2H), 7.44-7.49 (m, 2H), 7.69-7.72 (m, 2H), 7.77 (d, J = 2.3 Hz, 1H), 7.78 (d, J = 2.8 Hz, 1); ESI MS m/z 539, (M+Na)⁺.** |
| **52** | N-{1-[(7-{[[2-(Dimethylamino)ethyl](methyl)amino] methyl}-2-naphthyl)methyl]piperidin-4-yl}-3-methoxybenzamide | **¹H NMR (600 MHz, CDCl₃,** δ**): 1.52-1.61 (m, 2H), 1.99-2.05 (m, 2H), 2.17-2.29 (m, 2H), 221 (s, 6H), 2.27 (s, 3H), 2.42-2.49 (m, 2H), 2.50-2.56 (m, 2H), 2.88 (d, J = 11.9 Hz, 2H), 3.65 (s, 2H), 3.66 (s, 2H), 3.84 (s, 3H), 3.97-4.06 (m, 1H), 5.93 (d, J = 7.3 Hz, 1H), 7.00-7.03 (m, 1H), 7.23 (d, J = 7.8 Hz, 1H), 7.29-7.34 (m, 2H), 7.41-7.48 (m, 2H), 7.69 (s, 2H), 7.74-7.78 (m, 2H); ESI MS m/z 489, (M+H)⁺.** |
| **53** | N-[1-({7-[(4-Hydroxypiperidin-1-yl)methyl]-2-naphthyl}methyl)piperidin-4-yl]-3-methoxybenzamide | **¹H NMR (600 MHz, CDCl₃,** δ**): 1.51-1.66 (m, 4H), 1.82-1.94 (m, 2H), 1.98-2.06 (m, 2H), 2.13-2.28 (m, 4H), 2.71-2.84 (m, 2H), 2.89 (d, J = 11.5 Hz, 2H), 3.61-3.74 (m, 1H), 3.66 (s, 4H), 3.84 (s, 3H), 3.97-4.06 (m, 1H), 5.94 (d, J = 7.8 Hz, 1H), 7.02 (dd, J = 7.8, 2.3 Hz, 1H), 7.23 (d, J = 7.8 Hz, 1H), 7.29-7.33 (m, 2H), 7.45 (d, J = 8.3 Hz, 2H), 7.70 (s, 2H), 7.77 (d, J = 8.3 Hz, 2H); ESI MS m/z 488, (M+H)⁺.** |

**[Table 3-4]**

| | | |
|---|---|---|
| Example No. | Compound name | Physical data |
| **54** | 3-Methoxy-N-{1-[(7-{[(3-methylbutyl)amino]methyl}-2-naphthyl)methyl]piperidin-4-yl}benzamide | **¹H NMR (600 MHz, CDCl₃,** δ**): 0.88 (d, J = 6.4 Hz, 6H), 1.43 (q, J = 7.3 Hz, 2H), 1.52-1.68 (m, 3H), 1.99-2.04 (m, 2H), 2.19-2.26 (m, 2H), 2.67 (t, J = 7.3 Hz, 2H), 2.88 (d, J = 11.5 Hz, 2H), 3.66 (s, 2H), 3.84 (s, 3H), 3.94 (s, 2H), 3.97-4.06 (m, 1H), 5.94 (d, J = 7.8 Hz, 1H), 7.00-7.03 (m, 1H), 7.23 (d, J = 7.3 Hz, 1H), 7.28-7.34 (m, 2H), 7.43 (dd, J = 8.3, 1.8 Hz, 1H), 7.45 (d, J = 8.3 Hz, 1H), 7.70 (s, 1H), 7.72 (s, 1H), 7.75-7.79 (m, 2H); ESI MS m/z 474, (M+H)⁺.** |
| **55** | N-(1-{[7-({[3-(Dimethylamino)propyl]amino}methyl)-2-naphthyl]methyl}piperidin-4-yl)-3-methoxybenzamide | **¹H NMR (600 MHz, CDCl₃,** δ**): 1.52-1.61 (m, 2H), 1.68-1.74 (m, 2H), 1.99-2.05 (m, 2H), 2.19-2.26 (m, 8H), 2.32 (t, J = 7.1 Hz, 2H), 2.72 (t, J = 7.1 Hz, 2H), 2.85-2.91 (m, 2H), 3.66 (s, 2H), 3.84 (s, 3H), 3.95 (s, 2H), 3.97-4.05 (m, 1H), 5.91-5.9*5* (m, 1H), 7.00-7.03 (m, 1H), 7.23 (d, J = 7.8 Hz, 1H), 7.29-7.34 (m, 2H), 7.40-7-44 (m, 1H), 7.44-7.47 (m, 1H), 7.69 (s, 1H), 7.72 (s, 1H), 7.77 (d, J = 5.0 Hz, 1H), 7.78 (d, J = 5.5 Hz, 1H); ESI MS m/z 489, (M+H)⁺.** |
| **56** | N-(1-{[7-({[4-(Dimethylamino)butyl]amino}methyl)-2-naphthyl]methyl}piperidin-4-yl)-3-methoxybenzamide | **¹H NMR (600 MHz, COCl₃,** δ**): 1.47-1.71 (m, 6H), 1.99-2.05 (m, 2H), 2.14-2.29 (m, 10H), 2.61-2.72 (m, 2H), 2.85-2.91 (m, 2H), 3.66 (s, 2H), 3.84 (s, 3H), 3.96 (s, 2H), 3.97-4.05 (m, 1H), 5.91- 5.96 (m, 1H), 7.00-7.03 (m, 1H), 7.23 (d, J = 7.8 Hz, 1H), 7.29-7.34 (m, 2H), 7.45 (t, J = 9.2 Hz, 2H), 7.70 (s, 1H), 7.73 (s, 1H), 7.76-7.80 (m, 2H); ESI MS m/z 503, (M+H)⁺.** |
| **57** | 3-Methoxy-N-{1-[(7-{[(3-pyrrolidin-1-ylpropyl)amino]methyl}-2-naphthyl)methyl]piperidin-4-yl}benzamide | **¹H NMR (600 MHz, CDCl₃,** δ**): 1.49-1.58 (m, 2H), 1.58-1.79 (m, 3H), 1.96-2.02 (m, 2H), 2.16-2.23 (m, 2H), 2.45-2.54 (m, 4H), 2.68-2.74 (m, 2H), 2-82-2.88 (m, 2H), 3.62 (s, 2H), 3.81 (s, 3H), 3.92 (s, 2H), 3.94-4.02 (m, 1H), 5.88-5.92 (m, 1H), 6.97-7.00 (m, 1H), 7.19-7.21 (m, 1H), 7.28 (d, J = 8.3 Hz, 1H), 7.29-7.30 (m, 1H), 7.38 (dd, J = 8.3, 1.8 Hz, 1H), 7.40-7.44 (m, 1H), 7.66 (s, 1H), 7.68 (s, 1H), 7.74 (d, J = 4.1 Hz, 1H), 7.75 (d, J = 4.1 Hz, 1H); ESI MS m/z 515, (M+H)⁺.** |
| **58** | 3-Methoxy-N-(1-{[7-(morpholin-4-ylmethyl]-2-naphthyl]methyl}piperidin-4-yl)benzamide dihydrochloride | **¹H NMR (600 MHz, DMSO-d₆** δ**): 1.89-2.01 (m, 3H), 2.01-2.11 (m, 1H), 3.04-3.17 (m, 4H), 3.19-3.26 (m, 2H), 3.32-3.40 (m, 2H), 3.74-3.81 (m, 5H), 3.86-4.15 (m, 3H), 4.41-4.53 (m, 4H), 7.03-7.11 (m, 1H), 7.32 (t, J = 8.0 Hz, 1H), 7.35-7.38 (m, 1H), 7.39-7.47 (m, 1H), 7.78-7.85 (m, 2H), 8.01-8.06 (m, 2H), 8.15-8.21 (m, 2H), 8.34-8.55 (m, 1H), 10.78-11.04 (m, 1H), 11.32-11.46 (m, 1H); ESI MS m/z 474 [M (free)+H]⁺.** |

**[Table 3-5]**

| Example No. | Compound name | Physical data |
|---|---|---|
| **59** | N-{1-[(7-{[(Cyclopropylmethyl)amino]methyl}-2-naphthyl)methyl]piperidin-4-yl}-3-methoxybenzamide | **¹H NMR (600 MHz, CDCl₃,** δ**): 0.08-0.12 (m, 2H), 0.46-0.50 (m, 2H), 0.97-1.05 (m, 1H), 1.52-1.70 (m, 2H), 1.99-2.05 (m, 2H), 2.19-2.26 (m, 2H), 2.52 (d, J = 6.9 Hz, 2H), 2.85-2.91 (m, 2H), 3.66 (s, 2H), 3.84 (s, 3H), 3.97 (s, 2H), 3.98-4.05 (m, 1H), 5.92-5.96 (m, 1H), 7.00-7.03 (m, 1H), 7.22-7.25 (m, 1H), 7.31 (d, J = 8.3 Hz, 1H), 7.32-7.34 (m, 1H), 7.41-7.47 (m, 2H), 7.70 (s, 1H), 7.72 (s, 1H), 7.77 (d, J = 4.6 Hz, 1H), 7.78 (d, J = 4.6 Hz, 1H); ESI MS m/z 458, (M+H)⁺.** |
| **60** | 3-Methoxy-N-{1-[(7-{[(2-methoxyethyl)amino]methyl}-2-naphthyl)methyl]piperidin-4-yl}benzamide | **¹H NMR (600 MHz, CDCl₃** δ**): 1.50-1.63 (m, 2H), 1.95-2.06 (m, 2H), 2.23 (t, J = 10.8 Hz, 2H), 2.83 (t, J = 5.0 Hz, 2H), 2.89 (d, J = 11.5 Hz, 2H), 3.35 (s, 3H), 3.53 (t, J = 5.0 Hz, 2H), 3.66 (s, 2H), 3.84 (s, 3H), 3.97 (s, 2H), 398-4.05 (m, 1H), 5.95 (d, J = 7.8 Hz, 1H), 7.02 (dd, J = 7.8, 1.8 Hz, 1H), 7.23 (d, J = 7.8 Hz, 1H), 7.29-7.33 (m, 2H), 7.42-7.46 (m, 2H), 7.70 (s, 1H), 7.73 (s, 1H), 7.75-7.80 (m, 2H); ESI MS m/z 462, (M+H)⁺.** |
| **61** | 3-Methoxy-N-{1-[(7-{[(2-piperidin-1-ylethyl)amino]methyl}-2-naphthyl)methyl]piperidin-4-yl}benzamide | **¹H NMR (600 MHz, CDCl₃,** δ**): 1.39-1.46 (m, 2H), 1.53-1.61 (m, 6H), 1.68-1.79 (m, 1H), 1.99-2.05 (m, 2H), 2.20-2.26 (m, 2H), 2.33-2.44 (m, 3H), 2.50 (t, J = 6.0 Hz, 2H), 2.75 (t, J = 6.2 Hz, 2H), 2.85-2.91 (m, 2H), 3.66 (s, 2H), 3.84 (s, 3H), 3.96 (s, 2H), 3.98-4.05 (m, 1H), 5.91-5.96 (m, 1H), 7.00-7.03 (m, 1H), 7.22-7.25 (m, 1H), 7.31 (d, J = 8.3 Hz, 1H), 7.32-7.33 (m, 1H), 7.41-7.47 (m, 2H), 7.70 (s, 1H), 7.72 (s, 1H), 7.77 (d, J = 4.6 Hz, 1H), 7.78 (d, J = 4.6 Hz, 1H); ESI MS m/z 515, (M+H)⁺.** |
| **62** | Benzyl N-{[7-({4-[(3-methoxybenzoyl)amino]piperidin-1-yl}methyl)-2-naphthyl]methyl}-beta-alaninate | **¹H NMR (600 MHz, CDCl₃,** δ**): 1.52-1.61 (m, 2H), 1.98-2.04 (m, 2H), 2.22 (t, J = 11.0 Hz, 2H), 2.60 (t, J = 6.4 Hz, 2H), 2.88 (d, J = 11.0 Hz, 2H), 2.95 (t, J = 6.4 Hz, 2H), 3.66 (s, 2H), 3.84 (s, 3H), 3.94 (s, 2H), 3.97-4.05 (m, 1H), 5.12 (s, 2H), 5.94 (d, J = 7.8 Hz, 1H), 7.02 (dd, J = 8.3, 2.8 Hz, 1H), 7.23 (d, J = 7.8 Hz, 1H), 7.28-7.36 (m, 7H), 7.40 (dd, J = 8.3, 1.9 Hz, 1H), 7.45 (d, J = 6.9 Hz, 1H), 7.69 (s, 1H), 7.70 (s, 1H), 7.77 (d, J = 8.3 Hz, 2H); ESI MS m/z 566, (M+H)⁺.** |

### Example 63: Synthesis of 3-methoxy-N-{1-[(7-{[(piperidin-4-ylmethyl)amino]methyl}-2-naphthyl)methyl]piperidin-4-yl}benzamide trihydrochloride

Step 63-1: tert-Butyl 4-[({[7-({4-[(3-methoxybenzoyl)amino]piperidin-1-yl}methyl)-2-naphthyl]methyl}amino)methyl]piperidine-1-carboxylate (560 mg) was obtained from the compound obtained in Example 5 (500 mg) and tert-butyl 4-(aminomethyl)piperidine-1-carboxylate (293 mg) by the same method as in Step 1-5.
¹H NMR (600 MHz, CDCl₃, δ): 1.04-1.14 (m, 2H), 1.41 (s, 9H), 1.45-1.65 (m, 3H), 1.67-1.73 (m, 2H), 1.97-2.03 (m, 2H), 2.18-2.25 (m, 2H), 2.52 (d, J = 6.4 Hz, 2H), 2.60-2.72 (m, 2H), 2.84-2.90 (m, 2H), 3.64 (s, 2H), 3.82 (s, 3H), 3.91 (s, 2H), 3.95-4.17 (m, 3H), 5.91-5.95 (m, 1H), 6.98-7.01 (m, 1H), 7.21 (d, J = 7.8 Hz, 1H), 7.28 (d, J = 7.8 Hz, 1H), 7.29-7.31 (m, 1H), 7.38-7.41 (m, 1H), 7.42-7.45 (m, 1H), 7.68 (s, 1H), 7.68 (s, 1H), 7.75 (d, J = 2.3 Hz, 1H), 7.76 (d, J = 2.8 Hz, 1H); ESI MS m/z 601, (M+H)⁺.

Step 63-2: 4 M HCl/EtOAc solution (5.00 mL) was added to a suspension of the compound obtained in Step 63-1 (545 mg) in EtOAc (5.00 mL) with ice-cooling, and the mixture was stirred at room temperature for three hours. The generated solid was collected by filtration to obtain the title compound (518 mg, colorless solid).
¹H NMR (600 MHz, DMSO-d₆, δ): 1.32-1.42 (m, 2H), 1.88-2.00 (m, 5H), 2.01-2.10 (m, 2H), 2.76-2.87 (m, 4H), 3.05-3.14 (m, 2H), 3.18-3.26 (m, 2H), 3.31-3.38 (m, 2H), 3.74-3.80 (m, 3H), 3.93-4.14 (m, 1H), 4.27-4.33 (m, 2H), 4.40-4.53 (m, 2H), 7.03-7.11 (m, 1H), 7.32 (t, J = 7.8 Hz, 1H), 7.34-7.38 (m, 1H), 7.39-7.47 (m, 1H), 7.76-7.83 (m, 2H), 7.98-8.04 (m, 2H), 8.11-8.21 (m, 2H), 8.35-8.56 (m, 1H), 8.66-8.76 (m, 1H), 8.82-8.95 (m, 1H), 9.45-9.61 (m, 1H), 10.86-11.10 (m, 1H); ESI MS m/z 501, [M (free)+H]⁺.

The compounds of Example 64 to Example 70 were obtained by the same method as in Example 63.

**[Table 4-1]**

| Example No. | Compound name | Physical data |
|---|---|---|
| 64 | N-{1-[(7-{[(2-Aminoethyl)amino]methyl}-2-naphthyl)methyl]piperidin-4-yl}-3-methoxybenzamide trihydrochloride 7.71-7.74 (m, 1H), | **¹H NMR (600 MHz, CD₃OD, δ): 1.86-1.98 (m, 2H), 2.11-2.24 (m, 2H), 3.17-3.25 (m, 2H), 3.32-3.47 (m, 4H), 3.50-3.61 (m, 2H), 3.73-3.83 (m, 3H), 4.05-4.25 (m, 1H), 4.44-4.59 (m, 4H), 7.01-7.10 (m, 1H), 7.26-7.42 (m, 3H), 7.68 (d, J = 8.7 Hz, 1H), 8.00-8.05 (m, 2H), 8.16 (s, 1H), 8.19 (s, 1H); ESI MS m/z 447 [M (free)+H]⁺.** |
| 65 | N-{1-[(7-{[4-(Aminomethyl)piperidin-1-yl]methyl}-2-naphthyl)methyl]piperidin-4-yl}-3-methoxybenzamide trihydrochloride | **¹H NMR (600 MHz, DMSO-d₆, δ); 1.32-3.44 (m, 18H), 3.69-4.17 (m, 5H), 4.36-4.58 (m, 4H), 6.97-7.13 (m, 1H), 7.25-7.51 (m, 3H), 7.80-7.85 (m, 2H), 7.99-8.22 (m, 7H), 8.36-8.55 (m, 1H), 10.85-11.10 (m, 1H); ESI MS m/z S01 [M (free)+H]⁺.** |
| 66 | 3-Methoxy-N-(1-{[7-(piperazin-1-ylmethyl)-2-naphthyl]methyl}piperidin-4-yl)benzamide trihydrochloride | **¹H NMR (600 MHz, DMSO-d_{6,} δ): 1.66-3,66(m, 15H), 3.71-3.82 (m, 3H), 3.90-4.67 (m, 5H), 6.97-7.12(m, 1H), 7.27-7.47 (m, 3H), 7.71-8.27 (m, 6H), 8.34-8.59 (m,1H), 9.67 (brs, 2H). 10.91-11.14 (m, 1H); ESI MS m/z 473 [M (free)+H]⁺.** |

**[Table 4-2]**

| Example No. | Compound name | Physical data |
|---|---|---|
| 67 | 3-Methoxy-N-(1-{[7-({[2-methylamino)ethyl]amino}methyl)-2-naphthyl]methyl}piperidin-4-yl)benzamide trihydrochloride | **¹H NMR (600 MHz, DMSO-d₆ δ): 1.61-3.41 (m, 13H), 3.65-3.78 (m,4H), 3.86-4.11 (m,1H), 4.26-4.53 (m, 5H), 6.94-7.12 (m, 1H), 7.21-7.47 (m, 3H), 7.63-7.81 (m, 2H), 7.91-8.18 (m, 4H), 8.28-8.57 (m, 1H), 9.28 (brs, 2H), 9.86 (brs, 2H), 10.84-11.04 (m, 1H); ESI MS m/z 461 [M(free)+H]⁺.** |
| 68 | N-[1-({7-[(3-Aminopyrrolidin-1-yl)methyl]-2-naphthyl}methyl)piperidin-4-yl]-3-methoxybenzamide trihydrochloride | **¹H NMR (600 MHz, DMSO-d₆, δ): 1.55-1.66 (m, 1H), 1.89-2.00 (m, 4H), 2.00-2.10 (m, 1H), 2.14-2..32 (m, 1H), 3.06-3.15 (m, 2H), 3.18-3.32 (m, 2H), 3.32-3.41 (m, 2H), 3.48-3.75 (m, 2H), 3.74-3.80 (m, 3,4), 3.86-4.15 (m, 2H), 4.40-4.53 (m, 2H), 4.56 4.74 (m,2H), 7.03-7.11 (m, 1H), 7.32 (t, J = 8.0 Hz, 1H), 7.34-7.38 (m, 1H), 7.39-7.47 (m, 1H), 7.76-7.87 (m, 2H), 8.01-8.07 (m, 2H), 8.12-8.21 (m, 2H), 8.34-8.56 (m, 2H), 8.62-8.74 (m, 1H), 10.77-11.08 (m, 1H), 11.47-11.93 (m, 1H): ESI MS m/z 473 [M (free)+H]⁺.** |
| 69 | 3-Methoxy-N-[1-({7-[(pyrrolidin-3-ylamino)methyl]-2-naphthyl}methyl)piperidin-4-yl]benzamide trihydrochloride | **¹H NMR (600 MHz, DMSO-d_{6,} δ): 1.82-3.58 (m, 12H), 3.72-3.82 (m, 4H), 3.84-4.15 (m, 2H), 4.29-4.54 (m, 5H), 6.96-7.14 (m, 1H), 7.26-8.21 (m, 8H), 8.32-8.57 (m, 1H), 9.33-9.61 (m, 2H), 10.00-10.23 (m,2H), 10.82-11.06 (m, 1H); ESI MS m/z 473 [M (free)+H]⁺.** |
| 70 | 3-Methoxy-N-{1-[(7-{[(pyrrolidin-2-ylmethyl)amino]methyl}-2-naphthyl)methyl]piperidin-4-yl}benzamide trihydrochloride | **¹H NMR (600 MHz, DMSO-d_{6,} δ): 1.61-3.54 (m, 16H), 3.66-3.82 (m,3H), 3.87-4.19 (m,2H), 4.27-4.57 (m,4H), 6.97-7.14 (m, 1H), 726-7.50 (m, 3H), 7.71-7.88 (m, 2H), 7.96-8.22 (m, 4H), 8.32-8.57 (m, 1H), 9.36-9.54 (m, 2H), 9.77-9.99 (m, 2H), 10.76-10.99 (m,1H); ESI MS m/z 487 [M (free)+H]⁺.** |

### Example 71: Synthesis of N-{[7-({4-[(3-methoxybenxoyl)amino]piperidin-1-yl}methyl)-2-naphthyl]methyl}-beta-alanine

2 M aqueous NaOH solution (1.38 mL) was added to a solution of the compound obtained in Example 62 (311 mg) in THF (3.10 mL) at room temperature and the mixture was stirred at room temperature for one hour. MeOH (3.10 mL) was added to the reaction suspension, and the mixture was further stirred at room temperature for one hour. The organic solvent was concentrated under reduced pressure, followed by adjustment to pH = 1 with 1 M aqueous HCl with ice-cooling. After washing with CHCl₃ twice, the aqueous layer was adjusted to pH = 7 with 6 M aqueous NaOH solution, followed by stirring at room temperature for one hour. The generated solid was collected by filtration and washed with H₂O. IPA was added to the resulting solid at room temperature, followed by stirring for one hour. The solid was collected by filtration and washed with IPA to obtain the title compound (212 mg).
¹H NMR (600 MHz, DMSO-d₆, δ): 1.53-1.62 (m, 2H), 1.70-1.78 (m, 2H), 2.06 (t, J = 11.0 Hz, 2H), 2.41 (t, J = 6.6 Hz, 2H), 2.81-2.86 (m, 2H), 2.88 (t, J = 6.6 Hz, 2H), 3.62 (s, 2H), 3.70-3.78 (m, 1H), 3.76 (s, 3H), 4.04 (s, 2H), 7.02-7.05 (m, 1H), 7.30-7.35 (m, 2H), 7.36-7.39 (m, 1H), 7.48 (t, J = 8.0 Hz, 2H), 7.74 (s, 1H), 7.82-7.88 (m, 3H), 8.21 (d, J = 7.8 Hz, 1H); ESI MS m/z 476, (M+H)⁺.

### Example 72: Synthesis of 4-methoxy-N-[1-({7-[(2-methoxyethoxy)methyl]-2-naphthyl}methyl)piperidin-4-yl]benzamide

Step 72-1: tert-Butyl [1-({7-[(2-methoxyethoxy)methyl]-2-naphthyl}methyl)piperidin-4-yl]carbamate (62.9 g, yellow solid) was obtained from the compound obtained in Step 1-3 (33.5 g) and tert-butyl piperidin-4-ylcarbamate (27.5 g) by the same method as in Step 1-5.
¹H NMR (600 MHz, CDCl₃, δ): 1.38-1.49 (m, 2H), 1.43 (s, 9H), 1.90 (d, J = 11.0 Hz, 2H), 2.08-2.17 (m, 2H), 2.78-2.88 (m, 2H), 3.38 (s, 3H), 3.43-3.53 (m, 1H), 3.55-3.60 (m, 2H), 3.62 (s, 2H), 3.63-3.66 (m, 2H), 4.39-4.46 (m, 1H), 4.72 (s, 2H), 7.42-7.48 (m, 2H), 7.69 (s, 1H), 7.73-7.83 (m, 3H); ESI/APCI MS m/z 429, (M+H)⁺.

Step 72-2: l-({7-[(2-Methoxyethoxy)methyl]-2-naphthyl}methyl)piperidin-4-amine (43.8 g, yellow oil) was obtained from the compound obtained in Step 72-1 (62.9 g) by the same method as in Step 7-5.
¹H NMR (600 MHz, CDCl₃, δ): 1.35-1.43 (m, 2H), 1.74-1.82 (m, 2H), 2.01-2.09 (m, 2H), 2.62-2.68 (m, 1H), 2.81-2.88 (m, 2H), 3.40 (s, 3H), 3.56-3.60 (m, 2H), 3.62-3.66 (m, 2H), 3.63 (s, 2H), 4.72 (s, 2H), 7.42-7.47 (m, 2H), 7.69 (s, 1H), 7.74 (s, 1H), 7.75-7.81 (m, 2H); ESI/APCI MS m/z 329, (M+H)⁺.

Step 72-3: The title compound (104 mg, colorless solid) was obtained from the compound obtained in Step 72-2 (250 mg) and 4-methoxybenzoic acid (139 mg) by the same method as in Step 7-6.
¹H NMR (600 MHz, CDCl₃, δ): 1.51-1.62 (m, 2H), 1.98-2.05 (m, 2H), 2.18-2.26 (m, 2H), 2.85-2.91 (m, 2H), 3.40 (s, 3H), 3.57-3.60 (m, 2H), 3.64-3.67 (m, 2H), 3.66 (s, 2H), 3.84 (s, 3H), 3.95-4.05 (m, 1H), 4.73 (s, 2H), 5.86 (d, J = 7.8 Hz, 1H), 6.88-6.93 (m, 2H), 7.42-7.49 (m, 2H), 7.68-7.73 (m, 3H), 7.74-7.82 (m, 3H); ESI/APCI MS m/z 463, (M+H)⁺.

The compounds of Example 73 to Example 108 were obtained by the same method as in Example 72.

**[Table 5-1]**

| Example No. | Compound name | Physical data |
|---|---|---|
| 73 | 2-Methoxy-N-[1-({7-[(2-methoxyethoxy)methyl]-2-naphthyl}methyl)piperidin-4-yl]benzamide hydrochloride | **¹H NMR(600 MHz, CDCl₃, δ): 1.53-1.64 (m, 2H), 1.99-2.07 (m, 2H), 2.21-2.31 (m, 2H), 2.77-2.88 (m, 2H), 3.40 (s, 3H), 3.57-3.60 (m, 2H), 3.63-3.66 (m, 2H), 3.67 (s, 2H), 3.91 (s, 3H), 4.03-4.13 (m, 1H), 4.73 (s, 2H), 6.95 (d, J = 8.3 Hz, 1H), 7.06 (t, J = 6.9 Hz, 1H), 7.39-7.49 (m, 3H), 7.72 (s, 1H), 7.76 (s, 1H), 7.77-7.81 (m,2H), 7.82-7.86(m, 1H), 8.19 (dd, J = 7.8, 1.8 Hz, 1H); ESI/APCI MS m/z 463 [M (free)+H]⁺.** |
| 74 | 4-Ethoxy-N-[1-({7-[(2-methoxyethoxy)methyl]-2-naphthyl}methyl)piperidin-4-yl]benzamide | **¹H NMR (600 MHz, COCl₃, δ): 1.42 (t, J = 6.9 Hz, 3H), 1.51-1.62 (m, 2H), 1.99-2.04 (m, 2H), 2.22 (t, J = 10.8 Hz, 2H), 2.88 (d, J = 11.5Hz, 2H), 3.40 (s, 3H), 3.56-3.60 (m, 2H), 3.63-3.66 (m, 2H), 3.66 (s, 2H), 3.96-4.03 (m, 1H), 4.06 (q, J = 6.9 Hz, 2H), 4.72 (s, 2H), 5.86 (d, J = 7.8 Hz, 1H), 6.87-6,91 (m, 2H), 7.43-7.49 (m, 2H), 7.66-7.70(m, 2H), 7.71 (s, 1H), 7.75 (s, 1H), 7.78(d, J = 8.3 Hz, 1H), 7.80 (d, J = 8.3 Hz, 1H); ESI/APCI MS m/z 477, (M+H)⁺.** |
| 75 | 3-Ethoxy-N-[1-({7-[(2-methoxyethoxy)methyl]-2-naphthyl}methyl)piperidin-4-yl]benzamide | **¹H NMR (600 MHz, CDCl₃, δ): 1.41 (t, J = 7.2 Hz, 3H), 1.51-1.62 (m, 2H), 1.99-2.04 (m, 2H), 2.22 (t, J = 10.8 Hz, 2H), 2.88 (d, J = 11.5 Hz, 2H), 3.40 (s, 3H), 3.57-3.60 (m, 2H), 3.63-3.66 (m, 2H), 3.66 (s, 2H), 3.96-4.05 (m, 1H), 4.07 (q, J = 7.2 Hz, 2H), 4.73 (s, 2H), 5.94 (d, J = 7.8 Hz, 1H), 6.98-7.02 (m, 1H), 7.23 (d, J = 7.8 Hz, 1H), 7.28-7.32 (m, 2H), 7.43-7.49 (m, 2H), 7.71 (s, 1H), 7.76 (s, 1H), 7.78 (d, J = 8.7 Hz, 1H), 7.80 (d, J = 8.7Hz, 1H); ESI/APCI MS m/z 477, (M+H)⁺.** |
| 76 | Methyl 3-({[1-({7-[(2-methoxyethoxy)methyl]-2-naphthyl}methyl)piperidin-4-yl]amino}carbonyl)benzoate | **¹H NMR (600 MHz, CDCl₃, δ): 1.57-1.66 (m, 2H), 2.01-2.07 (m, 2H), 2.23 (t, J = 11.0 Hz, 2H), 2.85-2.97 (m, 2H), 3.41 (s, 3H), 3.55-3.61 (m, 2H), 3.64-3.67 (m, 2H), 3.67 (s, 2H), 3.95 (s, 3H), 3.97-4.08 (m, 1H), 4.73 (s, 2H), 6.05 (d, J = 7.8 Hz, 1H), 7.41-7.50 (m, 2H), 7.53 (t, J = 7.8Hz, 1H), 7.72 (s, 1H), 7.71 (s, 1H), 7.78-7.83 (m, 2H), 8.02 (d, J = 7.8 Hz, 1H), 8.16 (d, J = 7.8Hz. 1H), 8.33 (s, 1H); ESI/APCI MS m/z 491, (M+H)⁺.** |
| 77 | 2-Ethoxy-N-[1-({7-[(2-methoxyethoxy)methyl]-2-naphthyl}methyl)piperidin-4-yl]benzamide hydrochloride | **¹H NMR (600 MHz, CDCl₃, δ): 1.50 (t, J = 6.9 Hz, 3H), 1.53-1.63 (m, 2H), 2,01-2.07 (m, 2H), 2.21-2.30 (m, 2H), 2.80-2.90 (m, 2H), 3.40 (s, 3H), 3.56-3.60 (m, 2H), 3.63-3.66 (m, 2H), 3.67 (s, 2H), 3,99-4.11 (m, 1H), 4.16 (q, J = 6.9 Hz, 2H), 4.73 (s, 2H), 6.92 (d, J = 7.6 Hz, 1H), 7.05 (t, J = 7.6 Hz, 1H), 7.37-7.42 (m, 1H), 7.43-7.49 (m, 2H), 7.72 (s. 1H), 7.76 (s, 1H), 7.77-7.82 (m, 2H), 8.06 (d, J = 7.3 Hz, 1H), 8.19 (dd, J = 7.8, 1.8 Hz, 1H); ESI/APCI MS m/z 477 [M (free)+H]⁺.** |

**[Table 5-2]**

| Example No. | Compound name | Physical data |
|---|---|---|
| 78 | 3-Isopropoxy-N-[1-({7-[(2-methoxyethoxy)methyl]-2-naphthyl}methyl)piperidin-4-yl]benzamide | **¹H NMR (600 MHz, CDCl₃, δ): 1.33 (d, J = 6.4 Hz, 6H), 1.53-1.63 (m, 2H), 1.98-2.04 (m, 2H), 2.19-2.26 (m, 2H), 2.84-2.92 (m, 2H), 3.40 (s, 3H), 3.56-3.60 (m, 2H), 3.62-3.66 (m, 2H), 3.67 (s, 2H), 3.96-4.05 (m, 1H), 4.57-4.64 (m, 1H), 4.73 (s, 2H), 5.93 (d, J = 7.8 Hz, 1H), 6.95-7.01 (m, 1H), 7.22 (d, J = 8.3 Hz, 1H), 7.30 (s, 2H), 7.43-7.49 (m, 2H), 7.71 (s, 1H), 7.76 (s, 1H), 7.78 (d, J = 8.3 Hz, 1H), 7.80 (d, J = 8.3 Hz, 1H); ESI/APCI MS m/z 491, (M+H)⁺.** |
| 79 | 4-(Hydroxymethyl)-N-[1-({7-[(2-methoxyethoxy)methyl]-2-naphthyl}methyl)piperidin-4-yl]benzamide | **¹H NMR (600 MHz, CDCl₃, δ): 1.52-1.65 (m, 2H), 1.98-2.03 (m, 2H), 2.17-2.25 (m, 2H), 2.88 (d, J = 11.5 Hz, 2H), 3.38 (s, 3H), 3.53-3.59 (m, 2H), 3.62-3.64 (m, 2H), 3.65 (s, 2H), 3.96-4.05 (m, 1H), 4.71 (s, 2H), 4.73 (s, 2H), 5.94 (d, J = 7.8Hz, 1H), 7.34-7.50 (m, 4H), 7.68-7.81 (m, 6H); ESI/APCI MS m/z 463, (M+H)⁺.** |
| 80 | 3-(Acetylamino)-N-[1-({7-[(2-methoxyethoxy)methyl]-2-naphhthyl}methyl)piperidin-4-yl]benzamide | **¹H NMR (600 MHz, CDC1₃, δ): 1.53-1.68 (m, 2H), 2.00-2.06 (m, 2H), 2.21 (s, 3H), 2.22-2.28 (m, 2H), 2.89-2.95 (m, 2H). 3.42 (s, 3H), 3.59-3.62 (m, 2H), 3.65-3.68 (m, 2H), 3.69 (s, 2H), 3.99- 4.07 (m, 1H), 4.75 (s, 2H), 6.03-6.10 (m, 1H), 7.35 (s, 1H), 7.39 (t, J = 7.8 Hz, 1H), 7.47 (d, J = 8.3 Hz, 2H), 7.49-7.52 (m, 1H), 7.72-7.79 (m, 3H), 7.81 (t, J = 8.9 Hz, 2H), 7.86 (s, 1H); ESI/APCI MS m/z 490, (M+H)⁺.** |
| 81 | 6-Methoxy-N-[1-({7-[(2-methoxyethoxy)methyl]-2-naphthyl}methyl)piperidin-4-yl]nicotinamide | **¹H NMR (600 MHz, CDCl₃, δ): 1.50-1.65 (m, 2H), 1.99-2.06 (m, 2H), 2.19-2.28 (m, 2H), 2.87-2.92 (m, 2H), 3.40 (s, 3H), 3.57-3.60 (m, 2H), 3.63-3.66 (m, 2H), 3.67 (s, 2H), 3.96 (s, 3H), 3.98-4.06 (m, 1H), 4.73 (s, 2H), 5.82-5.86 (m, 1H), 6.75-6.77 (m, 1H), 4.06 (m, 1H), 4.73 (s, 2H), 5.82-5.86 (m, 1H), 6.75-6.77 (m, 1H), 7.45 (dd, J = 8.3, 1.8 Hz, 1H), 7.46-7.49 (m, 1H), 7.72 (s, 1H), 7.75-7.81 (m, 3H), 7.95 (dd, J = 8.5, 2.5 Hz, 1H), 8.53 (d, J = 1.8 Hz, 1H); ESI/APCI MS m/z 464, (M+H)⁺.** |
| 82 | 3-(Dimethylamino)-N-[1-({7-[(2-methoxyethoxy)methyl]-2-naphthyl}methyl)piperidin-4-yl]benzamide | **¹H NMR (600 MHz, CDCl₃, δ): 1.54-1.63 (m, 2H), 1.99-2.05 (m, 2H), 2.20-2.27 (m, 2H), 2.85-2.92 (m, 2H), 2.98 (s, 6H), 3.40 (s, 3H), 3.57-3.60 (m, 2H), 3.63-3.66 (m, 2H), 3.67 (s, 3H), 3.98-4.06 (m, 1H), 4.73 (s, 2H), 5.92-5.97 (m, 1H), 6.81 (dd, J = 8.0, 2.5 Hz, 1H), 6.90-6.93 (m, 1H), 7.15-7.17 (m, 1H), 7.22-7.26 (m, 1H), 7.43-7.46 (m, 1H), 7.46-7.49 (m, 1H), 7.71 (s, 1H), 7.76 (s, 1H), 7.78 (d, J = 8.7 Hz, 1H), 7.80 (d, J = 8.3 Hz, 1H); ESI/APCI MS m/z 476, (M+H)⁺.** |

**[Table 5-3]**

| Example | Compound name | Physical data |
|---|---|---|
| **83** | N-[1-({7-[(2-Methoxyethoxy)methyl]-2-naphthyl}methyl)piperidin-4-yl]-3-(trifluromethyl)benzamide | **¹H NMR (600 MHz, CDCl₃, δ): 1.51-1.68 (m, 2H), 2.01-2.07 (m, 2H), 2.21-2.29 (m, 2H), 2.89-2.96 (m, 2H), 3.40 (s, 3H), 3.57-3.60 (m, 2H), 3.63-3.68 (m, 2H), 3.69 (s, 2H), 4.00-4.08 (m, 1H), 4.73 (s, 2H), 5.95-6.00 (m, 1H), 7.43-7.47 (m, 1H), 7.48 (d, J = 8.3 Hz, 1H), 7.56 (t, J = 7.8 Hz, 1H), 7.71-7.77 (m, 3H), 7.80 (t, J = 8.9 Hz, 2H), 7.92 (d, J = 7.8 Hz, 1H), 7.99 (s, 1H); ESI/APCI MS m/z 501, (M+H)⁺.** |
| **84** | N-[1-({7-[(2-Methoxyethoxy)methyl]-2-naphthyl}methyl)piperidin-4-yl]-3-(trifluoromethoxy)benzamide | **¹H NMR (600 MHz, CDCl₃, δ): 1.52-1.66 (m, 2H), 2.00-2.06 (m, 2H), 2.20-2.27 (m, 2H), 2.88-2.94 (m, 2H), 3.40 (s, 3H), 3.57-3.60 (m, 2H), 3.63-3.66 (m, 2H), 3.68 (s, 2H), 3.98-4.06 (m, 1H), 4.73 (s, 2H), 5.91-5.96 (m, 1H), 7.32-7.36 (m, 1H), 7.44-7.49 (m, 3H), 7.61 (s, 1H), 7.62-7.64 (m, 1H), 7.72 (s, 1H) 7.76 (s, 1H), 7.77-7.82 (m, 2H); ESI/APCI MS m/z 517, (M+H)⁺.** |
| **85** | N-[1-({-7-[(2-Methoxyethoxy)methyl]-2-naphthyl}methyl)piperidin-4-yl]-3-methylbenzamide | **¹H NMR (600 MHz, CDCl₃, δ): 1.51-1.64 (m, 2H), 2.00-2.06 (m, 2H), 2.20-2.28 (m, 2H), 2.39 (s, 3H), 2.86-2.93 (m, 2H), 3.40 (s, 3H), 3.57-3.60 (m, 2H), 3.63-3.66 (m, 2H), 3.61 (s, 2H), 3.98-4.07 (m, 1H), 4.73 (s, 2H), 5.90-5.95 (m, 1H), 7.27-7.32 (m, 2H), 7.43-7.46 (m, 1H), 7.46-7.49 (m, 1H), 7.49-7.51 (m, 1H), 7.56 (s, 1H), 7.72 (s, 1H), 7.76 (s, 1H), 7.77-7.81 (m, 2H); ESI/APCI MS m/z 447, (M+H)⁺.** |
| **86** | 3-Cyano-N-[1-({7-[(2-methoxyethoxy)methyl]-2-naphthyl}methyl)piperidin-4-yl]benzamide | **¹H NMR (600 MHz, CDCl₃, δ): 1.54-1.65 (m, 2H), 2.00-2.06 (m, 2H), 2.20-2.27 (m, 2H), 2.88-2.94 (m, 2H), 3.40 (s, 3H), 3.57-3.60 (m, 2H), 3.64-3.66 (m, 2H), 3.68 (s, 2H) 3.98-4.06 (m, 1H) 4.73 (s, 2H), 5.94-5.98 (m, 1H), 7.44-7.46 (m, 1H), 7.47 (dd, J = 8.3, 1.8 Hz, 1H), 7.56 (t, J = 7.8 Hz, 1H), 7_{.}72 (s, 1H), 7.75-7.82 (m, 4H), 7.95-7.98 (m, 1H), 8.02-8.04 (m, 1H); ESI/APCI MS m/z 458, (M+H)⁺.** |
| **87** | N-[1-({7-[(2-Methoxyethoxy)methyl]-2-naphthyl}methyl)piperidin-4-yl]-3-phenoxybenzamide | **¹H NMR (600 MHz, CDCl₃,** δ**): 1.52-1.65 (m, 2H), 1.98-2.05 (m, 2H), 2.19-2.26 (m, 2H), 2.85-2.92 (m, 3H), 3.57-3.61 (m, 2H), 3.63-3.68 (m, 4H), 3.97-4.05 (m, 1H), 4.73 (s, 2H). 5.90-5.94 (m, 1H), 6.99-7.03 (m, 2H), 7.10-7.16 (m, 2H), 7.33-7.41 (m, 4H), 7.42-7.49 (m, 3H), 7.72 (s, 1H), 7.76 (s, 1H), 7.79 (d, J = 8.7 Hz, 1H), 7.91 (d, J = 8.7 Hz, 1H); ESI/APCI MS m/z 525, (M+H)⁺.** |

**[Table 5-4]**

| Example No. | Compound name | Physical data |
|---|---|---|
| **88** | N-[1-({7-[(2-Meuloxyethoxy)methyl]-2-naphthyl}methyl)piperidin-4-yl]-3-vinylbenzamide | **¹H NMR (600 MHz, CDCl₃,** δ)**: 1.51-1.66 (m, 2H), 2.00-2.07 (m, 2H), 2.20-2.29 (m, 2H), 2.87-2.94 (m, 2H), 3.40 (s, 3H), 3.57-3.60 (m, 2H), 3.63-3.66 (m, 2H), 3.68 (s, 2H), 3.99-4.07 (m, 1H), 4.73 (s, 2H), 5.32 (d, J = 11.0 Hz, 1H), 5.81 (d, J = 17.9 Hz, 1H), 5.92-5.98 (m, 1H), 6.73 (dd, J = 17.4, 11.0 Hz, 1H), 7.37 (t, J = 7.8 Hz, 1H), 7.43-7.47 (m, 1H), 7.47-7.49 (m, 1H), 7.51-7.54 (m, 1H), 7.56-7.60 (m, 1H), 7.72 (s, 1H), 7.75-7.77 (m, 2H), 7.77-7.82 (m, 2H); ESI/APCI MS m/z 459, (M+H)⁺.** |
| **89** | N-[1-({7-[(2-Methoxyethoxy)methyl]-2-naphthyl}methyl)piperidin-4-yl]-2-(3-methoxyphenyl)acetamide | **¹H NMR (600 MHz, CDCl₃, δ): 1.28-1.38 (m, 2H), 1.80-1.87 (m, 2H), 2.08-2.17 (m, 2H), 2.70-2.79 (m, 2H), (s, 3H), 3.51 (s, 2H), 3.56-3.61 (m, 4H), 3.62-3.65 (m, 2H), 3.74-3.84 (m, 4H), 4.71 (s, 2H), 5.20-5.26 (m, 1H), 6.75-6.78 (m, 1H), 6.78-6.83 (m, 2H), 7.22-7.26 (m, 1H), 7.38-7.43 (m, 1H), 7.43-7.45 (m, 1H), 7.66 (s, 1H), 7.73 (s, 1H), 7.75 (d, J = 8.3 Hz, 1H), 7.78 (d, J = 8.3 Hz, 1H); ESI/APCI MS m/z 477, (M+H)⁺.** |
| **90** | 3-Acetyl-N-[1-({7-[(2-methoxyethoxy)methyl]-2-naphthyl}methyl)piperidin-4-yl]benzamide | **¹H NMR (600 MHz, CDCl₃,** δ): **1.56-1.69 (m, 2H), 2.00-2.06 (m, 2H), 2.19-2.27 (m, 2H), 2.64 (s, 3H), 2.88-2.94 (m, 2H), 3.40 (s, 3H), 3.57-3.60 (m, 2H), 3.63-3.66 (m, 2H), 3.67 (s, 2H), 3.99-4.08 (m, 1H), 4.73 (s, 2H), 6.04-6.09 (m, 1H), 7.43-7.46 (m, 1H), 7.46-7.49 (m, 1H), 7.54 (t, J = 7.8 Hz, 1H), 7.72 (s, 1H), 7.76 (s, 1H), 7.79 (t, J = 8.9 Hz, 2H), 7.96-8.00 (m, 1H), 8.04-8.08 (m, 1H), 8.28-8.30 (m, 1H); ESI/APCI MS m/z 475, (M+H)⁺.** |
| **91** | 3-Benzoyl-N-[1-({7-[(2-methoxyethoxy)methyl]-2-naphthyl}methyl)piperidin-4-yl]benzamide | **¹H NMR (600 MHz, CDCl₃, δ): 1.55-1.72 (m, 2H), 1.99-2.06 (m, 2H), 2.19-2.27 (m, 2H), 2.86-2.94 (m, 2H), 3.40 (s, 3H), 3.57-3.60 (m, 2H), 3.63-3.66 (m, 2H), 3.67 (s, 2H), 3.98-4.07 (m, 1H), 4.72 (s, 2H), 6.04-6.10 (m, 1H), 7.43-7.52 (m, 4H), 7.55 (t, J = 7.8 Hz, 1H), 7.59-7.63 (m, 1H), 7.71 (s, 1H), 7.76 (s, 1H), 7.77-7.82 (m, 4H), 7.86-7.89 (m, 1H), 8.00-8.04 (m, 1H), 8.12-8.15 (m, 1H); ESI/APCI MS m/z 537, (M+H)⁺.** |
| **92** | N-[1-({7-[(2-Methoxyethoxy)methyl]-2-naphthyl}methyl)piperidin-4-yl]-3-(1H-pyrrol-1-yl)benzamide | **¹H NMR (600 MHz, CDCl₃,** δ**): 1.54-1.64 (m, 2H), 2.01-2.07 (m, 2H), 2.20-2.27 (m, 2H), 2.87-2.93 (m, 2H), 3.40 (s, 3H), 3.57-3.60 (m, 2H), 3.63-3.66 (m, 2H), 3.67 (s, 2H), 4.00-4.07 (m, 1H), 4.73 (s, 2H), 5.96-6.01 (m, 1H), 6.36 (t, J = 2.1 Hz, 2H), 7.12 (t, J = 2.3 Hz, 2H), 7.43-7.49 (m, 3H), 7.49-7.54 (m, 2H), 7.72 (s, 1H), 7.76 (s, 1H), 7.77-7.82 (m, 3H); ESI/APCI MS m/z 498, (M+H)⁺.** |

**[Table 5-5]**

| Example No. | Compound name | Physical data |
|---|---|---|
| **93** | N-[1-({7-[(2-Methoxyethoxy)methyl]-2-naphthyl}methyl)piperidin-4-yl]-3-nitrobenzamide | **¹H NMR (600 MHz, CDCl₃,** δ**): 1.57-1.66 (m, 2H), 2.01-2.07 (m, 2H), 2.20-2.27 (m, 2H), 2.89-2.94 (m, 2H), 3.40 (s, 3H), 3.57-3.60 (m, 2H), 3.64-3.67 (m, 2H), 3.68 (s, 2H), 4.00-4.08 (m, 1H), 4.73 (s, 2H), 6.03-6.09 (m, 1H), 7.45 (dd, J = 8.3, 1.8 Hz, 1H), 7.46-7.49 (m, 1H), 7.64 (t, J = 8.0 Hz, 1H), 7.72 (s, 1H), 7.76 (s, 1H), 7.79 (t, J = 8.9 Hz, 2H), 8.13 (d, J = 7.8 Hz, 1H), 8.32-8.36 (m, 1H), 8.54-8.56 (m, 1H); ESI/APCI MS m/z 478, (M+H)⁺.** |
| **94** | 3-Chloro-N-[1-({7-[(2-methoxyethoxy)methy]-2-naphthyl}methyl)piperidin-4-yl]benzamide | **¹H NMR (600 MHz, CDCl₃,** δ)**: 1.54-1.70 (m, 2H), 1.99-2.05 (m, 2H), 2.19-2.27 (m, 2H), 2.87-2.94 (m, 2H), 3.40 (s, 3H), 3.56-3.60 (m, 2H), 3.63-3.66 (m, 2H), 3.67 (s, 2H), 3.96-4.05 (m, 1H), 4.73 (s, 2H), 5.91-5.95 (m, 1H), 7.36 (t, J = 7.8 Hz, 1H), 7.43-7.49 (m, 3H), 7.58-7.61 (m, 1H), 7.70-7.73 (m, 2H), 7.76 (s, 1H), 7.77-7.82 (m, 2H); ESI MS m/z 465, (M-H)⁻.** |
| **95** | N-[1-({7-[(2-Methoxythoxy)methyl]-2-naphthyl}methyl)piperidin-4-yl]-3-(methylthio)benzamide | **¹H NMR (600 MHz, CDCl₃,** δ**): 1.55-1.71 (m, 2H), 1.99-2.06 (m, 2H), 2.19-2.27 (m, 2H), 2.51 (s, 3H), 2.87-2.93 (m, 2H), 3.40 (s, 3H), 3.57-3.60 (m, 2H), 3.63-3.66 (m, 2H), 3.97-4.05 (m, 1H), 4.73 (s, 2H), 5.91-5.97 (m, 1H), 7.30-7.37 (m, 2H), 7.41-7.44 (m, 1H), 7.44-7.45 (m, 1H), 7.46-7.49 (m, 1H), 7.62-7.64 (m, 1H). 7.72 (s, 1H), 7.76 (s, 1H), 7.77-7.82 (m, 2H); ESI MS m/z 477, (M-H)⁻.** |
| **96** | N-[1-({7-[(2-Methoxyethoxy)methyl]-2-naphthyl}methyl)piperidin-4-yl]-3-(methylsulfonyl)benzamide | **¹H NMR (600 MHz, CDCl₃,** δ**): 1.56-1.65 (m, 2H), 1.99-2.05 (m, 2H), 2.19-2.25 (m, 2H), 2.89-2.95 (m, 2H), 3.08 (s, 3H), 3.40 (s, 3H), 3.57-3.60 (m, 2H), 3.63-3.66 (m, 2H), 3.67 (s, 2H), 3.98-4.06 (m, 1H), 4.73 (s, 2H), 6.09-6.14 (m, 1H), 7.45 (dd, J = 8.3, 1.8 Hz, 1H), 7.46-7.49 (m, 1H), 7.66 (t, J = 7.8 Hz, 1H), 7.71 (s, 1H), 7.76 (s, 1H), 7.79 (t, J = 8.9 Hz, 2H), 8.04-8.07 (m, 1H), 8.07-8.10 (m, 1H), 8.23-8.25 (m, 1H); ESI MS m/z 509, (M-H)⁻.** |
| **97** | 2-Methoxy-N-[1-({7-[(2-methoxyethoxy)methyl]-2-naphthyl}methyl)piperidin-4-yl]isonicotinamide | **¹H NMR (600 MHz, CDCl₃,** δ**): 1.50-1.65 (m, 2H), 1.98-2.04 (m, 2H), 2.19-2.26 (m, 2H), 2.86-2.93 (m, 2H), 3.40 (s, 3H), 3.57-3.60 (m, 2H), 3.63-3.66 (m, 2H), 3.67 (s, 2H), 3.95 (s, 3H), 3.96-4.04 (m, 1H), 4.73 (s, 2H), 5.93-6.00 (m, 1H), 6.99-7.02 (m, 1H),** 7.11-7.15 (m, **1H), 7.43-7.49 (m, 2H), 7.71 (s, 1H), 7.76 (s,** 1H), **7.77-7.82 (m, 2H), 8.24 (d, J = 6.0 Hz, 1H); ESI/APCI MS m/z 464, (M+H)⁺.** |

**[Table 5-6]**

| Example No. | Compound name | Physical data |
|---|---|---|
| **98** | 4-Acetyl-N-[1-({7-[(2-methoxyethoxy)methyl]-2-naphthyl}methyl)piperidin-4-yl]benzamide | **¹H NMR (600 MHz, CDCl₃, δ): 1.52-1.66 (m, 2H), 2.01-2.07 (m, 2H), 2.21-2.28 (m, 2H), 2.63 (s. 3H), 2.88-2.94 (m, 2H), 3.40 (s, 3H), 3.57-3.60 (m, 2H), 3.63-3.66 (m, 2H), 3.68 (s, 2H), 3.99-4.08 (m, 1H), 4.73 (s, 2H), 5.98-6.03 (m, 1H), 7.44-7.46 (m, 1H), 7.46.7.49 (m, 1H), 7.72(s. 1H), 7.76(s, 1H), 7.77-7.83 (m, 4H), 7.98-8.01 (m, 2H); ESI/APCI MS m/z 475, (M+H)⁺.** |
| **99** | 5-Chloro-N-[1-({7-[(2-methoxyethoxy)methyl]-2-naphthyl}methyl)piperidin-4-yl]nicotinamide | **¹H NMR (600 MHz, CDCl₃, δ): 1.53-1.65 (m, 2H), 2.00-2.05 (m, 2H), 2.20-2.27 (m, 2H), 2.88-2.93 (m, 2H), 3,40 (s, 3H), 3.57-3.60 (m, 2H), 3.63-3.66 (m, 2H), 3.67 (s, 2H), 3.98-4.06 (m, 1H), 4.73 (s, 2H), 5.97-6.01 (m, 1H), 7.43-7.48 (m, 2H), 7.71 (3, 1H), 7.76 (s, 1H), 7.79 (t, J = 9.2 Hz, 2H), 8.06-8.09 (m, 1H), 8.67 (d, J = 2.3 Hz, 1H), 8.78 (d, J = 1.8 Hz, 1H); ESI/APCI MS m/z 468, (M+H)⁺.** |
| **100** | N-[1-({7-[(2-Methoxyethoxy)methyl]-2-naphthyl}methyl)piperidin-4-yl]-3-(methoxymethyl)benzamide | **¹H NMR (600 MHz, CD₃OD, δ): 1.62-1.70 (m, 2H), 1.87-1.92 (s, 3H, 3.54-3.57 (m, 2H), 3.61-3.65 (m, 2H), 3.67 (s, 2H), 3.82-(s, 3H), 3.54-3.57 (m, 2H), 3.61-3.65 (m, 2H), 3.67 (s, 2H), 3.82-3.89 (m, 1H), 4.45 (s, 2H), 4.66(s, 2H), 7.38 (t, J = 7.8 Hz, 1H), 7.41-7.48 (m, 3H), 7.68 (d, J = 7.8 Hz, 1H), 7.73 (s, 1H), 7.74 (s, 1H), 7.76 (s, 1H), 7.78 (d, J = 3.7 Hz, 1H), 7.79 (d, J = 3.7 Hz, 1H); ESI/APCI MS m/z 477, (M+H)⁺.** |
| **101** | N-[1-({7-[(2-Methoxyethoxy)methyl]-2-naphthyl}methyl)piperidin-4-yl]benzamide | **¹H NMR (600 MHz, CDCl₃, δ): 1.52-1.64 (m 2H), 2.00-2.06 (m, 2H), 2.21-2.2.7 (m, 2H), 2.87.2.94 (m, 2H), 3.40 (s, 3H), 3.57-3.60 (m, 2H), 3.63-3.66 (m, 2H), 3.67 (s, 2H), 3.99-4.08 (m, 1H), 4.73 (s, 2H), 5.94-5.98 (m, 1H), 7.40-7.44 (m, 2H), 7.44-7.46 (m, 1H), 7.46-7.50 (m,2H), 7.70-7.75 (m. 3H), 7.76 (s, 1H), 7.77-7.81 (m, 2H); ESI/APCI MS m/z 433, (M+H)⁺.** |
| **102** | 3-Hydroxy-N-[1-({7-[(2-methoxyethoxy)methyl]-2- naphthyl}methyl)piperidin-4-yl]benzamide | **¹H NMR (600 MHz, CD₃OD, δ): 1.61-1.69 (m, 2H), 1.86-1.92 3.54-3.53 (m. 2H). 3.62-3.66 (m, 2M) 3.68 (s. 2M) 3.90-3.87 (m, 1H), 4.67 (s, 2H), 6.85-6.90 (m, 1H), 7.14-7.17 (m, 1H), 7.17-7.22 (m. 2H), 7.44 (dd, *J* = 83, 1.8 Hz, 1H), 7.47 (dd, J = 8.3, 1.8 Hz, 1H). 7.75 (s, 1H), 7.77 (s. 1H), 7.78-7.82 (m, 2H); ESI/APCI MS m/z 449, (M+H)⁺.** |

**[Table 5-7]**

| Examples No. | Compound name | Physical data |
|---|---|---|
| **103** | 3-({[1-({7-[(2-Methoxyethoxy)methyl]-2-naphthyl}methyl)piperidin-4-yl]amino}carbonyl)phenyl acetate | **¹H NMR (600 MHz, CDCl₃, δ): 1.53-1.65 (m, 2H), 1.98-204 (m, 2H), 2.18-2-25 (m, 2H), 2.31 (s, 3H), 2.85-2.93 (m, 2H), 3.40 (s, 3H), 3.57-3.60 (m, 2H), 3.63-3.68 (m, 4H), 3.97-4.04 (m, 1H), 4.73 (s, 2H), 5.94-6.00 (m, 1H), 7.20-7.23 (m. 1H), 7.41-7.49 (m, 4H), 7.57-7.60 (m. 1H), 7.71 (s, 1H), 7.76 (s, 1H), 7.79 (dd, J = 10.6, 8.7 Hz, 2H); ESI/APCI MS m/z 491, (M+H)⁺.** |
| 104 | N-[1-({7-[(2-Methoxyethoxy)methyl]-2-naphthyl}methyl)piperidin-4-yl]pyrimidine-5-carboxamide | **¹H NMR (600 MHz, CDCl₃, δ): 1.55-1.65 (m, 2H), 1.99-206 (m, 2H), 2.17-2.26 (m, 2H), 2.87-2.94 (m, 2H), 3.40 (s, 3H), 3.56-3.60 (m, 2H), 3.62-3.68 (m. 4H), 3.99-4.08 (m, 1H), 4.72 (s. 2H), 6.08-6.13 (m, 1H), 7.43-7.48 (m, 2H), 7,70 (s, 1H), 1.75 (s, 1H), 7.79 (t, J = 8.9 Hz, 2H), 9.07 (s. 2H), 9.30 (s, 1H); ESI/APCI MS m/z 435, (M+H)⁺.** |
| **106** | N-[1-({7-[(2-Methoxyethoxy)methyl]-2-naphthyl}methyl)piperidin-4-yl]pyrimidme-2-carboxamide | **¹H NMR (600 MHz, CD₃OD, δ): 1.75-1.85 (m, 2H), 1.97-2-05 (m, 2H), 2.43-2.54 (m, 2H), 3.06-3.15 (m, 2H), 3.36 (s, 3H), 3.56-3.61 (m 2H), 3.64-3.69 (m. 2H), 3.88 (s, 2H), 3.96-4.05 (m, 1H), 4.70 (s, 2H), 7.48 (d, J = 8.3 Hz, 1H), 7.51 (d, J = 8.7 Hz. 1H), 7.60 (t, J = 4.8 Hz, 1H), 7.80-7.88 (m, 4H), 8.90 (s, 1H), 8.91 (s, 1H); ESI/APCI MS m/z 435, (M+H)⁺.** |
| **106** | N-[1-({7-[(2-Methoxyethoxy)methyl]-2- naphthyl}methyl)piperidin-4-yl]biphenyl-3-carboxamide | **¹H NMR.(600 MHz, CDCl₃, δ): 1.55-1.70 (m, 2H), 2.01-2.07 (m, 2H), 2.21-2.29 (m, 2H), 2.88-2.95 (m, 2H), 3.39 (s, 3H), 3.55-3.59 (m 2H), 3.62-3.66 (m, 2H), 3.68 (s, 2H), 4.01-4.09 (m, 1H),** 4.72 **(s, 2H), 6.00-6.05 (m, 1H), 7.34-7.38 (m, 1H), 7.42-7.46 (m, 3H, 7.46-7.50 (m, 2H), 7.57-7.61 (m, 2H), 7.66-7.71 (m, 2H), 7.72 (s, 1H), 7.75 (s, 1H), 7.79 (t, J = 8.5 Hz, 2H), 7.93-7.96 (m, 1H); ESI/APCI MS m/z 531, (M+Na)⁺.** |
| **107** | N-[1-({7-[(2-Methoxyethoxy)methyl]-2-naphthyl}methyl)piperidin-4-yl]-2-naphthamide | **¹H NMR (600 MHz, CDCl₃, δ): 1.53-1.74 (m, 2H), 2.05-2.11 (m, 2H), 2.25-2.32 (m, 2H), 2.92-2.98 (m, 2H), 3.40 (s, 3H), 3.57-3.60 (m, 2H), 3.63-3.67 (m, 2H), 3.71 (s, 2H), 4.06-4.14 (m, 1H), 4.73 (s, 2H), 6.11-6.16 (m, 1H), 7.44-7-47 (m, 1H), 7.48-7.51 (m, 1H), 7.51-7.57 (m, 2H), 7.74 (s, 1H), 7.77 (s, 1H), 7.78-7.82 (m, 3H), 7.84-7.87 (m, 1H), 7.88 (d, J = 8.7 Hz, 1H), 7.90-7.93 (m, 1H), 8.24-8.26 (m 1H); ESI/APCI MS m/z 483, (M+H)⁺.** |

**[Table 5-8]**

| Example No. | Compound name | Physical dada |
|---|---|---|
| **108** | N-[1-({7-[(2-Methoxyethoxy)methy]-2-naphthyl}methyl)piperidin-4-yl]-1-naphthamide | **¹H NMR (600 MHz, CDCl₃, δ): 1.54-1.73 (m, 2H), 2.10-2.15 (m, 2H), 2.26-2.34 (m, 2H). 2.90-2.98 (m, 2H). 3.40 (s, 3H), 3.57. 3.60 (m, 2H), 3.63-3.67 (m, 2H), 3.71 (s, 2H), 4.12-4.20 (m, 1H), 4.73 (s, 2H), 5.86-5.91 (m, 1H), 7.43-7.47 (m, 2H), 7.48-7.56 (m, 3H), 7.56-7.59 (m, 1H), 7.74 (s, 1H), 7.77 (s, 1H), 7.80 (t, J = 8.5 Hz, 2H), 7.84-7.87 (m, 1H), 7.90 (d, J = 8.3 Hz, 1H), 8.24-8.28 (m, 1H); ESI/APCI MS m/z 483, (M+H)⁺.** |

### Example 109: Synthesis of 3-({[1-({7-[(2-methoxyethoxy)methyl]-2-naphthyl}methyl)piperidin-4-yl]amino}carbonyl)benzoic acid

The title compound (2.41 g, pale yellow amorphous) was obtained from the compound obtained in Example 76 (2.50 g) by the same method as in Example 71.
¹H NMR (600 MHz, MeOH -d₃, δ): 1.85-1.98 (m, 2H) 2.17 (d, J = 11.5 Hz, 2H) 3.11 (t, J = 11.5Hz, 2 H) 3.36 (s, 3H) 3.44-3.52 (m, 2H) 3.56-3.62 (m, 2H) 3.65-3.70 (m, 2H) 4.08-4.17 (m, 1H) 4.38 (s, 2H) 4.72 (s, 2H) 7.47-7.61 (m, 3H) 7.86-8.03 (m, 5H) 8.14 (d, J = 7.8 Hz, 1H) 8.42 (s, 1H) ; ESI/APCI MS m/z 477, (M+H)⁺.

### Example 110: Synthesis of N-[l-({7-[(2-methoxyethoxy)methyl]-2-naphthyl}methyl)piperidin-4-yl]-N'-methylisophthalamide

The title compound (116 mg, colorless solid) was obtained from the compound obtained in Example 109 (260 mg) and 2 M MeNH₂/THF (475 µL) by the same method as in Step 7-6.
¹H NMR (600 MHz, CDCl₃, δ): 1.52-1.67 (m, 2H), 1.98-2.05 (m, 2H), 2.22 (t, J = 10.8 Hz, 2H), 2.86-2.94 (m, 2H), 3.02 (d, J = 5.0 Hz, 3H), 3.40 (s, 3H), 3.56-3.60 (m, 2H), 3.63-3.66 (m, 2H), 3.67 (s, 2H), 3.96-4.07 (m, 1H), 4 .73 (s, 2H), 6.13 (d, J = 7.8 Hz, 1H), 6.28 (brs, 1H), 7.40-7.53 (m, 3H), 7.71 (s, 1H), 7.76 (s, 1H), 7.77-7.82 (m, 2H), 7.84-7.92 (m, 2H), 8.13 (s, 1H); ESI/APCI MS m/z 490, (M+H)⁺.

### Example 111: Synthesis of N'-[1-({7-[(2-methoxyethoxy)methyl]-2-naphthyl}methyl)piperidin-4-yl]-N,N-dimethylisophthalamide

The title compound (112 mg, colorless solid) was obtained from the compound obtained in Example 109 (260 mg) and Me₂NH·HCl (77.0 mg) by the same method as in Step 7-6.
¹H NMR (600 MHz, CDCl₃, δ): 1.53-1.64 (m, 2H), 1.98-2.04 (m, 2H), 2.22 (t, J = 11.2 Hz, 2H), 2.85-2.92 (m, 2H), 2.96 (s, 3H), 3.12 (s, 3H), 3.40 (s, 3H), 3.56-3.61 (m, 2H), 3.63-3.67 (m, 2H), 3.66 (s, 2H), 3.96-4.05 (m, 1H), 4.73 (s, 2H), 6.02 (d, J = 8.3 Hz, 1H), 7.42-7.53 (m, 4H), 7.71 (s, 1H), 7.74-7.84 (m, 5H); ESI/APCI MS m/z 504, (M+H)⁺.

### Example 112: Synthesis of N-{1-[(7-hydroxy-2-naphthyl)methyl]piperidin-4-yl}-3-methoxybenzamide

Step 112-1: TBSCl (21.2 g) was added in two portions to a solution of naphthalene-2,7-diol (25.0 g) and imidazole (10.6 g) in DMF (150 mL) under nitrogen atmosphere with ice-cooling. After stirring at the same temperature for 2.5 hours, the reaction solution was added to H₂O, followed by extraction with Et₂O twice. The combined organic layers were washed with brine, dried over MgSO₄ and then concentrated under reduced pressure. The residue was purified by column chromatography (Chromatorex NH, mobile phase: EtOAc/hexane = 10/90 to 50/50; v/v) to obtain 7-{[tert-butyl(dimethyl)silyl]oxy}-2-naphthol (23.2 g, colorless oil).
¹H NMR (200 MHz, CDCl₃, δ): 0.24 (s, 6H), 1.01 (s, 9H), 5.23 (s, 1H), 6.87-6.97 (m, 2H), 6.98-7.01 (m, 1H), 7.01-7.04 (m, 1H), 7.62 (d, J = 4.8 Hz, 1H), 7.66 (d, J = 4.8 Hz, 1H); ESI MS m/z 273, (M-H)⁻.

Step 112-2: Py (9.94 g) and Tf₂O (28.4 g) were added to a solution of the compound obtained in Step 112-1 (23.0 g) in CHCl₃ (230 mL) under nitrogen atmosphere with ice-cooling, and the mixture was stirred at the same temperature for one hour. H₂O was added to the reaction solution, followed by extraction with CHCl₃ three times. The combined organic layers were washed with brine, dried over MgSO₄ and then concentrated under reduced pressure. The residue was purified by column chromatography (Silica gel 60N, mobile phase: EtOAc/hexane = 0/100 to 10/90; v/v) to obtain 7-{[tert-butyl(dimethyl)silyl]oxy}-2-naphthyl trifluoromethanesulfonate (25.3 g, colorless oil).
¹H NMR (200 MHz, CDCl₃, δ): 0.26 (s, 6H), 1.02 (s, 9H), 7.10-7.26 (m, 3H), 7.57-7.60 (m, 1H), 7.76 (d, J = 8.8 Hz, 1H), 7.82 (d, J = 9.2 Hz, 1H); ESI MS m/z 405, (M-H)⁻.

Step 112-3: Et₃N (13.7 g), dppp (1.27 g) and Pd(OAc)₂ (693 mg) were added to a mixed solution of the compound obtained in Step 112-2 (25.1 g) in DMSO (176 mL) and MeOH (126 mL), and the mixture was stirred in a carbon monoxide atmosphere at 75°C for three hours. H₂O was added to the reaction solution, followed by extraction with Et₂O three times. The combined organic layers were washed with brine, dried over MgSO₄ and then concentrated under reduced pressure. The residue was purified by column chromatography (Silica gel 60N, mobile phase: EtOAc/hexane = 0/100 to 10/90; v/v) to obtain methyl 7-{[tert-butyl(dimethyl)silyl]oxy}-2-naphthoate (13.8 g, colorless oil).
¹H NMR (200 MHz, CDCl₃, δ): 0.26 (s, 6H), 1.02 (s, 9H), 3.96 (s, 3H), 7.17 (dd, J = 8.8, 2.6 Hz, 1H), 7.27-7.31 (m, 1H), 7.71-7.82 (m, 2H), 7.88-7.95 (m, 1H), 8.44-8.48 (m, 1H); ESI/APCI MS m/z 317, (M+H)⁺.

Step 112-4: A solution of the compound obtained in Step 112-3 (9.00 g) in THF (45.0 mL) was added to a suspension of LiAlH₄ (1.08 g) in THF (270 mL) at 10°C or lower. After stirring at 0°C for one hour, Na₂SO₄·10H₂O was added and the mixture was filtered through celite. The filtrate was concentrated under reduced pressure, and the residue was dissolved in CHCl₃ (135 mL), followed by addition of MnO₂ (24.7 g). After stirring at room temperature for 18 hours, the reaction solution was filtered through celite. The filtrate was concentrated under reduced pressure to obtain 7-{[tert-butyl(dimethyl)silyl]oxy}-2-naphthaldehyde (colorless oil).
¹H NMR (200 MHz, CDCl₃, δ): 0.23-0.28 (m, 6H), 1.01-1.04 (m, 9H), 7.17-7.25 (m, 1H), 7.29-7.36 (m, 1H), 7.65-7.89 (m, 3H), 8.18-8.21 (m, 1H), 10.13 (s, 1H); EI MS m/z 286, M⁺.

Step 112-5: N-{1-[(7-{[tert-Butyl(dimethyl)silyl]oxy}-2-naphthyl)methyl]piperidin-4-yl}-3-methoxybenzamide (pale yellow solid) was obtained from The compound obtained in Step 112-4 and the compound obtained in Step 1-4 (9.04 g) by the same method as in Step 1-5.
¹H NMR (200 MHz, CDCl₃ δ): 0.25 (s, 6H), 1.02 (s, 9H), 1.48-1.71 (m, 2H), 1.96-2.12 (m, 2H), 2.13-2.32 (m, 2H), 2.85-2.98 (m, 2H), 3.65 (s, 2H), 3.84 (s, 3H), 3.93-4.13 (m, 1H), 5.93-6.02 (m, 1H), 6.99-7.08 (m, 2H), 7.15-7.37 (m, 5H), 7.59 (s, 1H), 7.69 (d, J = 4.8 Hz, 1H), 7.73 (d, J = 4.8 Hz, 1H); ESI MS m/z 505, (M+H)⁺.

Step 112-6: K₂CO₃ (39.3 g) was added to a mixed solution of the compound obtained in Step 112-5 in MeOH (110 mL) and THF (55.0 mL), and the mixture was stirred at room temperature for 1.5 hours. The reaction solution was filtered and the filtrate was concentrated under reduced pressure. The residue was dissolved in CHCl₃ and saturated aqueous NH₄Cl solution was added. The organic layer was separated, and then the aqueous layer was extracted with CHCl₃ twice. The combined organic layers were washed with brine, dried over MgSO₄ and then concentrated under reduced pressure. The residue was suspended in EtOAc (100 mL), and the mixture was stirred at room temperature for one hour. Then, the solid was collected by filtration to obtain the title compound (8.66 g, colorless solid).
¹H NMR (600 MHz, DMSO-d₆, δ): 1.52-1.61 (m, 2H), 1.70-1.77 (m, 2H), 1.97-2.06 (m, 2H), 2.79-2.86 (m, 2H), 3.53 (s, 2H), 3.70-3.79 (m, 4H), 6.98-7.02 (m, 1H), 7.02-7.06 (m, 2H), 7.19-7.23 (m, 1H), 7.30-7.35 (m, 2H), 7.36-7.39 (m, 1H), 7.51 (s, 1H), 7.68 (d, J = 8.7 Hz, 2H), 8.17-8.21 (m, 1H), 9.65 (s, 1H); ESI/APCI MS m/z 391, (M+H)⁺.

### Example 113: Synthesis of 3-methoxy-N-(1-{[7-(2-methoxyethoxy)-2-naphthyl]methyl}piperidin-4-yl)benzamide

K₂CO₃ (212 mg) and 1-bromo-2-methoxyethane (117 mg) were added to a solution of the compound obtained in Example 112 (300 mg) in DMF (3.00 mL), and the mixture was stirred at 100°C for 2.5 days. The reaction solution was added to H₂O, followed by extraction with EtOAc. The organic layer was dried over MgSO₄ and then concentrated under reduced pressure. The residue was purified by column chromatography (Silica gel 60 N, mobile phase: MeOH/CHCl₃ = 0/100 to 50/50; v/v). The resulting solid was suspended in a mixed solution of EtOAc/hexane (1/1; v/v), and the mixture was stirred at room temperature for one hour. Then, the solid was collected by filtration to obtain the title compound (179 mg, colorless solid).
¹H NMR (600 MHz, CDCl₃, δ): 1.54-1.69 (m, 2H), 2.00-2.05 (m, 2H), 2.19-2.26 (m, 2H), 2.86-2.92 (m, 2H), 3.48 (s, 3H), 3.64 (s, 2H), 3.80-3.83 (m, 2H), 3.84 (s, 3H), 3.98-4.05 (m, 1H), 4.21-4.25 (m, 2H), 5.92-5.97 (m, 1H), 7.02 (dd, J = 8.0, 2.5 Hz, 1H), 7.11 (d, J = 2.3 Hz, 1H), 7.16 (dd, J = 8.9, 2.5 Hz, 1H), 7.22-7.25 (m, 1H), 7.31 (d, J = 7.8 Hz, 1H), 7.32-7.35 (m, 2H), 7.62 (s, 1H), 7.70 (d, J = 4.1 Hz, 1H), 7.72 (d, J = 4.1 Hz, 1H); ESI/APCI MS m/z 449, (M+H)⁺.

The compounds of Example 114 to Example 118 were obtained by the same method as in Example 113.

**[Table 6-1]**

| Example No. | Compound name | Physical data |
|---|---|---|
| **114** | 3-Methoxy-N-[1-({7-[2-(2-methoxyethoxy)ethoxy]-2-naphthyl}methyl}piperidin-4-yl]benzamide | **¹H NMR (600 MHz, COCl₃, δ): 1.60-1.78 (m, 2H), 2.06-2.12 (m, 2H), 2,26-233 (m 2H), 2.93-2.99 (m. 2H), 3.46 (s, 3H), 3.64-3.67 (m, 2H), 3.71 (s, 3H), 3.80-3.83 (m, 2H), 3.90 (s. 2H), 3.97-4.00 (m, 2H), 4.04-4.12 (m, 1H), 4.30-4.33 (m, 2H), 5.99-6.04 (m, 1H), 7.08 (dd, J = 8.0, 2.5 Hz, 1H), 7.17 (d, J = 2.3 Hz, 1H), 7.20 (dd, J = 8.9, 2.5 Hz, 1H), 7.28-7.31 (m, 1H), 736-7.41 (m, 3H), 7.68 (s, 1H), 7.76 (d, J = 6.4 Hz, 1H), 7.78 (d, J = 6.0Hz. 1H); ESI/APCI MS m/z 493, (M+H)⁺.** |
| **115** | 3-Methoxy-N-(1-{[7-(3-methoxypropoxy)-2-naphthyl]methyl}piperidin-4-yl)benzamide | **¹H NMR (600 MHz, CDCl₃, δ); 1.54-1.67 (m, 2H), 1.99-2.05 (m, 2H), 2.08-2.14 (m, 2H), 2.20-2.27 (m, 2H), 2.86-2.93 (m, 2H), 3.37 (s, 3H), 3.59 (t, J = 6.2 Hz, 2H), 3.65 (s, 2H), 3.84 (s, 3H), 3.98-4.05 (m, 1H), 4.16 (t J = 6.4 Hz, 2H), 5.92-5.97 (m, 1H), 7.00-7.04 (m, 1H), 7.09-7.13 (m, 2H), 7.22-7.25 (m, 1H), 7.31 (d, J = 8.3 Hz, 1H), 7.32-7.35 (m, 2H), 7.62 (s, 1H), 7,70 (d, J = 5.5 Hz, 1H), 7.71 (d, J = 5.0 Hz, 1H); ESI/APCI MS m/z 463, (M+H)⁺.** |

**[Table 6-2]**

| Example No. | Compound name | Physical data |
|---|---|---|
| **116** | 3-Methoxy-N-(1-{[7-(2-pyrrolidin-1-ylethoxy)-2-naphthyl]methyl}piperidin-4-yl)benzamide | **¹H NMR (600 NHz, CDCl₃, δ): 1.52-1.65 (m, 2H), 1.79-1.86 (m, 4H), 1.99-2.05 (m, 2H), 2.18-2.25 (m, 2H), 2.63-2.71 (m, 4H), 2.85-2.91 (m, 2H), 2.95-3.00 (m, 2H), 3.63 (s, 2H), 3.84 (s, 3H), 3.97-4.05 (m, 1H), 4.23 (t, J = 5.7 Hz, 2H), 5.91-5.95 (m, 1H), 7.00-7.03 (m, 1H), 7.11 (d, J = 2.3 Hz, 1H), 7.14 (dd, J = 8.9, 2.5 Hz, 1H), 7.22-7.24 (m, 1H), 7.29-7.34 (m, 3H), 7.6 (s, 1H), 7.69 (d, J = 5.5 Hz, 1H), 7.71 (d, J = 5.0 Hz, 1H); ESI/APCI MS m/z 488, (M+H)⁺.** |
| **117** | N(1-{[7-(3-Hydroxypropoxy)-2-naphthyl]methyl}piperidin-4-yl)-3-methoxybenzamide | **¹H NMR (600 MHz, CDCl₃, δ): 1.53-1.86 (m, 3H), 1.99-2.05 (m, 2H), 2.09-2.14 (m, 2H), 2.22 (t, J = 11.0 Hz, 2H), 2.85-2.93 (m, 2H), 3.64 (s, 2H), 3.84 (s, 3H), 3.90 (t, J = 5.7 Hz, 2H), 3.97-4.05 (m, 1H), 4.24 (t, J = 6.0 Hz, 2H), 5.96 (d, J = 7.3 Hz 1H), 6.99-7.04 (m, 1H), 7.08-7.14 (m, 2H), 7.21-7.26 (m, 1H), 7.29-7.36 (m, 3H), 7.62 (s, 1H), 7.71 (dd, J = 8.5, 3.9 Hz, 2H); ESI MS m/z 449, (M+H)⁺.** |
| **118** | N-[1-({7-[3-(Dimethylamino)propoxy]-2-naphthyl}methyl)piperidin-4-yl]-3-methoxybenzamide | **¹H NMR (600 MHz, CDCl₃, δ): 1.53-1.63 (m, 2H), 1.99-2.07 (m, 4H), 2.19-2.26 (m, 2H), 2.28 (s, 6H), 2.50 (t, J = 7.1 Hz, 2H), 2.84-2.94 (m, 2H), 3.65 (s, 2H), 3.85 (s, 3H), 3.98-4.07 (m. 1H), 4.13 (t, J = 6.4 Hz, 2H), 5.96 (d, J = 8.3 Hz, 1H), 7.01-7.05 (m, 1H), 7.10-1.14 (m, 2H), 7.21-7.26 (m, 1H), 7.31-7.36 (m, 3H), 7.61 (s, 1H), 7.71 (dd, J = 8.5, 5.3 Hz, 2H); ESI MS m/z 476, (M+H)⁺.** |

### Example 119: Synthesis of 3-methoxy-N-(1-{[7-(2-oxoethyl)-2-naphthyl]methyl}piperidin-4-yl)benzamide

Step 119-1: A solution of 1.04 M phenyllithium in cyclohexane/Et₂O was added to a suspension of chloro(methoxymethyl)triphenylphosphorane (1.76 g) in THF (5.00 mL) under nitrogen atmosphere, and the mixture was stirred at room temperature for 10 minutes. A solution of the compound obtained in Example 5 (1.00 g) in THF (10.0 mL) was added to the reaction solution, and the mixture was stirred at room temperature for 18 hours. Saturated aqueous NaHCO₃ solution was added, followed by extraction with EtOAc three times. The combined organic layers were washed with brine, dried over Na₂SO₄ and then concentrated under reduced pressure. The residue was purified by column chromatography (Silica gel 60 N, mobile phase: MeOH/CHCl₃ = 1/120 to 1/60; v/v) to obtain 3-methoxy-N-[1-({7-[2-methoxyviny]-2-naphthyl}methyl)piperidin-4-yl]benzamide (960 mg, pale orange solid).
¹H NMR (600 MHz, CDCl₃, δ): 1.50-1.67 (m, 2H), 2.00-2.05 (m, 2H), 2.21-2.28 (m, 2H), 2.88-2.95 (m, 2H), 3.67 (s, 2H), 3.73 (s, 1.5H), 3.84 (s, 4.5H), 3.98-4.06 (m, 1H), 5.36 (d, J = 6.9 Hz, 0.5H), 5.94-5.99 (m, 1.5H), 6.22 (d, J = 6.9 Hz, 0.5H), 7.02 (dd, J = 8.3, 2.8 Hz, 1H), 7.17 (d, J = 13.3 Hz, 0.5H), 7.22-7.24 (m, 1H), 7.30-7.33 (m, 2H), 7.38-7.42 (m, 1.5H), 7.55 (s, 0.5H), 7.64 (s, 0.5H), 7.68-7.74 (m, 3H), 7.99 (s, 0.5H); ESI MS m/z 431, (M+H)⁺.

Step 119-2: Concentrated hydrochloric acid (5.60 mL) was added to a solution of the compound obtained in Step 119-1 (950 mg) in THF (14.0 mL), and the mixture was stirred at room temperature for 2.5 hours. A saturated NaHCO₃ solution was added, followed by further addition of a mixed solution of MeOH/CHCl₃ (1/9; v/v) and H₂O. The organic layer was separated, and then the aqueous layer was extracted with a mixed solution of MeOH/CHCl₃ (1/9; v/v) four times. The combined organic layers were washed with brine, dried over Na₂SO₄ and then concentrated under reduced pressure. The residue was purified by column chromatography (Silica gel 60 N, mobile phase: MeOH/CHCl₃ = 1/100 to 1/50; v/v) to obtain the title compound (530 mg, pale yellow solid).
¹H NMR (600 MHz, CDCl₃, δ): 1.50-1.68 (m, 2H), 2.00-2.06 (m, 2H), 2.22-2.31 (m, 2H), 2.89-2.96 (m, 2H), 3.70 (s, 2H), 3.84 (s, 3H), 3.85 (d, J = 1.8 Hz, 2H), 4.00-4.06 (m, 1H), 5.94-6.00 (m, 1H), 7.00-7.04 (m, 1H), 7.22-7.25 (m, 1H), 7.28-7.33 (m, 3H), 7.49-7.53 (m, 1H), 7.66 (s, 1H), 7.73 (s, 1H), 7.81 (d, J = 8.7 Hz, 1H), 7.83 (d, J = 8.7 Hz, 1H), 9.82 (t, J = 2.3 Hz, 1H); ESI MS m/z 415, (M-H)⁻.

### Example 120: Synthesis of N-(1-{[7-(2-hydroxyethyl)-2-naphthyl]methyl}piperidin-4-yl)-3-methoxybenzamide

NaBH₄ (48.0 mg) was added to a mixed solution of the compound obtained in Example 119 (264 mg) in MeOH (5.30 mL) and THF (2.60 mL), and the mixture was stirred at room temperature for 14.5 hours. Saturated aqueous NH₄Cl solution was added to the reaction solution, followed by concentration under reduced pressure. Saturated aqueous NaHCO₃ solution was added to the residue, followed by extraction with CHCl₃ three times. The combined organic layers were washed with brine, dried over Na₂SO₄ and then concentrated under reduced pressure. The residue was purified by column chromatography (Silica gel 60 N, mobile phase: MeOH/CHCl₃ = 1/50; v/v) to obtain the title compound (110 mg, colorless solid).
¹H NMR (600 MHz, CDCl₃, δ): 1.52-1.63 (m, 2H), 1.98-2.04 (m, 2H), 2.19-2.26 (m, 2H), 2.85-2.92 (m, 2H), 3.02 (t, J = 6.7 Hz, 2H), 3.66 (s, 2H), 3.83 (s, 3H), 3.93 (t, J = 6.7 Hz, 2H), 3.97-4.04 (m, 1H), 5.92-5.97 (m, 1H), 6.99-7.02 (m, 1H), 7.21-7.23 (m, 1H), 7.28-7.34 (m, 3H), 7.43-7.46 (m, 1H), 7.64 (s, 1H), 7.68 (s, 1H), 7.76 (d, J = 3.7 Hz, 1H), 7.77 (d, J = 3.7 Hz, 1H); ESI/APCI MS m/z 419, (M+H)⁺.

### Example 121: Synthesis of 3-methoxy-N-[1-({7-[2-(2-methoxyethoxy)ethyl]-2-naphthyl}methyl)piperidin-4-yl]benzamide

A solution of the compound obtained in Example 120 (93.0 mg) in DMF (500 µL) was added to a suspension of 30% KH (360 mg) in DMF (1.40 mL) under nitrogen atmosphere with ice-cooling, and the mixture was stirred until gas stopped to generate. 1-Bromo-2-methoxyethane (230 µL) and TBAI (160 mg) were added and the mixture was stirred at room temperature for 19 hours. H₂O (6.50 mL) was added, followed by extraction with a mixed solution of MeOH/CHCl₃ (1/20; v/v) four times. The combined organic layers were washed with brine, dried over Na₂SO₄ and then concentrated under reduced pressure. The residue was sequentially purified by column chromatography (Chromatorex NH, mobile phase: CHCl₃) and pTLC (MeOH/CHCl₃ = 1/20; v/v) to obtain the title compound (14.0 mg, pale yellow solid).
¹H NMR (600 MHz, CDCl₃, δ): 1.51-1.67 (m, 2H), 2.00-2.06 (m, 2H), 2.21-2.30 (m, 2H), 2.88-2.96 (m, 2H), 3.07 (t, J = 7.3 Hz, 2H), 3.38 (s, 3H), 3.53-3.56 (m, 2H), 3.61-3.64 (m, 2H), 3.68 (s, 2H), 3.76 (t, J = 7.3 Hz, 2H), 3.84 (s, 3H), 3.98-4.06 (m, 1H), 5.93-5.98 (m, 1H), 7.00-7.03 (m, 1H), 7.22-7.24 (m, 1H), 7.29-7.35 (m, 3H), 7.42-7.47 (m, 1H), 7.63 (s, 1H), 7.68 (s, 1H), 7.74 (d, J = 8.3 Hz, 1H), 7.76 (d, J = 8.3 Hz, 1H); ESI/APCI MS m/z 499, (M+Na)⁺.

### Example 122: Synthesis of 4-chloro-N-[1-({7-[[(2-methoxyethoxy)methyl]-2-naphthyl}methyl)piperidin-4-yl]pyridine-2-carboxamide dihydrochloride

Step 122-1: 4-Chloro-N-[1-({7-[(2-methoxyethoxy)methyl]-2-naphthyl}methyl)piperidin-4-yl]pyridine-2-carboxamide was obtained as a crude product from the compound obtained in Step 72-2 (250 mg) and 4-chloropyridine-2-carboxylic acid (144 mg) by the same method as in Step 7-6.
¹H NMR (600 MHz, CDCl₃, δ): 1.57-1.69 (m, 2H), 1.97-2.03 (m, 2H), 2.21-2.28 (m, 2H), 2.85-2.92 (m, 2H), 3.40 (s, 3H), 3.57-3.60 (m, 2H), 3.63-3.66 (m, 2H), 3.67 (s, 2H), 3.95-4.04 (m, 1H), 4.72 (s, 2H), 7.41 (dd, J = 5.3, 2.1 Hz, 1H), 7.45 (dd, J = 8.3, 1.8 Hz, 1H), 7.46-7.49 (m, 1H), 7.71 (s, 1H), 7.75 (s, 1H), 7.79 (dd, J = 10.6, 8.7 Hz, 2H), 7.87-7.91 (m, 1H), 8.18 (d, J = 2.3 Hz, 1H), 8.43 (d, J = 6.0 Hz, 1H); ESI/APCI MS m/z 468, (M+H)⁺.

Step 122-2: 4 M HCl/EtOAc solution (220 µL) was added to a solution of the crude product obtained in Step 122-1 in EtOAc (1.40 mL). Et₂O (1.40 mL) was added and the mixture was stirred at room temperature for three hours. The generated solid was collected by filtration to obtain the title compound (54.0 mg, pale yellow solid).
¹H NMR (600 MHz, CD₃OD, δ): 1.92-2.01 (m, 2H), 2.16-2.23 (m, 2H), 3.19-3.27 (m, 2H), 3.34-3.37 (m, 3H), 3.56-3.62 (m, 4H), 3.65-3.69 (m, 2H), 4.12-4.31 (m, 1H), 4.47-4.59 (m, 2H), 4.72 (s, 2H), 7.55-7.62 (m, 2H), 7.63-7.71 (m, 1H), 7.89-7.93 (m, 2H), 7.98 (d, J = 8.7 Hz, 1H), 8.05 (s, 1H), 8.08-8.16 (m, 1H), 8.54-8.63 (m, 1H); ESI/APCI MS m/z 468, [M (free)+H]⁺.

### Example 123: Synthesis of 3-(hydroxymethyl)-N-[1-({7-[(2-methoxyethoxy)methyl]-2-naphthyl}methyl)piperidin-4-yl]benzamide

The title compound (2.34 g, colorless solid) was obtained from the compound obtained in Example 76 (2.64 g) by the same method as in Step 1-1.
¹H NMR (600 MHz, CDCl₃, δ): 1.53-1.67 (m, 2H), 1.95 (brs, 1H), 1.98-2.04 (m, 2H), 2.19-2.25 (m, 2H), 2.86-2.92 (m, 2H), 3.39 (s, 3H), 3.56-3.59 (m, 2H), 3.63-3.65 (m, 2H), 3.66 (s, 2H), 3.97-4.05 (m, 1H), 4.72 (s, 2H), 4.73 (s, 2H), 5.97-6.01 (m, 1H), 7.39-7.42 (m, 1H), 7.43-7.49 (m, 3H), 7.63-7.67 (m, 1H), 7.71 (s, 1H), 7.73 (s, 1H), 7.75 (s, 1H), 7.79 (dd, J = 10.6, 8.7 Hz, 2H); ESI/APCI MS m/z 463, (M+H)⁺.

### Example 124: Synthesis of N-[1-({7-[(2-methoxyethoxy)methyl]-2-naphthyl}methyl)piperidin-4-yl]isophthalamide

30% H₂O₂ solution (69.0 µL) and K₂CO₃ (12.0 mg) were added to a solution of the compound obtained in Example 86 (272 mg) in DMSO (2.80 mL) with ice-cooling, and the mixture was stirred at room temperature for three hours. H₂O (20.0 mL) was added, followed by stirring for 30 minutes. The generated solid was collected by filtration to obtain the title compound (254 mg, colorless solid).
¹H NMR (600 MHz, CDCl₃, δ): 1.57-1.70 (m, 2H), 1.98-2.05 (m, 2H), 2.19-2.27 (m, 2H), 2.88-2.94 (m, 2H), 3.40 (s, 3H), 3.57-3.60 (m, 2H), 3.63-3.67 (m, 2H), 3.67 (s, 2H), 3.98-4.07 (m, 1H), 4.72 (s, 2H), 5.70 (brs, 1H), 6.17-6.22 (m, 1H), 6.28 (brs, 1H), 7.45 (dd, J = 8.3, 1.8 Hz, 1H), 7.46-7.49 (m, 1H), 7.52 (t, J = 7.8 Hz, 1H), 7.71 (s, 1H), 7.76 (s, 1H), 7.79 (t, J = 8.9 Hz, 2H), 7.91-7.95 (m, 2H), 8.20-8.22 (m, 1H); ESI/APCI MS m/z 498, (M+Na)⁺.

### Example 125: Synthesis of 3-formyl-N-[1-({7-[(2-methoxyethoxy)methyl]-2-naphthyl}methyl)piperidin-4-yl]benzamide

The title compound (945 mg, colorless solid) was obtained from the compound obtained in Example 123 (1.68 g) by the same method as in Step 1-3.
¹H NMR (600 MHz, CDCl₃, δ): 1.56-1.65 (m, 2H), 2.00-2.06 (m, 2H), 2.19-2.26 (m, 2H), 2.87-2.93 (m, 2H), 3.40 (s, 3H), 3.57-3.60 (m, 2H), 3.63-3.66 (m, 2H), 3.67 (s, 2H), 4.00-4.07 (m, 1H), 4.72 (s, 2H), 6.09-6.14 (m, 1H), 7.45 (dd, J = 8.3, 1.8 Hz, 1H), 7.46-7.49 (m, 1H), 7.62 (t, J = 7. 6 Hz, 1H), 7.71 (s, 1H), 7.76 (s, 1H), 7.79 (t, J = 8.9 Hz, 2H), 7.98-8.01 (m, 1H), 8.05-8.08 (m, 1H), 8.20-8.22 (m, 1H), 10.06 (s, 1H); ESI/APCI MS m/z 483, (M+Na)⁺.

### Example 126: Synthesis of 3-(1-hydroxypropyl)-N-[1-({7-[(2-methoxyethoxy)methyl]-2-naphthyl}methyl)piperidin-4-yl]benzamide

1 M EtMgBr/THF solution (5.54 mL) was added to a solution of the compound obtained in Example 125 (729 mg) in THF (15.0 mL) with ice-cooling, followed by stirring for one hour. The mixture was stirred at room temperature for 12 hours and 2 M aqueous HCl solution was added. CHCl₃ and 2 M aqueous NaOH solution were added with ice-cooling, followed by filtration through celite. The organic layer was separated in the filtrate, and the aqueous layer was extracted with CHCl₃ twice. The combined organic layers were dried over Na₂SO₄ and then concentrated under reduced pressure. The residue was purified by column chromatography (Chromatorex NH, mobile phase: CHCl₃/hexane = 100/1 to 20/1; v/v) to obtain the title compound (541 mg, colorless solid).
¹H NMR (600 MHz, CDCl₃, δ): 0.92 (t, J = 7.3 Hz, 3H), 1.52-1.65 (m, 2H), 1.72-1.86 (m, 2H), 1.94 (brs, 1H), 1.99-2.06 (m, 2H), 2.20-2.27 (m, 2H), 2.87-2.93 (m, 2H), 3.40 (s, 3H), 3.57-3.60 (m, 2H), 3.63-3.66 (m, 2H), 3.67 (s, 2H), 3.98-4.06 (m, 1H), 4.66 (t, J = 6.4 Hz, 1H), 4.73 (s, 2H), 5.95-6.00 (m, 1H), 7.40 (t, J = 7.6 Hz, 1H), 7.43-7.49 (m, 3H), 7.62-7.65 (m, 1H), 7.72 (s, 2H), 7.76 (s, 1H), 7.77-7.82 (m, 2H); ESI/APCI MS m/z 491, (M+H)⁺.

### Example 127: Synthesis of 3-ethyl-N-[1-({7-[(2-methoxyethoxy)methyl]-2-naphthyl}methyl)piperidin-4-yl]benzamide

A Pd/C-ethylenediamine complex (88.0 mg) was added to a mixed solution of the compound obtained in Example 88 (175 mg) in MeOH (1.80 mL) and THF (900 µL), and the mixture was stirred under hydrogen atmosphere at room temperature for 1.5 hours. The reaction solution was filtered through celite and the filtrate was concentrated under reduced pressure. The residue was purified by column chromatography (Silica gel 60 N, mobile phase: MeOH/CHCl₃ = 1/120 to 1/45; v/v) to obtain the title compound (79.0 g, colorless solid).
¹H NMR (600 MHz, CDCl₃, δ): 1.25 (t, J = 7.6 Hz, 3H), 1.52-1.65 (m, 2H), 2.00-2.06 (m, 2H), 2.21-2.29 (m, 2H), 2.68 (q, J = 7.6 Hz, 2H), 2.88-2.94 (m, 2H), 3.40 (s, 3H), 3.57-3.60 (m, 2H), 3.63-3.66 (m, 2H), 3.68 (s, 2H), 3.99-4.07 (m, 1H), 4.73 (s, 2H), 5.92-5.97 (m, 1H), 7.30-7.35 (m, 2H), 7.44-7.47 (m, 1H), 7.47-7.53 (m, 2H), 7.58 (s, 1H), 7.72 (s, 1H), 7.76 (s, 1H), 7.79 (t, J = 8.9 Hz, 2H); ESI MS m/z 461, (M+H)⁺.

### Example 128: Synthesis of 3-chloro-4-fluoro-N-{1-[(6-methoxy-2-naphthyl)methyl]piperidin-4-yl}benzamide

The title compound (300 mg, colorless solid) was obtained from the compound obtained in Step 6-3 (300 mg) and 6-methoxy-2-naphthaldehyde (261 mg) by the same method as in Step 1-5.
¹H NMR (600 MHz, CDCl₃, δ): 1.52-1.61 (m, 2H), 1.97-2.03 (m, 2H), 2.15-2.24 (m, 2H), 2.89 (d, J = 11.5 Hz, 2H), 3.62 (s, 2H), 3.91 (s, 3H), 3.93-4.02 (m, 1H), 5.92 (d, J = 7.8 Hz, 1H), 7.10-7.19 (m, 3H), 7.43 (dd, J = 8.5, 1.6 Hz, 1H), 7.61 (ddd, J = 8.7, 4.6, 2.3 Hz, 1H), 7.65 (s, 1H), 7.69 (dd, J = 8.5, 2.1 Hz, 2H), 7.81 (dd, J = 6.9, 1.8 Hz, 1H); ESI MS m/z 427, (M+H)⁺.

### Example 129: Synthesis of 3-chloro-4-fluoro-N-{1-[(6-hydroxy-2-naphthyl)methyl]piperidin-4-yl}benzamide

The title compound (263 mg) was obtained from the compound obtained in Step 6-3 (300 mg) and 6-hydroxy-2-naphthaldehyde (241 mg) by the same method as in Step 1-5.
¹H NMR (600 MHz, CDCl₃, δ): 1.57-1.70 (m, 2H), 1.99-2.09 (m, 2H), 2.25 (t, J = 11.2 Hz, 2H), 3.03 (d, J = 11.5 Hz, 2H), 3.63 (s, 2H), 3.98-4.07 (m, 1H), 6.04 (d, J = 7.8 Hz, 1H), 6.89 (d, J = 2.3 Hz, 1H), 6.93 (dd, J = 8.7, 2.3 Hz, 1H), 7.15 (t, J = 8.5 Hz, 1H), 7.31 (dd, J = 8.5, 1.6 Hz, 1H), 7.40 (d, J = 8.3 Hz, 1H), 7.51 (d, J = 8.7 Hz, 1H), 7.57 (s, 1H), 7.61 (ddd, J = 8.6, 4.5, 2.1 Hz, 1H), 7.81 (dd, J = 7.1, 2.1 Hz, 1H); ESI MS m/z 411, (M-H)⁻.

### Example 130: Synthesis of 3-chloro-4-fluoro-N-{1-[1-(6-methoxy-2-naphthyl)ethyl]piperidin-4-yl}benzamide

1-(6-Methoxy-2-naphthyl)ethanone (280 mg), AcOH (84.0 mg) and NaBH₃CN (111 mg) were added to a solution of the compound obtained in Step 6-3 (300 mg) in MeOH (3.00 mL), and the mixture was heated under reflux for 10 hours. After leaving to cool to room temperature, saturated aqueous NaHCO₃ solution was added, followed by extraction with CHCl₃ three times. The combined organic layers were dried over MgSO₄ and then concentrated under reduced pressure. The residue was purified by column chromatography (Silica gel 60 N, mobile phase: MeOH/CHCl₃ = 1/100 to 1/20; v/v) to obtain the title compound (48.0 g, colorless solid).
¹H NMR (600 MHz, CDCl₃, δ): 1.42-1.64 (m, 5H), 1.92-1.97 (m, 1H), 2.02-2.08 (m, 1H), 2.12-2.26 (m, 2H), 2.78-2.85 (m, 1H), 3.02-3.09 (m, 1H), 3.58 (q, J = 6.4 Hz, 1H), 3.89-3.97 (m, 1H), 3.92 (s, 3H), 5.85 (d, J = 7.3 Hz, 1H), 7.11-7.16 (m, 2H), 7.18 (t, J = 8.5 Hz, 1H), 7.47 (dd, J = 8.5, 1.6 Hz, 1H), 7.61 (ddd, J = 8.7, 4.6, 2.3 Hz, 1H), 7.64 (s, 1H), 7.71 (dd, J = 8.5, 2.5 Hz, 2H), 7.80 (dd, J = 6.9, 2.3 Hz, 1H); ESI MS m/z 441, (M+H)⁺.

The compounds of Example 131 and Example 132 were obtained by the same method as in Example 130.

**[Table 7]**

| Example No. | Compound name | Physical data |
|---|---|---|
| **131** | 3-Chloro-4-fluoro-N-{1-[1-(6-isopropyl-2-naphthyl)ethyl]piperidin-4-yl}benzamide | **¹H NMR (600 MHz, CDCl₃, δ): 1.33 (d, J = 7.34 Hz, 6H), 1.41-1.65 (m, 5H), 1.88 - 1.96 (m, 1H), 2.01 - 2.07 (m, 1H), 2.10 - 2.25 (m, 2H), 2.76 - 2.85 (m, 1H), 3.01 - 3.10 (m, 1H), 3.53 - 3.61 (m, 1H), 3.87 - 3.96 (m, 1H), 5.83 (d, J = 7.8 Hz, 1H), 1.16 (t, J = 8.5 Hz, 1H), 7.38 (dd, J = 8.5, 1.6 Hz, 1H), 7.47 (dd, J = 8.3, 1.4 Hz, 1H), 7.59 (ddd, J = 8.4, 4.5, 2.3 Hz, 1H), 7.62 (s, 1H), 7.66 (s, 1H), 7.75 (dd, J = 8.5, 2.1 Hz, 2H), 7.79 (dd, J = 6.9, 2.3 Hz, 1H); ESI MS m/z 453, (M+H)⁺.** |
| **132** | 3-Chloro-4-fluoro-N-{1-[1-(6-methyl-2-naphthyl)ethyl]piperidin-4-yl}benzamide | **¹H NMR (600 MHz, CDCl₃, δ): 1.40 - 1.69 (m, 5H), 1.90 - 1.97 (m, 1H), 2.01 - 2.08 (m, 1H), 2.12 - 2.27 (m, 2H), 2.51 (s, 3H), 2.77 - 2.86 (m, 1H), 3.02 - 3.12 (m, 1H), 3.53 - 3.63 (m, 1H), 3.87 - 3.99 (m, 1H), 5.84 (d, J = 6.9 Hz, 1H), 7.18 (t, J = 8.5 Hz, 1H), 7.29 - 733 (m, 1H), 7,45 - 7.50 (m, 1H), 7.58 - 7.63 (m, 2H), 7.66 (s, 1H), 7.72 (dd, J = 8.3, 3.2 Hz, 2H), 7.80 (dd, J = 7.1, 2.1 Hz, 1H); ESI MS m/z 425, (M+H)⁺.** |

### Example 133: Synthesis of 3-chloro-4-fluoro-N-(1-{[6-(methoxymethyl)-2-naphthyl]methyl}piperidin-4-yl)benzamide

Step 133-1: MeOH/THF (1/1; v/v, 800 µL) was added to a suspension of KOtBu (178 mg) in THF (4.00 mL), and the mixture was stirred at room temperature for one hour. 2,6-Bis(bromomethyl)naphthalene (500 mg) was added and the mixture was stirred at room temperature for 15 hours. Water was added, followed by extraction with CHCl₃ three times. The combined organic layers were dried over MgSO₄ and then concentrated under reduced pressure. The residue was purified by column chromatography (Silica gel 60 N, mobile phase: EtOAc/hexane = 1/20 to 1/3; v/v) to obtain 2-(bromomethyl)-6-(methoxymethyl)naphthalene (50.0 mg, colorless solid).
¹H NMR (600 MHz, CDCl₃, δ): 3. 42 (s, 3H), 4.61 (s, 2H), 4.66 (s, 2H), 7.44-7.52 (m, 2H), 7.74-7.84 (m, 4H).

Step 133-2: The compound obtained in Step 133-1 (50.0 mg) and Et₃N (23.0 mg) were added to a solution of the compound obtained in Step 6-3 (54.0 mg) in CHCl₃ (1.00 mL), and the mixture was stirred at room temperature for 12 hours. Saturated aqueous NaHCO₃ solution was added, followed by extraction with CHCl₃ three times. The combined organic layers were dried over MgSO₄ and then concentrated under reduced pressure. The residue was purified by column chromatography (Chromatorex NH, mobile phase: EtOAc/hexane = 1/10 to 1/1; v/v) to obtain the title compound (48.0 mg, colorless solid).
¹H NMR (600 MHz, CDCl₃, δ): 1.52-1.62 (m, 2H), 1.95-2.06 (m, 2H), 2.22 (t, 2H), 2.85-2.95 (m, 2H), 3.42 (s, 3H), 3.66 (s, 2H), 3.94-4.03 (m, 1H), 4.61 (s, 2H), 5.86 (d, J = 7.8 Hz, 1H), 7.18 (t, J = 8.7 Hz, 1H), 7.44 (d, J = 8.3 Hz, 1H), 7.48 (d, J = 8.3 Hz, 1H), 7.62 (ddd, J = 8.6, 4.5, 2.1 Hz, 1H), 7.72 (s, 1H), 7.76 (s, 1H), 7.77-7.83 (m, 3H); ESI MS m/z 441, (M+H)⁺.

### Example 134: Synthesis of 3-chloro-4-fluoro-N-(1-{[7-(methoxymethyl)-2-naphthyl]methyl}piperidin-4-yl)benzamide

Step 134-1: [7-(Methoxymethyl)-2-naphthyl]methanol (570 mg, colorless solid) was obtained from the compound obtained in Step 1-1 (3.50 g) by the same method as in Step 1-2.
¹H NMR (200 MHz, CDCl₃, δ): 3.43 (s, 3H), 4. 62 (s, 2H), 4.84 (s, 2H), 7.41-7.50 (m, 2H), 7.68-7.93 (m, 4H); ESI MS m/z 201. (M-H)⁻.

Step 134-2: CBr₄ (590 mg) and PPh₃ (467 mg) were added to a solution of the compound obtained in Step 134-1 (300 mg) in CHCl₃ (3.00 mL), and the mixture was stirred at room temperature for 12 hours. Water was added, followed by extraction with CHCl₃ three times. The combined organic layers were dried over MgSO₄ and then concentrated under reduced pressure. The residue was purified by column chromatography (Silica gel 60 N, mobile phase: EtOAc/hexane = 1/20 to 1/10; v/v) to obtain 2-(bromomethyl)-7-(methoxymethyl)naphthalene (210 mg, colorless solid).
¹H NMR (200 MHz, CDCl₃, δ): 3.43 (s, 3H), 4. 62 (s, 2H), 4.66 (s, 2H), 7.42-7.54 (m, 2H), 7.72-7.88 (m, 4H).

Step 134-3: The title compound (160 mg, colorless solid) was obtained from the compound obtained in Step 134-2 (205 mg) and the compound obtained in Step 6-3 (218 mg) by the same method as in Step 133-2.
¹H NMR (600 MHz, CDCl₃, δ): 1.51-1.63 (m, 2H), 1.96-2.06 (m, 2H), 2.21 (t, J = 10.8 Hz, 2H), 2.84-2.94 (m, 2H), 3.42 (s, 3H), 3.66 (s, 2H), 3.93-4.03 (m, 1H), 4.61 (s, 2H), 5.88 (d, J = 7.8 Hz, 1H), 7.17 (t, J = 8.5 Hz, 1H), 7.43 (d, J = 8.3 Hz, 1H), 7.47 (d, J = 8.3 Hz, 1H), 7.62 (ddd, J = 8.6, 4.5, 2.1 Hz, 1H), 7.71 (s, 1H), 7.74 (s, 1H), 7.77-7.85 (m, 3H); ESI/APCI MS m/z 441, (M+H)⁺.

### Example 135: Synthesis of 3-methoxy-N-[1-(1-{7-[(2-methoxyethoxy)methyl]-2-naphthyl}ethyl)piperidin-4-yl]benzamide hydrochloride

Step 135-1: 1 M MeMgBr/THF solution (3.00 mL) was added to a solution of the compound obtained in Step 1-3 (250 mg) in THF (5.00 mL), and the mixture was stirred at room temperature for one hour. Saturated aqueous NH₄Cl solution was added, followed by extraction with EtOAc. The combined organic layers were dried over MgSO₄ and then concentrated under reduced pressure. The residue was purified by column chromatography (Silica gel 60 N, mobile phase: EtOAc/hexane = 1/4 to 1/1; v/v) to obtain 1-{7-[(2-methoxyethoxy)methyl]-2-naphthyl}ethanol (259 mg, colorless oil).
¹H NMR (600 MHz, CDCl₃, δ): 1.57 (d, J = 6.4 Hz, 3H), 1.90 (brs, 1H), 3.40 (s, 3H), 3.57-3.60 (m, 2H), 3.63-3.67 (m, 2H), 4.73 (s, 2H), 5.06 (q, J = 6.7 Hz, 1H), 7.44-7.51 (m, 2H), 7.75-7.84 (m, 4H); ESI/APCI MS m/z 283, (M+Na)⁺,

Step 135-2: 1-{7-[(2-Methoxyethoxy)methyl]-2-naphthyl}ethanone (198 mg, colorless oil) was obtained from the compound obtained in Step 135-1 (259 mg) by the same method as in Step 1-3.
¹H NMR (600 MHz, CDCl₃, δ): 2.71 (s, 3H), 3.40 (s, 3H), 3.57-3.63 (m, 2H), 3.65-3.70 (m, 2H), 4.75 (s, 2H), 7.56-7.62 (m, 1H), 7.86 (t, J = 8.7 Hz, 2H), 7.91 (s, 1H), 7.98-8.03 (m, 1H), 8.44 (s, 1H); ESI/APCI MS m/z 259, (M+H)⁺.

Step 135-3: 3-Methoxy-N-[1-(1-{7-[(2-methoxyethoxy)methyl]-2-naphthyl}ethyl)piperidin-4-yl]benzamide (105 mg, colorless oil) was obtained from the compound obtained in Step 135-2 (198 mg) and the compound obtained in Step 1-4 (361 mg) by the same method as in Example 130.
¹H NMR (600 MHz, CDCl₃, δ): 1.49 (d, J = 6.4 Hz, 3H), 1.53-1.99 (m, 3H), 2.03-2.08 (m, 1H), 2.18-2.30 (m, 2H), 2.80-2.91 (m, 1H), 3.06-3.18 (m, 1H), 3.40 (s, 3H), 3.57-3.60 (m, 2H), 3.63-3.70 (m, 3H), 3.83 (s, 3H), 3.92-4.00 (m, 1H), 4.73 (s, 2H), 5.96-6.05 (m, 1H), 6.99-7.03 (m, 1H), 7.21-7.24 (m, 1H), 7.29-7.33 (m, 2H), 7.43-7.52 (m, 2H), 7.70 (s, 1H) 7.77 (s, 1H), 7.80 (dd, J = 8.3, 5.5 Hz, 2H); ESI MS m/z 477, (M+H)⁺.

Step 135-4: The title compound (83.9 mg, colorless solid) was obtained from the compound obtained in Step 135-3 (105 mg) by the same method as in Step 122-2.
¹H NMR (600 MHz, DMSO-d₆, δ): 1.78-2.17 (m, 8H), 2.81-2.93 (m, 1H), 2.94-3.03 (m, 1H), 3.17-3.23 (m, 1H), 3.28 (s, 3H), 3.50-3.55 (m, 2H), 3.60-3.64 (m, 2H), 3.79 (s, 3H), 3.88-3.97 (m, 1H), 4.56-4.64 (m, 1H), 4.69 (s, 2H), 7.05-7.12 (m, 1H), 7.28-7.47 (m, 3H), 7.54 (d, J = 8.7 Hz, 1H), 7.78-7.90 (m, 2H), 7.96 (d, J = 8.3 Hz, 1H), 8.00-8.06 (m, 1H), 8.11-8.18 (m, 1H), 8.52-8.60 (m, 1H); ESI/APCI MS m/z 477, [M (free)+H]⁺.

### Example 136: Synthesis of N-[1-({7-[3-(1,3-dioxolan-2-yl)propyl]-2-naphthyl}methyl)piperidin-4-yl]-3-methoxybenzamide

Step 136-1: 2.66 M BuLi/hexane solution (5.13 mL) was added dropwise to a suspension of bromo[2-(1,3-dioxolan-2-yl)ethyl]triphenylphosphorane (6.06 g) in THF (40.0 mL) with ice-cooling. The mixture was stirred at the same temperature for 30 miuntes, followed by addition of the compound obtained in Example 5 (5.00 g). The mixture was stirred at room temperature for 15 hours and then saturated aqueous NH₄Cl solution was added. H₂O was added, followed by extraction with CHCl₃. The organic layer was dried over MgSO₄ and then concentrated under reduced pressure. The residue was purified by column chromatography (Silica gel 60 N, mobile phase: EtOAc/hexane = 67/33; v/v) to obtain N-[1-({7-[3-(1,3-dioxolan-2-yl)prop-1-en-1-yl]-2-naphthyl}methyl)piperidin-4-yl]-3-methoxybenzamide (5.73 g, colorless solid).
¹H NMR (600 MHz, CDCl₃, δ): 1.53-4.09 (m, 20H), 5.01 (q, J = 4.6 Hz, 0.6H), 5.17-5.23 (m, 0.4H), 5.78-5.87 (m, 0.4H), 5.95-6.07 (m, 0.6H), 6.29-6.39 (m, 1H), 6.65 (d, J = 16.1 Hz, 0.6H), 6.73 (d, J = 11.5 Hz, 0.4H), 6.98-7.94 (m, 10H); ESI/APCI MS m/z 485, (M-H)⁻.

Step 136-2: 10% Pd/C (948 mg) was added to a suspension of the compound obtained in Step 136-1 (3.16 g) in MeOH (20.0 mL) and CHCl₃ (10.0 mL), and the mixture was stirred under hydrogen atmosphere at room temperature for three days. The reaction solution was filtered through celite and the filtrate was concentrated under reduced pressure. The residue was purified by column chromatography (Chromatorex NH, mobile phase: EtOAc/hexane = 50/50; v/v). The resulting solid was collected by filtration from Et₂O/EtOAc to obtain the title compound (2.05 g, colorless solid).
¹H NMR (600 MHz, CDCl₃, δ): 1.55-1.63 (m, 2H), 1.65-1.71 (m, 2H), 1.75-1.82 (m, 2H), 2.00-2.07 (m, 2H), 2.20-2.28 (m, 2H), 2.80 (t, J = 7.3 Hz, 2H), 2.90 (d, J = 11.0 Hz, 2H), 3.31 (s, 5H), 3.67 (s, 2H), 3.86 (s, 3H), 3.98-4.09 (m, 1H), 5.96 (d, J = 7.8 Hz, 1H), 7.04 (dd, J = 8.3, 2.8 Hz, 1H), 7.23-7.28 (m, 1H), 7.29-7.36 (m, 3H), 7.40-7.47 (m, 1H), 7.59 (s, 1H), 7.67 (s, 1H), 7.73-7.79 (m, 2H); FAB MS m/z 489, (M+H)⁺.

### Example 137: Synthesis of 3-methoxy-N-(1-{[7-(4-oxobutyl)-2-naphthyl]methyl}piperidin-4-yl)benzamide

1 M aqueous HCl solution (6.00 mL) was added to a suspension of the compound obtained in Example 136 (648 mg) in THF (6.00 mL), and the mixture was stirred at room temperature for 15 hours. The reaction solution was basified with 1 M aqueous NaOH solution with ice-cooling. The generated solid was collected by filtration to obtain the title compound (326 mg, colorless solid).
¹H NMR (600 MHz, CDCl₃, δ): 1.47-1.86 (m, 2H), 2.01-2.08 (m, 4H), 2.27-2.39 (m, 2H), 2.49 (td, J = 7.3, 1.4 Hz, 2H), 2.81 (t, J = 7.3 Hz, 2H), 2.95-3.06 (m, 2H), 3.73-3.80 (m, 2H), 3.84 (s, 3H), 4.00-4.11 (m, 1H), 6.02-6.11 (m, 1H), 7.00-7.04 (m, 1H), 7.22-7.26 (m, 1H), 7.29-7.34 (m, 3H), 7.48 (d, J = 8.7 Hz, 1H), 7.59 (s, 1H), 7.72 (s, 1H), 7.77 (dd, J = 12.8, 8.3 Hz, 2H), 9.77 (t, J = 1.6 Hz, 1H); ESI/APCI MS m/z 445, (M+H)⁺.

### Example 138: Synthesis of N-(1-{[7-(4-hydroxybutyl)-2-naphthyl]methyl}piperidin-4-yl)-3-methoxybenzamide

The title compound (135 mg, colorless solid) was obtained from the compound obtained in Example 137 (300 mg) by the same method as in Example 120.
¹H NMR (600 MHz, CDCl₃, δ): 1.50-1.68 (m, 5H), 1.74-1.83 (m, 2H), 1.97-2.06 (m, 2H), 2.18-2.27 (m, 2H), 2.80 (t, J = 7.6 Hz, 2H), 2.84-2.92 (m, 2H), 3.63-3.71 (m, 4H), 3.84 (s, 3H), 3.97-4.06 (m, 1H), 5.94 (d, J = 7.3 Hz, 1H), 7.02 (dd, J = 7.8, 2.3 Hz, 1H), 7.21-7.25 (m, 1H) 7.28-7.34 (m, 3H), 7.40-7.45 (m, 1H), 7.58 (s, 1H), 7.66 (s, 1H), 7.75 (t, J = 9.2 Hz, 2H); ESI/APCI MS m/z 447, (M+H)⁺.

### Example 139: Synthesis of ethyl 3-[7-({4-[(3-methoxybenzoyl)amino]piperidin-1-yl}methyl)-2-naphthyl]propanoate

Step 139-1: NaH (109 mg) was added to a solution of ethyl (diethoxyphosphoryl)acetate (550 µL) in DMF (12.0 mL) with ice-cooling, followed by stirring for 30 minutes. The compound obtained in Example 5 (1.00 g) was added and the mixture was stirred at room temperature for 15 hours, followed by addition of saturated aqueous NH₄Cl solution. H₂O was added, followed by extraction with CHCl₃. The organic layer was dried over MgSO₄ and then concentrated under reduced pressure. The residue was collected by filtration with Et₂O to obtain ethyl 3-[7-({4-[(3-methoxybenzoyl)amino]piperidin-1-yl}methyl)-2-naphthyl]acrylate (1.02 g, colorless solid).
¹H NMR (600 MHz, DMSO-d₆, δ): 1.28 (t, J = 7.1 Hz, 3H), 1.53-1.67 (m, 2H), 1.72-1.86 (m, 2H), 1.99-2.15 (m, 2H), 2.80-2.93 (m, 2H), 3.59-3.67 (m, 2H), 3.73-3.85 (m, 4H), 4.22 (q, J = 6.9 Hz, 2H), 6.72-6.82 (m, 1H), 7.04-7.12 (m, 1H), 7.31-7.39 (m, 2H), 7.38-7.44 (m, 1H), 7.50-7.60 (m, 1H), 7.77-7.85 (m, 2H), 7.86-7.97 (m, 3H), 8.16-8.30 (m, 2H); ESI/APCI MS m/z 473, (M+H)⁺.

Step 139-2: The title compound (966 mg, colorless solid) was obtained from the compound obtained in Step 139-1 (941 mg) by the same method as in Step 136-2.
¹H NMR (600 MHz, CDCl₃, δ): 1.37 (t, J = 7.1 Hz, 3H), 1.58-1.78 (m, 4H), 2.11-2.22 (m, 2H), 2.46-2.61 (m, 2H), 2.79-2.94 (m, 2H), 3.52-3.63 (m, 2H), 3.84 (s, 3H), 4.20-4.38 (m, 5H), 6.68-6.81 (m, 1H), 7.00-7.07 (m, 1H), 7.28-7.36 (m, 3H), 7.70-8.13 (m, 6H); ESI/APCI MS m/z 475, (M+H)⁺.

### Example 140: Synthesis of N-(1-{[7-(3-hydroxypropyl)-2-naphthyl]methyl}piperidin-4-yl)-3-methoxybenzamide

The title compound (296 mg, colorless solid) was obtained from the compound obtained in Example 139 (516 mg) by the same method as in Step 1-1.
¹H NMR (600 MHz, CDCl₃, δ): 1.49-1.68 (m, 4H), 1.94-2.06 (m, 4H), 2.18-2.28 (m, 2H), 2.80-2.95 (m, 4H), 3.65 (s, 2H), 3.71 (t, J = 6.4 Hz, 1H), 3.84 (s, 3H), 3.96-4.06 (m, 1H), 5.95 (d, J = 6.9 Hz, 1H), 7.02 (dd, J = 8.3, 2.8 Hz, 1H), 7.23 (d, J = 7.8 Hz, 1H), 7.29-7.34 (m, 3H) , 7.43 (dd, J = 8.3, 1.8 Hz, 1H), 7. 60 (s, 1H), 7.66 (s, 1H), 7.73-7.78 (m, 2H); ESI/APCI MS m/z 433, (M+H)⁺.

### Example 141: Synthesis of 3-[7-({4-[(3-methoxybenzoyl)amino]piperidin-1-yl}methyl)-2-naphthyl]propanoic acid

LiOH·H₂O (151 mg) was added to a mixed solution of the compound obtained in Example 139 (343 mg) in THF (3.00 mL), MeOH (1.50 mL) and H₂O (0.50 mL), and the mixture was stirred at room temperature for 15 hours. 1 M aqueous HCl solution (3.50 mL) was added dropwise with ice-cooling. H₂O was added, followed by extraction with CHCl₃ three times. The combined organic layers were dried over MgSO₄ and then concentrated under reduced pressure. The residue was collected by filtration from CHCl₃/Et₂O to obtain the title compound (34.5 mg, colorless solid).
¹H NMR (600 MHz, CDCl₃, δ): 1.69-2.13 (m, 7H), 2.42-2.56 (m, 2H), 3.26-3.36 (m, 2H), 3.82 (s, 4H), 3.90 (s, 3H), 4.10-4.22 (m, 1H), 6.31 (m, 1H), 6.96-7.04 (m, 1H), 7.21-8.11 (m, 8H); ESI/APCI MS m/z 447, (M+H)⁺. Example 142: Synthesis of N-(1-{[7-(1-hydroxyethyl)-2-naphthyl]methyl}piperidin-4-yl)-3-methoxybenzamide

The title compound (582 mg, colorless solid) was obtained from the compound obtained in Example 5 (1.00 g) and MeMgBr (3.00 mL) by the same method as in Example 126.
¹H NMR (600 MHz, CDCl₃, δ): 1.52-1.61 (m, 5H), 1.99-2.05 (m, 2H), 2.19-2.28 (m, 2H), 2.84-2.92 (m, 2H), 3.66 (s, 2H), 3.84 (s, 3H), 3.97-4.06 (m, 1H), 5.03-5.10 (m, 1H), 5.94 (d, J = 7.3 Hz, 1H), 7.02 (dd, J = 8.3, 1.8 Hz, 1H), 7.20-7.25 (m, 1H), 7.30-7.34 (m, 2H), 7.45-7.50 (m, 2H), 7.72 (s, 1H), 7.76-7.84 (m, 3H); ESI/APCI MS m/z 419, (M+H)⁺.

### Example 143: Synthesis of 7-({4-[(3-methoxybenzoyl)amino]piperidin-1-yl}methyl)-2-naphthoic acid

The title compound (1.42 g, colorless solid) was obtained from the compound obtained in Example 3 (1.47 g) by the same method as in Example 71.
¹H NMR (600 MHz, DMSO-d₆, δ): 1.54-1.62 (m, 2H), 1.71-1.78 (m, 2H), 2.02-2.08 (m, 2H), 2.81-2.86 (m, 2H), 3.62 (s, 2H), 3.71-3.79 (m, 4H), 7.02-7.05 (m, 1H), 7.30-7.35 (m, 2H), 7.37-7.40 (m, 1H), 7.51-7.58 (m, 1H), 7.85-7.92 (m, 3H), 7.94-7.99 (m, 1H), 8.22 (d, J = 7.8 Hz, 1H), 8.46-8.50 (m, 1H); ESI MS m/z 419, (M+H)⁺.

### Example 144: Synthesis of 7-({4-[(3-methoxybenzoyl)amino]piperidin-1-yl}methyl)-N-(2-pyrrolidin-1-ylethyl)-2-naphthamide dihydrochloride

Step 144-1: 7-({4-[(3-Methoxybenzoyl)amino]piperidin-1-yl}methyl)-N-(2-pyrrolidin-1-ylethyl)-2-naphthamide was obtained from the compound obtained in Example 143 (250 mg) and 2-pyrrolidin-1-ylethanamine (82.0 mg) by the same method as in Step 7-6.
¹H NMR (600 MHz, CDCl₃, δ): 1.53-1.61 (m, 2H), 1.78-1.85 (m, 4H), 2.00-2.06 (m, 2H), 2.20-2.27 (m, 2H), 2.56-2.61 (m, 4H), 2.75 (t, J = 6.0 Hz, 2H), 2.85-2.91 (m, 2H), 3.59-3.63 (m, 2H), 3.67 (s, 2H), 3.84 (s, 3H), 3.98-4.06 (m, 1H), 5.94-5.98 (m, 1H), 6.99-7.04 (m, 2H), 7.22-7.25 (m, 1H), 7.31 (d, J = 7.8 Hz, 1H), 7.32-7.34 (m, 1H), 7.55-7.58 (m, 1H), 7.80-7.84 (m, 3H), 7.85-7.88 (m, 1H), 8.27-8.28 (m, 1H).

Step 144-2: The title compound (140 mg, colorless solid) was obtained from the compound obtained in Step 144-1 by the same method as in Step 122-2.
¹H NMR (600 MHz, DMSO-d₆, δ): 1.81-1.91 (m, 2H), 1.91-2.10 (m, 5H), 2.98-3.06 (m, 2H), 3.06-3.15 (m, 2H), 3.20-3.29 (m, 1H), 3.32-3.40 (m, 4H), 3.59-3.69 (m, 4H), 3.74-3.79 (m, 3H), 3.93-4.14 (m, 1H), 4.42-4.54 (m, 2H), 7.03-7.11 (m, 1H), 7.32 (t, J = 8.0 Hz, 1H), 7.34-7.46 (m, 2H), 7.83-7.87 (m, 1H), 8.02-8.08 (m, 3H), 8.22-8.26 (m, 1H), 8.35-8.55 (m, 1H), 8.56 (s, 1H), 9.08-9.13 (m, 1H), 10.37-10.45 (m, 1H), 10.74-10.97 (m, 1H); ESI MS m/z 515, [M (free)+H]⁺.

The compounds of Example 145 to Example 147 were obtained by the same method as in Example 144.

**[Table 8]**

| Example No. | Compound name | Physical data |
|---|---|---|
| **145** | 7-({4-[(3-Methoxybenzoyl)amino]pipendin-1-yl}methyl)-N-(2-methoxyethyl)-2-naphthamide hydrochloride | **¹H NMR (600 MHz, DMSO-d₆,** δ**): 1.86-1.94 (m, 2H), 1.94-2.10 (m, 2H), 3.06-3.15 (m, 2H), 3.24-3.27 (m, 3H), 3.36-3.42 (m, 2H), 3.44-3.49 (m, 4H), 3.75-3,78 (m, 3H), 3.93-4.14 (m, 1H), 4.42-4.52 (m, 2H), 7.03-7.11 (m, 1H), 7.33 (t, J = 7.8 Hz, 1H), 7.34-7.38 (m, 1H), 7.39-7.43 (m, 1H), 7.79-7.83 (m, 1H), 7.98-8.01 (m, 1H), 8.01-8.06 (m, 2H), 8.18-8.21 (m, 1H), 8.44-8.47 (m, 1H), 8.51 (d, *J*=7.3 Hz, 1H), 8.64-8.79 (m, 1H), 10.48-10.70 (m, 1H); ESI MS m/z 476 [M (free)+H]⁺.** |
| **146** | N-Benzyl-7-({4-[(3-methoxybenzoyl)amino]piperidin-1-yl}methyl)-2-naphthamide hydrochloride | **¹H NMR (600 MHz, DMSO-d₆,** δ**): 1.86-1.95 (m, 2H), 1.95-2.10 (m, 2H), 3.07-3.15 (m, 2H), 3.36-3.42 (m, 2H), 3.75-3.78 (m, 3H), 3.93-4.14 (m, 1H), 4.42-4.55 (m, 4H), 7.04-7.11 (m, 1H), 7.21-7.25 (m, 1H), 7.29-7.38 (m, 6H), 7.39-7.44 (m, 1H), 7.81-7.85 (m, 1H), 8.02-8.08 (m, 3H), 8.19-8.34 (m, 1H), 8.50-8.53 (m, 2H), 9.28-9.33 (m, 1H), 10.50-10.72 (m, 1H); ESI MS m/z 508 [M (free)+H]⁺.** |
| **147** | N-(Cyclopropylmethyl)-7-({4-[(3-methoxybenzoyl)amino]piperidin-1-yl}methyl)-2-naphthamide hydrochloride | **¹H NMR (600 MHz, DMSO-d₆,** δ**): 0.21.0.26 (m, 2H), 0.40-0.45 (m, 2H), 1.01-1.08 (m, 1H), 1.86-2.10 (m, 4H), 3.06-3.15 (m, 2H), 3.16-3.20 (m, 2H), 3.36-3.42 (m, 2H), 3.75-3.78 (m, 3H), 3.93-4.14 (m, 1H), 4.42-4.52 (m, 2H), 7.04-7.10 (m, 1H), 7.33 (t, J = 7.8 Hz, 1H), 7.34-7.38 (m, 1H), 7.38-7.43 (m, 1H), 7.79-7.83 (m, 1H), 7.98-8.07 (m, 3H), 8.18-8.21 (m, 1H), 8.31-8.53 (m, 2H), 8.79-8.83 (m, 1H), 10.50-10.71 (m, 1H); ESI MS m/z 472 [M (free)+H]⁺.** |

### Reference Example 1: Synthesis of 7-[(2-methoxyethoxy)methyl]-2-naphthaldehyde

Step 1-1: Sodium 7-(carbomethoxy)naphthalene-2-carboxylate (12.6 g) obtained according to a method described in JP-A-6-107599 was suspended in toluene (126 mL), and toluene was evaporated under reduced pressure. The compound was suspended again in toluene (126 mL), followed by heating to 79°C. Thionyl chloride (7.14 g) was added dropwise, followed by stirring at 79°C for 20 minutes. Then, thionyl chloride (16.7 g) and N,N-dimethylformamide (0.500 g) were added dropwise. After stirring at 79°C for 1.5 hours, the reaction solution was concentrated under reduced pressure. The concentrated residue was suspended in N,N-dimethylformamide (63.0 mL) to obtain a suspension A. N-methoxymethanamine hydrochloride (5.37 g) was suspended in N,N-dimethylformamide (37.8 mL), and then triethylamine (12.7 g) was added to obtain a suspension B. The suspension B was added to the suspension A. After stirring at room temperature for one hour, triethylamine (2.02 g) and N-methoxymethanamine hydrochloride (0.490 g) were added and the mixture was further stirred for 30 minutes. The reaction solution was separated with water (300 mL) and toluene (100 mL). The lower layer was extracted with toluene (100 mL) twice. The organic layers were combined, washed with water (100 mL) three times and then concentrated under reduced pressure to obtain methyl 7-[methoxy(methyl)carbamoyl]naphthalene-2-carboxylate (11.3 g, pale brown solid).
¹H NMR (300 MHz, CDCl₃, δ): 3.43 (s, 3H), 3.57 (s, 3H), 3.99 (s, 3H), 7.84-7.94 (m, 3H), 8.13 (dd, J = 8.7, 1.5 Hz, 1H), 8.34 (d, J = 0.9 Hz, 1H), 8.66 (brs, 1H); ESI MS m/z 274, (M+H)⁺.

Step 1-2: The compound obtained at the step 1-1 (11.3 g) was dissolved in tetrahydrofuran (113 mL). The solution was added dropwise to a suspension of lithium aluminum hydride (1.88 g) in tetrahydrofuran (226 mL) in an argon atmosphere while maintaining the temperature at -24 to -19°C. The mixture was gradually warmed up to 0°C. After one hour, ethyl acetate (50.0 mL) was added dropwise. After dropwise addition of 3 M hydrochloric acid (150 mL), the mixture was separated with toluene (200 mL). The lower layer was extracted with toluene (100 mL). The organic layers were combined, washed with water (100 mL) three times and then concentrated under reduced pressure to obtain 7-(hydroxymethyl)naphthalene-2-carbaldehyde (7.34 g, pale brown solid).
¹H NMR (300 MHz, CDCl₃, δ): 1.99 (t, J = 6.0 Hz, 1H), 4.91 (d, J = 5.1 Hz, 2H), 7.64 (dd, J = 8.7, 1.8 Hz, 1H), 7.87-8.01 (m, 4H), 8.31 (d, J = 0.9 Hz, 1H), 10.14 (s, 1H); EI MS m/z 186, M⁺.

Step 1-3: The compound obtained at the step 1-2 (7.32 g) was dissolved in methanol (146 mL). Trimethoxymethane (14.6 g) and pyridine p-toluenesulfonate (0.500 g) were added to the solution, and the mixture was heated under reflux for 1.5 hours. After cooling to room temperature, sodium carbonate (1.00 g) was added and the mixture was concentrated under reduced pressure. Toluene (140 mL), saturated aqueous sodium bicarbonate solution (20.0 mL), water (60.0 mL) and ethyl acetate (140 mL) were added to the concentrated residue, and the mixture was stirred and dissolved. The upper layer was separated and the lower layer was extracted with ethyl acetate (70.0 mL). The organic layers were combined, washed with water (30.0 mL) three times and then concentrated under reduced pressure. The residue was redissolved in toluene (100 mL), followed by concentration under reduced pressure, to obtain [7-(dimethoxymethyl)naphthalen-2-yl]methanol (9.09 g, pale brown solid).
¹H NMR (300 MHz, CDCl₃, δ): 1.91 (t, J = 5.9 Hz, 1H), 3.37 (s, 6H), 4.85 (d, J = 5.1 Hz, 2H), 5.54 (s, 1H), 7.42-7.60 (m, 2H), 7.75-7.96 (m, 4H); TOF MS EI+ m/z, 232, M⁺.

Step 1-4: NaH (60% in oil, 3.14 g) was washed with hexane (5.00 mL) twice in an argon atmosphere and suspended in dimethyl sulfoxide (45.6 mL). A solution of the compound obtained in Step 1-3 (9.09 g) in dimethyl sulfoxide (45.6 mL) was added dropwise to the suspension at room temperature. After stirring at room temperature overnight, 1-bromo-2-methoxyethane (10.9 g) was added dropwise while maintaining the temperature at 24 to 32°C. After stirring at room temperature for 1.5 hours, 1-bromo-2-methoxyethane (10.9 g) was added. Then, NaH (60% in oil, 3.14 g) was washed with hexane (5.00 mL) twice and added. The mixture was heated to 80°C over four hours and further heated at 80°C for 1.5 hours. After leaving to cool to room temperature, methanol (10.0 mL) and water (50.0 mL) were added. The mixture was adjusted to pH < 1 with 2 M hydrochloric acid (120 mL) and stirred at room temperature for 10 minutes. After addition of toluene (100 mL) and stirring, the upper layer was separated. The lower layer was extracted with toluene (100 mL). The organic layers were combined, washed with water (100 mL) three times and then concentrated under reduced pressure to obtain a brown oil (9.59 g). 4.79 g of the resulting brown oil was distilled under reduced pressure and purified to obtain 7-[(2-methoxyethoxy)methyl]-2-naphthaldehyde (3.64 g, pale yellow oil) as a fraction at 170 to 176 °C (1.5 hPa).
¹H NMR (300 MHz, CDCl₃, δ): 3.42 (s, 3H), 3.58-3.66 (m, 2H), 3.67-3.74 (m, 2H), 4.77 (s, 2H), 7.64 (dd, J = 8.6, 1.7 Hz, 1H) 7.85-8.00 (m, 4H), 8.32 (brs, 1H), 10.15 (s, 1H); EI MS m/z 244, M⁺.

### Reference Example 2: Synthesis of 2-(dimethoxymethyl)-7-[(2-methoxyethoxy)methyl]naphthalene

NaH (60% in oil, 0.224 g) was washed with hexane (1.00 mL) twice in an argon atmosphere and suspended in dimethyl sulfoxide (2.00 mL). A solution of [7-(dimethoxymethyl)naphthalen-2-yl]methanol (0.652 g) obtained by the same method as in Step 1-3 of Example 1 in dimethyl sulfoxide (2.00 mL) was added dropwise to the suspension at room temperature. After stirring for 30 minutes, 1-bromo-2-methoxyethane (0.778 g) was added dropwise. The mixture was stirred at room temperature overnight and then heated at 50°C for 30 minutes. After leaving to cool to room temperature, toluene (20.0 mL) and water (10.0 mL) were added. After stirring, the upper layer was separated. The upper layer was washed with water (5.00 mL) three times and then concentrated under reduced pressure. The residue was redissolved in toluene (20.0 mL), followed by concentration under reduced pressure, to obtain 2-(dimethoxymethyl)-7-[(2-methoxyethoxy)methyl]naphthalene (0.633 g, colorless oil).
¹H NMR (300 MHz, CDCl₃, δ): 3.37 (s, 6H), 3.41 (s, 3H), 3.57-3.62 (m, 2H), 3.63-3.68 (m, 2H), 4.74 (s, 2H), 5.55 (s, 1H), 7.46-7.57 (m, 2H), 7.76-7.94(m, 4H); EI MS m/z 290, M⁺.

The compounds of Example 148 to Example 163 were obtained by the same method as in Example 72.

**[Table 9-1]**

| Example No. | Compound name | Physical data |
|---|---|---|
| **148** | 2-(3,4-Dicbiorophenyl)-N-(1-{[7-(methoxymethyl)naphthalen-2-yl]methyl}piperidin-4-yl)acetamide | **¹H NMR (600 MHz, CDCl₃, δ): 1.34-1.44 (m, 2H), 1.84-1.90 (m, 2H), 2.14 (t, J=10.8 Hz, 2H), 2.75-2.84 (m, 2H), 3.42 (s, 3H), 3.45(s, 2H), 3.61 (s, 2H), 3.75-3.84 (m, 1H), 4.60 (s, 2H), 5.22 (d, J = 7.8 Hz, 1H), 7.07-7.11 (m, 1H), 7.36 (d, J = 1.6 Hz, 1H), 7.39 (d, J = 8.3 Hz, 1H), 7.43 (dt, J = 8.3, 1.6 Hz, 2H), 7.68 (s, 1H), 7.73 (s. 1H), 7.76 (d, J = 8.3 Hz, 1H), 7.79 (d, J = 8.3 Hz, 1H); ESI/APCI MS m/z 471 (M+H)⁺.** |
| **149** | N-[1-{(7-[(2-Methoxyethoxy)methyl]naphthalen-2-yl}cnethyl)piperidin-4-yl]-2,2-diphenylacetamide | **¹H NMR (600 MHz, CDCl₃, δ): t.32-t.42 (m, 2H). 1.86-1.92 (m, 2H), 2.14 (t, J = 10.5 Hz, 2H), 2.68-2.78 (m, 2H, 3.40 (s, 3H), 3.55-3.61 (m, 4H), 3.62-3.66 (m, 2H), 3.85-3.93 (m, 1H), 4.72 (s, 2H), 4.89 (s, 1H), 5.40 (d, J = 7.8 Hz, 1H), 7.29-7.27 (m, 6H), 7.28-7.33 (m, 4H), 7.38-7.46 (m, 2H), 7.66 (s, 1H), 7.73 (s, 1H), 7.75 (d, J = 8.3 Hz, 1H), 7.78 (d, J = 8.3 Hz, 1H); ESI/APCI MS m/z 523 (M+H)⁺.** |
| **150** | N-[1-({7-[(2-Methoxyethoxy)methyl]naphthalen-2-yl}methyl)piperidin-4-yl]-3-(3-methoxyphenyl)propanamide | **¹H NMR (600 MHz, CDCl₃, δ): 1.29-1.39 (m, 2H). 1.79-1.86 (m, 2H), 2.13 (t, J = 10.8 Hz, 2H), 2.43 (t, J = 7.6 Hz, 2H), 2.76 (d, J = 9.6 Hz, 2H), 2.92 (t, J = 7.6 Hz, 2H), 3.40 (s, 3H, 3.56-3.66 (m, 6H), 3.74-3.82 (m, 4H, 4.72 (s, 2H), 5.12 (d, J = 8.3 Hz, 1H), 6.71-6.75 (m, 2H), 6.77 (d, J = 7.8 Hz, 1H), 7.15-7.20 (m, 1H), 7.41-7.46 (m, 2H), 7.68 (s, 1H), 7.74 (s, 1H), 7.76 (d, J = 8.3 Hz, 1H), 7.79 (d, J = 8.3 Hz, 1H); ESI/APCI MS m/z 491 (M+H)⁺.** |
| **151** | (2S)-N-[1-({7-[(2-Methoxyethoxy)methyl]naphthalen-2-yl}methyl)piperidin-4-yl]-2-phenylpropanamide | **¹H NMR (600 MHz, CDCl₃, δ): 1.22-1.36 (m, 2H), 1.50 (d, J = 7.0 Hz, 3H), 1.74-1.88 (m, 2H), 2.06-2.16 (m, 2H), 2.67-2.77 (m, 2H), 3.40 (s, 3H), 3.51 (q, J = 7.0 Hz, 1H), 3.55-3.60 (m, 4H) 3.61-3.66 (m, 2H), 3.72-3.80 (m, (q, J = 7.0 (s, 2H), 5.11 (d, J = 7.8 Hz, 1H, 7.22-7.28 (m, 3H), 7.29-7.34 (m, 1H), 4.71 (s, 2H), 5.11 (d, J = 7.8 Hz, 1H), 7.22-7.28 (m, 3H), 7.29-7.34 (m. 2H), 7.38-7.45 (m, 2H), 7.65 (s, 1H), 7.73 (s, 1H), 7.74 (d, J=8.3Hz. 1H), 7.78 (d, J = 8.3 Hz, 1H); ESI/APCI MS m/z 461 (M+H)⁺.** |
| **152** | (2R)-N-[1-({7-m[(2-Methoxyethoxy)methyl]naphthalen-2-yl}methyl)piperidin-4-yl]-2-phenylpropanamide | **¹H NMR (600 MHz, CDCl₃, δ): 1.22-1.37 (m, 2H), 1.50 (d, J = 7.2 Hz, 3H), 1.75-1.87 (m, 2H). 2.06-2.16 (m, 2H), 2.66-2.77 (m 2H), 3.40 (s, 3H), 3.51 (q, J = 7.2 Hz, 1H), 3.56-3,60 (m, 4H), 3.62-3.66 (m, 2H), 3.74-3.80 (m, (q, J = 7.2 Hz, 1H), 3.56-3.60 (m, 4H), 3.62-3.66 (m 2H), 3.74-3.80 (m, 1H), 4.71 (s, 2H), 5,11 (d, J = 8.3 Hz, 1H), 7.23-7.27 (m, 3H), 7.30-7.34 (m. 2H), 7.39-7.45 (m, 2H), 7.65 (s, 1H), 7.73 (s. 1H), 7.74 (d, J = 8.7 Hz, 1H), 7.78 (d, J = 8.7 Hz, 1H); ESI/APCI MS m/z 461 (M+H)⁺.** |

**[Table 9-2]**

| Example No. | Compound name | Physical data |
|---|---|---|
| **153** | 2,2-Bis(4-chlorophenyl)-N-[1-({7-methoxyethoxy)methyl]naphthalen-2-yl}methyl)piperidin-4-yl]acetamide | **¹H NMR (600 MHz, CDCl₃, δ): 1.34-1.43 (m, 2H, 1.85-1.92 (m, 2H, 2.14 (t, J = 10.3 Hz, 2H), 2.73-2.81 (m, 2H), 3.40 (s, 3H), 3.56-3.60 (m, 2H), 3.60 (s, 2H), 3.62-3.67 (m, 2H), 3.82-3.89 (m, 1H), 4.72 (s, 2H), 4.76 (s, 1H) 5.38 (d, J=7.8 Hz, 1H), 7.13-7.17 (m, 4H), 7.26-7.30 (m, 4H), 7.40-7.45 (m, 2H), 7.67 (s, 1H), 7.74 (s, 1H), 7.75 (d, J = 8.7 Hz, 1H), 7.78 (d, J = 8.7 Hz, 1H); ESI/APCI MS m/z 591 (M+H)⁺.** |
| **154** | N-[1-({7-[(2-Methoxyethoxy)methyl]naphthalen-2-yl}methyl)piperidin-4-yl]-2-methyl-2-phenylpropanamide hydrochloride | **¹H NMR (600 MHz, CDCl₃, δ): 1.51 (s, 6H), 1.91 (d, J = 13.8 Hz, 2H), 2.06-2.18 (m, 2H), 2.65-2.74 (m, 2H), 3.40 (s, 3H), 3.41-3.47 (m, 2H), 3.57-3.62 (m, 2H), 3.64-3.69 (m. 2H), 3.88-3.98 (m. 1H), 4.20-4.27 (m, 2H), 4.73 (s, 2H), 5.21 (d, J = 8.3 Hz, 1H), 7.22-7.28 (m. 3H). 7.28-7.34 (m, 2H). 7.56 (d, J = 10.1 Hz, 1H), 7.74 (d, J = 8.7 Hz, 1H), 7.81-1.86 (m, 2H), 7.89 (d, J = 8.7 Hz, 1H), 7.99 (s, 1H), 12.68 (br. s., 1H); ESI/APCI MS m/z 475 [M (free)+H]⁺.** |
| **156** | N-[1-({7-[(2-Methoxyethoxy)methyl]naphthalen-2-yl}methyl)piperidin-4-yl]-1-phenylcyclopropanecarboxamide hydrochloride | **¹H NMR (600 MHz, CDCl₃, δ): 1.01-1.06 (m, 2H), 1.50-1.55 (m. 2H). 1.92 (d, J = 12.8 Hz, 2H), 2.02-2.12 (m, 2H), 2.65-2.73 (m 2H). 3.40 (s, 3H), 3.40-3.45 (m, 2H), 3.57-3.61 (m, 2H), 3.64-3.68 (m, 2H), 3.83-3.92 (m, 1H), 4.19-4.27 (m, 2H), 4.72 (s, 2H), 5.32 (d, J = 6.9 Hz, 1H), 7.28-7.38 (m, 5H), 7.55 (d, J = 8.3 Hz, 1H), 7.74 (d, J = 8.3 Hz, 1 H), 7.81-7.85 (m, 2H), 7.88 (d, J = 8.3 Hz, 1H), 7.98 (s, 1H), 12.64 (br. s., 1H); ESI/APCI MS m/z 473 [M (free)+H]⁺.** |
| **156** | Methoxyethoxy)methyl]naphthalen-2-yl}methyl)piperidin-4-yl]cyclohexanecarboxamide | **N-[1-({7-[(2- ¹H NMR (600 MHz, CDCl₃, δ): 1.14-1.30 (m, 3H), 1.33-1.47 (m, 4H), 1.63-1.68 (m, 1H), 1.72-1.93 (m, 6H, 1.97-2.05 (m, 1H), 2.11-219 (m, 2H), 2.82 (d, J = 10.5 Hz, 2H), 3.40 (s, 3H), 3.56-3.60 (m, 2H), 3.61-3.66 (m, 4H), 3.76-3.84 (m, 1H), 4.72 (s, 2H), 5.25 (d, J = 7.8 Hz, 1H), 7.42-7.46 (m, 2H), 7.69 (s, 1H), 7.75 (s, 1H), 7.77 (d, J = 8.7 Hz, 1H), 7.79 (d, J = 8.7 Hz, 1H); BSI/APCI MS m/z 439 (M+H)⁺.** |
| **157** | N-[1-({7-[(2-Methoxyethoxy)methyl]naphthalen-2-yl}methyl)piperidin-4-yl]quinoline-3-carboxamide | **¹H NMR (600 MHz, CDCl₃, δ): 1.61-1.70 (m, 2H), 2.06-2.12 (m, 2H), 2.27 (t, J = 10.8 Hz, 2H), 2.90-2.97 (m, 2H), 3.40 (s, 3H), 3.57-3.6 (m, 2H), 3.63-3.67 (m. 2H), 3.69 (s, 2H), 4.07-4.14 (m, 1H), 4.73 (s, 2H), 6. 10 (d, J = 7.3 Hz, 1H). 7.44-7.47 (m, 1H), 7.47-7.50 (m, 1H), 7.59-7.63 (m, 1H), 7.73 (s, 1H), 7.77 (s, 1H), 7.78-7.82 (m, 3H), 7.91 (d, J = 7.3 Hz, 1H), 8.15 (d, J 8.7 Hz, 1H), 8.55 (d, J = 2.3 Hz, 1H). 9.23 (d, J = 2.3 Hz, 1H); ESI/APCI MS m/z 484 (M+H)⁺.** |
| **158** | N-[1-({7-[(2-Methoxyethoxy)methyl]naphthalen-2-yl}methyl)piperidin-4-yl]-1H-pyrrole-3-carboxamide | **¹H NMR (600 MHz, CDCl₃, δ): 1.48-1.61 (m. 2H), 1.97-2.03 (m, 2H), 2.21 (t, J = 11.0 Hz, 2H), 2.83-2.90 (m, 2H), 3.40 (s, 3H), 3.56-3.60 (m, 2H), 3.62-3.68 (m, 4H), 3.95-4.03 (m, 1H), 4.72 (s, 2H), 5.61 (d, J = 7.8 Hz, 1H), 6.38-6.40 (m, 1H), 6.73-6.76 (m, 3H), 7.33-73S (m, 1H), 7.42-7.49 (m, 2H). 7.71 (s, 1H), 7.75 (s, 1H), 7.78 (d, J = 8.3 Hz, 1H), 7.80 (d, J = 8.7 Hz, 1H), 8.47 (br. s., 1H); ESI/APCI MS m/z 422 (M+H)⁺.** |

**[Table 9-3]**

| Example No. | Compound name | Physical data |
|---|---|---|
| **159** | N-[1-({7-[(2-Methoxyethoxy)methyl]naphthalen-2-yl}methyl)piperidin-4-yl]furan-3-carboxamide | **¹H NMR (600 MHz. CDCl₃, δ): 1.48-1.58 (m, 2H), 1.96-2.02 (m, 2H), 2.20 10.8 Hz, 2H), 2.81-2.92 (m, 2H), 3.40 (s, 3H), 3.55-3.60 (m, 2H), 3.62-3.67 (m, 4H), 3.92-4.02 (m, 1H), 4.72 (s, 2H), 5.56 (d, J = 7.8 Hz, 1H), 3.62-3.67 (m, 4H), 3.92-4.02 (m, 1H), 4.72 (s, 2H), 5.56 (d, J = 7.8 Hz, 1H), 6.54-6.58 (m, 1H), 7.42 (t, J = 1.8 Hz, 1H), 7.43-7.48 (m, 2H), 7.70 (s, 1H), 7.75 (s, 1H), 7.78 (d, J = 8.3 Hz, 1H), 7.80 (d, J = 8.7 Hz, 1H), 7.89 (s, 1H); ESI/APCI MS m/z 423 (M+H)⁺.** |
| **160** | N-[1({7-[(2-Methoxyethoxy)mtethyl]naphthalen- 2-yl}methyl)piperidin-4-yl]thiophene-3-carboxamide | **¹H NMR (600 MHz, CDCl₃, δ): 1.49-1.63 (m, 2H), 1.99-2.04 (m, 2H), 2.16-2.26 (m, 2H), 2.84-2.93 (m, 2H). 3.40 (s, 3H), 3.56-3.60 (m, 2H), 3.62-3.69 (m, 4H), 3.94-4.03 (m, 1H), 4.73 (s, 2H), 5.76 (d, J = 8.7 Hz, 1H), 7.30-7.36 (m, 2H), 7.42-7.43 (m, 2H), 7.71 (s, 1H), 7.73-7.84 (m, 4H); ESI/APCI MS m/z 439 (M+H)⁺,** |
| **161** | N-[1-({7-[(2-Methoxyethoxy)methyl]naphthalen-2-yl}methyl)piperidin-4-yl]-1H-indole-2-carboxamide | **¹H NMR (600 MHz, CDCl₃, δ): 1.53-1.67 (m, 2H), 1.99-2.07 (m, 2H), 2.24 (t, J = 11.0 Hz, 2H), 2.86-2.95 (m, 2H), 3.40 (s, 3H), 3.57-3.61 (m, 2H), 3.63-3.70 (m, 4H), 3.99-4.08 (m, 1H), 4.73. (s, 2H), 5.99 (d, J = 8.3 Hz, 1H), 6.79-6.32 (m, 1H). 7.11-7.16 (m, 1H), 7.26-7.30 (m, 1H), 7.38-7.51 (m, 3H), 7.64 (d, J = 7.8 Hz, 1H), 7.72 (s, 1H), 7.75-7.83 (m, 3H), 9.12 (br. s., 1H); ESI/APCI MS m/z 472 (M+H)⁺.** |
| **162** | N-[1-({7-[(2-Methoxyethoxy)methyl]naphthalen-2-yl}methyl)piperidin-4-yl]-9H-xanthene-9-carboxamide | **¹H NMR (600 MHz, CDCl₃, δ): 1.16-1.26 (m, 2H), 1.69-1.77 (m, 2H), 2.07 (t, J = 10.5 Hz, 2H), 2.54-2.67 (m, 2H), 3.39 (s, 3H), 3.54 (s, 2H), 3.56-3.59 (t, J = 10.5 Hz, 2H), 2.54-2.67 (m, 2H), 3.39 (s, 3H), 3.54 (s, 2H), 3.56-3.59 (m, 2H), 3.61-3.65 (m, 2H), 3.65-3.73 (m, 1H), 4.71 (s, 2H), 4.83 (s, 1H), (m, 2H), 3.61-3.65 (m, 2H), 3.65-3.73 (m, 1H), 4.71 (s, 2H), 4.83 (s, 1H), 5.08 (d, J=7.3 Hz, 1H), 7.05-7.14 (m, 4H), 7.26-7.31 (m, 2H), 7.34-7.39 (m, 3H), 7.43 (d, J 8,3 Hz, 1H), 7.62 (s, 1H), 7.70-7.74 (m, 2H), 7.77 (d, J = 8.3 Hz, 1H); ESI/APCI MS m/z 537 (M+H)⁺.** |
| **163** | N-[1-({7-[(2-Methoxyethoxy)methyl]naphthalen-2-yl}methyl)piperidin-4-yl]-9H-fluorene-9-carboxamide | **¹H NMR (600 MHz, CDCl₃, δ): 1.17-1.26 (m, 2H), 1.73-1.79 (m, 2H), 2.03-2.11 (m, 2H), 2.63-2.70 (m, 2H), 3.39 (s, 3H), 3.55 (s, 2H), 3.56-3.60 (m, 2H), 3.61-3.65 (m, 2H), 3.70-3.79 (m, 1H), 4.71 (s, 2H). 4.75 (s, 1H), 5.04-5.11 (m, 1H), 7.30-7.46 (m, 6H), 7.60-7.79 (m, 8H); ESI/APCIMS m/z 521 (M+H)⁺.** |

### Example 164: Synthesis of 3-methoxy-N-{1-[(6-methoxynaphthalen-2-yl)methyl]piperidin-4-yl}benzamide

The title compound (331 mg, colorless solid) was obtained from 6-methoxy-2-naphthaldehyde (199 mg) and the compound obtained in Step 1-4 (250 mg) by the same method as in Step 1-5.
¹H NMR (600 MHz, CDCl₃, δ): 1.50-1.62 (m, 2H), 1.99-2.05 (m, 2H), 2.22 (t, J = 11.0 Hz, 2H), 2.88 (d, J = 11.5 Hz, 2H), 3. 63 (s, 2H), 3.84 (s, 3H), 3.91 (s, 3H), 3.97-4.05 (m, 1H), 5.92 (d, J = 7.8 Hz, 1H), 7.12 (s, 1H), 7.13-7.15 (m, 2H), 7.23 (d, J = 7.8 Hz, 1H), 7.28-7.34 (m, 2H), 7.41-7.46 (m, 1H), 7.65 (s, 1H), 7.69 (d, J = 4.1 Hz, 1H), 7.70 (d, J = 4.1 Hz, 1H); ESI/APCI MS 405, (M+H)⁺.

### Example 165: Synthesis of N-{1-[(6-hydroxynaphthalen-2-yl)methyl]piperidin-4-yl}-3-methoxybenzamide

The title compound (268 mg, pale yellow solid) was obtained from 6-hydroxy-2-naphthaldehyde (184 mg) and the compound obtained in Step 1-4 (250 mg) by the same method as in Step 1-5.
¹H NMR (600 MHz, CDCl₃, δ): 1.54-1.65 (m, 2H), 2.00-2.08 (m, 2H), 2.25 (t, J = 11.0 Hz, 2H), 2.95 (d, J = 11.5 Hz, 2H), 3.63 (s, 2H), 3.83 (s, 3H), 3.99-4.07 (m, 1H), 5.96 (d, J = 7.8 Hz, 1H), 6.98-7.03 (m, 3H), 7.23 (d, J = 7.8 Hz, 1H), 7.30 (d, J = 8.3 Hz, 1H), 7.31-7.33 (m, 1H), 7.35-7.39 (m, 1H), 7.51 (d, J = 8.7 Hz, 1H), 7.58-7.62 (m, 2 H); ESI/APCI MS 391, (M+H)⁺.

### Example 166: Synthesis of 3-methoxy-N-{1-[(7-methoxynaphthalen-2-yl)methyl]piperidin-4-yl}benzamide

Step 166-1: K₂CO₃ (5.44 g) and MeI (3.34 mL) were added to a solution of the compound obtained in Step 112-1 (9.82 g) in DMF (98.0 mL) under nitrogen atmosphere, and the mixture was stirred at room temperature for 14 hours. Saturated aqueous NH₄Cl solution and H₂O were added to the reaction solution, followed by extraction with a mixed solution of EtOAc/hexane (1/1; v/v). The organic layer was washed with H₂O, dried over Na₂SO₄ and then concentrated under reduced pressure. Hexane (50.0 mL) was added to the residue, and the mixture was stirred at room temperature for 30 minutes. The solid was separated by filtration, and then the filtrate was concentrated under reduced pressure. The residue was purified by column chromatography (Chromatorex NH, mobile phase: EtOAc/hexane = 10/90 to 50/50; v/v) to obtain a crude pale yellow oily compound (5.04 g). K₂CO₃ (27.6 g) was added to a solution of the resulting crude product in MeOH (40.0 mL) and THF (20.0 mL), and the mixture was stirred at room temperature for one day. The solid was separated by filtration and then the filtrate was concentrated under reduced pressure. The residue was purified by column chromatography (Silica gel 60 N, mobile phase: MeOH/CHCl₃ = 2/98 to 5/95; v/v) to obtain a pale brown solid (1.40 g).

Step 166-2: A colorless oily compound (2.40 g) was obtained from the pale brown solid obtained in Step 166-1 (1.38 g) by the same method as in Step 112-2.

Step 166-3: A colorless solid (1.47 g) was obtained from the colorless oily compound obtained in Step 166-2 (2.38 g) by the same method as in Step 112-3.

Step 166-4: 7-Methoxynaphthalene-2-carbaldehyde (1.12 g, pale yellow solid) was obtained from the colorless solid obtained in Step 166-3 (1.45 g) by the same method as in Step 112-4.
¹H NMR (200 MHz, CDCl₃, δ): 3.96 (s, 3H), 7.24-7.34 (m, 2H), 7.74-7.91 (m, 3H), 8.24 (s, 1H), 10.14 (s, 1H); ESI/APCI MS 187, (M+H)⁺.

Step 166-5: The title compound (382 mg, colorless solid) was obtained from the compound obtained in Step 166-4 (199 mg) and the compound obtained in Step 1-4 (250 mg) by the same method as in Step 1-5.
¹H NMR (600 MHz, CDCl₃, δ): 1.51-1.62 (m, 2H), 1.99-2.05 (m, 2H), 2.19-2.26 (m, 2H), 2.89 (d, J = 11.5 Hz, 2H), 3.65 (s, 2H), 3.84 (s, 3H), 3.91 (s, 3H), 3.98-4.05 (m, 1H), 5.93 (d, J = 7.8 Hz, 1H), 7.00-7.04 (m, 1H), 7.09-7.13 (m, 2H), 7.21-7.24 (m, 1H), 7.29-7.36 (m, 3H), 7.64 (s, 1H), 7.69-7.73 (m, 2H); ESI/APCI MS 405, (M+H)⁺.

### Example 167: Synthesis of 3-methoxy-N-{1-[(8-methoxynaphthalen-2-yl)methyl]piperidin-4-yl}benzamide

Step 167-1: Acetic anhydride (14.2 mL) was added to a solution of 1,7-dihydroxynaphthalene (10.0 g) in pyridine (50.0 mL) under nitrogen atmosphere with ice-cooling, and the mixture was stirred at room temperature for 18 hours. Ice water was added to the reaction solution with ice-cooling, and the mixture was stirred at the same temperature for one hour. Then, the solid was collected by filtration to obtain a pale brown solid (14.8 g).

Step 167-2: H₂O (10.3 mL) and lipase PS "Amazon" SD (573 mg) were added to a solution of the pale brown solid obtained in Step 167-1 (7.00 g) in t-butyl methyl ether (431 mL), and the mixture was stirred at 25°C for 36 minutes. The reaction solution was filtered through celite and the filtrate was concentrated under reduced pressure. The residue was purified by column chromatography (Silica gel 60 N, mobile phase: MeOH/CHCl₃ = 1/99 to 3/97; v/v) to obtain a pale brown solid (2.61 g).

Step 167-3: A colorless oily compound (1.56 g) was obtained from the pale brown solid obtained in Step 167-2 (984 mg) by the same method as in Step 112-2.

Step 167-4: A crude pale yellow solid compound (1.00 g) was obtained from the colorless oily compound obtained in Step 167-3 (1.53 g) by the same method as in Step 112-3. K₂CO₃ (1.13 g) was added to a solution of the resulting crude product in MeOH (10.0 mL), and the mixture was stirred at room temperature for 45 minutes. The solid was separated by filtration and washed with EtOAc. Then, the filtrate was concentrated under reduced pressure. The residue was diluted with CHCl₃, followed by washing with 1 M aqueous HCl solution. The aqueous layer was extracted with CHCl₃ twice. The combined organic layers were dried over Na₂SO₄ and then concentrated under reduced pressure. The residue was purified by column chromatography (Silica gel 60 N, mobile phase: MeOH/CHCl₃ = 1/99 to 2/98; v/v) to obtain a pale brown solid (761 mg).

Step 167-5: A pale yellow solid (505 mg) was obtained from the pale brown solid obtained in Step 167-4 (520 mg) by the same method as in Step 166-1.

Step 167-6: 8-Methoxynaphthalene-2-carbaldehyde (360 mg, pale brown oil) was obtained from the pale yellow solid obtained in Step 167-5 (491 mg) by the same method as in Step 112-4.
¹H NMR (600 MHz, CDCl₃, δ): 4.05 (s, 3H), 6.90(d, J = 8.0 Hz, 1H), 7.46 (d, J = 8.0 Hz, 1H), 7.55 (t, J = 8.0 Hz, 1H), 7.86 (d, J = 8.5 Hz, 1H), 7.96 (dd, J = 8.5, 1.6 Hz, 1H), 8.77 (s, 1H), 10.14 (s, 1H); ESI/APCI MS 187, (M+H)⁺.

Step 167-7: The title compound (243 mg, colorless solid) was obtained from the compound obtained in Step 167-6 (159 mg) and the compound obtained in Step 1-4 (200 mg) by the same method as in Step 1-5.
¹H NMR (600 MHz, CDCl₃, δ): 1.52-1.63 (m, 2H), 1.97-2.05 (m, 2H), 2.19-2.27 (m, 2H), 2.88 (d, J = 11.0 Hz, 2H), 3.68 (s, 2H), 3.84 (s, 3H), 3.97-4.05 (m, 1H), 4.00 (s, 3H), 5.93 (d, J = 7.8 Hz, 1H), 6.81 (d, J = 7.8 Hz, 1H), 6.98-7.03 (m, 1H), 7.21-7.24 (m, 1H), 7.28-7.34 (m, 2H), 7.36 (d, J = 7.8 Hz, 1H), 7.38-7.41 (m, J = 8.3 Hz, 1H), 7.49-7.53 (m, 1H), 7.76 (d, J = 8.7 Hz, 1H), 8.12 (s, 1H); ESI/APCI MS 405, (M+H)⁺.

### Example 168: Synthesis of 3-methoxy-N-{1-[(5-methoxynaphthalen-2-yl)methyl]piperidin-4-yl}benzamide

The title compound was obtained by the same method as in Example 167.
1H NMR (600 MHz, CDCl₃, δ): 1.51-1.63 (m, 2H), 1.98-2.06 (m, 2H), 2.17-2.28 (m, 2H), 2.88 (d, J = 11.5 Hz, 2H), 3.66 (s, 2H), 3.84 (s, 3H), 3.95-4.06 (m, 1H), 3.99 (s, 3H), 5.93 (d, J = 7.8 Hz, 1H), 6.79 (d, J = 6.9 Hz, 1H), 7.02 (dd, J = 8.3, 1.8Hz, 1H), 7.23 (d, J = 8.3 Hz, 1H), 7.29-7.40 (m, 4H), 7.47 (d, J = 8.7 Hz, 1H), 7.69 (s, 1H), 8.20 (d, J = 8.3 Hz, 1H); ESI/APCI MS 405, (M+H)⁺.

### Example 169: Synthesis of 3-methoxy-N-(1-{2-[7-(methoxymethyl)naphthalen-2-yl]ethyl}piperidin-4-yl)benzamide

Step 169-1: Phenyllithium solution (1.04 M cyclohexane-diethyl ether solution, 5.04 mL) was added to a suspension of (methoxymethyl)triphenylphosphonium chloride (1.80 g) in THF (5.00 mL) under nitrogen atmosphere, and the mixture was stirred at room temperature for 10 minutes. A solution of the compound obtained in Step 7-3 (1.00 g) in THF (10.0 mL) was added to the reaction solution, and the mixture was stirred at room temperature for four hours. In another reaction vessel, phenyllithium solution (1.04 M cyclohexane-diethyl ether solution, 2.40 mL) was added to a suspension of (methoxymethyl)triphenylphosphonium chloride (855 mg) in THF (2.50 mL) under nitrogen atmosphere, and the mixture was stirred at room temperature for 10 minutes. The mixture was added to the reaction solution, followed by further stirring at room temperature for 30 minutes. The reaction solution was filtered through celite and the filtrate was concentrated under reduced pressure. The residue was purified by column chromatography (Silica gel 60 N, mobile phase: EtOAc/hexane = 25/75 to 30/70; v/v) to obtain a crude pale yellow solid (1.08 g).

Step 169-2: Concentrated sulfuric acid (310 µL) was added to a solution of the crude pale yellow solid obtained in Step 169-1 (310 mg) in acetic acid (3.10 mL), and the mixture was stirred at room temperature for nine minutes. The reaction solution was diluted with CHCl₃ and ice water was added, followed by extraction with CHCl₃ twice. The combined organic layers were dried over MgSO₄ and then concentrated under reduced pressure to obtain a crude brown oily compound (401 mg).

Step 169-3: The title compound (11 mg, colorless solid) was obtained from the crude brown oily compound obtained in Step 169-2 (401 mg) and the compound obtained in Step 1-4 (319 mg) by the same method as in Step 1-5.
¹H NMR (600 MHz, CDCl₃, δ): 1.53-1.64 (m, 2H), 2.05-2.11 (m, 2H), 2.23-2.32 (m, 2H), 2.66-2.73 (m, 2H), 2.93-3.04 (m, 4H), 3.42 (s, 3H), 3.85 (s, 3H), 3.98-4.06 (m, 1H), 4.61 (s, 2H), 5.94 (d, J = 7.8 Hz, 1H), 7.00-7.05 (m, 1H), 7.21-7.27 (m, 1H), 7.28-7.35 (m, 3H), 7.38-7.42 (m, 1H), 7.63 (s, 1H), 7.71 (s, 1H), 7.75 (d, J = 8.3 Hz, 1H), 7.79 (d, J = 8.3 Hz, 1H); ESI/APCI MS 433, (M+H)⁺.

### Example 170: Synthesis of 3-methoxy-N-[1-({7-[(3-methoxybutoxy)methyl]naphthalen-2-yl}methyl-)piperidin-4-yl]benzamide

Step 170-1: Pyridine (2.91 mL) and Tf₂O (4.85 mL) were added to a solution of 3-methoxy-1-butanol (2.50 g) in CHCl₃ (50.0 mL) under nitrogen atmosphere with ice-cooling, and the mixture was stirred at the same temperature for 15 minutes. H₂O was added to the reaction solution with ice-cooling, followed by extraction with CHCl₃. The organic layer was washed with H₂O three times, dried over Na₂SO₄ and then concentrated under reduced pressure to obtain a crude pale brown oily compound (2.86 g).

Step 170-2: The title compound (308 mg, pale brown solid) was obtained from the compound obtained in Example 4 (500 mg) and the crude pale brown oily compound obtained in Step 170-1 (351 mg) by the same method as in Step 1-2.
¹H NMR (600 MHz, CDCl₃, δ): 1.15 (d, J = 6.0 Hz, 3H), 1.52-1.64 (m, 2H), 1.70-1.77 (m, 1H), 1.80-1.88 (m, 1H), 2.03 (d, J = 11.0 Hz, 2H), 2.23 (t, J = 11.0 Hz, 2H), 2.89 (d, J = 10.1 Hz, 2H), 3.31 (s, 3H), 3.45-3.53 (m, 1H), 3.54-3.64 (m, 2H), 3.67 (s, 2H), 3.84 (s, 3H), 3.97-4.06 (m, 1H), 4.65 (s, 2H), 5.93 (d, J = 7.3 Hz, 1H), 6.99-7.04 (m, 1H), 7.21-7.25 (m, 1H), 7.29-7.33 (m, 2H), 7.43 (dd, J = 8.3, 1.8 Hz, 1H), 7.47 (d, J = 8.3 Hz, 1H), 7.69-7.82 (m, 4H); ESI/APCI MS 491, (M+H)⁺.

### Example 171: Synthesis of 3-methoxy-N-[1-({7-[(2-methoxyethoxy)methyl]naphthalen-2-yl}methyl)-1-oxidopiperidin-4-yl]benzamide

3-Chloroperbenzoic acid (574 mg) was added to a solution of the compound obtained in Step 1-5 (1.00 g) in CHCl₃ (5.00 mL) with ice-cooling, and the mixture was stirred at the same temperature for 30 minutes. Saturated aqueous sodium thiosulfate solution and saturated aqueous NaHCO₃ solution were added to the reaction solution with ice-cooling, followed by extraction with CHCl₃. The organic layer was washed with 1 M aqueous NaOH solution twice, dried over Na₂SO₄ and then concentrated under reduced pressure. The residue was purified by column chromatography (Silica gel 60 N, mobile phase: MeOH/CHCl₃ = 0/100 to 1/99; v/v) to obtain the title compound (904 mg, colorless solid).
¹H NMR (600 MHz, CDCl₃, δ): 1.90-1.96 (m, 2H), 2.55-2.65 (m, 2H), 3.20-3.33 (m, 4H), 3.41 (s, 3H), 3.57-3.62 (m, 2H), 3.64-3.68 (m, 2H), 3.82 (s, 3H), 3.95-4.04 (m, 1H), 4.58 (s, 2H), 4.75 (s, 2H), 6.53 (d, J = 6.9 Hz, 1H), 6.99-7.03 (m, 1H), 7.29-7.32 (m, 2H), 7.36-7.38 (m, 1H), 7.54 (dd, J = 8.3, 1.8 Hz, 1H), 7.62 (dd, J = 8.3, 1.8 Hz, 1H), 7.83 (s, 1H), 7.85 (d, J = 6.0 Hz, 1H), 7.86 (d, J = 6.0 Hz, 1H), 7.91 (s, 1H); ESI/APCI MS 479, (M+H)⁺.

**[Table 10-1]**

| | | | | | | |
|---|---|---|---|---|---|---|
| 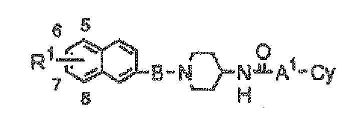 | | | | | | |

| Example | R¹ substitution position | R¹ | B | A¹ | Cy | salt |
|---|---|---|---|---|---|---|
| 1 | 7-Poeition | 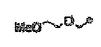 | 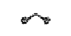 | Bond | 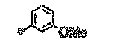 | HCl |
| 2 | 7-Position | 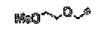 | 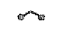 | Bond | 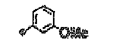 | HBr |
| 3 | 7-Position | 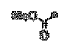 | 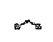 | Bond | 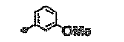 | free |
| 4 | 7-Position | 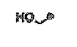 | 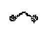 | Bond | 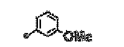 | free |
| 5 | 7-position | 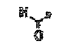 | 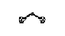 | Bond | 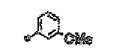 | free |
| 6 | 7-Position | 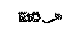 | 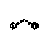 | Bond | 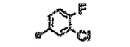 | free |
| 7 | 7-Position | 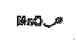 | 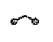 | Bond | 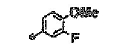 | free |
| 8 | 7-Position | 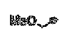 | 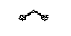 | Bond | 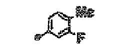 | free |
| 9 | 7-Position | 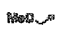 | 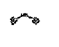 | Bond | 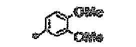 | free |
| 10 | 7-position | 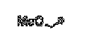 | 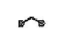 | Bond | 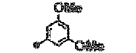 | free |
| 11 | 7-Position | 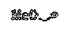 | 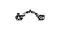 | Bond | 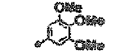 | free |
| 12 | 7-position | 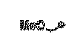 | 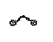 | Bond | 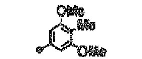 | free |
| 13 | 7-Position | 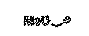 | 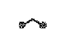 | Bond | 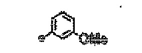 | free |
| 14 | 7-Position | 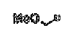 | 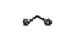 | Bond | 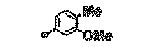 | free |
| 15 | 7-position | 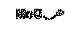 | 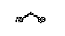 | Bond | 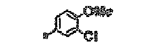 | free |
| 16 | 7-Position | 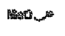 | 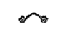 | Bond | 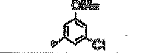 | free |
| 17 | 7-Position | 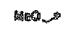 | 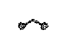 | Bond | 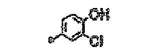 | free |
| 18 | 7-Position | 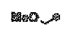 | 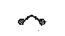 | Bond | 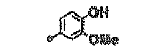 | free |

**[Table 10-2]**

| | | | | | | |
|---|---|---|---|---|---|---|
| 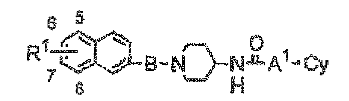 | | | | | | |

| Example | R¹ substitution position | R¹ | B | A¹ | Cy | salt |
|---|---|---|---|---|---|---|
| 19 | 7-Position | 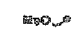 | 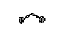 | Bond | 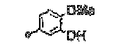 | free |
| 20 | 7-position | 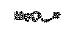 | 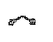 | Bond | 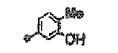 | free |
| 21 | 7-Position | 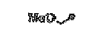 | 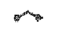 | Bond | 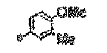 | free |
| 22 | 7-Position | 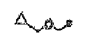 | 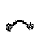 | Bond | 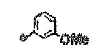 | free |
| 23 | 7-Position | 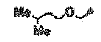 | 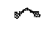 | Bond | 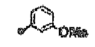 | free |
| 24 | 7-Position | 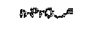 | 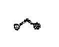 | Bond | 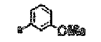 | free |
| 25 | 7-Position | 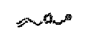 | 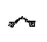 | Bond | 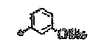 | free |
| 26 | 7-Position | 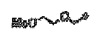 | 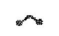 | Bond | 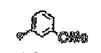 | free |
| 27 | 7-Position | 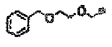 | 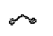 | Bond | 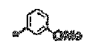 | free |
| 28 | 7-Position | 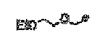 | 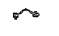 | Bond | 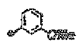 | free |
| 29 | 7-position | 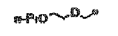 | 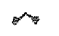 | Bond | 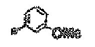 | free |
| 30 | 7-Position | 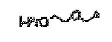 | 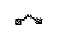 | Bond | 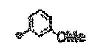 | free |
| 31 | 7-Position | 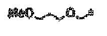 | 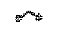 | Bond | 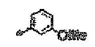 | free |
| 32 | 7-Position | 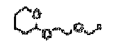 | 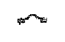 | Bond | 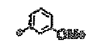 | free |
| 33 | 7-Position | 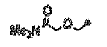 | 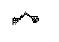 | Bond | 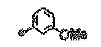 | free |
| 34 | 7-Position | 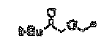 | 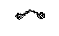 | Bond | 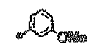 | free |
| 35 | 7-Position | 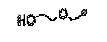 | 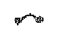 | Bond | 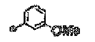 | free |
| 36 | 7-Position | 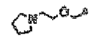 | 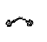 | Bond | 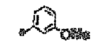 | free |

**[Table 10-3]**

| | | | | | | |
|---|---|---|---|---|---|---|
| 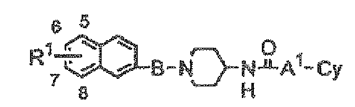 | | | | | | |

| Example | R¹ substitution position | R¹ | B | A¹ | Cy | salt |
|---|---|---|---|---|---|---|
| 37 | 7-Position | 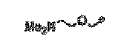 | 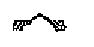 | Bond | 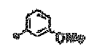 | free |
| 38 | 7-position | 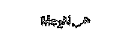 | 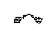 | Bond | 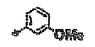 | free |
| 39 | 7-Position | 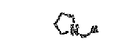 | 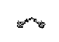 | Bond | 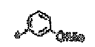 | free |
| 40 | 7-Position | 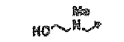 | 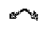 | Bond | 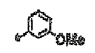 | free |
| 41 | 7-Position | 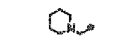 | 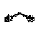 | Bond | 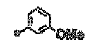 | free |
| 42 | 7-Position | 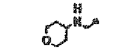 | 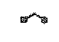 | Bond | 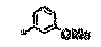 | free |
| 43 | 7-Position | 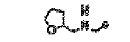 | 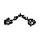 | Bond | 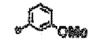 | free |
| 44 | 7-Position | 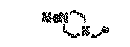 | 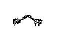 | Bond | 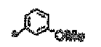 | free |
| 45 | 7-Position | 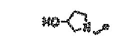 | 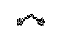 | Bond | 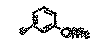 | free |
| 46 | 7-Position | 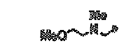 | 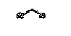 | Bond | 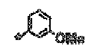 | free |
| 47 | 7-Position | 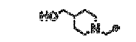 | 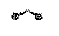 | Bond | 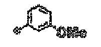 | free |
| 48 | 7-Position | 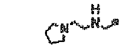 | 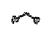 | Bond | 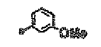 | free |
| 49 | 7-Position | 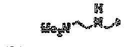 | 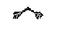 | Bond | 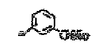 | free |
| 50 | 7-Position | 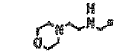 | 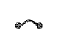 | Bond | 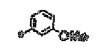 | free |
| 51 | 7-Position | 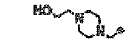 | 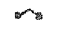 | Bond | 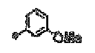 | free |
| 52 | 7-Position | 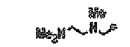 | 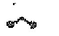 | Bond | 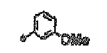 | free |
| 53 | 7-Position | 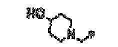 | 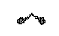 | Bond | 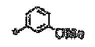 | free |
| 54 | 7-Position | 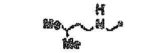 | 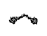 | Bond | 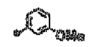 | free |

**[Table 10-4]**

| | | | | | | |
|---|---|---|---|---|---|---|
| 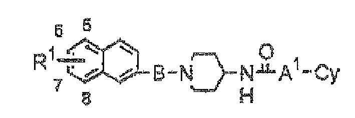 | | | | | | |

| Example | R¹ substitution position | R¹ | B | A¹ | Cy | salt |
|---|---|---|---|---|---|---|
| 55 | 7-Position | 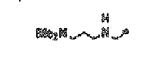 | 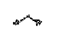 | Bond | 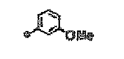 | free |
| 56 | 7-Position | 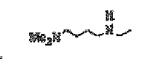 | 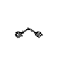 | Bond | 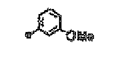 | free |
| 57 | 7-Position | 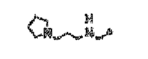 | 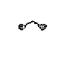 | Bond | 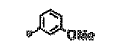 | free |
| 58 | 7-Position | 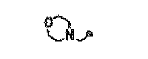 | 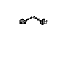 | Bond | 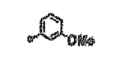 | 2HCl |
| 59 | 7-Position | 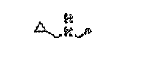 | 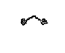 | Bond | 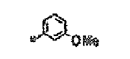 | free |
| 60 | 7-Position | 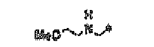 | 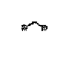 | Bond | 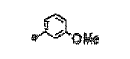 | free |
| 61 | 7-Position | 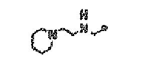 | 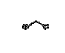 | Bond | 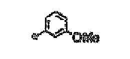 | free |
| 62 | 7-Position | 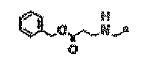 | 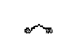 | Bond | 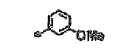 | free |
| 63 | 7-Position | 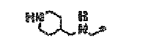 | 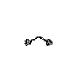 | Bond | 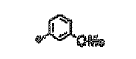 | 3HCl |
| 64 | 7-Position | 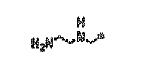 | 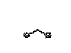 | Bond | 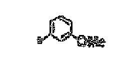 | 3HCl |
| 65 | 7-Position | 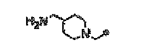 | 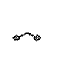 | Bond | 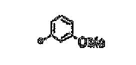 | 3HCl |
| 66 | 7-Position | 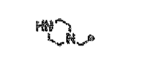 | 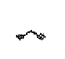 | Bond | 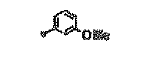 | 3HCl |
| 67 | 7-Position | 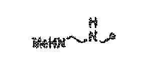 | 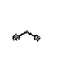 | Bond | 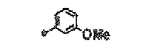 | 3HCl |
| 68 | 7-Position | 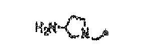 | 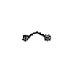 | Bond | 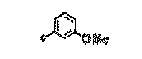 | 3HCl |
| 69 | 7-Position | 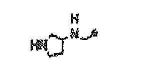 | 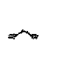 | Bond | 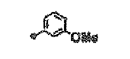 | 3HCl |
| 70 | 7-Position | 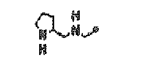 | 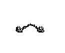 | Bond | 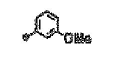 | 3HCl |
| 71 | 7-Position | 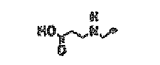 | 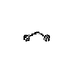 | Bond | 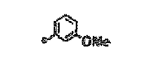 | free |
| 72 | 7-Position | 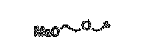 | 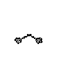 | Bond | 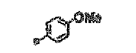 | free |

**[Table 10-5]**

| | | | | | | |
|---|---|---|---|---|---|---|
| 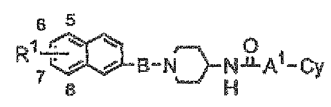 | | | | | | |

| Example | R¹ substitution position | R¹ | B | A¹ | Cy | salt |
|---|---|---|---|---|---|---|
| 73 | 7-Position | 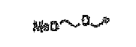 | 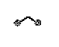 | Bond | 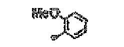 | HCl |
| 74 | 7-Position | 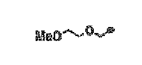 | 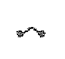 | Bond | 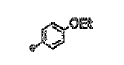 | free |
| 75 | 7-Position | 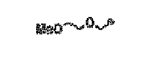 | 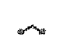 | Bond | 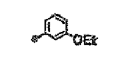 | free |
| 76 | 7-Position | 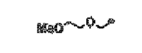 | 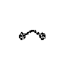 | Bond | 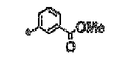 | free |
| 77 | 7-Position | 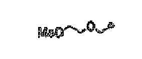 | 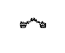 | Bond | 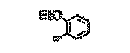 | HCl |
| 78 | 7-Position | 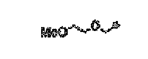 | 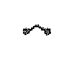 | Bond | 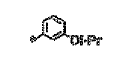 | free |
| 79 | 7-Position | 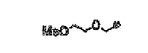 | 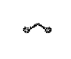 | Bond | 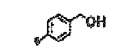 | free |
| 80 | 7-Position | 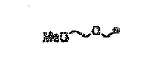 | 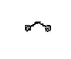 | Bond | 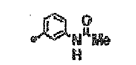 | free |
| 81 | 7-Position | 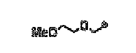 | 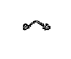 | Bond | 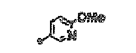 | free |
| 82 | 7-Position | 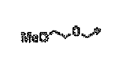 | 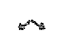 | Bond | 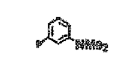 | free |
| 83 | 7-Position | 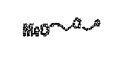 | 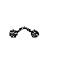 | Bond | 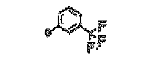 | free |
| 84 | 7-Position | 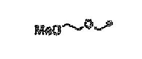 | 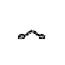 | Bond | 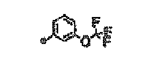 | free |
| 85 | 7-Position | 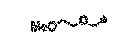 | 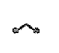 | Bond | 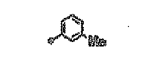 | free |
| 86 | 7-Position | 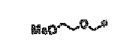 | 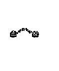 | Bond | 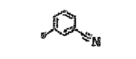 | free |
| 87 | 7-Position | 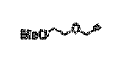 | 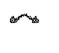 | Bond | 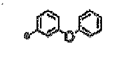 | free |
| 88 | 7-Position | 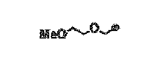 | 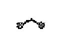 | Bond | 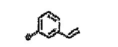 | free |
| 89 | 7-Position | 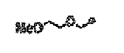 | 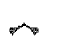 | 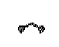 | 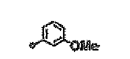 | free |
| 90 | 7-Position | 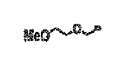 | 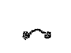 | Bond | 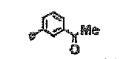 | free |

**[Table 10-6]**

| | | | | | | |
|---|---|---|---|---|---|---|
| 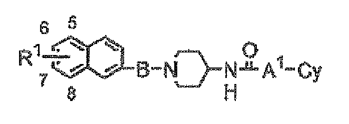 | | | | | | |

| Example | R¹ substitution position | R¹ | B | A¹ | Cy | salt |
|---|---|---|---|---|---|---|
| 91 | 7-Position | 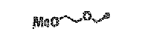 | 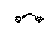 | Bond | 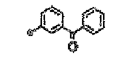 | free |
| 92 | 7-Position | 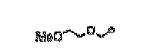 | 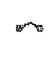 | Bond | 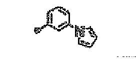 | free |
| 93 | 7-Position | 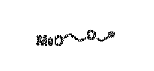 | 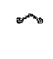 | Bond | 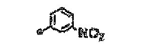 | free |
| 94 | 7-Position | 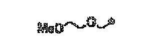 | 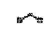 | Bond | 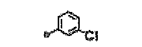 | free |
| 95 | 7-Position | 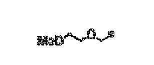 | 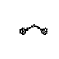 | Bond | 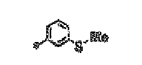 | free |
| 96 | 7-Position | 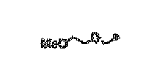 | 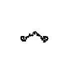 | Bond | 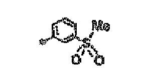 | free |
| 97 | 7-Position | 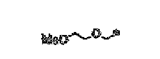 | 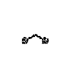 | Bond | 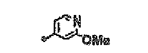 | free |
| 98 | 7-Position | 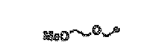 | 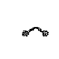 | Bond | 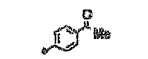 | free |
| 99 | 7-Position | 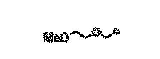 | 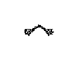 | Bond | 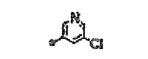 | free |
| 100 | 7-Position | 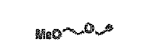 | 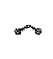 | Bond | 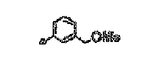 | free |
| 101 | 7-Position | 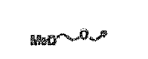 | 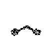 | Bond | 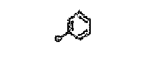 | free |
| 102 | 7-Position | 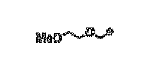 | 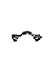 | Bond | 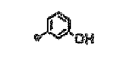 | free |
| 103 | 7-Position | 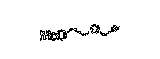 | 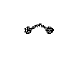 | Bond | 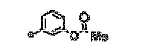 | free |
| 104 | 7-Position | 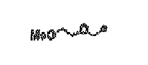 | 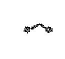 | Bond | 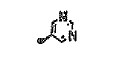 | free |
| 105 | 7-Position | 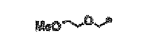 | 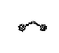 | Bond | 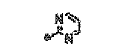 | free |
| 106 | 7-Position | 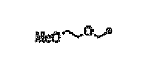 | 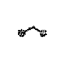 | Bond | 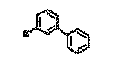 | free |
| 107 | 7-Position | 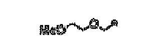 | 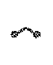 | Bond | 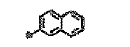 | free |
| 108 | 7-Position | 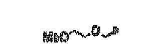 | 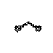 | Bond | 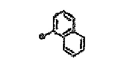 | free |

**[Table 10-7]**

| | | | | | | |
|---|---|---|---|---|---|---|
| 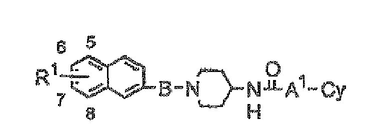 | | | | | | |

| Example | R¹ substitution position | R¹ | B | A¹ | Cy | salt |
|---|---|---|---|---|---|---|
| 109 | 7-Position | 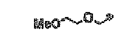 | 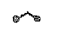 | Bond | 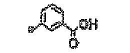 | free |
| 110 | 7-Position | 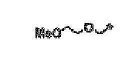 | 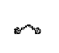 | Bond | 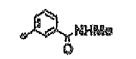 | free |
| 111 | 7-Position | 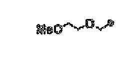 | 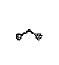 | Bond | 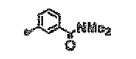 | free |
| 112 | 7-Position | 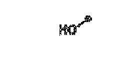 | 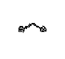 | Bond | 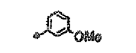 | free |
| 113 | 7-Position | 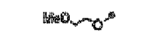 | 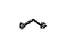 | Bond | 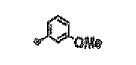 | free |
| 114 | 7-Position | 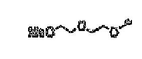 | 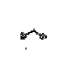 | Bond | 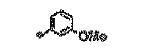 | free |
| 115 | 7-Position | 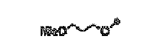 | 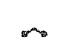 | Bond | 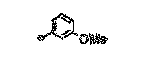 | free |
| 116 | 7-Position | 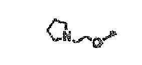 | 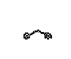 | Bond | 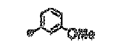 | free |
| 117 | 7-Position | 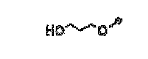 | 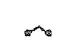 | Bond | 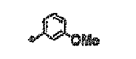 | free |
| 118 | 7-Position | 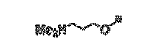 | 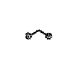 | Bond | 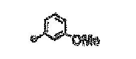 | free |
| 119 | 7-Position | 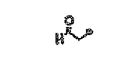 | 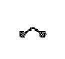 | Bond | 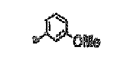 | free |
| 120 | 7-Position | 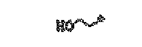 | 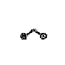 | Bond | 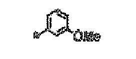 | free |
| 121 | 7-Position | 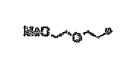 | 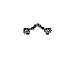 | Bond | 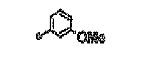 | free |
| 122 | 7-Position | 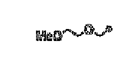 | 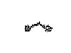 | Bond | 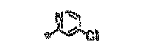 | 2HCl |
| 123 | 7-Position | 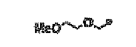 | 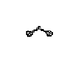 | Bond | 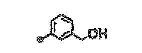 | free |
| 124 | 7-Position | 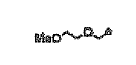 | 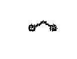 | Bond | 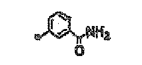 | free |
| 125 | 7-Position | 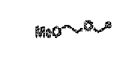 | 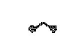 | Bond | 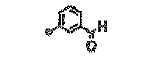 | free |
| 126 | 7-Position | 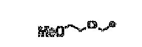 | 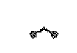 | Bond | 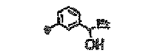 | free |

**[Table 10-8]**

| | | | | | | |
|---|---|---|---|---|---|---|
| 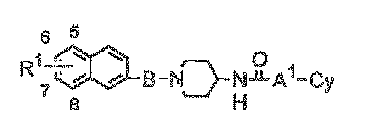 | | | | | | |

| Example | R¹ substitution position | | B | A¹ | Cy | salt |
|---|---|---|---|---|---|---|
| 127 | 7-Posvition | 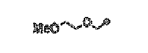 | 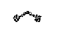 | Bond | 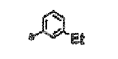 | free |
| 128 | 6-Posvition | 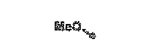 | 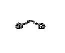 | Bond | 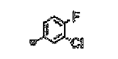 | free |
| 129 | 6-Posvition | 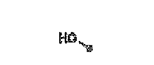 | 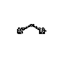 | Bond | 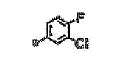 | free |
| 130 | 7-Posvition | 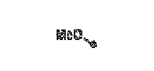 | 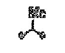 | Bond | 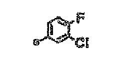 | free |
| 131 | 6-Posvition | 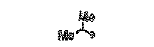 | 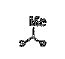 | Bond | 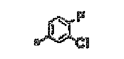 | free |
| 132 | 6-Posvition | 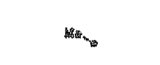 | 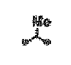 | Bond | 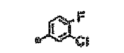 | free |
| 133 | 6-Posvition | 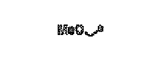 | 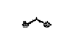 | Bond | 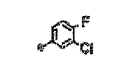 | free |
| 134 | 6-Posvition | 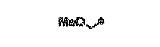 | 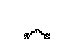 | Bond | 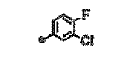 | free |
| 135 | 6-Posvition | 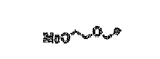 | 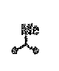 | Bond | 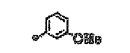 | HCI |
| 136 | 7-Posvition | 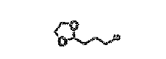 | 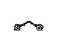 | Bond | 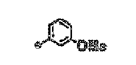 | free |
| 137 | 7-Posvition | 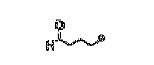 | 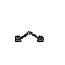 | Bond | 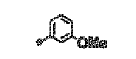 | free |
| 138 | 7-Posvition | 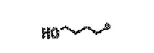 | 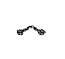 | Bond | 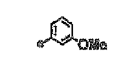 | free |
| 139 | 7-Posvition | 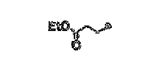 | 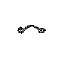 | Bond | 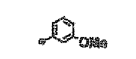 | free |
| 140 | 7-Posvition | 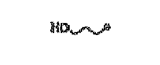 | 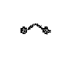 | Bond | 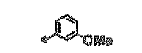 | free |
| 141 | 7-Posvition | 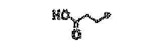 | 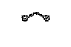 | Bond | 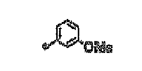 | free |
| 142 | 7-Posvition | 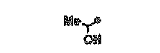 | 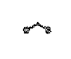 | Bond | 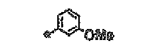 | free |
| 143 | 7-Posvition | 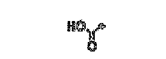 | 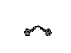 | Bond | 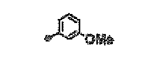 | free |
| 144 | 7-Posvition | 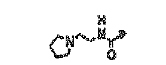 | 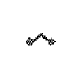 | Bond | 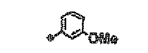 | 2HCI |

**[Table 10-9]**

| | | | | | | |
|---|---|---|---|---|---|---|
| 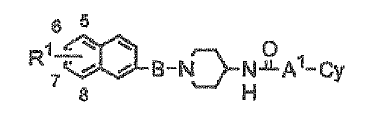 | | | | | | |

| Example | R¹ substitution position | R¹ | B | A¹ | Cy | salt |
|---|---|---|---|---|---|---|
| 145 | 7-Position | 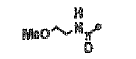 | 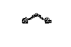 | Bond | 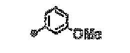 | HCl |
| 146 | 7-Position | 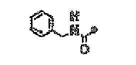 | 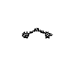 | Bond | 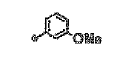 | HCl |
| 147 | 7-Position | 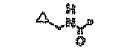 | 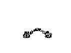 | Bond | 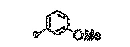 | HCl |
| 148 | 7-Position | 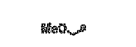 | 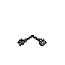 | 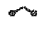 | 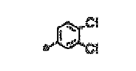 | free |
| 149 | 7-Position | 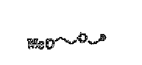 | 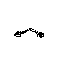 | 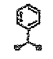 | 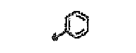 | free |
| 150 | 7-Position | 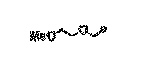 | 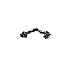 | 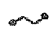 | 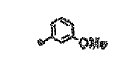 | free |
| 151 | 7-Position | 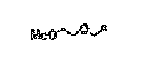 | 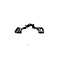 | 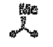 | 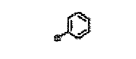 | free |
| 152 | 7-Position | 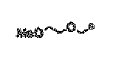 | 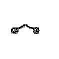 | 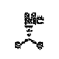 | 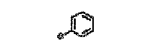 | free |
| 153 | 7-Position | 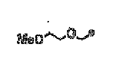 | 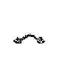 | 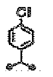 | 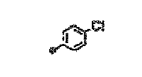 | free |
| 154 | 7-Position | 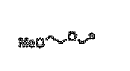 | 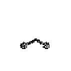 | 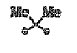 | 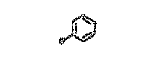 | HCl |
| 155 | 7-Position | 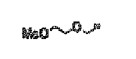 | 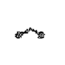 | 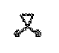 | 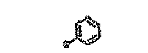 | HCl |
| 156 | 7-Position | 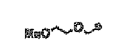 | 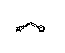 | Bond | 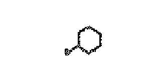 | free |
| 157 | 7-Position | 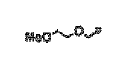 | 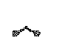 | Bond | 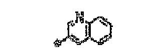 | free |
| 158 | 7-Position | 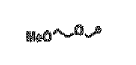 | 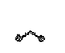 | Bond | 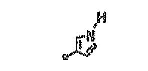 | free |
| 159 | 7-Position | 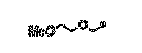 | 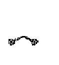 | Bond | 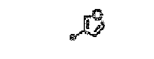 | free |
| 160 | 7-Position | 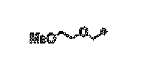 | 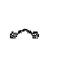 | Bond | 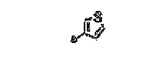 | free |
| 161 | 7-Position | 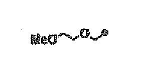 | 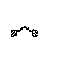 | Bond | 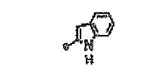 | free |

**[Table 10-10]**

| | | | | | | |
|---|---|---|---|---|---|---|
| 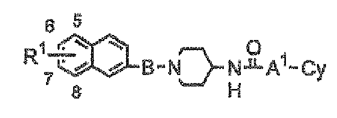 | | | | | | |

| Example | R¹ substitution position | R¹ | B | A¹ | Cy | salt |
|---|---|---|---|---|---|---|
| 162 | 7-Position | 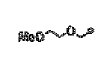 | 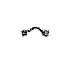 | Bond | 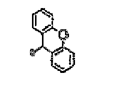 | free |
| 163 | 7-Position | 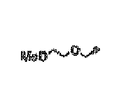 | 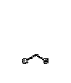 | Bond | 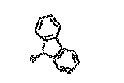 | free |
| 164 | 6-Position | 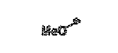 | 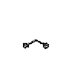 | Bond | 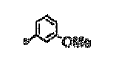 | free |
| 165 | 6-position | 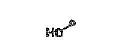 | 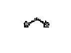 | Bond | 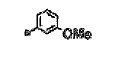 | free |
| 166 | 7-Position | 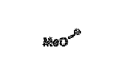 | 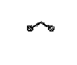 | Bond | 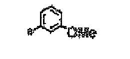 | free |
| 167 | 8-Position | 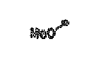 | 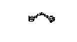 | Bond | 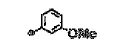 | free |
| 168 | 5-Position | 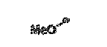 | 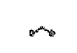 | Bond | 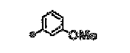 | free |
| 169 | 7-Position | 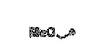 | 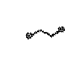 | Bond | 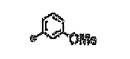 | free |
| 170 | 7-Position | 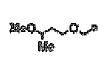 | 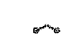 | Bond | 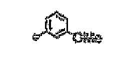 | free |
| 171*^{a}* | 7-Position | 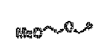 | 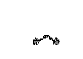 | Bond | 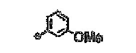 | free |

| | | | | | | |
|---|---|---|---|---|---|---|
| a: Piperidine N-oxide | | | | | | |

### Example 172 (MCH1R calcium evaluation test)

An FDSS assay can measure the intracellular calcium concentration and can evaluate the Gq-coupled receptor activity using the calcium concentration as an index. For example, the assay can determine whether an analyte is an antagonist, an inverse agonist or an agonist for a Gq-coupled receptor. The FDSS6000^{™} system (Hamamatsu Photonics K.K.) is designed to perform evaluation based on functionality such as measurement of intracellular calcium for high-throughput screening. Intracellular calcium release by activation of a Gq-coupled receptor can be fluorometrically measured by incorporating a calcium indicator (such as Fluo4) into cells. On the other hand, the assay cannot measure the activation of Gi- and Go-coupled receptors, because the activation is not associated with calcium signaling pathways.

Intracellular fluorescence can be rapidly and successively measured in a 96-well microplate or a 384-well microplate using a fluorometric imaging plate reader system. FDSS6000^{™} can simultaneously measure fluorescence in all wells sensitively, accurately and by seconds. This system is ideal for functional analysis in cells such as monitoring of an intracellular calcium flow generated within several seconds after activation of a Gq-coupled receptor.

### Test method

On the day before the test, cells stably expressing non-endogenous active MCH1R were seeded into a 96-well microplate at 3 × 10⁴ cells per well. 100 µL per well of a medium (Dulbecco's modified Eagle medium containing 10% fetal bovine serum, 2 mM glutamine, 1 mM sodium pyruvate and 0.5 mg/mL G418, pH 7.4) was used for culture. On the day of the test, the medium was removed and an assay buffer {Hank's balanced salt solution containing 20 mM HEPES, 0.5 mM probenecid, 0.05 mg/mL amaranth and 0.2% bovine serum albumin (BSA), pH 7.4} containing 2 µM Fluo4-AM and 0.04% Pluronic F127 was added at 100 µL per well, followed by incubation in a 5% CO₂ incubator at 37°C for one hour. Thereafter, the buffer was removed and an assay buffer containing each concentration of the test compound was newly added at 150 µL per well, followed by incubation in a 5% CO₂ incubator at 37°C for 30 minutes. An assay buffer containing each concentration of MCH was added at 50 µL per well, and transient changes in the intracellular calcium concentration induced by MCH were monitored using FDSS6000^{™} at Ex. 488 nm and Em. 530 nm for 180 seconds. In testing the antagonistic activity of the analyte, MCH was added to a final concentration of 50 nM. An inhibition curve was prepared with various concentrations of the analyte, and the concentration of the analyte inhibiting 50% of the increase in intracellular calcium when 50 nM MCH was added (IC₅₀ value) was calculated using data analysis software Origin Ver. 6.

The compounds of the present invention having an IC₅₀ value of 30 nM or less are shown below.

Compounds Nos. 2, 3, 4, 5, 6, 10, 12, 13, 16, 22, 23, 24, 25, 26, 27, 28, 29, 30, 31, 32, 35, 36, 37, 48, 49, 54, 59, 60, 61, 64, 66, 67, 68, 70, 82, 83, 84, 88, 90, 93, 94, 95, 112, 113, 114, 115, 116, 117, 118, 119, 120, 121, 127, 129, 134, 135, 138, 139, 140, 149, 153, 166 and 170

Further, the IC₅₀ values of the compounds of the present invention are illustrated in Table 10.

**[Table 11]**

| Example No. | IC₅₀ (nM) | | Example No. | IC₅₀ (nM) |
|---|---|---|---|---|
| 6 | 3.54 | | 48 | 9.68 |
| 10 | 4.99 | | 49 | 7.08 |
| 13 | 10.4 | | 64 | 7.14 |
| 26 | 5.67 | | 67 | 6.51 |
| 36 | 6.11 | | 90 | 7.69 |
| 37 | 4.89 | | 120 | 7.41 |

### INDUSTRIAL APPLICABILITY

The compound of the present invention has an MCH receptor antagonistic activity and can be used as a prophylactic and therapeutic agent for a disease associated with MCH, specifically, a prophylactic and therapeutic agent for depression, anxiety disorders (such as generalized anxiety disorder, posttraumatic stress disorder, panic disorder, obsessive-compulsive disorder or social anxiety disorder), attention deficit disorder, mania, manic-depressive illness, schizophrenia, mood disorders, stress, sleep disorder, attacks, memory impairment, cognitive impairment, dementia, amnesia, delirium, obesity, eating disorder, appetite disorder, hyperphagia, bulimia, cibophobia, diabetes, cardiovascular diseases, hypertension, dyslipidemia, myocardial infarction, movement disorder (such as Parkinson's disease, epilepsy, convulsion or tremor), drug abuse, drug addiction or the like.

## Claims

1. A compound represented by the formula (I): 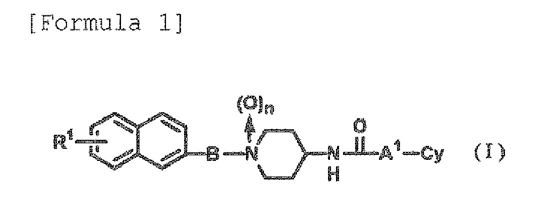 or a pharmaceutically acceptable salt thereof,
wherein in the formula (I), R¹ is a substituent selected from the group consisting of the formula (II): 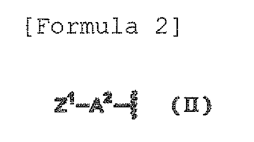 [wherein in the formula (II), Z¹ is a hydroxyl group, a C₁₋₆ alkoxy group (wherein the C₁₋₆ alkoxy group may be substituted with a C₃₋₆ cycloalkyl group), a C₂₋₆ alkenyloxy group, a mono-C₁₋₆ alkylamino group (wherein the mono-C₁₋₆ alkylamino group may be substituted with a C₃₋₆ cycloalkyl group), a di-C₁₋₆ alkylamino group, a formyl group, a carboxyl group, a C₁₋₆ alkoxycarbonyl group or a heterocyclic group {wherein the heterocyclic group is substituted with a hydroxyl group, a C₁₋₆ alkyl group (wherein the C₁₋₆ alkyl group may be substituted with a hydroxyl group or an amino group) or an amino group} and
A² is a C₁₋₆ alkylene group (wherein the C₁₋₆ alkylene group may be substituted with a C₁₋₆ alkyl group), provided that A² may be a bond when Z¹ is a formyl group],
the formula (III): 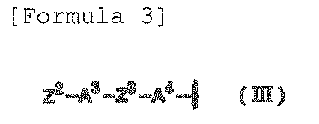 {wherein in the formula (III), Z² is a hydroxyl group, a C₁₋₆ alkoxy group, a C₇₋₁₀ aralkyloxy group, a heterocycloxy group, an amino group, a mono-C₁₋₆ alkylamino group, a di-C₁₋₆ alkylamino group, a carboxyl group, a C₂₋₆ alkanoyl group, a C₇₋₁₀ aralkyloxycarbonyl group, a di-C₁₋₆ alkylaminocarbonyl group or a heterocyclic group,
Z³ is -O- or -NR³-, wherein R³ is a hydrogen atom or a
C₁₋₆ alkyl group, and
A³ and A⁴ are each independently a C₁₋₆ alkylene group (wherein the C₁₋₆ alkylene group may be substituted with a C₁₋₆ alkyl group), provided that A³ may be a bond when Z² is a heterocyclic group} and
the formula (IV): 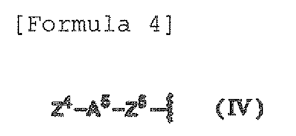 {wherein in the formula (IV), Z⁴ is a C₃₋₆ cycloalkyl group, a C₁₋₆ alkoxy group (wherein the C₁₋₆ alkoxy group may be substituted with a C₁₋₆ alkoxy group), an aryl group or a heterocyclic group, provided that Z⁴ is not a heterocyclic group when Z⁵ is -O-,
Z⁵ is -O- or -NH-CO- and
A⁵ is a C₁₋₆ alkylene group (wherein the C₁₋₆ alkylene group may be substituted with a C₁₋₆ alkyl group)},
B is a C₁₋₆ alkylene group (wherein the C₁₋₆ alkylene group may be substituted with a C₁₋₆ alkyl group),
A¹ is a bond or a C₁₋₆ alkylene group {wherein the C₁₋₆ alkylene group may be substituted with one or two independent C₁₋₆ alkyl groups (wherein the C₁₋₆ alkyl groups may be bonded together to form a ring) or aryl groups (wherein the aryl groups may each be substituted with a halogen atom)},
Cy is a C₃₋₆ cycloalkyl group, an aryl group or a heteroaryl group {wherein the aryl group or the heteroaryl group may have 1 to 3 substituents selected from substituent group X consisting of a halogen atom, a cyano group, a nitro group, a C₁₋₆ alkyl group (wherein the C₁₋₆ alkyl group may be substituted with a halogen atom, a hydroxyl group or a C₁₋₆ alkoxy group), a C₂₋₆ alkenyl group, a hydroxyl group, a C₁₋₆ alkoxy group (wherein the C₁₋₆ alkoxy group may be substituted with a halogen atom), an aryloxy group, a di-C₁₋₆ alkylamino group, a formyl group, a carboxyl group, a carbamoyl group, a C₂₋₆ alkanoyl group, an arylcarbonyl group, a C₁₋₆ alkoxycarbonyl group, a mono-C₁₋₆ alkylcarbonylamino group, a C₁₋₆ alkylcarbonyloxy group, a mono-C₁₋₆ alkylaminocarbonyl group, a di-C₁₋₆ alkylaminocarbonyl group, a C₁₋₆ alkylthio group, a C₁₋₆ alkylsulfonyl group, an aryl group and a heteroaryl group} and
n is 0 or 1.

2. The compound or pharmaceutically acceptable salt thereof according to claim 1, wherein the compound is represented by the formula (V): 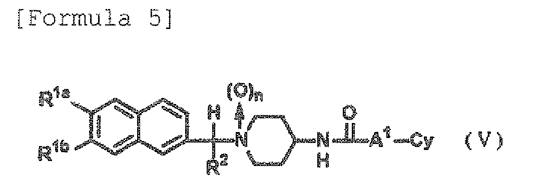 wherein in the formula (V), any one of R^{1a} and R^{1b} is a hydrogen atom and the other is a substituent selected from the group consisting of the formula (II), the formula (III) and the formula (IV),
R² is a hydrogen atom or a C₁₋₆ alkyl group and
A¹, Cy and n are as defined in claim 1.

3. The compound or pharmaceutically acceptable salt thereof according to claim 2, wherein in the formula (V),
R^{1a} is a hydrogen atom,
R^{1b} is a substituent selected from the group consisting of the formula (II), the formula (III) and the formula (IV) and
R² is a hydrogen atom.

4. The compound or pharmaceutically acceptable salt thereof according to claim 3, wherein in the formula (V),
Cy is a C₃₋₆ cycloalkyl group, an aryl group {wherein the aryl group may have 1 to 3 substituents selected from the group consisting of a halogen atom, a cyano group, a nitro group, a C₁₋₆ alkyl group (wherein the C₁₆ alkyl group may be substituted with a halogen atom, a hydroxyl group or a C₁₋₆ alkoxy group), a C₂₋₆ alkenyl group, a hydroxyl group, a C₁₋₆ alkoxy group (wherein the C₁₋₆ alkoxy group may be substituted with a halogen atom), an aryloxy group, a di-C₁₋₆ alkylamino group, a formyl group, a C₂₋₆ alkanoyl group, an arylcarbonyl group, a C₁₋₆ alkoxycarbonyl group, a mono-C₁₋₆ alkylcarbonylamino group, a C₁₋₆ alkylcarbonyloxy group, a mono-C₁₋₆ alkylaminocarbonyl group, a C₁₋₆ alkylthio group, an aryl group and a heteroaryl group} or a heteroaryl group (wherein the heteroaryl group may be substituted with a halogen atom).

5. The compound or pharmaceutically acceptable salt thereof according to claim 4, wherein in the formula (V),
A¹ is a bond or a methylene group {wherein the methylene group may be substituted with one or two independent C₁₆ alkyl groups (wherein the C₁₋₆ alkyl groups may be bonded together to form a ring) or aryl groups (wherein the aryl groups may each be substituted with a halogen atom)},
Cy is an aryl group {wherein the aryl group may have 1 to 3 substituents selected from the group consisting of a halogen atom, a cyano group, a nitro group, a C₁₋₆ alkyl group (wherein the C₁₋₆ alkyl group may be substituted with a halogen atom), a C₂₋₆ alkenyl group, a C₁₋₆ alkoxy group (wherein the C₁₋₆ alkoxy group may be substituted with a halogen atom), an aryloxy group, a di-C₁₋₆ alkylamino group, a C₂₋₆ alkanoyl group, a C₁₋₆ alkoxycarbonyl group and a C₁₋₆ alkylthio group} or a heteroaryl group (wherein the heteroaryl group may be substituted with a halogen atom) and n is 0.

6. The compound or pharmaceutically acceptable salt thereof according to claim 5, wherein in the formula (V),
Cy is an aryl group {wherein the aryl group may have 1 to 3 substituents selected from the group consisting of a halogen atom, a nitro group, a C₁₋₆ alkyl group (wherein the C₁₋₆ alkyl group may be substituted with a halogen atom), a C₂₋₆ alkenyl group, a C₁₋₆ alkoxy group (wherein the C₁₋₆ alkoxy group may be substituted with a halogen atom), a di-C₁₋₆ alkylamino group, a C₂₋₆ alkanoyl group and a C₁₋₆ alkylthio group}, provided that Cy is 3-monosubstituted, 3,4-disubstituted, 3,5-disubstituted or 3,4,5-trisubstituted.

7. The compound or pharmaceutically acceptable salt thereof according to claim 6, wherein in the formula (V),
R^{1b} is a substituent selected from the group consisting of the formula (II)
[wherein in the formula (II), Z¹ is a hydroxyl group, a C₁₋₆ alkoxy group (wherein the C₁₋₆ alkoxy group may be substituted with a C₃₋₆ cycloalkyl group), a C₂₋₆ alkenyloxy group, a mono-C₁₋₆ alkylamino group (wherein the mono-C₁₋₆ alkylamino group may be substituted with a C₃₋₆ cycloalkyl group), a formyl group, a C₁₋₆ alkoxycarbonyl group or a heterocyclic group {wherein the heterocyclic group is substituted with a C₁₋₆ alkyl group (wherein the C₁₋₆ alkyl group may be substituted with a hydroxyl group) or an amino group} and
A² is a C₁₋₆ alkylene group, provided that A² may be a bond when Z¹ is a formyl group],
the formula (III)
{wherein in the formula (III), Z² is a hydroxyl group, a C₁₋₆ alkoxy group, a C₇₋₁₀ aralkyloxy group, an amino group, a mono-C₁₋₆ alkylamino group, a di-C₁₋₆ alkylamino group, a C₇₋₁₀ aralkyloxycarbonyl group or a heterocyclic group,
Z³ is -O- or -NR³-, wherein R³ is a hydrogen atom or a
C₁₋₆ alkyl group, and
A³ and A⁴ are each independently a C₁₋₆ alkylene group (wherein the C₁₋₆ alkylene group may be substituted with a C₁₋₆ alkyl group)} and
the formula (IV)
{wherein in the formula (IV), Z⁴ is a C₁₋₆ alkoxy group
(wherein the C₁₋₆ alkoxy group may be substituted with a C₁₋₆ alkoxy group) or a heterocyclic group, provided that Z⁴ is not a heterocyclic group when Z⁵ is -O-,
z⁵ is -O- or -NH-CO- and
A⁵ is a C₁₋₆ alkylene group}.

8. The compound or pharmaceutically acceptable salt thereof according to claim 7, wherein in the formula (V),
R^{1b} is a substituent selected from the group consisting of the formula (II)
{wherein in the formula (II), Z¹ is a hydroxyl group, a C₁₋₆ alkoxy group (wherein the C₁₋₆ alkoxy group may be substituted with a C₃₋₆ cycloalkyl group), a C₂₋₆ alkenyloxy group, a mono-C₁₋₆ alkylamino group (wherein the mono-C₁₋₆ alkylamino group may be substituted with a C₃₋₆ cycloalkyl group), a formyl group, a C₁₋₆ alkoxycarbonyl group or a heterocyclic group (wherein the heterocyclic group is substituted with an amino group) and
A² is a C₁₋₆ alkylene group, provided that A² may be a bond when Z¹ is a formyl group},
the formula (III)
{wherein in the formula (III), Z² is a hydroxyl group, a C₁₋₆ alkoxy group, a C₇₋₁₀ aralkyloxy group, an amino group, a mono-C₁₋₆ alkylamino group, a di-C₁₋₆ alkylamino group or a heterocyclic group,
Z³ is -O- or -NH- and
A³ and A⁴ are each independently a C₁₋₆ alkylene group (wherein the C₁₋₆ alkylene group may be substituted with a C₁₋₆ alkyl group)} and
the formula (IV)
{wherein in the formula (IV), Z⁴ is a C₁₋₆ alkoxy group
(wherein the C₁₋₆ alkoxy group may be substituted with a C₁₋₆ alkoxy group),
Z⁵ is -O- and
A⁵ is a C₁₋₆ alkylene group} and
A¹ is a bond or a methylene group {wherein the methylene group may be substituted with an aryl group (wherein the aryl group may be substituted with a halogen atom)}.

9. The compound or pharmaceutically acceptable salt thereof according to claim 8, wherein in the formula (V),
R^{1b} is a substituent selected from the group consisting of the formula (II)
{wherein in the formula (II), Z¹ is a C₁₋₆ alkoxy group
(wherein the C₁₋₆ alkoxy group may be substituted with a C₃₋₆ cycloalkyl group) or a C₂₋₆ alkenyloxy group and
A² is a C₁₋₆ alkylene group},
the formula (III)
{wherein in the formula (III), Z² is a hydroxyl group or a C₁₋₆ alkoxy group,
Z³ is -O- and
A³ and A⁴ are each independently a C₁₋₆ alkylene group (wherein the C₁₋₆ alkylene group may be substituted with a C₁₋₆ alkyl group)} and
the formula (IV)
{wherein in the formula (IV), Z⁴ is a C₁₋₆ alkoxy group
(wherein the C₁₋₆ alkoxy group may be substituted with a C₁₋₆ alkoxy group),
_{Z}⁵ is -O- and
A⁵ is a C₁₋₆ alkylene group} and
A¹ is a bond.

10. The compound or pharmaceutically acceptable salt thereof according to claim 1, wherein the compound represented by the formula (I) is
3-methoxy-N-[1-({7-[(2-methoxyethoxy)methyl]-2-naphthyl}methyl)piperidin-4-yl]benzamide,
N-(1-{[7-(hydroxymethyl)-2-naphthyl]methyl}piperidin-4-yl)-3-methoxybenzamide,
N-{1-[(7-formyl-2-naphthyl)methyl]piperidin-4-yl}-3-methoxybenzamide,
3-chloro-N-(1-{[7-(ethoxymethyl)-2-naphthyl]methyl}piperidin-4-yl)-4-fluorobenzamide,
3,5-dimethoxy-N-(1-{[7-(methoxymethyl)-2-naphthyl]methyl}piperidin-4-yl)benzamide,
3,5-dimethoxy-N-(1-{[7-(methoxymethyl)-2-naphthyl]methyl}piperidin-4-yl)-4-methylbenzamide,
3-methoxy-N-(1-{[7-(methoxymethyl)-2-naphthyl]methyl}piperidin-4-yl)benzamide,
3-methoxy-N-(1-{[7-(methoxymethyl)-2-naphthyl]methyl}piperidin-4-yl)-4-methylbenzamide,
3-chloro-5-methoxy-N-(1-{[7-(methoxymethyl)-2-naphthyl]methyl}piperidin-4-yl)benzamide,
N-[1-({7-[(cyolopropylmethoxy)methyl]-2-naphthyl}methyl)piperidin-4-yl]-3-methoxybenzamide,
3-methoxy-N-[1-({7-[(3-methylbutoxy)methyl]-2-naphthyl}methyl)piperidin-4-yl]benzamide,
3-methoxy-N-(1-{[7-(propoxymethyl)-2-naphthyl]methyl}piperidin-4-yl)benzamide,
N-[1-({7-[(allyloxy)methyl]-2-naphthyl}methyl)piperidin-4-yl]-3-methoxybenzamide,
3-methoxy-N-[1-({7-[(2-methoxyethoxy)methyl]-2-naphthyl}methyl)piperidin-4-yl]benzamide,
N-{1-[(7-{[2-(benzyloxy)ethoxy]methyl}-2-naphthyl)methyl]piperidin-4-yl}-3-methoxybenzamide,
N-[1-({7-[(2-ethoxyethoxy)methyl]-2-naphthyl}methyl)piperidin-4-yl]-3-methoxybenzamide,
3-methoxy-N-[1-({7-[(2-propoxyethoxy)methyl]-2-naphthyl}methyl)piperidin-4-yl]benzamide,
N-[1-({7-[(2-isopropoxyethoxy)methyl]-2-naphthyl}methyl)piperidin-4-yl]-3-methoxybenzamide,
3-methoxy-N-[1-({7-[(3-methoxypropoxy)methyl]-2-naphthyl}methyl)piperidin-4-yl]benzamide,
3-methoxy-N-{1-[(7-{[2-(tetrahydro-2H-pyran-2-yloxy)ethoxy]methyl}-2-naphthyl)methyl]piperidin-4-yl}benzamide,
N-[1-({7-[(2-hydroxyethoxy)methyl]-2-naphthyl}methyl)piperidin-4-yl]-3-methoxybenzamide,
3-methoxy-N-[1-({7-[(2-pyrrolidin-1-ylethoxy)methyl]-2-naphthyl}methyl)piperidin-4-yl]benzamide,
N-{1-[(7-{[2-(dimethylamino)ethoxy]methyl}-2-naphthyl)methyl]piperidin-4-yl}-3-methoxybenzamide,
3-methoxy-N-{1-[(7-{[(tetrahydrofuran-2-ylmethyl)amino]methyl}-2-naphthyl)methyl]piperidin-4-yl}benzamide,
3-methoxy-N-[1-({7-[(4-methylpiperazin-1-yl)methyl]-2-naphthyl}methyl)piperidin-4-yl]benzamide,
3-methoxy-N-{1-[(7-{[(2-pyrrolidin-1-ylethyl)amino]methyl}-2-naphthyl)methyl]piperidin-4-yl}benzamide,
N-(1-{[7-({[2-(dimethylamino)ethyl]amino}methyl)-2-naphthyl]methyl}piperidin-4-yl)-3-methoxybenzamide,
3-methoxy-N-{1-[(7-{[(2-morpholin-4-ylethyl)amino]methyl}-2-naphthyl)methyl]piperidin-4-yl}benzamide,
N-{1-[(7-{[4-(2-hydroxyethyl)piperazin-1-yl]methyl}-2-naphthyl)methyl]piperidin-4-yl}-3-methoxybenzamide,
N-{1-[(7-{[[2-(dimethylamino)ethyl] (methyl)amino]methyl}-2-naphthyl)methyl]piperidin-4-yl}-3-methoxybenzamide,
3-methoxy-N-{1-[(7-{[(3-methylbutyl)amino]methyl}-2-naphthyl)methyl]piperidin-4-yl}benzamide,
N-{1-[(7-{[(cyclopropylmethyl)amino]methyl}-2-naphthyl)methyl]piperidin-4-yl}-3-methoxybenzamide,
3-methoxy-N-{1-[(7-{[(2-methoxyethyl)amino]methyl}-2-naphthyl)methyl]piperidin-4-yl}benzamide,
3-methoxy-N-{1-[(7-{[(2-piperidin-1-ylethyl)amino]methyl}-2-naphthyl)methyl]piperidin-4-yl}benzamide,
benzyl N-{[7-({4-[(3-methoxybenzoyl)amino]piperidin-1-yl}methyl)-2-naphthyl]methyl}-beta-alaninate,
N-{1-[(7-{[(2-aminoethyl)amino]methyl}-2-naphthyl)methyl]piperidin-4-yl}-3-methoxybenzamide,
3-methoxy-N-(1-{[7-({[2-(methylamino)ethyl]amino}methyl)-2-naphthyl]methyl}piperidin-4-yl)benzamide,
N-[1-({7-[(3-aminapyrrolidin-1-yl)methyl]-2-naphthyl}methyl)piperidin-4-yl]-3-methoxybenzamide,
3-methoxy-N-[1-({7-[(pyrrolidin-3-ylamino)methyl]-2-naphthyl}methyl)piperidin-4-yl]benzamide,
3-methoxy-N-{1-[(7-{[(pyrrolidin-2-ylmethyl)amino]methyl}-2-naphthyl)methyl]piperidin-4-yl}benzamide,
3-ethoxy-N-[1-({7-[(2-methoxyethoxy)methyl]-2-naphthyl}methyl)piperidin-4-yl]benzamide,
methyl 3-({[1-({7-[(2-methoxyethoxy)methyl]-2-naphthyl}methyl)piperidin-4-yl]amino}carbonyl)benzoate,
3-(dimethylamino)-N-[1-({7-[(2-methoxyethoxy)methyl]-2-naphthyl}methyl)piperidin-4-yl]benzamide,
N-[1-({7-[(2-methoxyethoxy)methyl]-2-naphthyl}methyl)piperidin-4-yl]-3-(trifluoromethyl)benzamide,
N-[1-({7-[(2-methoxyethoxy)methyl]-2-naphthyl}methyl)piperidin-4-yl]-3-(trifluoromethoxy)benzamide,
N-[1-({7-[(2-methoxyethoxy)methyl]-2-naphthyl}methyl)piperidin-4-yl]-3-methylbenzamide,
3-cyano-N-[1-({7-[(2-methoxyethoxy)methyl]-2-naphthyl}methyl)piperidin-4-yl]benzamide,
N-[1-({7-[(2-methoxyethoxy)methyl]-2-naphthyl}methyl)piperidin-4-yl]-3-phenoxybenzamide,
N-[1-({7-[(2-methoxyethoxy)methyl]-2-naphthyl}methyl)piperidin-4-yl]-3-vinylbenzamide,
3-acetyl-N-[1-({7-[(2-methoxyethoxy)methyl]-2-naphthyl}methyl)piperidin-4-yl]benzamide,
N-[1-({7-[(2-methoxyethoxy)methyl]-2-naphthyl}methyl)piperidin-4-yl]-3-nitrobenzamide,
3-chloro-N-[1-({7-[(2-methoxyethoxy)methyl]-2-naphthyl}methyl)piperidin-4-yl]benzamide,
N-[1-({7-[(2-methoxyethoxy)methyl]-2-naphthyl}methyl)piperidin-4-yl]-3-(methylthio)benzamide,
5-chloro-N-[1-({7-[(2-methoxyethoxy)methyl]-2-naphthyl}methyl)piperidin-4-yl]nicotinamide,
3-methoxy-N-(1-{[7-(2-methoxyethoxy)-2-naphthyl]methyl}piperidin-4-yl)benzamide,
3-methoxy-N-[1-({7-[2-(2-methoxyethoxy)ethoxy]-2-naphthyl}methyl)piperidin-4-yl]benzamide,
3-methoxy-N-(1-{[7-(3-methoxypropoxy)-2-naphthyl]methyl}piperidin-4-yl)benzamide,
3-methoxy-N-(1-{[7-(2-oxoethyl)-2-naphthyl]methyl}piperidin-4-yl)benzamide,
N-(1-{[7-(2-hydroxyethyl)-2-naphthyl]methyl}lpiperidin-4-yl)-3-methoxybenzamide,
3-methoxy-N-[1-({7-[2-(2-methoxyethoxy)ethyl]-2-naphthyl}methyl)piperidin-4-yl]benzamide,
3-ethyl-N-[1-({7-[(2-methoxyethoxy)methyl]-2-naphthyl}methyl)piperidin-4-yl]benzamide,
3-chloro-4-fluoro-N-(1-{[7-(methoxymethyl)-2-naphthyl]methyl}piperidin-4-yl)benzamide,
3-methoxy-N-[1-(1-{7-[(2-methoxyethoxy)methyl]-2-naphthyl}ethyl)piperidin-4-yl]benzamide,
3-methoxy-N-(1-{[7-(4-oxobutyl)-2-naphthyl]methyl}piperidin-4-yl)benzamide,
N-(1-{[7-(4-hydroxybutyl)-2-naphthyl]methyl}piperidin-4-yl)-3-methoxybenzamide,
ethyl 3-[7-({4-[(3-methoxybenzoyl)amino]piperidin-1-yl}methyl)-2-naphthyl]propanoate,
N-(1-{[7-(3-hydroxypropyl)-2-naphthyl]methyl}piperidin-4-yl)-3-methoxybenzamide,
7-({4-[(3-methoxybenzoyl)amino]piperidin-1-yl}methyl)-N-(2-pyrrolidin-1-ylethyl)-2-naphthamide,
2-(3,4-dichlorophenyl)-N-(1-{[7-(methoxymethyl)naphthalen-2-yl]methyl}piperidin-4-yl)acetamide,
N-[1-({7-[(2-methoxyethoxy)methyl]naphthalen-2-yl}methyl)piperidin-4-yl]-2,2-diphenylacetamide,
2,2-bis(4-chlorophenyl)-N-[1-({7-[(2-methoxyethoxy)methyl]naphthalen-2-yl}methyl)piperidin-4-yl]acetamide,
N-[1-({7-[(2-methoxyethoxy)methyl]naphthalen-2-yl}methyl)piperidin-4-yl]thiophene-3-carboxamide,
N-[1-({7-[(2-methoxyethoxy)methyl]naphthalen-2-yl}methyl)piperidin-4-yl]-9H-fluorene-9-carboxamide or
3-methoxy-N-[1-({7-[(3-methoxybutoxy)methyl]naphthalen-2-yl}methyl)piperidin-4-yl]benzamide.

11. A pharmaceutical composition comprising the compound or pharmaceutically acceptable salt thereof according to any one of claims 1 to 10 as an active ingredient.

12. The pharmaceutical composition according to claim 11, which is an MCH receptor antagonist.

13. A pharmaceutical composition for preventing or treating a disease selected from the group consisting of depression, anxiety disorders, attention deficit disorder, mania, manic-depressive illness, schizophrenia, mood disorders, stress, sleep disorder, attacks, memory impairment, cognitive impairment, dementia, amnesia, delirium, obesity, eating disorder, appetite disorder, hyperphagia, bulimia, cibophobia, diabetes, cardiovascular diseases, hypertension, dyslipidemia, myocardial infarction, movement disorder, drug abuse and drug addiction, which comprises the compound or pharmaceutically acceptable salt thereof according to any one of claims 1 to 10 as an active ingredient.
